# EUROPÄISCHE PATENTSCHRIFT

(11) **EP 1 604 029 B1**
(45) Veröffentlichungstag und Bekanntmachung des Hinweises auf die Patenterteilung: **02.09.2009**
(21) Anmeldenummer: 04718952.7
(22) Anmeldetag: 10.03.2004
(51) Int. Cl.: C12N 15/82, C07K 14/415, C12N 15/29, A01H 5/00

(54) **VERFAHREN ZUR ERHÖHUNG DER RESISTENZ GEGEN STRESSFAKTOREN IN PFLANZEN**
METHOD FOR INCREASING RESISTANCE AGAINST STRESS FACTORS IN PLANTS
PROCEDES POUR AUGMENTER LA RESISTANCE DE VEGETAUX PAR RAPPORT A DES FACTEURS DE STRESS

(30) Priorität: 12.03.2003 DE 10311118
(43) Veröffentlichungstag der Anmeldung: 14.12.2005
(73) Patentinhaber: BASF Plant Science GmbH, 67056 Ludwigshafen (DE)
(72) Erfinder: FRANK, Markus, 68163 Mannheim (DE); KOGEL, Karl-Heinz, 35457 Lollar (DE); HÜCKELHOVEN, Ralph, 35392 Giessen (DE)
(74) Vertreter: Bieberbach, Andreas
(86) Internationale Anmeldenummer: PCT/EP2004/002436
(87) Internationale Veröffentlichungsnummer: WO 2004/081217

(56) Entgegenhaltungen:
- EP-A- 0 864 650
- WO-A-00/26391
- WO-A-02/101079
- US-A1- 2003 009 785
- LINCOLN JAMES E ET AL: "Expression of the antiapoptotic baculovirus p35 gene in tomato blocks programmed cell death and provides broad-spectrum resistance to disease." PROCEEDINGS OF THE NATIONAL ACADEMY OF SCIENCES OF THE UNITED STATES, Bd. 99, Nr. 23, 12. November 2002 (2002-11-12), Seiten 15217-15221, XP002286824 November 12, 2002 ISSN: 0027-8424 (ISSN print)
- HUECKELHOVEN RALPH ET AL: "Overexpression of barley BAX inhibitor 1 induces breakdown of mlo-mediated penetration resistance to Blumeria graminis." PROCEEDINGS OF THE NATIONAL ACADEMY OF SCIENCES OF THE UNITED STATES, Bd. 100, Nr. 9, 29. April 2003 (2003-04-29), Seiten 5555-5560, XP002286823 April 29, 2003 ISSN: 0027-8424 (ISSN print)
- CHAE H-J ET AL: "Evolutionarily conserved cytoprotection provided by Bax Inhibitor-1 homologs from animals, plants, and yeast" GENE: AN INTERNATIONAL JOURNAL ON GENES AND GENOMES, ELSEVIER SCIENCE PUBLISHERS, BARKING, GB, Bd. 323, 24. Dezember 2003 (2003-12-24), Seiten 101-113, XP004477034 ISSN: 0378-1119

## Beschreibung

Die Erfindung betrifft Verfahren zur Erzeugung oder Erhöhung der Resistenz gegen mindestens einen biotischen Streßfaktor in Pflanzen, bevorzugt gegen pflanzliche Pathogene, durch Erhöhung der Expression mindestens eines Bax-Inhibitor 1 (BI1) Proteins in mindestens einem pflanzlichen Gewebe mit der Maßgabe, dass die Expression in der Blattepidermis im wesentlichen unverändert bleibt.

Ziel biotechnologischer Arbeiten an Pflanzen ist die Herstellung von Pflanzen mit vorteilhaften, neuen Eigenschaften zum Beispiel zur Steigerung der landwirtschaftlichen Produktivität, zur Qualitätssteigerung bei Nahrungsmitteln oder zur Produktion bestimmter Chemikalien oder Pharmazeutika. Oft sind die natürlichen Abwehrmechanismen der Pflanze gegen Pathogene unzureichend. Allein Pilzerkrankungen führen zu Ernteverlusten in der Höhe von vielen Milliarden US-$ jährlich. Die Einführung fremder Gene aus Pflanzen, Tieren oder mikrobiellen Quellen kann die Abwehr verstärken. Beispiele sind der Schutz gegen Insektenfraß in Tabak durch Expression von *Bacillus thuringiensis* Endotoxinen (Vaeck et al. (1987) Nature 328:33-37) oder der Schutz des Tabaks gegen Pilzbefall durch Expression einer Chitinase aus der Bohne (Broglie et al. (1991) Science 254:1194-1197). Die meisten der beschriebenen Ansätze gewähren jedoch nur eine Resistenz gegen ein einzelnes Pathogen oder gegen ein schmales Spektrum von Pathogenen.

Es gibt nur wenige Ansätze, die Pflanzen eine Resistenz gegen ein breiteres Spektrum von Pathogenen, vor allem Pilzpathogene, verleihen. Die systemische erworbene Resistenz ("systemic acquired resistance"; SAR) - ein Abwehrmechanismus bei verschiedenen Pflanze/Pathogen-Interaktionen - kann durch Applikation von endogene Botenstoffe wie Jasmonat (JA) oder Salizylsäure (SA) vermittelt werden (Ward et al. (1991) Plant Cell 3:1085-1094; Uknes et al. (1992) Plant Cell 4(6):645-656). Ähnliche Effekte können auch durch synthetische Verbindungen wie 2,6-Dichlorisonikotinsäure (DCINA) oder Benzo(1, 2, 3)thiadiazol-7-thiocarbonsäure-S-methylester (BTH; Bion^{®}) (Friedrich et al. (1996) Plant J 10(1):61-70; Lawton et al. (1996) Plant J 10:71-82) bewirkt werden. Auch die Expression der im Rahmen eines SAR hochregulierten "pathogenesis related" (PR) Proteine vermag zum Teil eine Pathogenresistenz zu bewirken.

In Gerste ist der Mlo-Locus als negativer Regulator der Pathogenabwehr beschrieben. Der Verlust oder Funktionsverlust ("loss-of-function") des Mlo-Gens bedingt eine erhöhte, rassenunspezifische Resistenz gegen zahlreiche Mehltauisolate (Büschges R et al. (1997) Cell 88:695-705; Jorgensen JH (1977) Euphytica 26:55-62; Lyngkjaer MF et al. (1995) Plant Pathol 44:786-790).
Das Mlo-Gen ist beschrieben (Büschges R et al. (1997) Cell 88:695-705; WO 98/04586; Schulze-Lefert P, Vogel J (2000) Trends Plant Sci. 5:343-348). Verschiedene Mlo-Homologe aus anderen Getreidearten wurden isoliert. Verfahren unter Verwendung dieser Gene zum Erzielen einer Pathogenresistenz sind beschrieben (WO .98/04586; WO 00/01722; WO 99/47552). Nachteilig ist, dass Mlodefiziente Pflanzen auch in Abwesenheit eines Pathogens den o.g. Abwehrmechanismus initiieren, was sich in einem spontanen Absterben von Blattzellen äußert (Weiter M et al. (1993) Mol Gen Genet 239:122-128). Dadurch erleiden mlo-resistente Pflanzen eine Ertragseinbuße von ca. 5% (Jörgensen JH (1992) Euphytica 63: 141-152). Das spontane Absterben der Blattzellen bedingt ferner eine nachteilige Hypersuszeptibilität gegen nekrotrophe und hemibiotrophe Pathogene wie *Magnaporte grisea* (*M*. *grisea*) oder *Cochliobolus sativus* (Bipolaris sorokiniana) (Jarosch B et al. (1999) Mol Plant Microbe Interact 12:508-514; Kumar J et al. (2001) Phytopathology 91:127-133).

Faktoren die einen der mlo-Resistenz vergleichbaren Effekt gegen nekrotrophe Pilze vermitteln, konnten bislang nicht identifiziert werden. Dies mag an dem besonderen Infektionsmechanismus der nekrotrophen Pilze liegen: Anstelle einer Appressorien-vermittelten Penetration infundieren sie zunächst die pflanzliche Wirtszelle mit Mykotoxinen und Enzymen, was zu einem Absterben der Zelle führt. Erst danach wird die Zelle penetriert (Shirasu K and Schulze-Lefert P (2000) Plant Mol Biol 44:371-385). Ähnliche Infektionsstrategien verfolgen bakterielle Pathogene wie Erwinina carotovora (Whitehead NA et al. (2002) Antonie van Leeuwenhoek 81: 223-231). Eine Penetrationsresistenz mit Hilfe von Papillenbildung etc. stellt hier keine effiziente Abwehrstrategie dar.

Apoptose, auch als programmierter Zelltod bezeichnet, ist ein essentieller Mechanismus zur Aufrechterhaltung der Gewebehomöostase und steht damit der Zellteilung als negativ regulierender Mechanismus gegenüber. Im vielzelligen Organismus ist die Apoptose ein natürlicher Bestandteil der Ontogenese und u.a. an der Entwicklung der Organe und der Beseitigung von gealterten, infizierten oder mutierten Zellen beteiligt. Durch die Apoptose wird eine effiziente Elimination von unerwünschten Zellen erreicht. Eine Störung oder Inhibition der Apoptose trägt zur Pathogenese verschiedener Erkrankungen bei, unter anderem zur Karzinogenese. Die Haupteffektoren der Apoptose sind Aspartat-spezifische Cystein-Proteasen, die sogenannten Caspasen. Ihre Aktivierung kann durch mindestens zwei Apoptose-Signalwege stattfinden: Zum einen durch die Aktivierung der TNF-(Tumor Necrosis Factor) Rezeptorfamilie, zum anderen spielen Mitochondrien eine zentrale Rolle. Die Aktivierung des mitochondrialen Apoptose-Signalweges wird durch Proteine der Bcl-2-Familie reguliert. Diese Proteinfamilie besteht aus anti-apoptotischen sowie pro-apoptotischen Proteinen wie z.B. Bax. Im Falle eines apoptotischen Stimulus findet eine allosterische Konformationsänderung des Bax-Proteins statt, welche zur Verankerung des Proteins in der mitochondrialen Außenmembran und seiner Oligomerisierung führt. Durch diese Oligomere werden pro-apoptotischen Moleküle aus den Mitochondrien ins Zytosol freigesetzt, die eine apoptotische Signalkaskade und letztlich die Degradierung spezifischer zellulärer Substrate bedingen, was den Zelltod zur Folge hat. Der Bax Inhibitor-1 BI1 wurde über seine Eigenschaft isoliert, die pro-apoptotische Wirkung von BAX zu inhibieren (Xu Q & Reed JC (1998) Mol Cell 1(3): 337-346). BI1 stellt ein hochkonserviertes Protein dar. Es findet sich überwiegend als integraler Bestandteil intrazellulärer Membranen. BI1 interagiert mit bcl-2 und bcl-xl. Überexpression von BI1 in Säugetierzellen unterdrückt die pro-apoptotische Wirkung von BAX, Etoposid und Staurosporin, aber nicht von Fas-Antigen (Roth W and Reed JC (2002) Nat Med 8: 216-218). Die Inhibition von BI1 durch antisense-RNA hingegen induziert Apoptose (Xu Q & Reed JC (1998) Mol Cell 1(3):337-346). Die ersten pflanzlichen Homologen von BI1 wurden aus Reis und Arabidopsis isoliert (Kawai et al. (1999) FEBS Lett 464:143-147; Sanchez et al (2000) Plant J 21:393-399). Diese pflanzlichen Proteine supprimieren BAX-induzierten Zelltod in Hefe. Die Aminosäure-Sequenzhomologie zu menschlichem BI1 beträgt ca. 45%. Das Arabidopsis-Homolog AtBI1 vermag in rekombinanten Pflanzen die pro-apoptotische Wirkung von BAX aus Maus zu supprimieren (Kawai-Yamada et al. (2001) Proc Natl Acad Sci USA 98(21):12295-12300). Das Reis (Oryza sativa) BI1-Homolog OsBI1 wird in allen pflanzlichen Geweben exprimiert (Kawai et al. (1999) FEBS Lett 464: 143-147). Beschrieben sind ferner BI1-Gene aus Gerste (Hordeum vulgare; GenBank Acc.-No.: AJ290421), Reis (GenBank Acc.-No.: AB025926), Arabidopsis (GenBank Acc.-No.: AB025927), Tabak (GenBank Acc.-No.: AF390556) und Raps (GenBank Acc.-No.: AF390555, Bolduc N et al. (2003) Planta 216:377-386). Die Expression von BI1 in Gerste wird infolge einer Infektion mit Mehltau hochreguliert (Hückelhoven R et al. (2001) Plant Mol Biol 47(6):739-748). WO 00/26391 beschreibt die Überexpression der anti-apoptotischen Gene Ced-9 aus C. elegans, sfIAP aus Spodoptera frugiperda, bcl-2 aus Mensch sowie bcl-xl aus Huhn in Pflanzen zur Erhöhung der Resistenz gegen nekrotrophe bzw. hemibiotrophe Pilze. Pflanzliche BI1 Homologe werden nicht offenbart. Die Expression erfolgt unter Kontrolle konstitutiver Promotoren. Beschrieben ist ferner die Expression eines BI1 Proteins aus Arabidopsis unter dem starken konstitutiven 35S CaMV Promotor in Reiszellen und eine dadurch induzierte Resistenz gegen Zelltod induzierende Substanzen aus Magnaporthe grisea (Matsumura H et al. (2003) Plant J 33:425-434).

Überraschenderweise wurde im Rahmen dieser Erfindung gefunden, dass eine konstitutive Expression eines BI1-Proteins zwar eine Resistenz gegen nekrotrophe Pilze bedingt, jedoch ein Brechen der mlo-vermittelten Resistenz gegen obligat-biotrophen Echten Mehltau (siehe Vergleichsversuch 1) zur Folge hat. Dies stellt den wirtschaftlichen Nutzen der im Stand der Technik beschriebenen Verfahren in Frage.

Es bestand die Aufgabe, Verfahren zur Pathogenabwehr in Pflanzen bereitzustellen, die eine effiziente Abwehr pflanzlicher Pathogene (bevorzugt nekrotropher Pathogene) ermöglichen, ohne eine ggf. bestehende Resistenz gegen andere Pathogene (wie beispielsweise biotrophe Pathogene) zu brechen. Diese Aufgabe wird durch das erfindungsgemäße Verfahren gelöst.

Ein erster Gegenstand der Erfindung betrifft Verfahren zur Erzeugung oder Erhöhung der Resistenz gegen mindestens einen biotischen Streßfaktor in Pflanzen, wobei nachfolgende Arbeitsschritte umfaßt sind
a) Erhöhung der Proteinmenge mindestens eines Bax Inhibitor-1 (BI1) Proteins in mindestens einem pflanzlichen Gewebe mit der Maßgabe, dass die Expression in der Blattepidermis im wesentlichen unverändert bleibt oder reduziert wird, und
b) Auswahl der Pflanzen, bei denen im vergleich zur Ausgangspflanze eine Resistenz gegen mindestens einen biotischen oder abiotischen Streßfaktor besteht oder erhöht ist.

Bevorzugt ist der biotische Streßfaktor ein Pathogen, besonders bevorzugt ein Pathogen ausgewählt aus der Gruppe der nekrotrophen und hemibiotrophen Pathogene.

Unter der Epidermis versteht der Fachmann das vorherrschende Abschlussgewebe primärer oberirdischer Pflanzenteile, so des Sprosses, der Blätter, Blüten, Früchte und Samen. Nach außen scheiden die Epidermiszellen eine wasserabstoßende Schicht, die Kutikula ab. Die Wurzeln sind von der Rhizodermis umgeben, welche der Epidermis in vieler Hinsicht ähnelt, jedoch auch markante Unterschiede zu ihr aufweist. Die Epidermis entsteht aus der äußersten Schicht des Apikalmeristems. Die Ableitung der Rhizodermis hingegen ist weniger klar. Je nach Art kann sie entwicklungsgeschichtlich entweder der Wurzelhaube oder der primären Rinde zugerechnet werden. Der Epidermis können zahlreiche Funktionen zugeschrieben werden: Sie bietet der Pflanze Schutz vor Austrocknung und regelt die Transpirationsrate Sie schützt die Pflanze vor den verschiedensten chemischen und physikalischen Fremdeinflüssen sowie vor Tierfraß und Befall durch Parasiten. Sie ist am Gasaustausch, an der Sekretion bestimmter Stoffwechselprodukte und an der Absorption von Wasser beteiligt. In ihr sind Rezeptoren für Licht und mechanische Reize enthalten. Sie wirkt damit als ein Signalwandler zwischen Umwelt und Pflanze. Entsprechend den verschiedenen Funktionen enthält die Epidermis eine Anzahl unterschiedlich differenzierter Zellen. Hinzu kommen artspezifische Varianten und unterschiedliche Organisation der Epidermen in den einzelnen Teilen einer Pflanze. Im wesentlichen besteht sie aus drei Kategorien von Zellen: den "eigentlichen" Epidermiszellen, den Zellen der Stomata (Spaltöffnungen) und den Trichomen (griech.: Trichoma, Haar), epidermalen Anhangsgebilden verschiedener Form, Struktur und Funktion.
Die "eigentlichen", d.h., die am wenigsten spezialisierten Epidermiszellen machen die Hauptmasse der Zellen des Abschlussgewebes aus. Sie sind in der Aufsicht entweder polygonal (von platten- oder tafelförmiger Gestalt) oder gestreckt. Die zwischen ihnen ausgebildeten Wände sind vielfach gewellt oder gebuchtet. Wodurch diese Form während der Entwicklung induziert wird, ist unbekannt, die vorliegenden Hypothesen erklären den Sachverhalt nur unbefriedigend. Gestreckte Epidermiszellen findet man an Organen oder Organteilen, die selbst gestreckt sind, so z.B. an Stengeln, Blattstielen und Blattrippen sowie an den Blättern der meisten Monokotyledonen. Ober- und Unterseite von Blattspreiten können von unterschiedlich strukturierten Epidermen bedeckt sein wobei sowohl die Form der Zellen, die Dicke der Wände als auch die Verteilung und Zahl spezialisierter Zellen (Stomata und/oder Trichome) pro Flächeneinheit variieren kann. Große Variationen findet man auch innerhalb einzelner Familien, z. B. bei den Crassulaceen. Meist ist die Epidermis einschichtig, jedoch sind bei Arten aus mehreren Familien (Moraceae: hier die meisten Ficus-Arten, Piperaceae: Peperonia [Peperonie], Begoniaceae, Malvaceae u.a.) mehrschichtige wasserspeichernde Epidermen nachgewiesen worden. Epidermiszellen sondern nach außen eine Cutinschicht (Kutikula) ab, die als ein ununterbrochener Film alle epidermalen Oberflächen überzieht. Sie kann entweder glatt oder durch Vorwölbungen, Leisten, Falten und Furchen strukturiert sein. Doch nicht immer beruht eine durch Betrachtung der Oberfläche sichtbare Faltung der Kutikula auf der Ausbildung von Kutikularleisten. Es gibt durchaus Fälle, wo eine Kutikulafaltung nur der Ausdruck der darunterliegenden Ausstülpungen der Zellwand ist. Epidermale Anhangsgebilde verschiedener Form, Struktur und Funktion werden als Trichome bezeichnet und hierin ebenfalls unter dem Begriff "Epidermis" verstanden.. Sie treten als Schutz-, Stütz- und Drüsenhaare in Form von Schuppen, verschiedenen Papillen und bei Wurzeln als absorbierende Haare auf. An ihrer Bildung sind allein Epidermiszellen beteiligt. Oft entsteht ein Trichom aus nur einer solchen Zelle, manchmal sind an der Entstehung mehrere beteiligt. Ebenfalls umfasst unter dem Begriff "Epidermis" sind Papillen. Papillen sind Ausstülpungen der Epidermisoberfläche. Das Lehrbuchbeispiel hierfür sind die Papillen auf Blütenoberflächen des Stiefmütterchens (Viola tricolor) sowie die Blattoberflächen vieler Arten im tropischen Regenwald. Sie verleihen der Oberfläche eine samtartige Konsistenz. Einige Zellen von Epidermen können als Wasserspeicher ausgebildet sein. Ein typisches Beispiel stellen die Blasenzellen an Oberflächen vieler Mittagsblumenarten und anderer Sukkulenten dar. Bei manchen Pflanzen, z.B. bei der Glockenblume (Campanula persicifolia) sind die Außenwände der Epidermis linsenförmig verdickt.

Die Hauptmasse aller Gewebe bildet das Grundgewebe oder Parenchym. Zu den parenchymatischen Geweben gehört das Mesophyll, das in Blättern in Palisadenparenchym und Schwammparenchym differenziert sein kann.

Folglich versteht der Fachmann unter Mesophyll ein parenchymatisches Gewebe. Parenchymatisehe Zellen sind durchweg lebend, meist isodiametrisch, seltener gestreckt. Das Mark der Sprosse, die Speichergewebe der Früchte, Samen, der Wurzel und anderer unterirdischer Organe sind ebenso als Parenchyme zu betrachten wie das Mesophyll.

Das Mesophyll ist in den Blättern der meisten Farne und Phanerogamen, besonders ausgeprägt bei den Dikotyledonen und vielen Monokotyledonen, in Palisaden- und Schwammparenchym untergliedert. Ein "typischen" Blatt ist dorsiventral gebaut. Das Palisadenparenchym liegt dabei meist an der Blattoberseite unmittelbar unter der Epidermis. Das Schwammparenchym füllt den darunterliegenden Raum aus. Es ist von einem voluminösen Interzellularsystem durchsetzt, dessen Gasraum über die Spaltöffnungen in direktem Kontakt zur Außenwelt steht.
Das Palisadenparenchym besteht aus langgestreckten, zylindrischen Zellen. Bei einigen Arten sind die Zellen irregulär, gelegentlich sind sie gegabelt (Y-förmig: Armpalisadenparenchym). Solche Varianten kommen bei Farnen, Coniferen und einigen wenigen Angiospermen (z.B. bei einigen Ranunculaceen- und Caprifoliaceenarten [Beispiel: Holunder]) vor. Neben der eben beschriebenen, am weitesten verbreiteten Organisationsform sind die folgenden Varianten nachgewiesen worden:
Palisadenparenchym an der Blattunterseite. Besonders auffällig bei Schuppenblättern. Beispiel: Lebensbaum (Thuja), sowie bei den Blättern des Bärlauchs (Allium ursinum).
Palisadenparenchym an beiden Blattseiten (Ober- und Unterseite). Häufig bei Pflanzen trockener Standorte (Xerophyten). Beispiel: Kompasspflanze (Lactuca serriola);
Ringförmig geschlossenes Palisadenparenchym: In zylindrisch organisierten Blättern und in Nadeln der Koniferen.

Die Variabilität der Schwammparenchymzellen und die Ausbildung des Schwammparenehyms selbst sind noch vielgestaltiger als die des Palisadenparenchyms. Es wird meist als Durchlüftungsgewebe bezeichnet, denn es enthält eine Vielzahl untereinander verbundener Interzellularen.
Das Mesophyll kann das so genannte Assimilationsgewebe umfassen, jedoch sind die Begriffe Mesophyll und Assimilationsgewebe nicht als Synonyme zu verwenden. Es gibt chloroplastenfreie Blätter, die sich in ihrem Aufbau nur unwesentlich von vergleichbaren, grünen Blättern unterscheiden. Folglich enthalten sie Mesophyll, doch eine Assimilation unterbleibt; umgekehrt findet eine Assimilation z.B. auch in Sproßabschnitten statt. Weitere Hilfsmittel zur Charakterisierung von Epidermis und Mesophyll findet der Fachmann z.B. in v. GUTTENBERG, H.: Lehrbuch der Allgemeinen Botanik. Berlin: Akademie-Verlag 1955 (5. Aufl.), HABERLANDT, G.: Physiologische Pflanzenanatomie. Leipzig: W. Engelmann 1924 (6. Aufl.); TROLL, W.: Morphologie der Pflanzen. Band 1: Vegetationsorgane. Berlin: Gebr. Borntraeger, 1937; TROLL, W.: Praktische Einführung in die Pflanzenmorphologie. Jena: VEB G. Thieme Verlag 1954/1957; TROLL, W., HÖHN, K.: Allgemeine Botanik. Stuttgart: F. Enke Verlag, 1973 (4. Aufl.)

In einer Ausführungsform wird die Epidermis biochemisch charakterisiert. Die Epidermis kann in einer Ausführungsform durch die Aktivität eines oder mehrer der folgenden Promotoren gekennzeichnet werden:
- WIR5 (=GstA1), acc. X56012, Dudler & Schweizer, unveröff.
- GLP4, acc. AJ310534; Wei,Y.; Zhang, Z.; Andersen,C.H.; Schmelzer, E.; Gregersen,P.L.; Collinge,D.B.; Smedegaard-Petersen,V.; Thordal-Christensen,H. (1998) An epidermis/papilla-specific oxalate oxidase-like protein in the defence response of barley attacked by the powdery mildew fungus. Plant Molecular Biology 36, 101-112.
- GLP2a, acc. AJ237942, Schweizer, P., Christoffel,A. and Dudler,R. (1999). Transient expression of members of the germin-like gene family in epidermal cells of wheat confers disease resistance, Plant J 20, 541-552.
- Prx7, acc. AJ003141, Kristensen BK, Ammitzböll H, Rasmussen SK & Nielsen KA. 2001. Transient expression of a vacuolar peroxidase increases susceptibility of epidermal barley cells to powdery mildew. Molecular Plant Pathology, 2(6), 311-317
- GerA, acc. AF250933 ; Wu S, Druka A, Horvath H, Kleinhofs A, Kannangara G & von Wettstein D, 2000. Functional characterization of seed coat-specific members of the barley germin gene family. Plant Phys Biochem 38, 685-698
- OsR0C1, acc. AP004656
- RTBV, acc. AAV62708, AAV62707 ; Klöti, A, Henrich C, Bieri S, He X, Chen G, Burkhardt PK, Wünn J, Lucca, P, Hohn, T, Potrykus I & Fütterer J, 1999, Upstream and downstream sequence elements determine the specificity of the rice tungro bacilliform virus promoter and influence RNA production after transcription initiation. PMB 40, 249-266

In einer Ausführungsform wird die Epidermis dadurch gekennzeichnet, dass alle genannten Promoter in dem Gewebe oder der Zelle aktiv sind. In einer anderen Ausführungsform wird die Epidermis dadurch gekennzeichnet, dass nur ein Teil der Promotoren aktiv ist, z.B. 2, 3, 5 oder 7 oder mehr, mindestens jedoch einer der oben aufgezählten.

In einer Ausführungsform wird das Mesophyll biochemisch charakterisiert. Das Mesophyll kann in einer Ausführungsform durch die Aktivität eines oder mehrer der folgenden Promotoren gekennzeichnet werden:
- PPCZm1 (=PEPC); Kausch,A.P., Owen, T.P., Zachwieja,S.J., Flynn, A.R. and Sheen,J. (2001) Mesophyll-specific, light and metabolic regulation of the C(4) PPCZm1 promoter in transgenic maize. Plant Mol. Biol. 45, 1-15
- OsrbcS, Kyozuka et al PlaNT Phys: 1993 102: Kyozuka J, NcElroy D, Hayakawa T, Xie Y, Wu R & Shimamoto K. 1993. Light-regulated and cell-specific expression of tomato rbcs-gusA and rice rbcs-gusA fusion genes in transgenic rice. Plant Phys 102, 991-1000
- OsPPDK, acc. AC099041, unveröff.
- TaGF-2.8, acc. M63223; Schweizer,P., Christoffel,A. and Dudler,R. (1999). Transient expression of members of the germin-like gene family in epidermal cells of wheat confers disease resistance, Plant J 20, 541-552.

- TaFBPase, acc. X53957; unveröff.
- TaWIS1, acc. AF467542; US 200220115849
- HvBIS1, acc. AF467539; US 200220115849
- ZmMIS1, acc. AF467514; US 200220115849

- HvPR1a, acc. X74939 ; Bryngelsson et al. Molecular Plant-Microbe Interactions (1994)
- HvPR1b, acc. X74940; Bryngelsson et al. Molecular Plant-Microbe Interactions (1994)

- HvB1, 3gluc; acc. AF479647; unveröff.
- HvPrx8, acc. AJ276227; Kristensen et al MPP 2001 (siehe oben)

- HvPAL, acc. X97313 ; Wei, Y.; Zhang,Z.; Andersen,C.H.; Schmelzer,E.; Gregersen,P.L.; Collinge,D.B.; Smedegaard-Petersen,V.; Thordal-Christensen,H. (1998) An epidermis/papilla-specific oxalate oxidase-like protein in the defence response of barley attacked by the powdery mildew fungus. Plant Molecular Biology 36, 101-112.

In einer Ausführungsform wird das Mesophyll dadurch gekennzeichnet, dass alle genannten Promoter in dem Gewebe oder der Zelle aktiv sind. In einer anderen Ausführungsform wird das Mesophyll dadurch gekennzeichnet, dass nur ein Teil der Promotoren aktiv ist, z.B. 2, 3, 5 oder 7 oder mehr, mindestens jedoch einer der oben aufgezählten.

In einer Ausführungsform sind in einer erfindungsgemäß verwendeten oder hergestellten Pflanze oder in einer erfindungsgemäßen Pflanze in der Epidermis und im Mesophyll alle genannten Promotoren aktiv. In einer Ausführungsform sind nur ein Teil der genannten Promotoren aktiv, z.B. 2, 5, 7 oder mehr, mindestens ist jedoch einer der oben aufgezählten Promotoren jeweils aktiv.

In einer bevorzugten Ausführungsform erfolgt die Erhöhung der Proteinmenge des BI1-Proteins wurzel-, knollen- oder mesophyll-spezifisch, besonders bevorzugt mesophyll-spezifisch, beispielsweise durch rekombinante Expression einer für besagtes BI1-Protein kodierenden Nukleinsäuresequenz unter Kontrolle eines wurzel-, knollen- oder mesophyll-spezifischen Promotors, bevorzugt unter Kontrolle eines mesophyll-spezifischen Promotors.

In einer Ausführungsform wird wie hierin beschrieben, die Expression oder Funktion des erfindungsgemäßen Proteins bzw. des hierin charakterisierten BI-1 im Mesophyll einer Pflanze erhöht. Eine Erhöhung der Expression kann wie unten beschrieben erreicht werden. Unter Erhöhung der Expression wird hierein sowohl die Aktivierung oder Steigerung der Expression des endogenen Proteins einschließlich einer de *novo* Expression als auch eine Erhöhung oder Steigerung durch die Expression eines transgenen Proteins oder Faktors verstanden.

In einer besonders bevorzugten Ausführungsform kann die Erhöhung der Proteinmenge mindestens eines pflanzlichen BI1-Proteins kombiniert werden mit einem mlo-resistenten Phänotyp oder mit der Inhibierung oder Reduzierung im Vergleich zu einer Kontrollpflanze der Expression von MLO, RacB und/oder NaOx in der Pflanze oder einem Teil davon, z.B. in einem Gewebe, besonders vorteilhaft jedoch mindestens in der Epidermis oder einem wesentlichen Teil der Epidermiszellen und/oder mit der Erhöhung der Expression von PEN2 und/oder PEN1 in der Pflanze, z.B. konstitutiv, oder einem Teil davon, z.B. in einem Gewebe, besonders vorteilhaft jedoch mindestens in der Epidermis oder einem wesentlichen Teil der Epidermiszellen wird, mit der Maßgabe, dass die Expression eines pflanzlichen BI1-Proteins in der Blattepidermis im wesentlichen unverändert bleibt oder reduziert wird und so eine kombinierte Resistenz gegen sowohl nekrotrophe als auch biotrophe Pathogene erreicht wird.

In Gerste ist der Mlo-Locus als negativer Regulator der Pathogenabwehr beschrieben. Der Verlust oder Funktionsverlust ("loss-of-function") des Mlo-Gens bedingt eine erhöhte, rassen-unspezifische Resistenz gegen zahlreiche Mehltauisolate (Büschges R et al. (1997) Cell 88:695-705; Jorgensen JH (1977) Euphytica 26:55-62; Lyngkjaer MF et al. (1995) Plant Pathol 44:786-790). Das Mlo-Gen ist beschrieben (Büschges R et al. (1997) Cell 88:695-705; WO 98/04586; Schulze-Lefert P, Vogel J (2000) Trends Plant Sci. 5:343-348). Verschiedene Mlo-Homologe aus anderen Getreidearten wurden isoliert.
Ein mlo-resistenter Phänotyp kann wie im Stand der Technik beschrieben erreicht werden. Verfahren unter Verwendung dieser Gene zum Erzielen einer Pathogenresistenz sind beschrieben u.a. in WO 98/04586; WO 00/01722; WO 99/47552.

Vorteilhaft kann in einer Ausführungsform der vorliegenden Erfindung die Aktivität, oder Expression von MLO, RacB und/oder NaOx in der Pflanze oder einem Teil davon, z.B. in einem Gewebe, besonders vorteilhaft jedoch mindestens in der Epidermis oder einem wesentlichen Teil der Epidermiszellen inhibiert oder im Vergleich zu einer Kontrollpflanze oder einem Teil davon reduziert werden. Durch die Reduzierung der Aktivität von MLO, RacB und/oder NaOx in der Pflanze oder einem Teil davon, z.B. in einem Gewebe, besonders vorteilhaft jedoch mindestens in der Epidermis oder einem wesentlichen Teil der Epidermiszellen wird vorzugsweise die Resistenz oder Widerstandskraft gegen biotrophe Pathogene bei erfindungsgemäß hergestellten Pflanzen erhöht. In Kombination mit einer Reduktion oder Unterdrückung des nekrotischen Zelltods ist dies besonders vorteilhaft. Die Aktivität von MLO, RacB und/oder NaOX kann analog wie für MLO in WO 98/04586; WO 00/01722; WO 99/47552 und den weiteren unten geannten Schriften beschrieben reduziert oder inhibiert werden insbesondere um die Aktivität und Inhibierung von MLO zu beschreiben. Die Beschreibung der genannten Schriften beschreibt Verfahren Methoden und besonders bevorzugte Ausführungsformen zur Erniedrigung oder Inhibierung der Aktivität oder Funktion von MLO, die Beispiele geben konkret an, wie dies ausgeführt werden kann.
Die Reduzierung der Aktivität, ggf. der Expression von RacB ist in der WO 2003020939 ausführlich beschrieben. Die Beschreibung der genannten Schrift beschreibt Verfahren und Methoden zur Erniedrigung oder Inhibierung der Aktivität von BI-1, die Beispiele geben konkret an, wie dies ausgeführt werden kann. Besonders bevorzugt wird die Reduktion oder Inhibierung der Aktivität oder Funktion von RacB gemäß den in der WO 2003020939 besonders bevorzugten Ausführungsformen und den Beispielen und in den dort als besonders bevorzugt dargestellten Organismen durchgeführt, insbesondere in einer Pflanze oder einem Teil davon, z.B. in einem Gewebe, besonders vorteilhaft jedoch mindestens in der Epidermis oder einem wesentlichen Teil der Epidermiszellen. Die Reduzierung der Aktivität, ggf. der Expression von RacB ist in der WO 2003020939 ausführlich beschrieben. Der Fachmann findet in der WO 2003020939 die Sequenzen, die für RacB Proteine codieren und kann mit dem in der WO 2003020939 zur Verfügung gestellten Verfahren auch RacB identifizieren. Die Reduzierung der Aktivität, ggf. der Expression von NaOX ist in der PCT/EP/03/07589 ausführlich beschrieben. Die Beschreibung der genannten Schrift beschreibt Verfahren und Methoden zur Erniedrigung oder Inhibierung der Aktivität von NaOx, die Beispiele geben konkret an, wie dies ausgeführt werden kann. Besonders bevorzugt wird die Reduktion oder Inhibierung der Aktivität von NaOx gemäß den in der PCT/EP/03/07589 besonders bevorzugten Ausführungsformen und den Beispielen und in den dort als besonders bevorzugt dargestellten Organismen durchgeführt insbesondere in einer Pflanze oder einem Teil davon, z.B. in einem Gewebe, besonders vorteilhaft jedoch mindestens in der Epidermis oder einem wesentlichen Teil der Epidermiszellen. Der Fachmann findet in der PCT/EP/03/07589 die Sequenzen, die für NaOx Proteine codieren und kann mit dem in der PCT/EP/03/07589 zur Verfügung gestellten Verfahren auch NaOx identifizieren.

Vorteilhaft kann in einer Ausführungsform der vorliegenden Erfindung die Aktivität, Expression von PEN1, PEN2 und/oder SNAP34 in der Pflanze, z.B. konstitutiv, oder einem Teil davon, z.B. in einem Gewebe, besonders vorteilhaft jedoch mindestens in der Epidermis oder einem wesentlichen Teil der Epidermiszellen erhöht werden. Die Erhöhung der Aktivität, die auch eine de novo Expression umschließt, von PEN1, PEN2 und/oder SNAP 34 in der Pflanze, z.B. konstitutiv, oder einem Teil davon, z.B. in einem Gewebe, besonders vorteilhaft jedoch mindestens in der Epidermis oder einem wesentlichen Teil der Epidermiszellen wird vorzugsweise die Resistenz oder Widerstandskraft gegen biotrophe Pathogene bei erfindungsgemäß hergestellten Pflanzen erhöhen. In Kombination mit einer Reduktion oder Unterdrückung des nekrotischen Zelltods ist dies besonders vorteilhaft. Die Erhöhung der Aktivität, ggf. der Expression von PEN2 ist in der WO03074688 ausführlich beschrieben. Die Beschreibung der genannten Schrift beschreibt Verfahren und Methoden zur Erniedrigung oder Inhibierung der Aktivität oder Funktion von PEN2, die Beispiele geben konkret an, wie dies ausgeführt werden kann. Besonders bevorzugt wird die Reduktion oder Inhibierung der Aktivität oder Funktion von PEN2 gemäß den in der WO03074688 besonders bevorzugten Ausführungsformen und den Beispielen und in den dort als besonders bevorzugt dargestellten Organismen durchgeführt, insbesondere in Pflanzen, z.B. konstitutiv, oder einem Teil davon, z.B. in einem Gewebe, besonders vorteilhaft jedoch mindestens in der Epidermis oder einem wesentlichen Teil der Epidermiszellen. Der Fachmann findet in der WO03074688 die Sequenzen, die für PEN2 Proteine codieren und kann mit dem in der WO03074688 zur Verfügung gestellten Verfahren auch PEN2 identifizieren. Die Expression von PEN1 und SNAP34 kann analog zu den in der WO03074688 beschriebenen Verfahren erhöht werden. Der Fachmann kann aufgrund des allgemeinen Fachwissens und des ihm bekannten Stands der Technik PEN1 und SNAP34-Nukleinsäuresequenzen und - Proteinsequenzen isolieren und überexprimieren. Seq ID NO.: 39 beschreibt die Nukleinsäuresequenz, die für PEN1 aus Gerste kodiert, die Proteinsequenz ist in Seq ID No.: 40 beschrieben. Seq ID NO.: 41 beschreibt die Nukleinsäuresequenz, die für PEN1 aus Arabidopsis thaliana kodiert, die Proteinsequenz ist in Seq ID No.: 42 beschrieben. PEN1 aus Arabidopsis thaliana ist veröffentlicht unter den accession numbers NM 202559 und NM 112015. Das Homolog aus Gerste wird als ROR2 offenbart in accession numbers AY246907 und AY246906. Es handelt sich um Mitglieder der recht großen Syntaxin-Proteinfamilie. Der Fachmann kann somit durch einfache Homologievergleiche weitere Syntaxin-Proteine identifzieren, die als potentielle Resistenzgene in dem erfindungsgemäßen Verfahren exprimiert werden.

Seq ID NO.: 43 beschreibt die Nukleinsäuresequenz, die für SNAP34 aus Gerste kodiert, die Proteinsequenz ist in Seq ID No.: 44 beschrieben. Das SNAP-34 Homolog aus Gerste ist auch veröffentlicht unter AY 247208 (SNAP-34). Homologe unbekannter Funktion, die eine Rolle in der Resistenz spielen könnten, sind veröffentlicht unter AY 247209 (SNAP-28) und AY 247210 (SNAP-25). Folgende Arabidopsis-Gene zeigen eine höhere Homologie zu Gerste SNAP34 als Gersten SNAP-28 bzw. SNAP-25 zu SNAP-34 und können somit als potentielle Resistenz-vermittelnde Gene vorteilhaft coüberexprimiert werden:
AAM 62553 - Arabidopsis SNAP25a
NP 200929 - Arabidopsis SNAP33b
NP 172842 - Arabidopsis SNAP30
NP 196405 - Arabidopsis SNAP29

Folglich betrifft die Erfindung auch eine Pflanze, in der mindestens weiterhin in der Epidermis ein Polypeptide überexprimiert wird, das codiert wird von einem Nukleinsäuremolekül, das die in Seq. ID No.: 39, 41 oder 43 oder eine der in den genannten Datenbankveröffentlichungen gezeigten Sequenzen umfasst oder das die in Seq. ID No.: 40, 42 oder 44 gezeigte oder eine der in den genannten Datenbankveröffentlichungen gezeigten Aminosäuresequenzen umfasst, oder das ein funktionelles Äquivalent davon ist oder das eine Homologie von mindestens 50 %, bevorzugt 70 %, mehr bevorzugt 80 %, noch mehrbevorzugt 90 % oder mehr zu den genannten Sequenzen auf Ebene des codierenden Nukleinsäuremoleküls oder bevorzugt auf Aminosäureebene hat, oder eine Pflanze, in der konstitutiv oder in einem Teil, z.B. in einem Gewebe, besonders vorteilhaft jedoch mindestens in der Epidermis oder einem wesentlichen Teil der Epidermiszellen das vorhergehend charakterisierte Polypeptide aktiviert wird oder dessen Aktivität erhöht wird.

Eine Reduktion oder Expression oder Aktivität kann durch dem Fachmann geläufige Verfahren bewirkt werden, z.B. Mutagenese, z.B. EMS, ggf. Tilling, iRNA; Ribozyme, Silencing, Knockout, etc. Verfahren zur Reduzierung sind insbesondere besschrieben in WO 2003020939, dessen Methoden an die hierin beschriebenen Sequenzen leicht angepasst werden können.

Die Reduzierung oder Verminderung der Expression eines BI-1-Proteins, oder der BI-1-Aktivität kann auf vielfältige Art und Weise realisiert werden.

"Reduzierung", reduzieren", "Verminderung" oder "vermindern" ist im Zusammenhang mit einem BI-1 Protein, oder einer BI-1 Aktivität, weit auszulegen und umfasst die teilweise oder im wesentlichen vollständige, auf unterschiedliche zellbiologische Mechanismen beruhende Unterbindung oder Blockierung der Funktionalität eines BI-1-Proteins.
Eine Verminderung im Sinne der Erfindung umfasst auch eine mengenmäßige Verringerung eines BI-1-Proteins bis hin zu einem im wesentlichen vollständigen Fehlen des BI-1-Proteins (d.h. fehlende Nachweisbarkeit von BI-1-Aktivität bzw. BI-1-Funktion oder fehlende immunologische Nachweisbarkeit des BI-1-Proteins). Dabei wird die Expression eines bestimmten BI-1-Proteins oder die BI-1-Aktivität in einer Zelle oder einem Organismus bevorzugt um mehr als 50 %, besonders bevorzugt um mehr als 80 %, ganz besonders bevorzugt um mehr als 90 % vermindert.

Erfindungsgemäß sind verschiedene Strategien zur Verminderung der Expression eines BI-1-Proteins, oder der BI-1-Aktivität umfasst. Der Fachmann erkennt, dass eine Reihe verschiedener Methoden zur Verfügung stehen, um die Expression eines BI-1-Proteins, oder die BI-1-Aktivität in gewünschter Weise zu beeinflussen.

Eine Verminderung der BI-1-Aktivität wird bevorzugt durch eine verminderte Expression eines endogenen BI-1-Proteins erreicht.

Eine Verminderung der BI-1-Proteinmenge, oder BI-1-Aktivität kann unter Verwendung nachfolgender Verfahren realisiert werden:
a) Einbringung einer doppelsträngigen BI-1 RNA-Nukleinsäuresequenz (BI-1-dsRNA) oder einer deren Expression gewährleistenden Expressionskassette oder Expressionskassetten;
b) Einbringung einer BI-1 antisense-Nukleinsäuresequenzen oder einer deren Expression gewährleistenden Expressionskassette. Umfasst sind solche Verfahren, bei denen die antisense-Nukleinsäuresequenz gegen ein BI-1-Gen (also genomische DNA-Sequenzen) oder ein BI-1-Gentranskript (also RNA-Sequenzen) gerichtet ist. Umfasst sind auch α-anomere Nukleinsäuresequenzen.
c) Einbringung einer BI-1 antisense-Nukleinsäuresequenzen kombiniert mit einem Ribozym oder einer deren Expression gewährleistenden Expressionskassette.
d) Einbringung von BI-1 sense-Nukleinsäuresequenzen zur Induktion einer Kosuppression oder einer deren Expression gewährleistenden Expressionskassette.
e) Einbringung einer Nukleinsäuresequenz kodierend für dominant-negatives BI-1 Protein oder einer deren Expression gewährleistenden Expressionskassette.
f) Einbringung DNA-oder Protein-bindende Faktoren gegen BI-1 - Gene, -RNAs oder -Proteine oder einer deren Expression gewährleistenden Expressionskassette.
g) Einbringung von den BI-1 RNA-Abbau bewirkende virale Nukleinsäuresequenzen und Expressionskonstrukten oder einer deren Expression gewährleistenden Expressionskassette.
h) Einbringung von Konstrukten zur Induktion einer homologen Rekombination an endogenen BI-1-Genen beispielsweise zur Erzeugung von Knockout-Mutanten.
i) Einführung von Mutationen in endogenen BI-1 Gene zur Erzeugung eines Funktionsverlustes (z.B. Generierung von Stopp-Kodons, Verschiebungen im Leseraster etc.)
wobei jedes der genannten Verfahren Epidermis-spezifisch durchgeführt werden muss, d.h. die Expression im Epidermisgewebe bleibt unverändert oder wird reduziert. Dabei kann jedes einzelne dieser Verfahren eine Verminderung der BI-1-Expression, oder BI-1-Aktivität im Sinne der Erfindung bewirken. Auch eine kombinierte Anwendung ist denkbar. Weitere Methoden sind dem Fachmann bekannt und können die Behinderung oder Unterbindung der Prozessierung des BI-1-Proteins, des Transports des BI-1-Proteins oder dessen mRNPr, Hemmung der Ribosomenanlagerung, Hemmung des RNA-Spleißens, Induktion eines BI-1-RNA abbauenden Enzyms und/oder Hemmung der Translationselongation oder -termination umfassen.

Die Epidermis-sepzifische Reduktion kann z.B. durch eine transiente Anwendung der genannten Verfahren auf Epidermiszellen oder durch eine spezifische Transformation von im wesentlichen nur Epidermiszellen oder durch eine Expressionskontrolle der aufgeführten Konstrukte unter einem Epidermisspezifischen Promoter oder sonstigen epidermisspezifischen Kontrollelement erfolgt.

Die einzelnen bevorzugten Verfahren seien infolge kurz beschrieben:
a) Einbringung eines doppelsträngigen BI-1 RNA-Nukleinsäuremoleküls (BI-1-dsRNA)
   Das Verfahren der Genregulation mittels doppelsträngiger RNA ("double-stranded RNA interference"; dsRNAi) ist vielfach in tierischen und pflanzlichen Organismen beschrieben (z.B. Matzke MA et al. (2000) Plant Mol Biol 43:401-415; Fire A. et al (1998) Nature 391:806-811; WO 99/32619; WO 99/53050; WO 00/68374; WO 00/44914; WO 00/44895; WO 00/49035; WO 00/63364). Auf die in den angegebenen Zitaten beschriebenen Verfahren und Methoden wird ausdrücklich Bezug genommen. Eine effiziente Gensuppression kann auch bei transienter Expression oder nach transienter Transformation beispielsweise infolge einer biolistischen Transformation gezeigt werden (Schweizer P et al. (2000) Plant J 2000 24: 895-903). dsRNAi-Verfahren beruhen auf dem Phänomen, dass durch gleichzeitiges Einbringen von komplementären Strang- und Gegenstrang eines Gentranskriptes eine hocheffiziente Unterdrückung der Expression des entsprechenden Gens bewirkt wird. Der bewirkte Phänotyp kommt dem einer entsprechenden knock-out Mutanten sehr ähnlich (Waterhouse PM et al. (1998) Proc Natl Acad Sci USA 95:13959-64).
   Das dsRNAi-Verfahren hat sich bei der Verminderung der Expression als besonders effizient und vorteilhaft erwiesen. Wie u.a. in WO 99/32619 beschrieben sind dsRNAi-Ansätze klassischen antisense-Ansätzen deutlich überlegen.
   Ein weiterer Gegenstand der Erfindung bezieht sich daher auf doppelsträngige RNA-Moleküle (dsRNA-Moleküle), die bei Einführung in eine Pflanze (oder eine davon abgeleitete Zelle, Gewebe, Organen, insbesondere Blattepidermis) die Verminderung eines BI-1 bewirken.
   Das doppelsträngiges RNA-Molekül zur Verminderung der Expression eines BI-1 Proteins ist dadurch gekennzeichnet, dass
   a) einer der beiden RNA Stränge im wesentlichen identisch ist zu zumindest einem Teil einer BI-1-Nukleinsäuresequenz, und
   b) der jeweils andere RNA Strang im wesentlichen identisch ist zu zumindest einem Teil des identisch ist zu zumindest einem Teil des komplementärenren Stranges einer BI-1 Nukleinsäuresequenzges einer BI-1-Nukleinsäuresequenz.

   In einer weiterhin bevorzugten Ausführungsform umfasst das doppelsträngige RNA-Molekül zur Verminderung der Expression eines BI-1 Proteins
   a) einen "sense"-RNA-Strang umfassend mindestens eine Ribonukleotidsequenz, die im wesentlichen identisch ist zu mindestens einem Teil des "sense"-RNA-Transkriptes einer Nukleinsäuresequenz kodierend für ein BI-1 Protein, und
   b) einen "antisense"-RNA-Strang, der zu dem RNA-sense-Strang unter a) im wesentlichen - bevorzugt vollständig - komplementären ist.

   In Bezugt auf die doppelsträngigen RNA-Moleküle meint BI-1-Nukleinsäuresequenz bevorzugt eine Sequenz gemäß SEQ ID NO: 1, 3, 5, 7, 9, 11, 13, 15, 17, 19, 21, 23, 25, 27, 29, 31, 33 oder 38 oder ein funktionelles Äquivalent derselben.
   "Im wesentlichen identisch" meint, dass die dsRNA Sequenz auch Insertionen, Deletionen sowie einzelne Punktmutationen im Vergleich zu der BI-1 Zielsequenz aufweisen kann und dennoch eine effizient Verminderung der Expression bewirken. Bevorzugt beträgt die Homologie nach obiger Definition mindestens 50 % oder 75 %, bevorzugt mindestens 80 %, ganz besonders bevorzugt mindestens 90 % am meisten bevorzugt 100 % zwischen dem "sense"-Strang einer inhibitorischen dsRNA und einem Teilabschnitt einer BI-1 Nukleinsäuresequenz (bzw. zwischen dem "antisense"-Strang dem komplementären Strang einer BI-1 Nukleinsäuresequenz).
   Die Länge des Teilabschnittes beträgt mindestens 10 Basen, bevorzugt mindestens 25 Basen, besonders bevorzugt mindestens 50 Basen, ganz besonders bevorzugt mindestens 100 Basen, am meisten bevorzugt mindestens 200 Basen oder mindestens 300 Basen. Alternativ, kann eine "im wesentlichen identisch" dsRNA auch als Nukleinsäuresequenz definiert werden, die befähigt ist, mit einem Teil eines BI-1 Gentranskriptes zu hybridisieren (z.B. in 400 mM NaCl, 40 mM PIPES pH 6,4, 1 mM EDTA bei 50°C oder 70°C für 12 bis 16 h).
   "Im wesentlichen komplementär" meint, dass der antisense"-RNA-Strang auch Insertionen, Deletionen sowie einzelne Punktmutationen im Vergleich zu dem Komplement des "sense"-RNA-Stranges aufweisen kann. Bevorzugt beträgt die Homologie mindestens 80 %, bevorzugt mindestens 90 %, ganz besonders bevorzugt mindestens 95 %, am meisten bevorzugt 100 % zwischen dem "antisense"-RNA-Strang und dem Komplement des "sense"-RNA-Strangs.
   "Teil des "sense"-RNA-Transkriptes" einer Nukleinsäuresequenz kodierend für ein BI-1 Protein oder ein funktionelles Äquivalent desselben meint Fragmente einer RNA oder mRNA transkibiert von einer für ein BI-1-Protein oder ein funktionelles Äquivalent desselben kodierenden Nukleinsäuresequenz, bevorzugt von einem BI-1-Gen. Dabei haben die Fragmente bevorzugt eine Sequenzlänge von mindestens 20 Basen, bevorzugt mindestens 50 Basen, besonders bevorzugt mindestens 100 Basen, ganz besonders bevorzugt mindestens 200 Basen, am meisten bevorzugt mindestens 500 Basen. Umfasst ist auch die vollständige transkribierte RNA oder mRNA.
   Umfasst ist auch die Verwendung der erfindungsgemäßen dsRNA-Moleküle in den erfindungsgemäßen Verfahren zur Erzeugung einer Pathogenresistenz in Pflanzen.
   Die dsRNA kann aus einem oder mehr Strängen polymerisierter Ribonukleotide bestehen. Es können ferner Modifikationen sowohl des Zucker-Phosphat-Gerüstes als auch der Nukleoside vorliegen. Beispielsweise können die Phosphodiesterbindungen der natürlichen RNA dahingehend modifiziert sein, dass sie zumindest ein Stickstoff oder Schwefel-Heteroatom umfassen. Basen können dahingehend modifiziert werden, dass die Aktivität beispielsweise von Adenosindearninase eingeschränkt wird. Solche und weitere Modifikationen sind weiter unten bei den Verfahren zur Stabilisierung von antisense-RNA beschrieben.
   Natürlich können, um den gleichen Zweck zu erreichen, auch mehrere individuelle dsRNA Moleküle, die jeweils einen der oben definierten Ribonukleotidsequenzabschnitte umfassen, in die Zelle oder den Organismus eingebracht werden.
   Die dsRNA kann enzymatisch oder ganz oder teilweise chemisch-synthetisch hergestellt werden.
   Die doppelsträngige dsRNA Struktur kann ausgehend von zwei komplementären, separaten RNA-Strängen oder - bevorzugt - ausgehend von einem einzelnen, selbstkomplementären RNA-Strang gebildet werden.
   Die doppelsträngige Struktur kann ausgehend von einem einzelnen, selbstkompleznentären Strang oder ausgehend von zwei komplementären Strängen gebildet werden. Bei einem einzelnen, selbstkomplementären Strang, können "sense"- und "antisense"-Sequenz durch eine verbindende Sequenz ("Linker") verknüpft sein und beispielsweise eine Haarnadelstruktur ausbilden. Bevorzugt kann die verbindende Sequenz ein Intron sein, das nach Synthese der dsRNA herausgespleißt wird. Die Nukleinsäuresequenz kodierend für eine dsRNA kann weitere Elemente beinhalten, wie beispielsweise Transkriptionsterminationssignale oder Polyadenylierungssignale. Sollen die zwei Stränge der dsRNA in einer Zelle oder Pflanze zusammengebracht werden, so kann dies auf verschiedene Art geschehen:
   Die Nukleinsäuresequenz kodierend für eine dsRNA kann weitere Elemente beinhalten, wie beispielsweise Transkriptionsterminationssignale oder Polyadenylierungssignale.
   Sollen die zwei Stränge der dsRNA in einer Zelle oder Pflanze zusammengebracht werden, so kann dies auf verschiedene Art geschehen:
   a) Transformation der Zelle oder Pflanze mit einem Vektor, der beide Expressionskassetten umfasst,
   b) Kotransformation der Zelle oder Pflanze mit zwei Vektoren, wobei der eine die Expressionskassetten mit dem "sense"-Strang, der andere die Expressionskassetten mit dem "antisense"-Strang umfasst.
   c) Kreuzung von zwei Pflanzen, die mit jeweils einem Vektor transformiert wurden, wobei der eine die Expressionskassetten mit dem "sense"-Strang, der andere die Expressionskassetten mit dem "antisense"-Strang umfasst.

   Die Bildung der RNA Duplex kann entweder außerhalb der Zelle oder innerhalb derselben initiiert werden. Wie in WO 99/53050 kann die dsRNA auch eine Haarnadelstruktur umfassen, indem "sense"- und "antisense"-Strang durch einen "Linker" (beispielsweise ein Intron) verbunden werden. Die selbstkomplementären dsRNA-Strukturen sind bevorzugt, da sie lediglich die Expression eines Konstruktes erfordern und die komplementären Stränge stets in einem äquimolaren Verhältnis umfassen.
   Die Expressionskassetten kodierend für den "antisense"- oder "sense"-Strang einer dsRNA oder für den selbstkomplementären-Strang der dsRNA, werden bevorzugt in einen Vektor insertiert und mit den unten beschriebenen Verfahren stabil (beispielsweise unter Verwendung von Selektionsmarkern) in das Genom einer Pflanze insertiert unter Kontrolle eines Epidermisspezifischen Promoters wie hierin aufgeführt, um eine dauerhafte Expression der dsRNA in der Epidermis zu gewährleisten.
   Die dsRNA kann unter Verwendung einer Menge eingeführt werden, die zumindest ein Kopie pro Zelle ermöglicht. Höhere Mengen (z.B. mindestens 5, 10, 100, 500 oder 1000 Kopien pro Zelle) können ggf. eine effizienter Verminderung bewirken.
   Wie bereits beschrieben, ist eine 100%ige Sequenzidentität zwischen dsRNA und einem BI-1 Gentranskript oder dem Gentranskript eines funktionell äquivalenten Gens nicht zwingend erforderlich, um eine effiziente Verminderung der BI-1 Expression zu bewirken. Demzufolge besteht der Vorteil, dass das Verfahren tolerant ist gegenüber Sequenzabweichungen, wie sie infolge genetischer Mutationen, Polymorphismen oder evolutionärer Divergenzen vorliegen können. So ist es beispielsweise möglich mit der dsRNA, die ausgehend von der BI-1 Sequenz des einen Organismus generiert wurde, die BI-1 Expression in einem anderen Organismus zu unterdrücken. Die hohe Sequenzhomologie zwischen den BI-1 Sequenzen aus Reis, Mais und Gerste lässt auf einen hohen Konservierungsgrad dieses Proteins innerhalb von Pflanzen schließen, so dass die Expression einer dsRNA abgeleitet von einer der offenbarten BI-1 Sequenzen gemäß SEQ ID NO: 1, 3 oder 5 auch einen vorteilhaften Effekt in anderen Pflanzenarten haben dürfte.
   Auch ist es aufgrund der hohen Homologie zwischen den einzelnen BI-1-Proteinen und ihren funktionellen Äquivalenten möglich mit einer einzigen dsRNA, die ausgehend von einer bestimmten BI-1-Sequenz eines Organismus generiert wurde, die Expression weiterer homologer BI-1-Proteine und/oder deren funktioneller Äquivalente des gleichen Organismus oder aber auch die Expression von BI-1-Proteinen in anderen verwandten Arten zu unterdrücken. Zu diesem Zweck umfasst die dsRNA bevorzugt Sequenzbereich von BI-1-Gentranskripten, die konservierten Bereichen entsprechen. Besagte konservierte Bereiche können aus Sequenzvergleichen leicht abgeleitet werden.
   Die dsRNA kann entweder in vivo oder in vitro synthetisiert werden. Dazu kann eine DNA-Sequenz kodierend für eine dsRNA in eine Expressionskassette unter Kontrolle mindestens eines genetischen Kontrollelementes (wie beispielsweise Promotor, Enhancer, Silencer, Splice-Donor oder -Akzeptor, Polyadenylierungssignal) gebracht werden, wobei eine Epidermis-spezifische Expression der dsRNA angestrebt wird. Entsprechend vorteilhafte Konstruktionen sind weiter unten beschrieben. Eine Polyadenylierung ist nicht erforderlich, ebenso müssen keine Elemente zur Initiierung einer Translation vorhanden sein.
   Eine dsRNA kann chemisch oder enzymatisch synthetisiert werden. Dazu können zelluläre RNA Polymerasen oder Bakteriophagen RNA Polymerasen (wie z.B. T3-, T7- oder SP6 RNA-Polymerase) verwendet werden. Entsprechende Verfahren zu in vitro Expression von RNA sind beschrieben (WO 97/32016; US 5,593,874; US 5,698,425, US 5,712,135, US 5,789,214, US 5,804,693). Eine chemisch oder enzymatisch in vitro syntetisierte dsRNA kann vor der Einführung in eine Zelle, Gewebe oder Organismus aus dem Reaktionsgemisch beispielsweise durch Extraktion, Präzipitation, Elektrophorese, chromatographie oder Kombinationen dieser Verfahren ganz oder teilweise aufgereinigt werden. Die dsRNA kann unmittelbar in die Zelle eingeführt werden oder aber auch extrazellulär (z.B. in den interstitialen Raum) appliziert werden.
   Bevorzugt wird die Pflanze jedoch stabil mit einem Expressionskonstrukt, das die Expression der dsRNA in der Epidermis realisiert, transformiert. Entsprechende Verfahren sind weiter unten beschrieben.
b) Einbringung eines BI-1 antisense-Nukleinsäuremoleküls
   Verfahren zur Suppression eines bestimmten Proteins durch Verhinderung der Akkumulation seiner mRNA durch die "antisense"-Technologie sind vielfach - auch in Pflanzen - beschrieben (Sheehy et al. (1988) Proc Natl Acad Sci USA 85: 8805-8809; US 4,801,340; Mol JN et al. (1990) FEBS Lett 268(2):427-430). Das antisense Nukleinsäuremolekül hybridisiert bzw. bindet mit der zellulären mRNA und/oder genomischen DNA kodierend für das zu supprimierende BI-1-Zielprotein. Dadurch wird die Transkription und/oder Translation des Zielproteins unterdrückt. Die Hybridisierung kann auf konventionelle Art über die Bildung einer stabilen Duplex oder - im Fall von genomischer DNA - durch Bindung des anti-sense Nukleinsäuremoleküls mit der Duplex der genomischen DNA durch spezifische Wechselwirkung in der großen Furche der DNA-Helix entstehen. Das Einbringen erfolgt so, dass die BI-1-Menge oder Funktion spezifisch in der Epidermis reduziert wird, z.B. durch transiente Transformation der Epidermis oder stabile Transformation unter Kontrolle der Expression eines entsprechenden Konstruktes mit einem Epidermis-spezifischen Promoters.
   Eine antisense Nukleinsäuresequenz geeignet zur Verminderung eines BI-1-Proteins kann unter Verwendung der für dieses Protein kodierenden Nukleinsäuresequenz, beispielsweise der Nukleinsäuresequenz gemäß SEQ ID NO: 1, 3, 5, 7, 9, 11, 13, 15, 17, 19, 21, 23, 25, 27, 29, 31, 33 oder 38 oder kodierend für ein funktionelles Äquivalent derselben nach den Basenpaarregeln von Watson und Crick abgeleitet werden. Die antisense Nukleinsäuresequenz kann zu der gesamten transkribierten mRNA des besagten Proteins komplementär sein, sich auf die kodierende Region beschränken oder nur aus einem Oligonukleotid bestehen, das zu einem Teil der kodierenden oder nicht-kodierenden Sequenz der mRNA komplementär ist. So kann das Oligonukleotid beispielsweise komplementär zu der Region sein, die den Translationsstart für das besagte Protein umfasst. Antisense-Nukleinsäuresequenzen können eine Länge von zum Beispiel 5, 10, 15, 20, 25, 30, 35, 40, 45 oder 50 Nukleotide haben, können aber auch länger sein und mindestens 100, 200, 500, 1000, 2000 oder 5000 Nukleotide umfassen. Antisense-Nukleinsäuresequenzen können rekombinant exprimiert oder chemisch bzw. enzymatisch unter Verwendung von dem Fachmann bekannten Verfahren synthetisiert werden. Bei der chemischen Synthese können natürlich oder modifizierte Nukleotide verwendet werden. Modifizierte Nukleotide können der antisense Nukleinsäuresequenz eine erhöhte biochemische Stabilität verleihen und zu einer erhöhten physikalischen Stabilität der Duplex gebildet aus antisense-Nukleinsäuresequenz und sense-Zielsequenz führen. Verwendet werden können beispielsweise Phosphorothioatderivative und Acridin-substitierte Nukleotide wie 5-Fluorouracil, 5-Bromouracil, 5-Chlorouracil, 5-Iodouracil, Hypoxanthin, Xanthin, 4-Acetylcytosin, 5-(Carboxyhydroxylmethyl)uracil, 5-Carboxymethylaminomethyl-2-thiouridin, 5-Carboxymethylaminomethyluracil, Dihydrouracil, β-D-Galactosylqueosin, Inosine, N6-Isopentenyladenin, 1-Methylguanin, 1-Methylinosin, 2,2-Dimethylguanin, 2-Methyladenin, 2-Methylguanin, 3-Methylcytosin, 5-Methylcytosin, N6-Adenin, 7-Methylguanin, 5-Methylaminomethyluracil, 5-Methoxyaminomethyl-2-thiouracil, β-D-mannosylqueosin, 5'-Methoxycarboxymethyluracil, 5-Methoxyuracil, 2-Methylthio-N6-isopentenyladenin, Uracil-5-oxyessigsäure, Pseudouracil, Queosine, 2-Thiocytosin, 5-Methyl-2-thiouracil, 2-Thiouracil, 4-Thiouracil, 5-Methyluracil, Uracil-5-oxyessigsäuremethylester, Uracil-5-oxyessigsäure, 5-Methyl-2-thiouracil, 3-(3-Amino-3-N-2-carboxypropyl)uracil und 2,6-Diaminopurin.
   In einer weiteren bevorzugten Ausführungsform kann die Expression eines BI-1-Proteins durch Nukleotidsequenzen inhibiert werden, die komplementär zu der regulatorischen Region eines BI-1-Gens (z.B. einem BI-1 Promoter und/oder Enhancer) sind und triple-helikale Strukturen mit der dortigen DNA-Doppelhelix ausbilden, so dass die Transkription des BI-1-Gens vermindert wird. Entsprechende Verfahren sind beschrieben (Helene C (1991) Anticancer Drug Res 6(6):569-84; Helene C et al. (1992) Ann NY Acad Sci 660:27-36; Maher LJ (1992) Bioassays 14(12):807-815). In einer weiteren Ausführungsform kann das antisense Nukleinsäuremolekül eine α-anomere Nukleinsäure sein. Derartige α-anomere Nukleinsäuremoleküle bilden spezifische doppelsträngige Hybride mit komplementärer RNA in denen - im Unterschied zu den konventionellen β-Nukleinsäuren - die beiden Stränge parallel zueinander verlaufen (Gautier C et al. (1987) Nucleic Acids Res 15:6625-6641). Das antisense Nukleinsäuremolekül kann ferner auch 2'-O-Methylribonukleotide (Inoue et al. (1987) Nucleic Acids Res 15:6131-6148) oder chimäre RNA-DNA Analoge beinhalten (Inoue et al. (1987) FEBS Lett 215:327-330).
c) Einbringung eines BI-1 antisense-Nukleinsäuremoleküls kombiniert mit einem Ribozym
   Vorteilhaft kann die oben beschriebene antisense-Strategie mit einem Ribozym-Verfahren gekoppelt werden. Katalytische RNA-Moleküle oder Ribozyme können an jede beliebige Ziel-RNA angepasst werden und spalten das Phosphodiester-Gerüst an spezifischen Positionen, wodurch die Ziel-RNA funktionell deaktiviert wird (Tanner NK (1999) FEMS Microbiol Rev 23(3):257-275). Das Ribozym wird dadurch nicht selber modifiziert, sondern ist in der Lage, weitere Ziel-RNA-Moleküle analog zu spalten, wodurch es die Eigenschaften eines Enzyms erhält. Der Einbau von Ribozymsequenzen in "antisense"-RNAs verleiht eben diesen "antisense"-RNAs diese enzymähnliche, RNA-spaltende Eigenschaft und steigert so deren Effizienz bei der Inaktivierung der Ziel-RNA. Die Herstellung und Verwendung entsprechender Ribozym-"antisense"-RNA-Moleküle ist beispielsweise beschrieben bei Haseloff et al. (1988) Nature 334: 585-591.
   Auf diese Art können Ribozyme (z.B. "Hammerhead"-Ribozyme; Haselhoff und Gerlach (1988) Nature 334:585-591) verwendet werden, um die mRNA eines zu supprimierenden Enzyms - z.B. BI-1 - katalytisch zu spalten und die Translation zu verhindern. Die Ribozym-Technologie kann die Effizienz einer antisense-Strategie erhöhen. Verfahren zur Expression von Ribozymen zur Verminderung bestimmter Proteine sind beschrieben in (EP 0 291 533, EP 0 321 201, EP 0 360 257). In pflanzlichen Zellen ist eine Ribozym-Expression ebenfalls beschrieben (Steinecke P et al. (1992) EMBO J 11(4):1525-1530; de Feyter R et al. (1996) Mol Gen Genet. 250(3):329-338). Geeignete Zielsequenzen und Ribozyme können zum Beispiel wie bei "Steinecke P, Ribozyme, Methods in Cell Biology 50, Galbraith et al. eds, Academic Press, Inc. (1995), S. 449-460" beschrieben, durch Sekundärstrukturberechnungen von Ribozym- und Ziel-RNA sowie durch deren Interaktion bestimmt werden (Bayley CC et al. (1992) Plant Mol Biol. 18(2):353-361; Lloyd AM and Davis RW et al. (1994) Mol Gen Genet. 242(6):653-657). Beispielsweise können Derivate der Tetrahymena L-19 IVS RNA konstruiert werden, die komplementäre Bereiche zu der mRNA des zu supprimierenden BI-1 Proteins aufweisen (siehe auch US 4,987,071 und US 5,116,742). Alternativ können solche Ribozyme auch über einen Selektionsprozess aus einer Bibliothek diverser Ribozyme identifiziert werden (Bartel D und Szostak JW (1993) Science 261:1411-1418). Die Expression erfolgt z.B. unter Kotrolle eines Epidermis-spezifischen Promoters.
d) Einbringung eines BI-1 sense-Nukleinsäuremoleküls zur Induktion eines Kosuppression
   Die Epidermis-spezifische Expression eines BI-1 Nukleinsäuremoleküls in sense-Orientierung kann zu einer Kosuppression des entsprechenden homologen, endogenen Gens in Epidermiszellen führen. Die Expression von sense-RNA mit Homologie zu einem endogenen Gen kann die Expression desselben vermindern oder ausschalten, ähnlich wie es für antisense Ansätze beschrieben wurde (Jorgensen et al. (1996) Plant Mol Biol 31(5):957-973; Goring et al. (1991) Proc Natl Acad Sci USA 88:1770-1774; Smith et al. (1990) Mol Gen Genet 224:447-481; Napoli et al. (1990) Plant Cell 2:279-289; Van der Krol et al. (1990) Plant Cell 2:291-99). Dabei kann das eingeführte Konstrukt das zu vermindernde, homologe Gen ganz oder nur teilweise repräsentieren. Die Möglichkeit zur Translation ist nicht erforderlich. Die Anwendung dieser Technologie auf Pflanzen ist beispielsweise beschrieben bei Napoli et al. (1990) The Plant Cell 2: 279-289 und in US 5,034,323.
   Bevorzugt wird die Kosuppression unter Verwendung einer Sequenz realisiert, die im wesentlichen identisch ist zu zumindest einem Teil der Nukleinsäuresequenz kodierend für ein BI-1-Protein oder ein funktionelles Äquivalent desselben, beispielsweise der Nukleinsäuresequenz gemäß SEQ ID NO: 1, 3, 5, 7, 9, 11, 13, 15, 17, 19, 21, 23, 25, 27, 29, 31, 33 oder 38 oder der Nukleinsäuresequenz kodierend für ein funktionelles Äquivalent derselben.
e) Einbringung von Nukleinsäuremolekülen kodierend für ein dominant-negatives BI-1 Protein
   Die Funktion oder Aktivität eines BI-1 Protein kann effektiv auch durch Epidermis-spezifische Expression einer dominant-negativen Variante dieses BI-1-Proteins in Epidermiszellen reduziert werden. Verfahren zur Verminderung der Funktion bzw. Aktivität eines Protein mittels Koexpression seiner dominant-negativen Form sind dem Fachmann bekannt (Lagna G und Hemmati-Brivanlou A (1998) Current Topics in Developmental Biology 36:75-98; Perlmutter RM und Alberola-Ila J (1996) Current Opinion in Immunology 8(2):285-90; Sheppard D (1994) American Journal of Respiratory Cell & Molecular Biology. 11(1):1-6; Herskowitz I (1987) Nature 329(6136):219-22).
   Die bevorzugt zu mutierende Aminosäure in BI-1 Homologen aus anderen Arten kann beispielsweise mittels Computer-unterstütztem Vergleich ("Alignment") ermittelt werden. Diese Mutationen zum Erreichen einer dominant-negativen BI-1 Variante werden bevorzugt auf der Ebene der Nukleinsäure-sequenz kodierend für BI-1 Proteine durchgeführt. Eine entsprechende Mutation kann beispielsweise durch PCR vermittelte in vitro Mutagenese unter Verwendung entsprechender Oligonukleotidprimer, durch welche die gewünschte Mutation eingeführt wird, realisiert werden. Dazu werden dem Fachmann geläufige Verfahren verwendet. Beispielsweise kann zu diesem Zweck der "LA PCR in vitro Mutagenesis Kit" (Takara Shuzo, Kyoto) verwendet werden. Ein Verfahren zur Herstellung einer dominant-negativen Variante eines RacB-Proteins aus Mais ist auch in WO 00/15815 (Beispiel 4, S. 69) beschrieben.
   Die Expression einer solchen Mutante kann dann z.B. unter Kontrolle eines Epidermis-spezifischen Promoters erfolgen.
f) Einbringung DNA-oder Protein-bindende Faktoren gegen BI-1 Gene, -RNAs oder Proteine
   Eine Verminderung einer BI-1 Genexpression in der Epidermis ist auch mit spezifischen DNA-bindenden Faktoren z.B. mit Faktoren vom Typus der Zinkfingertranskriptionsfaktoren möglich. Diese Faktoren lagern sich an die genomische Sequenz des endogenen Zielgens, bevorzugt in den regulatorischen Bereichen, an und bewirken eine Repression des endogenen Gens. Die Verwendung eines solchen Verfahrens ermöglicht die Verminderung der Expression eines endogenen BI-1 Gens, ohne dass dessen Sequenz gentechnisch manipuliert werden muss. Entsprechende Verfahren zur Herstellung entsprechender Faktoren sind beschrieben (Dreier B et al. (2001) J Biol Chem 276(31):29466-78; Dreier B et al. (2000) J Mol Biol 303(4):489-502; Beerli RR et al. (2000) Proc Natl Acad Sci USA 97 (4):1495-1500; Beerli RR et al. (2000) J Biol Chem 275(42):32617-32627; Segal DJ and Barbas CF 3rd (2000) Curr Opin Chem Biol 4(1):34-39; Kang JS and Kim JS (2000) J Biol Chem 275(12):8742-8748; Beerli RR et al. (1998) Proc Natl Acad Sci USA 95(25) :14628- 14633; Kim JS et al. (1997) Proc Natl Acad Sci USA 94(8):3616 -3620; Klug A (1999) J Mol Biol 293(2):215-218; Tsai SY et al. (1998) Adv Drug Deliv Rev 30(1-3):23-31; Mapp AK et al. (2000) Proc Natl Acad Sci USA 97(8):3930-3935; Sharrocks AD et al. (1997) Int J Biochem Cell Biol 29(12):1371-1387; Zhang L et al. (2000) J Biol Chem 275(43):33850-33860).
   Die Selektion dieser Faktoren kann unter Verwendung eines beliebigen Stückes eines BI-1-Gens erfolgen. Bevorzugt liegt dieser Abschnitt im Bereich der Promotorregion. Für eine Genunterdrückung kann er aber auch im Bereich der kodierenden Exons oder Introns liegen. Die entsprechenden Abschnitte sind für den Fachmann mittels Datenbankabfrage aus der Genbank oder - ausgehend von einer BI-1 cDNA, deren Gen nicht in der Genbank vorhanden ist, durch Durchmusterung einer genomischen Bibliothek nach korrespondierenden genomischen Klonen erhältlich. Die dazu erforderlichen Verfahren sind dem Fachmann geläufig, z.B. können diese Faktoren unter Kontrolle eines Epidermis-spezifischen Promoters oder anderer eine Epidermis-spezifische Expression vermittelnden Faktoren exprimiert werden.
   Ferner können Faktoren in eine Zelle eingebracht werden, die das BI-1 Zielprotein selber inhibieren. Die proteinbindenden Faktoren können z.B. Aptamere (Famulok M und Mayer G (1999) Curr Top Microbiol Immunol 243:123-36) oder Antikörper bzw. Antikörperfragmente oder einzelkettige Antikörper sein. Die Gewinnung dieser Faktoren ist beschrieben und dem Fachmann bekannt. Beispielsweise wurde ein cytoplasmatischer scFv Antikörper eingesetzt, um die Aktivität des Phytochrom A Proteins in gentechnisch veränderten Tabakpflanzen zu modulieren (Owen M et al. (1992) Biotechnology (N Y) 10(7):790-794; Franken E et al. (1997) Curr Opin Biotechnol 8(4):411-416; Whitelam (1996) Trend Plant Sci 1:286-272).
   Die Genexpression kann auch durch maßgeschneiderte, niedermolekulare synthetische Verbindungen unterdrückt werden, beispielsweise vom Polyamid-Typ (Dervan PB und Bürli RW (1999) Current Opinion in Chemical Biology 3:688-693; Gottesfeld JM et al. (2000) Gene Expr 9(1-2):77-91). Diese Oligomere bestehen aus den Bausteinen 3-(Dimethylamino)-propylamin, N-Methyl-3-hydroxypyrrol, N-Methylimidazol und N-Methylpyrrole und können an jedes Stück doppelsträngiger DNA so angepasst werden, dass sie sequenzspezifisch in die große Furche binden und die Expression der dortigen Gensequenzen blockieren. Entsprechende Verfahren sind beschrieben (siehe unter anderem Bremer RE et al. (2001) Bioorg Med Chem. 9(8):2093-103; Ansari AZ et al. (2001) Chem Biol. 8(6):583-92; Gottesfeld JM et al. (2001) J Mol Biol. 309(3):615-29; Wurtz NR et al. (2001) Org Lett 3(8):1201-3; Wang CC et al. (2001) Bioorg Med Chem 9(3): 653-7; Urbach AR und Dervan PB (2001) Proc Natl Acad Sci USA 98(8):4343-8; Chiang SY et al. (2000) J Biol Chem. 275(32):24246-54).
   Alle genannten Faktoren werden Epidermis-spezifisch eingebracht, um eine Reduktion der BI-1-Aktivität lediglich in Epidermiszellen zu gewährleisten, z.B. durch Expression unter Kontrolle eines Epidermis-spezifischen Promoters, wie sie z.B. oben genannt sind.
g) Einbringung von den BI-1 RNA-Abbau bewirkenden viralen Nukleinsäuremolekülen und entsprechenden Expressionskonstrukten
   Die BI-1 Expression in der Epidermis kann effektiv auch durch Induktion des spezifischen BI-1 RNA-Abbaus in Epidermiszellen mit Hilfe eines viralen Expressionssystems (Amplikon) (Angell, SM et al. (1999) Plant J. 20(3):357-362) realisiert werden. Diese Systeme - auch als "VIGS" (viral induced gene silencing) bezeichnet - bringen Nukleinsäuremoleküle mit Homologie zu den zu supprimierenden Transkripten mittels viraler Vektoren in die Pflanze ein. Die Transkription wird sodann - vermutlich mediiert durch pflanzliche Abwehrmechanismen gegen Viren - abgeschaltet. Entsprechende Techniken und Verfahren sind beschrieben (Ratcliff F et al. (2001) Plant J 25(2):237-45; Fagard M und Vaucheret H (2000) Plant Mol Biol 43(2-3):285-93; Anandalakshmi R et al. (1998) Proc Natl Acad Sci USA 95(22):13079-84; Ruiz MT (1998) Plant Cell 10(6): 937-46).
h) Einbringung von Konstrukten zur Induktion einer homologen Rekombination an endogenen BI-1-Genen beispielsweise zur Erzeugung von Knockout-Mutanten
   Zur Herstellung eines homolog rekombinanten Organismus mit verminderter BI-1-Aktivität in den Epidermiszellen verwendet man beispielsweise ein Nukleinsäurekonstrukt, das zumindest einen Teil eines endogenen BI-1 Gens enthält, das durch eine Deletion, Addition oder Substitution mindestens eines Nukleotids so verändert wird, so dass die Funktionalität vermindert oder gänzlich aufgehoben wird. Die Veränderung kann auch die regulativen Elemente (z.B. den Promotor) des Gens betreffen, so dass die kodierende Sequenz unverändert bleibt, eine Expression (Transkription und/oder Translation) jedoch unterbleibt und vermindert wird.
   Bei der konventionellen homologen Rekombination ist die veränderte Region an ihrem 5'- und 3'-Ende von weiteren Nukleinsäuresequenzen flankiert, die eine ausreichende Länge für die Ermöglichung der Rekombination aufweisen müssen. Die Länge liegt in der Regel in einem Bereich von mehreren einhundert Basen bis zu mehreren Kilobasen (Thomas KR und Capecchi MR (1987) Cell 51:503; Strepp et al. (1998) Proc Natl Acad Sci USA 95(8):43368-4373). Für die homologe Rekombination wird der Wirtsorganismus - zum Beispiel eine Pflanze - mit dem Rekombinationskonstrukt unter Verwendung der unten beschriebenen Verfahren transformiert und erfolgreich rekombinierte Klone unter Verwendung zum Beispiel einer Antibiotika- oder Herbizidresistenz selektioniert.
   Homologe Rekombination ist ein relativ seltenes Ereignis in höheren Eukaryoten, vor allem in Pflanzen. Zufällige Integrationen in das Wirtsgenom überwiegen. Eine Möglichkeit die zufällig integrierten Sequenzen zu entfernen und so Zellklone mit einer korrekten homologen Rekombination anzureichern, besteht in der Verwendung eines sequenzspezifischen Rekombinationssystems wie in US 6,110,736 beschrieben, durch welche unspezifisch integrierte Sequenzen wieder deletiert werden können, was die Selektion erfolgreich über homologe Rekombination integrierter Ereignisse erleichtert. Eine Vielzahl von sequenzspezifischen Rekombinationssystemen kann verwendet werden, beispielhaft sind das Cre/lox-System des Bacteriophagen P1, das FLP/FRT System der Hefe, die Gin Rekombinase des Mu Phagen, die Pin Rekombinase aus E. coli und das R/RS System des pSR1 Plasmids genannt. Bevorzugt sind das Bacteriophagen P1 Cre/lox und das Hefe FLP/FRT System. Das FLP/FRT und cre/lox Rekombinasesystem wurde bereits in pflanzlichen Systemen angewendet (Odell et al. (1990) Mol Gen Genet 223: 369-378). Die Epidermis-spezifische Rekombination kann z.B. dadurch gewährleistet werden, dass die Expression der die Rekombination vermittelnden Systeme und Enzyme unter Kontrolle eines Epidermis-spezifischen Promoters erfolgt.
i) Einführung von Mutationen in endogene BI-1 Gene zur Erzeugung eines Funktionsverlustes (z.B. Generierung von Stopp-Kodons, Verschiebungen im Leseraster etc.)
   Weitere geeignete Methoden zur Verminderung der BI-1-Aktivität sind die Einführung von Nonsense-Mutationen in endogene BI-1 Gene zum Beispiel mittels Einführung von RNA/DNA-Oligonukleotiden in die Epidermiszellen (Zhu et al. (2000) Nat Biotechnol 18(5):555-558) sowie die Generierung von Knock-out-Mutanten mit Hilfe von z.B. T-DNA-Mutagenese (Koncz et al. (1992) Plant Mol Biol 20(5):963-976), ENU-(N-Ethyl-N-nitrosoharnstoff) - Mutagenese oder homolger Rekombination (Hohn B und Puchta (1999) H Proc Natl Acad Sci USA 96:8321-8323.). Punktmutationen können auch mittels DNA-RNA Hybriden erzeugt werden, die auch als "chimeraplasty" bekannt sind (Cole-Strauss et al. (1999) Nucl Acids Res 27(5):1323-1330; Kmiec (1999) Gene therapy American Scientist 87(3):240-247).

Die Methoden der dsRNAi, der Kosuppression mittels sense-RNA und der "VIGS" ("virus induced gene silencing") werden auch als "post-transcriptional gene silencing" (PTGS) bezeichnet. PTGS-Verfahren wie auch die Verminderung der BI-1-Funktion oder Aktivität mit dominant-negativen BI-1-Varianten sind besonders vorteilhaft, weil die Anforderungen an die Homologie zwischen dem zu supprimierenden endogenem Gen und der transgen exprimierten sense- oder dsRNA-Nukleinsäuresequenz (bzw. zwischen dem endogenen Gen und seiner dominant-negativen Variante) geringer sind als beispielsweise bei einem klassischen antisense-Ansatz. Entsprechende Homologie-Kriterien sind bei der Beschreibung des dsRNAI-Verfahrens genannt und allgemein für PTGS-Verfahren oder dominant-negative Ansätze übertragbar. So kann man voraussichtlich unter Verwendung der BI-1-Nukleinsäuresequenzen auch die Expression von homologen BI-1-Proteinen in anderen Arten effektiv supprimieren, ohne dass die Isolierung und Strukturaufklärung der dort vorkommenden BI-1-Homologen zwingend erforderlich wäre. Dies erleichtert erheblich den Arbeitsaufwand. Analog kann man voraussichtlich auch unter Verwendung von dominant-negativen Varianten eines BI-1-Proteins die Funktion/Aktivität seines Homologs in anderen Pflanzenarten effektiv vermindern oder unterdrücken.

Alle Substanzen und Verbindungen die direkt oder indirekt eine Verminderung der Proteinmenge, RNA-Menge, Genaktivität oder Proteinaktivität eines BI-1-Proteins bewirken, seien infolge unter der Bezeichnung "anti-BI-1"-Verbindungen zusammengefasst. Der Begriff "anti-BI-1"-verbindung schließt explizit die in den oben beschriebenen Verfahren zum Einsatz kommenden Nukleinsäuresequenzen, Peptide, Proteine oder andere Faktoren ein.

"Einbringung" umfasst im Rahmen der Erfindung alle Verfahren, die dazu geeignet eine "anti-BI-1"-Verbindung, direkt oder indirekt, in der Epidermis oder einem wesentlichen Teil der Epidermiszellen, Kompartiment oder Gewebe derselben einzuführen oder dort zu generieren. Direkte und indirekte Verfahren sind umfasst. Die Einbringung kann zu einer vorübergehenden (transienten) Präsenz einer "anti-BI-1"-Verbindung (beispielsweise einer dsRNA) führen oder aber auch zu einer dauerhaften (stabilen).

Gemäß der unterschiedlichen Natur der oben beschriebenen Ansätze kann die "anti-BI-1"-Verbindung ihre Funktion direkt ausüben (zum Beispiel durch Insertion in ein endogenes BI-1 Gen). Die Funktion kann aber auch indirekt nach Transkription in eine RNA (zum Beispiel bei antisense Ansätzen) oder nach Transkription und Translation in ein Protein (zum Beispiel bei Bindungsfaktoren) ausgeübt werden. Sowohl direkte als auch indirekt wirkende "anti-BI-1"-Verbindungen sind erfindungsgemäß umfasst.

Einführen umfasst beispielsweise Verfahren wie Transfektion, Transduktion oder Transformation.

"Anti-BI-1" Verbindungen umfasst somit beispielsweise auch rekombinante Expressionskonstrukte, die eine Expression (d.h. Transkription und ggf. Translation) beispielsweise einer BI-1-dsRNA oder einer BI-1 "antisense"-RNA Epidermis-spezifisch bedingen.

In besagten Expressionskonstrukten steht ein Nukleinsäuremolekül, dessen Expression (Transkription und ggf. Translation) eine "anti-BI-1"-Verbindung generiert, bevorzugt in funktioneller Verknüpfung mit mindestens einem genetischen Kontrollelement (beispielsweise einem Promotor), das eine Expression in einem Organismus, bevorzugt in Pflanzen, gewährleistet, vorzugsweise Epidermis-spezifisch. Soll das Expressionskonstrukt direkt in die Pflanze eingeführt und die "anti-BI-1"-Verbindung (beispielsweise die BI-1 dsRNA) dort in plantae erzeugt werden, so sind pflanzenspezifische genetische Kontrollelemente (beispielsweise Promotoren) bevorzugt, wobei aus dem oben gesagten die Epidermis-spezifische Aktivität des Promoters für eine Epidermis-spezifische Reduktion von BI-1 in den meisten Ausführungsformen wie oben beschrieben zwingend ist. Die "anti-BI-1"-Verbindung kann jedoch auch in anderen Organismen oder in vitro erzeugt und dann in die Pflanze eingebracht werden. In diesem sind all prokaryotischen oder eukaryotischen genetischen Kontrollelemente (beispielsweise Promotoren) bevorzugt, die die Expression in den jeweils für die Herstellung gewählten Organismus erlauben.

Unter einer funktionellen Verknüpfung versteht man zum Beispiel die sequentielle Anordnung eines Promotors mit der zu exprimierenden Nukleinsäuresequenz (zum Beispiel einer "anti-BI-1"-Verbindung) und ggf. weiterer regulativer Elemente wie zum Beispiel einem Terminator derart, dass jedes der regulativen Elemente seine Funktion bei der transgenen Expression der Nukleinsäuresequenz, je nach Anordnung der Nukleinsäuresequenzen zu sense oder anti-sense RNA, erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben (cis- bzw. trans-Lokalisation). Bevorzugt sind Anordnungen, in denen die transgen zu exprimierende Nukleinsäuresequenz hinter der als Promoter fungierenden Sequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Die Herstellung einer funktionellen Verknüpfung als auch die Herstellung einer Expressionskassette kann mittels gängiger Rekombinations- und Klonierungstechniken realisiert werden, wie sie beispielsweise in Maniatis T, Fritsch EF und Sambrook J (1989) Molecular Cloning: A Laboratory Manual, Cold Spring Harbor Laboratory, Cold Spring Harbor (NY), in Silhavy TJ, Berman ML und Enquist LW (1984) Experiments with Gene Fusions, Cold Spring Harbor Laboratory, Cold Spring Harbor (NY), in Ausubel FM et al. (1987) Current Protocols in Molecular Biology, Greene Publishing Assoc. and Wiley Interscience und bei Gelvin et al. (1990) In: Plant Molecular Biology Manual beschrieben sind. Zwischen beide Sequenzen können aber auch weitere Sequenzen positioniert werden, die zum Beispiel die Funktion eines Linkers mit bestimmten Restriktionsenzymschnittstellen oder eines Signalpeptides haben. Auch kann die Insertion von Sequenzen zur Expression von Fusionsproteinen führen. Bevorzugt kann die Expressionskassette, bestehend aus einer Verknüpfung von Promoter und zu exprimierender Nukleinsäuresequenz, integriert in einem Vektor vorliegen und durch zum Beispiel Transformation in ein pflanzliches Genom insertiert werden. Die Kontrollelemente vermitteln bevorzugt eine Epidermis-spezifische Expression.

Die obengenannten Verfahren (a) bis (i) können auch für die Reduktion der Aktivität, insbesondere der Expression der anderen hier genannten Proteine eingesetzt werden, insbesondere zur Reduktion von MLO, RacB und NaOx verwendet werden.

Das BI1-Protein aus Gerste (hvBI1) wird vorwiegend im Mesophyll exprimiert (Beispiel 6)und infolge einer Infektion mit Blumeria (syn. Erysiphe) graminis f. sp.hordei hochreguliert (Beispiel 2). Die rekombinante mesophyll-spezifische Überexpression in *mlo*-resistenter Gerste führt - neben der Resistenz gegen insbesondere nekrotrophe und hemibiotrophe Pathogene - zu einer Blumeria (syn. Erysiphe) graminis f. sp.hordei -resistenten Pflanze, die keine nekrotischen Flecken ("mlo-Flecken"; negative Begleiterscheinung der mlo-Resistenz) zeigt. Unter Ausnutzen dieses Effekts lassen sich die negativen Begleiterscheinungen der mlo-vermittelten Resistenz (Ertragseinbuße von ca. 5%, Jörgensen JH (1992) Euphytica 63: 141-152); Hypersuszeptibilität gegen nekrotrophe Pilze, Jarosch B et al. (1999) Mol Plant Microbe Interact 12:508-514; Kumar J et al. (2001) Phytopathology 91:127-133) unterdrücken, ohne dass die mlo-Resistenz selber beeinträchtigt wird.

Ferner kann überraschenderweise gezeigt werden, dass eine Überexpression von BI1 eine Resistenz gegen Streßfaktoren wie nekrosen-auslösende Agenzien (isoliert z.B. aus nekrotrophen Schadpilzen; Beispiel 2) zur Folge hat.

Das erfindungsgemäße Verfahren bietet demnach eine effiziente biotechnologische Strategie der Resistenz gegen Nekrotisierung durch endogenen, abiotischen und biotischen Streß - beispielsweise mlo-Flecken, Ozonschäden, nekrotrophe und hemibiotrophe Schadorganismen.

BI1-Proteine scheinen zentrale Regulatoren der rasseunspezifischen Pilzresistenz in Pflanzen zu sein. Dies ermöglicht eine breite Einsetzbarkeit in biotechnologischen Strategien zur Erhöhung der Pathogenresistenz in Pflanzen insbesondere der Pilzresistenz. Das erfindungsgemäße Verfahren kann im Prinzip auf alle Pflanzenarten angewendet werden. BI1-Proteine wurden in zahlreichen Pflanzen - Monokotyledonen und Dikotyledonen - identifiziert (s.o.).

"Ungefähr" meint im Rahmen dieser Erfindung im Zusammenhang mit Zahlen- oder Größenangaben einen Zahlen- oder Größenbereich um den angegebenen Zahlen- oder Größenwert herum. Im allgemeinen meint der Begriff ungefähr einen Bereich von jeweils 20% des angegebenen Wertes nach oben und nach unten.

"Pflanze" im Rahmen der Erfindung meint alle Gattungen und Arten höherer und niedrigerer Pflanzen des Pflanzenreiches. Eingeschlossen unter dem Begriff sind die reifen Pflanzen, Saatgut, Sprossen und Keimlinge, sowie davon abgeleitete Teile, Vermehrungsgut, Pflanzenorgane, Gewebe, Protoplasten, Kallus und andere Kulturen, zum Beispiel Zellkulturen, sowie alle anderen Arten von Gruppierungen von Pflanzenzellen zu funktionellen oder strukturellen Einheiten. Reife Pflanzen meint Pflanzen zu jedem beliebigen Entwicklungsstadium jenseits des Keimlings. Keimling meint eine junge, unreife Pflanze in einem frühen Entwicklungsstadium.

"Pflanze" umfaßt alle einjährigen und mehrjährige, monokotyledonen und dikotyledonen Pflanzen und schließt beispielhaft jedoch nicht einschränkend solche der Gattungen Cucurbita, Rosa, Vitis, Juglans, Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solarium, Petunia, Digitalis, Majorana, Ciahorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Glycine, Pisum, Phaseolus, Lolium, Oryza, Zea, Avena, Hordeum, Secale, Triticum, Sorghum, Picea und Populus ein.

Der Begriff "Pflanze" umfaßt bevorzugt monokotyledone Kulturpflanzen, wie zum Beispiel Getreidearten wie Weizen, Gerste, Hirse, Roggen, Triticale, Mais, Reis, Sorghum oder Hafer sowie Zuckerrohr.

Ferner umfaßt der Begriff dikotyledonen Kulturpflanzen, wie zum Beispiel
- Brassicacae wie Raps, Rübsen, Kresse, Arabidopsis, Kohlarten,
- Leguminosae wie Soja, Alfalfa, Erbse, Bohnengewächsen oder Erdnuss
- Solanaceae wie Kartoffel, Tabak, Tomate, Aubergine oder Paprika,
- Asteraceae wie Sonnenblume, Tagetes, Salat oder Calendula,
- Cucurbitaceae wie Melone, Kürbis oder Zucchini,
sowie Lein (Flachs), Baumwolle, Hanf, Klee, Spinat, Roter Pfeffer, Möhre, Karotte, Rübe, Rettich, Zuckerrübe, Süßkartoffel, Gurke, Chicorée, Blumenkohl, Brokkoli, Spargel, Zwiebel, Knoblauch, Sellerie, Erdbeere, Himbeere, Brombeere, Ananas, Avocado, und den verschiedenen Baum-, Strauch-, Nuß- und Weinarten. Baumarten umfaßt bevorzugt Pflaume, Kirsche, Pfirsich, Nektarine, Aprikose, Banane, Papaya, Mango, Apfel, Birne, Quitte.

Im Rahmen der Erfindung sind solche Pflanzen bevorzugt, die als Nahrungs- oder Futtermittel zum Einsatz kommen, ganz besonders bevorzugt monokotyledone Gattungen und Arten mit landwirtschaftlicher Bedeutung wie Weizen, Hafer, Hirse, Gerste, Roggen, Mais, Reis, Buchweizen, Sorghum, Triticale, Dinkel, Leinsamen oder Zuckerrohr.

Der Begriff "Streßfaktor" umfaßt im Rahmen der vorliegenden Erfindung biotische Streßfaktoren (wie insbesondere die unten aufgeführten Pathogene)

"Streßresistenz" meint das Vermindern oder Abschwächen von Symptomen einer Pflanze infolge von Stress. Die Symptome können vielfältiger Art sein, umfassen aber bevorzugt solche die direkt oder indirekt zu einer Beeinträchtigung Qualität der Pflanze, der Quantität des Ertrages, der Eignung zur Verwendung als Futter- oder Nahrungsmittel führen oder aber auch Aussaat, Anbau, Ernte oder Prozessierung des Erntegutes erschweren.

"Pathogenresistenz" meint das Vermindern oder Abschwächen von Krankheitssymptomen einer Pflanze infolge eines Befalls durch mindestens ein Pathogen. Die Symptome können vielfältiger Art sein, umfassen aber bevorzugt solche die direkt oder indirekt zu einer Beeinträchtigung Qualität der Pflanze, der Quantität des Ertrages, der Eignung zur Verwendung als Futter- oder Nahrungsmittel führen oder aber auch Aussaat, Anbau, Ernte oder Prozessierung des Erntegutes erschweren.

"verleihen", "bestehen", "erzeugern" oder "erhöhen" einer Stress- oder Pathogenresistenz meint, dass die Abwehrmechanismen einer bestimmten Pflanzenart oder -sorte durch Anwendung des erfindungsgemäßen Verfahrens im Vergleich zu dem Wildtyp der Pflanze ("Ausgangspflanze"), auf den das erfindungsgemäße Verfahren nicht angewendet wurde, unter ansonsten gleichen Bedingungen (wie beispielsweise Klima- oder Anbaubedingungen, Stress- oder Pathogenart etc.) eine erhöhte Resistenz gegen ein und mehrere Stressfaktoren bzw. Pathogene aufweist. Dabei äußert sich die erhöhte Resistenz bevorzugt in einer verminderten Ausprägung der Stress- oder Krankheitssymptome, wobei Krankheitssymptome - neben den oben erwähnten Beeinträchtigungen - auch beispielsweise die Penetrationseffizienz eines Pathogens in die Pflanze oder pflanzliche Zellen oder die Proliferationseffizienz in oder auf denselben umfaßt. Dabei sind die Stress- oder Krankheitssymptome bevorzugt um mindestens 10 % oder mindestens 20 %, besonders bevorzugt um mindestens 40 % oder 60 %, ganz besonders bevorzugt um mindestens 70 % oder 80 %, am meisten bevorzugt um mindestens 90 % oder 95 % vermindert.

"Auswahl" meint in Bezug auf Pflanzen, bei denen - im Unterschied oder Vergleich zur Ausgangspflanze - die Resistenz gegen mindestens einen Stressfaktor oder Pathogen besteht oder erhöht ist, all die Verfahren, die eine zur Erkennung einer vorliegenden oder erhöhten Stress- bzw. Pathogenresistenz geeignet sind. Dies können beispiesweise Symptome der Pathogeninfektion sein (z.B. Nekrosen-Ausbildung bei Pilzinfektion) aber auch die oben beschriebenen Symptome umfassen, die die Qualität der Pflanze, die Quantität des Ertrages, die Eignung zur Verwendung als Futter- oder Nahrungsmittel usw. betreffen.

"Pathogene" meint im Rahmen der Erfindung beispielsweise jedoch nicht einschränkend Viren oder Viroide, Bakterien, Pilze, tierische Schädlinge, wie beispielsweise Insekten oder Nematoden. Besonders bevorzugt sind Pilze, insbesondere nekrotrophe oder hemibiotrophe Pilze. Es ist jedoch anzunehmen, dass die mesophyll-spezifische Expression eines BI1-Proteins auch eine Resistenz gegen weitere Pathogene bewirkt, da insgesamt eine Resistenz gegen Streßfaktoren erzeugt wird.

Beispielsweise jedoch nicht einschränkend seien nachfolgende Pathogene zu nennen:

### 1. Pilzpathogene oder pilz-ähnliche Pathogene:

Pilzpathogene oder pilz-ähnliche Pathogene (wie z.B. Chromista) stammen vorzugsweise aus der Gruppe umfassend Plasmodiophoramycota, Oomycota, Ascomycota, Chytridiomyceten, Zygomyceten, Basidiomycota und Deuteromyceten (Fungi imperfecti). Beispielhaft jedoch nicht einschränkend seien die in Tabelle 1 bis 4 genannten Pathogene und die mit ihnen in Zusammenhang gebrachten Erkrankungen zu nennen. Folgende englische und deutsche Termini können alternativ verwendet werden:

| | | |
|---|---|---|
| Ährenfäule | - | ear rot / head blight |
| Stengelfäule | - | stalk rot |
| Wurzelfäule | - | root rot |
| Rost | - | rust |
| Falscher Mehltau | | downy mildew |

Weiter Übersetzungen können beispielsweise bei http://www.bba.de/english/da.tabase/psmengl/pilz.htm gefunden werden.

**Tabelle 1: Erkrankungen hervorgerufen durch biotrophe, phytopathogene Pilze**

| **Erkrankung** | **Pathogen** |
|---|---|
| Braunrost | Puccinia recondita |
| Gelbrost | P. striiformis |
| Echter Mehltau | Erysiphe graminis / Blumeria graminis |
| Rost (gemeiner Mais) | Puccinia sorghi |
| Rost (südlicher Mais) | Puccinia polysora |
| Tabak Blattflecken | Cercospora nicotianae |
| Rost (tropischer Mais) | Physopella pallescens, P. zeae = Angiopsora zeae |

**Tabelle 2: Erkrankungen hervorgerufen durch nekrotrophe und/oder hemibiotrophe Pilze und Oomyceten**

| **Erkrankung** | **Pathogen** |
|---|---|
| Spelzenbräune | Septoria (Stagonospora) nodorum |
| Blattdürre | Septoria tritici |
| Ährenfusariosen | Fusarium spp. |
| Halmbruchkrankheit | Pseudocercosporella herpotrichoides |
| Flugbrand | Ustilago spp. |
| Kraut- und Knollenfäule | Phytohpthora infestans |
| Weizensteinbrand | Tilletia caries |
| Schwarzbeinigkeit | Gaeumannomyces graminis |
| Anthrocnose leaf blight Anthracnose stalk rot | Colletotrichum graminicola (teleomorph: Glomerella graminicola Politis); Glomerella tucumanensis (anamorph: Glomerella falcatum Went) |
| Aspergillus ear and kernel rot | Aspergillus flavus |
| Banded leaf and sheath spot ("Wurzeltöter") | Rhizoctonia solani Kuhn = Rhizoctonia microsclerotia J. Matz (telomorph: Thanatephorus cucumeris) |
| Black bundle disease | Acremonium strictum W. Gams = Cephalosporium acremonium Auct. non Corda |
| Black kernel rot | Lasiodiplodia theobromae = Botryodiplodia theobromae |
| Borde blanco | Marasmiellus sp. |
| Brown spot (black spot, stalk rot) | Physoderma maydis |
| Cephalosporium kernel rot | Acremonium strictum = Cephalosporium acremonium |
| Charcoal rot | Macrophomina phaseolina |
| Corticium ear rot | Thanatephorus cucumeris = Corticium sasakii |
| Curvularia leaf spot | Curvularia clavata, C. eragrostidis, = C. maculans (teleomorph: Cochliobolus eragrostidis), Curvularia inaequalis, C. intermedia (teleomorph: Cochliobolus intermedius), Curvularia lunata (teleomorph: Cochliobolus lunatus), Curvularia pallescens (teleomorph: Cochliobolus pallescens), Curvularia senegalensis, C. tuberculata (teleomorph: Cochliobolus tuberculatus) |
| Didymella leaf spot | Didymella exitalis |
| Diplodia Ähren- und Stengelfäule | Diplodia frumenti (teleomorph: Botryosphaeria festucae) |
| Diplodia Ähren- und Stengelfäule, seed rot and seedling blight | Diplodia maydis = Stenocarpella maydis |
| Diplodia leaf spot or streak | Stenocarpella macrospora = Diplodialeaf macrospora |
| Brown stripe downy mildew | Sclerophthora rayssiae var. zeae |
| Crazy top downy mildew | Sclerophthora macrospora = Sclerospora macrospora |
| Green ear downy mi ldew (graminicola downy mildew) | Sclerospora graminicola |
| Dry ear rot (cob, kernel and stalk rot) | Nigrospora oryzae (teleomorph: Khuskia oryzae) |
| Ährenfäulen (minor) | Alternaria alternata = A. tenuis, Aspergillus glaucus, A. niger, Aspergillus spp., Botrytis cinerea (teleomorph: Botryotinia fuckeliana), Cunninghamella sp., Curvularia pallescens, Doratomyces stemonitis = Cephalotrichum stemonitis, Fusarium culmorum, Gonatobotrys simplex, Pithomyces maydicus, Rhizopus microsporus Tiegh., R. stolonifer = R. nigricans, Scopulariopsis brumptii |
| Ergot(horse's tooth) | Claviceps gigantea (anamorph: Sphacelia sp.) |
| Eyespot | Aureobasidium zeae = Kabatiella zeae |
| Fusarium Ähren- und Stengelfäule F. | Fusarium subglutinans = moniliforme var.subglutinans |
| Fusarium kernel, root and stalk rot, seed rot and seedling blight | Fusarium moniliforme (teleomorph: Gibberella fujikuroi) |
| Fusarium Stengelfäule, seedling root rot | Fusarium avenaceum (teleomorph: Gibberella avenacea) |
| Gibberella Ähren- u. Stengelfäule | Gibberella zeae (anamorph: Fusarium graminearum) |
| Graue Ährenfäule | Botryosphaeria zeae = Physalospora zeae (anamorph: Macrophoma zeae) |
| Gray leaf spot (Cercospora leaf spot) | Cercospora sorghi = C. sorghi var. maydis, C. zeae-maydis |
| Helminthosporium root rot | Exserohilum pedicellatum = Helminthosporium pedicellatum (teleomorph: Setosphaeria pedicellata) |
| Hormodendrum Ährenfäule (Cladosporium Fäule) | Cladosporium cladosporioides = Hormodendrum cladosporioides, C. herbarum (teleomorph: Mycosphaerella tassiana) |
| Leaf spots, minor | Alternaria alternata, Ascochyta maydis, A. tritici, A. zeicola, Bipolaris victoriae = Helminthosporium victoriae (teleomorph: Cochliobolus victoriae), C. sativus (anamorph: Bipolaris sorokiniana = H. sorokinianum = H. sativum), Epicoccum nigrum, Exserohilum prolatum = Drechslera prolata (teleomorph: Setosphaeria prolata) Graphium penicillioides, Leptosphaeria maydis, Leptothyrium zeae, Ophiosphaerella herpotricha, (anamorph: Scolecosporiella sp.), Paraphaeosphaeria michotii, Phoma sp., Septoria zeae, S. zeicola, S. zeina |
| Northern corn leaf blight (white blast, crown stalk rot, stripe) | Setosphaeria turcica (anarnorph: Exserohilum turcicum = Helminthosporium turcicum) |
| Northern corn leaf spot Helminthosporium ear rot (race 1) | Cochliobolus carbonum (anamorph: Bipolaris zeicola = Helminthosporium carbonum) |
| Penicillium Ährenfäule (blue eye, blue mold) | Penicillium spp., P. chrysogenum, P. expansum, P. oxalicum |
| Phaeocytostroma Stengel- und Wurzelfäule | Phaeocytostroma ambiguum, = Phaeocytosporella zeae |
| Phaeosphaeria leaf spot | Phaeosphaeria maydis = Sphaerulina maydis |
| Physalospora Ährenfäule (Botryosphaeria Ährenfäule) | Botryosphaeria festucae = Physalospora zeicola (anamorph: Diplodia frumenti) |
| Purple leaf sheath | Hemiparasitic bacteria and fungi |
| Pyrenochaeta Stengel- und Wurzelfäule | Phoma terrestris = Pyrenochaeta terrestris |
| Pythium Wurzelfäule | Pythium spp., P. arrhenomanes, P. graminicola |
| Pythium Stengelfäule | Pythium aphanidermatum = P. butleri L. |
| Red kernel disease (ear mold, leaf and seed rot) | Epicoccum nigrum |
| Rhizoctonia Ährenfäule (sclerotial rot) | Rhizoctonia zeae (teleomorph: Waitea circinata) |
| Rhizoctonia Wurzel- und Stengelfäule | Rhizoctonia solani, Rhizoctonia zeae |
| Wurzelfäulen (minor) | Alternaria alternata, Cercospora sorghi, Dictochaeta fertilis, Fusarium acuminatum (teleomorph: Gibberella acuminata), F. equiseti (teleomorph: G. intricans), F. oxysporum, F. pallidoroseum, F. poae, F. roseum, G. cyanogena, (anamorph: F. sulphureum), Microdochium bolleyi, Mucor sp., Periconia circinata, Phytophthora cactorum, P. drechsleri, P. nicotianae var. parasitica, Rhizopus arrhizus |
| Rostratum leaf spot (Helminthosporium leaf disease, ear and stalk rot) | Setosphaeria rostrata, (anamorph: Exserohilum rostratum = He/minthosporium rostratum) |
| Falscher Java Mehltau | Peronosclerospora maydis = Sclerospora maydis |
| Falscher Philippinen Mehltau | Peronosclerospora philippinensis = Sclerospora philippinensis |
| Falscher Sorghum Mehltau | Peronosclerospora sorghi = Sclerospora sorghi |
| Spontaneum downy mildew | Peronosclerospora spontanea = Sclerospora spontanea |
| Falscher Zuckerrohr-Mehltau | Peronosclerospora sacchari = Sclerospora sacchari |
| Sclerotium Ährenfäule (southern blight) | Sclerotium rolfsii Sacc. (teleomorph: Athelia rolfsii) |
| Seed rot-seedling blight | Bipolaris sorokiniana, B. zeicola = Helminthosporium carbonum, Diplodia maydis, Exserohilum pedicillatum, Exserohilum turcicum = Helminthosporium turcicum, Fusarium avenaceum, F. culmorum, F. moniliforme, Gibberella zeae (anamorph: F. graminearum), Macrophomina phaseolina, Penicillium spp., Phomopsis sp., Pythium spp., Rhizoctonia solani, R. zeae, Sclerotium rolfsii, Spicaria sp. |
| Selenophoma leaf spot | Selenophoma sp. |
| Sheath rot | Gaeumannomyces graminis |
| Shuck rot | Myrothecium gramineum |
| Silage mold | Monascus purpureus, M ruber |
| Flugbrand (Smut, common) | Ustilago zeae = U. maydis |
| Smut, false | Ustilaginoidea virens |
| Kolbenbrand (Smut, head) | Sphacelotheca reiliana = Sporisorium holcisorghi |
| Southern corn leaf blight and stalk rot | Cochliobolus heterostrophus (anamorph: Bipolaris maydis = Helminthosporium maydis) |
| Southern leaf spot | Stenocarpella macrospora = Diplodia macrospora |
| Stengelfäulen (minor) | Cercospora sorghi, Fusarium episphaeria, F. merismoides, F. oxysporum Schlechtend, F. poae, F. roseum, F. solani (teleomorph: Nectria haematococca), F. tricinctum, Mariannaea elegans, Mucor sp., Rhopographus zeae, Spicaria sp. |
| Lagerfäulen (Storage rots) | Aspergillus spp., Penicillium spp. und weitere Pilze |
| Tar spot | Phyllachora maydis |
| Trichoderma ear rot and root rot | Trichoderma viride = T. lignorum teleomorph: Hypocrea sp. |
| White ear rot, root and stalk rot | Stenocarpella maydis = Diplodia zeae |
| Yellow leaf blight | Ascochyta ischaemi, Phyllosticta maydis (teleomorph: Mycosphaerella zeae-maydis) |
| Zonate leaf spot | Gloeocercospora sorghi |

**Tabelle 4: Erkrankungen hervorgerufen durch Pilze und Oomyceten mit unklarer Einstufung hinsichtlich biotrophen, hemibiotrophen bzw. nekrotrophen Verhaltens**

| **Erkrankung** | **Pathogen** |
|---|---|
| Hyalothyridium leaf spot | Hyalothyridium maydis |
| Late wilt | Cephalosporium maydis |

Besonders bevorzugt sind
- Plasmodiophoromycota wie Plasmodiophora brassicae (Kohlhernie), Spongospora subterranea, Polymyxa graminis,
- Oomycota wie Bremia lactucae (Falscher Mehltau an Salat), Peronospora (Falscher Mehltau) bei Löwenmaul (P. antirrhini), Zwiebel (P. destructor), Spinat (P. effusa), Sojabohne (P. manchurica), Tabak (Blauschimmel; P. tabacina) Alfalfa und Klee (P. trifolium), Pseudoperonospora humuli (Falscher Mehltau an Hopfen), Plasmopara (Falscher Mehltau bei Trauben) (P. viticola) und Sonnenblume (P. halstedii), Sclerophtohra macrospora (Falscher Mehltau bei Cerealien und Gäsern), Pythium (z.B.
   Wurzelbrand an Beta-Rübe durch P. debaryanum), Phytophthora infestans (Kraut- und Knollenfäule bei.Kartoffel, Braunfäule bei Tomate etc.), Albugo spec.
- Ascomycota wie Microdochium nivale (Schneeschimmel an Roggen und Weizen), Fusarium graminearum, Fusarium culmorum (Ährenfäule v.a. bei Weizen), Fusarium oxysporum (Fusarium-Welke an Tomate), Blumeria graminis (Echter Mehltau an Gerste (f.sp. hordei) und Weizen (f.sp. tritici)), Erysiphe pisi (Erbsenmehltau), Nectria galligena (Obstbaumkrebs), Unicnula necator (Echter Mehltau der Weinrebe), Pseudopeziza tracheiphila (Roter Brenner der Weinrebe), Claviceps purpurea (Mutterkorn an z.B. Roggen und Gräsern), Gaeumannomyces graminis (Schwarzbeinigkeit an Weizen, Roggen u.a. Gräsern), Magnaporthe grisea, Pyrenophora graminea (Streifenkrankheit an Gerste), Pyrenophora teres (Netzfleckenkrankheit an Gerste), Pyrenophora tritici-repentis (Blattfleckenkrankheit (Blattdürre) an Weizen), Venturia inaequalis (Apfelschorf), Sclerotinia sclerotium (Weißstengeligkeit, Rapskrebs), Pseudopeziza medicaginis (Klappenschorf an Luzerne, Weiß- und Rotklee).
- Basidiomyceten wie Typhula incarnata (Typhula-Fäule an Gerste, Roggen, Weizen), Ustilago maydis (Beulenbrand an Mais), Ustilago nuda (Flugbrand an Gerste), Ustilago tritici (Flugbrand an Weizen, Dinkel), Ustilago avenae (Flugbrand an Hafer), Rhizoctonia solani (Wurzeltöter an Kartoffeln), Sphacelotheca spp. (Kolbenbrand bei Sorghum), Melampsora lini (Rost bei Flachs), Puccinia graminis (Schwarzrost an Weizen, Gerste, Roggen, Hafer), Puccinia recondita (Braunrost an Weizen), Puccinia dispersa (Braunrost an Roggen), Puccinia hordei (Braunrost an Gerste), Puccinia coronata (Kronenrost an Hafer), Puccinia striiformis (Gelbrost an Weizen, Gerste, Roggen sowie zahlreichen Gräsern), Uromyces appendiculatus (Bohnenrost), Sclerotium rolfsii (Wurzel- und Stengelfäule bei zahlreichen Pflanzen).
- Deuteromyceten (Fungi imperfecti) wie Septoria (Stagonospora) nodorum (Spelzenbräune) an Weizen (Septoria tritici), Pseudocercosporella herpotrichoides (Halmbruchkrankheit an Weizen, Gerste, Roggen), Rynchosporium secalis (Blattfleckenkrankheit an Roggen und Gerste), Alternaria solani (Dürrfleckenkrankheit an Kartoffel, Tomate), Phoma betae (Wurzelbrand an Beta-Rübe), Cercospora beticola (Cercospora-Blattfleckenkrankheit an Beta-Rübe), (Alternaria brassicae (Rapsschwärze an Raps, Kohl u.a. Kreuzblütlern), Verticillium dahliae (Rapswelke und -stengelfäule), Colletotrichum lindemuthianum (Brennfleckenkrankheit an Bohne), Phoma lingam - Umfallkrankheit (Schwarzbeinigkeit an Kohl; Wurzelhals- oder Stengelfäule an Raps), Botrytis cinerea (Grauschimmel an Weinrebe, Erdbeere, Tomate, Hopfen etc.).

Am meisten bevorzugt sind Phytophthora infestans (Kraut- und Knollenfäule, Braunfäule bei Tomate etc.), Microdochium nivale (vormals Fusarium nivale; Schneeschimmel an Roggen und Weizen), Fusarium graminearum, Fusarium culmorum, Fusarium avenaceum und Fusarium poae (Ährenfäule an Weizen), Fusarium oxysporum (Fusarium-Welke an Tomate), Magnaporthe grisea (rice blast disease), Sclerotinia sclerotium (Weißstengeligkeit, Rapskrebs), Septoria (Stagonospora)nodorum und Septoria tritici (Spelzenbräune an Weizen), Alternaria brassicae (Rapsschwärze an Raps, Kohl u.a. Kreuzblütlern), Phoma lingam (Umfallkrankheit, Schwarzbeinigkeit an Wohl; Wurzelhals- oder Stengelfäule an Raps).

### 2. Bakterielle Pathogene:

Beispielhaft jedoch nicht einschränkend seien die in Tabelle 5 genannten Pathogene und die mit ihnen in Zusammenhang gebrachten Erkrankungen zu nennen.

**Tabelle 5: Bakterielle Erkrankungen**

| **Erkrankung** | **Pathogen** |
|---|---|
| Bacterial leaf blight and stalk rot | Pseudomonas avenae subsp. avenae |
| Bacterial leaf spot | Xanthomonas campestris pv. holcicola |
| Bakterielle Stengelfäule | Enterobacter dissolvens = Erwinia dissolvens |
| Schwarzbeinigkeit ("Bacterial stalk and top rot") | Erwinia carotovora subsp. carotovora, Erwinia chrysanthemi pv. zeae |
| Bacterial stripe | Pseudomonas andropogonis |
| Chocolate spot | Pseudomonas syringae pv. coronafaciens |
| Goss's bacterial wilt and blight (leaf freckles and wilt) | Clavibacter michiganensis subsp. nebraskensis = Corynebacterium michiganense pv.andnebraskense |
| Holcus spot | Pseudomonas syringae pv. syringae |
| Purple leaf sheath | Hemiparasitic bacteria |
| Seed rot-seedling blight | Bacillus subtilis |
| Stewart's disease (bacterial wilt) | Pantoea stewartii = Erwinia stewartii |
| Corn stunt (achapparramiento,maize stunt, Mesa Central or Rio Grande maize stunt) | Spiroplasma kunkelii |

Ganz besonders bevorzugt sind nachfolgende pathogene Bakterien: Corynebacterium sepedonicum (Bakterienringfäule an Kartoffel), Erwinia carotovora (Schwarzbeinigkeit an Kartoffel), Erwinia amylovora (Feuerbrand an Birne, Apfel, Quitte), Streptomyces scabies (Kartoffelschorf), Pseudomonas syringae pv. tabaci (Wildfeuer an Tabak), Pseudomonas syringae pv. phaseolicola (Fettfleckenkrankheit an Buschbohne), Pseudomonas syringae pv. tomato ("bacterial speck" an Tomate), Xanthomonas campestris pv. malvacearum (Blattfleckenkrankheit an Baumwolle) und Xanthomonas campestris pv. oryzae (Bakterienfäule an Reis und anderen Gräsern).

### 3. Virale Pathogene:

"Virale Pathogene" schließt sämtliche Pflanzenviren ein wie beispielsweise Tabak- oder oder Cucumber-Mosaiv Virus, Ringspot-Virus, Nekrose-Virus, Mais Dwarf-Mosaic Virus etc.

Beispielhaft jedoch nicht einschränkend sind die in Tabelle 6 genannten Pathogene und die mit ihnen in Zusammenhang gebrachten Erkrankungen zu nennen.

**Tabelle 6: Virale Erkrankungen**

| **Krankheit** | **Pathogen** |
|---|---|
| American wheat striate (wheat striate mosaic) | American wheat striate mosaic virus (AWSMV) |
| Barley stripe mosaic | Barley stripe mosaic virus (BSMV) |
| Barley yellow dwarf | Barley yellow dwarf virus (BYDV) |
| Brome mosaic | Brome mosaic virus (BMV) |
| Cereal chlorotic mottle | Cereal chlorotic mottle virus (CCMV) |
| Corn chlorotic vein banding (Braizilian maize mosaic) | Corn chlorotic vein banding virus (CCVBV) |
| Corn lethal necrosis | Viruskomplex aus Maize chlorotic mottle virus (MCMV) und Maize dwarf mosaic virus (MDMV) A oder B oder Wheat streak mosaic virus(WSMV) |
| Cucumber mosaic | Cucumber mosaic virus (CMV) |
| Cynodon chlorotic streak | Cynodon chlorotic streak virus (CCSV) |
| Johnsongrass mosaic | Johnsongrass mosaic virus (JGMV) |
| Maize bushy stunt | Mycoplasma-like organism (MLO) associated |
| Maize chlorotic dwarf | Maize chlorotic dwarf virus (MCDV) |
| Maize chlorotic mottle | Maize chlorotic mottle virus (MCMV) |
| Maize dwarf mosaic | Maize dwarf mosaic virus (MDMV) strains A, D, E and F |
| Maize leaf fleck | Maize leaf fleck virus (MLFV) |
| Maize line | Maize line virus (MLV) |
| Maize mosaic (corn leaf stripe, enanismo rayado) | Maize mosaic virus (MMV) |
| Maize mottle and chlorotic stunt | Maize mottle and chlorotic stunt virus |
| Maize pellucid ringspot | Maize pellucid ringspot virus (MPRV) |
| Maize raya gruesa | Maize raya gruesa virus (MRGV) |
| maize rayado fino (fine striping disease) | Maize rayado fino virus (MRFV) |
| Maize red leaf and red stripe | Mollicute |
| Maize red stripe | Maize red stripe virus (MRSV) |
| Maize ring mottle | Maize ring mottle virus (MRMV) |
| Maize rio IV | Maize rio cuarto virus (MRCV) |
| Maize rough dwarf (nanismo ruvido) | Maize rough dwarf virus (MRDV) (Cereal tillering disease virus) |
| Maize sterile stunt | Maize sterile stunt virus (strains of barley yellow striate virus) |
| Maize streak | Maize streak virus (MSV) |
| Maize stripe (maize chlorotic stripe, maize hoja blanca) | Maize stripe virus |
| Maize stunting | Maize stunting virus |
| Maize tassel abortion | Maize tassel abortion virus (MTAV) |
| Maize vein enation | Maize vein enation virus (MVEV) |
| Maize wallaby ear | Maize wallaby ear virus (MWEV) |
| Maize white leaf | Maize white leaf virus |
| Maize white line mosaic | Maize white line mosaic virus (MWLMV) |
| Millet red leaf | Millet red leaf virus (MRLV) |
| Northern cereal mosaic | Northern cereal mosaic virus (NCMV) |
| Oat pseudorosette (zakuklivanie) | Oat pseudorosette virus |
| Oat sterile dwarf | Oat sterile dwarf virus (OSDV) |
| Rice black-streaked dwarf | Rice black-streaked dwarf virus (RBSDV) |
| Rice stripe | Rice stripe virus (RSV) |
| Sorghum mosaic | Sorghum mosaic virus (SrMV) (auch: sugarcane mosaic virus (SCMV) Stämme H, I and M) |
| Sugarcane Fiji disease | Sugarcane Fiji disease virus (FDV) |
| Sugarcane mosaic | Sugarcane mosaic virus (SCMV) strains A, B, D, E, SC, BC, Sabi and MB (formerly MDMV-B) |
| Wheat spot mosaic | Wheat spot mosaic virus (WSMV) |

### 4. Tierische Schädlinge

### 4.1 Insekten Pathogene:

Beispielhaft jedoch nicht einschränkend seien Insekten wie beispielsweise Käfer, Raupen, Läuse oder Milben zu nennen. Bevorzugt sind Insekten der Gattungen Coleoptera, Diptera, Hymenoptera, Lepidoptera, Mallophaga, Homoptera, Hemiptera, Orthoptera, Thysanoptera. Dermaptera, Isoptera, Anoplura, Siphonaptera, Trichoptera, etc.. Besonders bevorzugt sind Coleoptera and Lepidoptera Insekten, wie beispielsweise den Maiszünsler (European Corn Borer), Diabrotica barberi, Diabrotica undecimpunctata, Diabrotica virgifera, Agrotis ipsilon , Crymodes devastator, Feltia ducens, Agrotis gladiaria, Melanotus spp., Aeolus mellillus, Aeolus mancus, Horistonotus uhlerii, Sphenophorus maidis, Sphenophorus zeae, Sphenophorus parvulus, Sphenophorus callosus, Phyllogphaga spp., Anuraphis maidiradicis, Delia platura, Colaspis brunnea, Stenolophus lecontei und Clivinia impressifrons.

Ferner sind zu nennen: Das Getreidehähnchen (Oulema melanopus), die Fritfliege (Oscinella frit), Drahtwürmer (Agrotis lineatus) und Blattläuse (wie z.B. Haferblattlaus Rhopalosiphum padi, Grosse Getreideblattlaus Sitobion avenae).

### 4.2 Nematoden:

Beispielhaft jedoch nicht einschränkend seien die in Tabelle 7 genannten Pathogene und die mit ihnen in Zusammenhang gebrachten Erkrankungen zu nennen.

**Tabelle 7: Parasitäre Nematoden**

| **Schädigung** | **Pathogene Nematode** |
|---|---|
| Awl | Dolichodorus spp., D. heterocephalus |
| Stengel- oder Stockälchen, Rübenkopfälchen | Ditylenchus dipsaci |
| Burrowing | Radopholus similis |
| Haferzystenälchen | Heterodera avenae, H. zeae, Punctodera chalcoensis |
| Dagger | Xiphinema spp., X. americanum, X. mediterraneum |
| False root-knot | Nacobbus dorsalis |
| Lance, Columbia | Hoplolaimus columbus |
| Lance | Hoplolaimus spp., H. galeatus |
| Lesion | Pratylenchus spp., P. brachyurus, P. crenatus, P. hexincisus, P. neglectus, P. penetrans, P. scribneri, P. thornei, P. zeae |
| Needle | Longidorus spp., L. breviannulatus |
| Ring | Criconemella spp., C. ornata |
| Wurzelgallenälchen | Meloidogyne spp., M. chitwoodi, M. incognita, M. javanica |
| Spiral | Helicotylenchus spp. |
| Sting | Belonolaimus spp., B. longicaudatus |
| Stubby-root | Paratrichodorus spp., P. christiei, P. minor, Quinisulcius acutus, Trichodorus spp. |
| Stunt | Tylenchorhynchus dubius |

Ganz besonders bevorzugt sind Globodera rostochiensis und G. pallida (Zystenälchen an Kartoffel, Tomate u.a. Nachtschattengewächsen), Heterodera schachtii (Rübenzystenälchen an Zucker- und Futterrübe, Raps, Kohl etc.), Heterodera avenae (Haferzystenälchen an Hafer u.a. Getreidearten), Ditylenchus dipsaci (Stengel- oder Stockälchen, Rübenkopfälchen an Roggen, Hafer, Mais, Klee, Tabak, Rübe), Anguina tritici (Weizenälchen, Radekrankheit an Weizen (Dinkel, Roggen), Meloidogyne hapla (Wurzelgallenälchen an Möhre, Gurke, Salat, Tomate, Kartoffel, Zuckerrübe, Luzerne).

Als für die einzelnen Sorten bevorzugte Pilz- oder Virus-Pathogene sind beispielsweise zu nennen:

### 1. Gerste:

Pilz-, bakterielle und virale Pathogene: Puccinia graminis f.sp. hordei, Blumeria (Erysiphe) graminis f.sp. hordei, barley yellow dwarf virus (BYDV),

Pathogene Insekten / Nematoden: Ostrinia nubilalis (European corn borer); Agrotis ipsilon; Schizaphis graminum; Blissus leucopterus leucopterus; Acrosternum hilare; Euschistus servus; Deliaplatura; Mayetiola destructor; Petrobia latens.

### 2. Sojabohne:

Pilz-, bakterielle oder virale Pathogene: Phytophthora megasperma fsp.glycinea, Macrophomina phaseolina, Rhizoctonia solani, Sclerotinia sclerotiorum, Fusarium oxysporum, Diaporthe phaseolorum var. sojae (Phomopsis sojae), Diaporthe phaseolorum var. caulivora, Sclerotium rolfsii, Cercospora kikuchii, Cercospora sojina, Peronospora manshurica, Colletotrichum dematium (Colletotrichum truncatum), Corynespora cassiicola, Septoria glycines, Phyllosticta sojicola, Alternaria alternata, Pseudomonas syringae p.v. glycinea, Xanthomonas campestris p.v. phaseoli, Microsphaera diffussa, Fusarium semitectum, Phialophora gregata, Sojabohnen Mosaikvirus, Glomerella glycines, Tobacco Ring spot virus, Tobacco Streak virus, Phakopsorapachyrhizi, Pythium aphanidermatum, Pythium ultimum, Pythium debaryanum, Tomato spotted wilt virus, Heterodera glycines Fusarium solani.

Pathogene Insekten / Nematoden: Pseudoplusia includens; Anticarsia gemmatalis; Plathypena scabra; Ostrinia nubilalis; Agrotis ipsilon; Spodoptera exigua; Heliothis virescens; Helicoverpa zea; Epilachna varivestis; Myzus persicae; Empoasca fabae; Acrosternum hilare; Melanoplus femurrubrum; Melanoplus differentialis; Hylemya platura; Sericothrips variabilis; Thrips tabaci; Tetranychus turkestani; Tetranychus urticae;

### 3. Raps:

Pilz-, bakterielle oder virale Pathogene: Albugo candida, Alternaria brassicae, Leptosphaeria maculans, Rhizoctonia solani, Sclerotinia sclerotiorum, Mycosphaerella brassiccola, Pythium ultimum, Peronospora parasitica, Fusarium roseum, Alternaria alternata.

### 4. Alfalfa:

Pilz,-, bakterielle oder virale Pathogene: Clavibater michiganese subsp. insidiosum, Pythium ultimum, Pythium irregulare, Pythium splendens, Pythium debaryanum, Pythium aphanidermatum, Phytophthora megasperma, Peronospora trifoliorum, Phoma medicaginis var. medicaginis, Cercospora medicaginis, Pseudopeziza medicaginis, Leptotrochila medicaginis, Fusarium, Xanthomonas campestris p.v. alfalfae, Aphanomyces euteiches, Stemphylium herbarum, Stemphylium alfalfae.

### 5. Weizen:

Pilz-,bakterielle oder virale Pathogene: Pseudomonas syringae p.v. atrofaciens, Urocystis agropyri, Xanthomonas campestris p.v. translucens, Pseudomonas syringae p.v. syringae, Alternaria alternata, Cladosporium herbarum, Fusarium graminearum, Fusarium avenaceum, Fusarium culmorum, Ustilago tritici, Ascochyta tritici, Cephalosporium gramineum, Collotetrichum graminicola, Erysiphe graminis f.sp. tritici, Puccinia graminis f.sp. tritici, Puccinia recondita f.sp. tritici, Puccinia striiformis, Pyrenophora tritici-repentis, Septoria (Stagonospora) nodorum, Septoria tritici, Septoria avenae, Pseudocercosporella herpotrichoides, Rhizoctonia solani, Rhizoctonia cerealis, Gaeumannomyces graminis var. tritici, Pythium aphanidermatum, Pythium arrhenomanes, Pythium ultimum, Bipolaris sorokiniana, Barley Yellow Dwarf Virus, Brome Mosaic Virus, Soil Borne Wheat Mosaic Virus, Wheat Streak Mosaic Virus, Wheat Spindle Streak Virus, American Wheat Striate Virus, Claviceps purpurea, Tilletia tritici, Tilletia laevis, Ustilago tritici, Tilletia indica, Rhizoctonia solani, Pythium arrhenomannes, Pythium gramicola, Pythium aphanidermatum, High Plains Virus, European Wheat Striate Virus, Puccinia graminis f.sp. tritici (Wheat stem rust), Blumeria (Erysiphe) graminis f.sp. tritici (Wheat Powdery Mildew).

Pathogene Insekten / Nematoden: Pseudaletia unipunctata; Spodoptera,frugiperda; Elasmopalpus lignosellus; Agrotis orthogonia; Elasmopalpus Zignosellus; Oulema melanopus; Hypera punctata; Diabrotica undecimpunctata howardi; Russian wheat aphid; Schizaphis graminum; Macrosiphum avenae; Melanoplus femurrubrum; Melanoplus differentialis; Melanoplus sanguinipes; Mayetiola destructor; Sitodiplosis mosellana; Meromyza americana; Hylemya coarctata; Frankliniella fusca; Cephus cinctus; Aceria tulipae;

### 6. Sonnenblume:

Pilz-, bakterielle oder virale Pathogene: Plasmophora halstedii, Sclerotinia sclerotiorum, Aster Yellows, Septoria helianthi, Phomopsis helianthi, Alternaria helianthi, Alternaria zinniae, Botrytis cinerea, Phoma macdonaldii, Macrophomina phaseolina, Erysiphe cichoracearum, Rhizopus oryzae, Rhizopus arrhizus, Rhizopus stolonifer, Puccinia helianthi, Verticillium dahliae, Erwinia carotovorum p.v. Carotovora, Cephalosporium acremonium, Phytophthora cryptogea, Albugo tragopogonis.

Pathogene Insekten / Nematoden: Suleima helianthana; Homoeosoma electellum; zygogramma exclamationis; Bothyrus gibbosus; Neolasioptera murtfeldtiana;

### 7. Mais:

Pilz-, bakterielle oder virale Pathogene: Fusarium moniliforme var. subglutinans, Erwinia stewartii, Fusarium moniliforme, Gibberella zeae (Fusarium graminearum), Stenocarpella maydi (Diplodia maydis), Pythium irregulare, Pythium debaryanum, Pythium graminicola, Pythium splendens, Pythium ultimum, Pythium aphanidermatum, Aspergillus flavus, Bipolaris maydis 0, T (Cochliobolus heterostrophus), Helminthosporium carbonum I, II & III (Cochliobolus carbonum), Exserohilum turcicum I, II & III, Helminthosporium pedicellatum, Physoderma maydis, Phyllosticta maydis, Kabatiella maydis, Cercospora sorghi, Ustilago maydis, Puccinia sorghi, Puccinia polysora, Macrophomina phaseolina, Penicillium oxalicum, Nigrospora oryzae, Cladosporium herbarum, Curvularia lunata, Curvularia inaequalis, Curvularia pallescens, Clavibacter michiganese subsp. nebraskense, Trichoderma viride, Maize Dwarf Mosaic Virus A & B, Wheat Streak Mosaic Virus, Maize Chlorotic Dwarf Virus, Claviceps sorghi, Pseudonomas avenae, Erwinia chrysanthemi p.v. Zea, Erwinia corotovora, Cornstunt spiroplasma, Diplodia macrospora, Sclerophthora macrospora, Peronosclerospora sorghi, Peronosclerospora philippinesis, Peronosclerospora maydis, Peronosclerospora sacchari, Spacelotheca reiliana, Physopella zeae, Cephalosporium maydis, Caphalosporium acremonium, Maize Chlorotic Mottle Virus, High Plains Virus, Maize Mosaic Virus, Maize Rayado Fino Virus, Maize Streak Virus (MSV, Maisstrichel-Virus), Maize Stripe Virus, Maize Rough Dwarf Virus.

Pathogene Insekten / Nematoden: Ostrinia nubilalis; Agrotis ipsilon; Helicoverpa zea; Spodoptera frugiperda; Diatraea grandiosella; Elasmopalpus lignosellus; Diatraea saccharalis; Diabrotica virgifera; Diabrotica longicornis barberi; Diabrotica undecimpunctata howardi; Melanotus spp.; Cyclocephala borealis; Cyclocephala immaculata; Popillia japonica; Chaetocnema pulicaria; Sphenophorus maidis; Rhopalosiphum maidis; Anuraphis maidiradicis; Blissus leucopterus leucopterus; Melanoplus femurrubrum; Melanoplus sanguinipes; Hylemva platura; Agromyza parvicornis; Anaphothrips obscrurus; Solenopsis milesta; Tetranychus urticae.

### 8. Sorghum:

Pilz-, bakterielle oder virale Pathogene: Exserohilum turcicum, Colletotrichum graminicola (Glomerella graminicola), Cercospora sorghi, Gloeocercospora sorghi, Ascochyta sorghina, Pseudomonas syringae p.v. syringae, Xanthomonas campestris p.v. holcicola, Pseudomonas andropogonis, Puccinia purpurea, Macrophomina phaseolina, Perconia circinata, Fusarium monilifonne, Alternaria alternate, Bipolaris sorghicola, Helminthosporium sorghicola, Curvularia lunata, Phoma insidiosa, Pseudomonas avenae (Pseudomonas alboprecipitans), Ramulispora sorghi, Ramulispora sorghicola, Phyllachara sacchari, Sporisorium reilianum (Sphacelotheca reiliana), Sphacelotheca cruenta, Sporisorium sorghi, Sugarcane mosaic H, Maize Dwarf Mosaic Virus A & B, Claviceps sorghi, Rhizoctonia solani, Acremonium strictum, Sclerophthona macrospora, Peronosclerospora sorghi, Peronosclerospora philippinensis, Sclerospora graminicola, Fusarium graminearum, Fusarium oxysporum, Pythium arrhenomanes, Pythium graminicola.

Pathogene Insekten / Nematoden: Chilo partellus; Spodoptera frugiperda; Helicoverpa zea; Elasmopalpus lignosellus; Feltia subterranea; Phvllophaga crinita ; Eleodes, Conoderus und Aeolus spp.; Oulema melanopus; Chaetocnema pulicaria; Sphenophorus maidis; Rhopalosiphum maidis; Siphaflava; Blissus leucopterus leucopterus; Contarinia sorghicola; Tetranychus cinnabarinus; Tetranychus urticae.

### 9. Baumwolle:

Pathogene Insekten / Nematoden: Heliothis virescens; Helicoverpa zea; Spodoptera exigua; Pectinophora gossypiella; Anthonomus grandis grandis; Aphis gossypii; Pseudatomoscelis seriatus; Trialeurodes abutilonea; Lygus lineolaris; Melanoplus femurrubrum; Melanoplus differentialis; Thrips tabaci (onion thrips); Franklinkiella fusca; Tetranychus cinnabarinus; Tetranychus urticae.

### 10. Reis:

Pathogene Insekten / Nematoden: Diatraea saccharalis; Spodoptera frugiperda; Helicoverpa zea; Colaspis brunnea; Lissorhoptrus oryzophilus; Sitophilus oryzae; Nephotettix nigropictus; Blissus Ieucopterus leucopterus; Acrosternum hilare.

### 11. Raps:

Pathogene Insekten / Nematoden: Brevicoryne brassicae; Phyilotreta cruciferae; Mamestra conjgurata; Plutella xylostella; Delia ssp..

"BI1-Protein" meint im Rahmen der Erfindung Polypeptide die mindestens eine Sequenz die eine Homologie von mindestens 50%, bevorzugt mindestens 80%, besonders bevorzugt mindestens 90%, ganz besonders bevorzugt 100% aufweisen zu einem BI1-Konsensusmotiv ausgewählt aus der Gruppe bestehend aus
a) H(L/I)KXVY
b) AXGA(Y/F)XH
c) NIGG
d) P(V/P)(Y/F)E(E/Q)(R/Q)KR
e) (E/Q)G(A/S)S(V/I)GPL
f) DP(S/G)(L/I)(I/L)
g) V(G/A)T(A/S)(L/I)AF(A/G)CF(S/T)
h) YL(Y/F)LGG, bevorzugt EYLYLGG
i) L(L/V)SS(G/W)L(S/T)(I/M)L(L/M)W
j) DTGX(I/V) (I/V)E

Besonders bevorzugt ist dabei das BI- Konsensusmotiv f) YL(Y/F)LGG, ganz besonders bevorzugt (EYLYLGG). Dieses Motiv ist charakteristisch für pflanzliche BI1-Proteine.

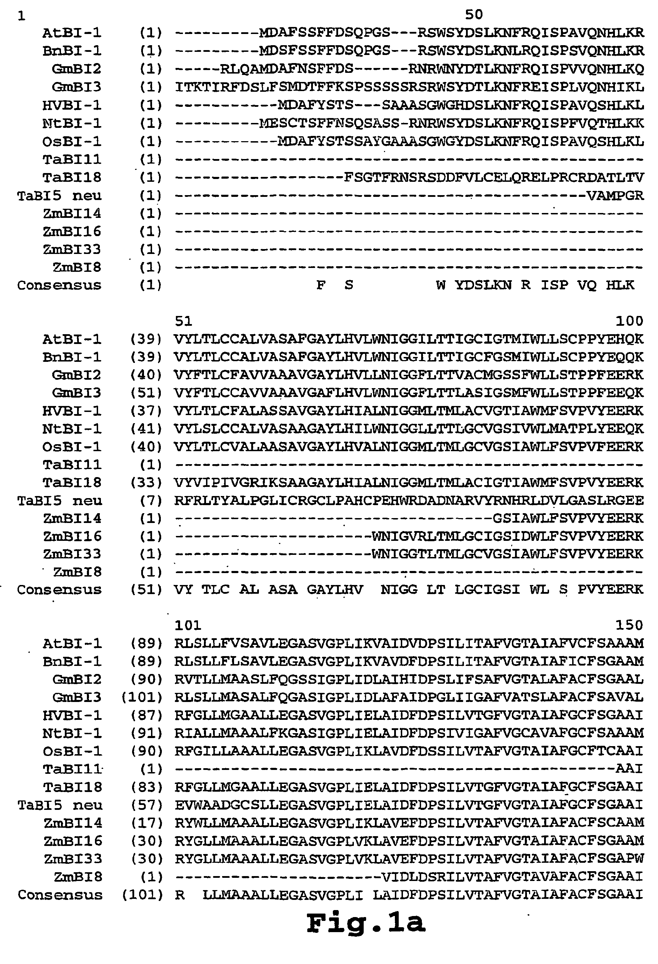

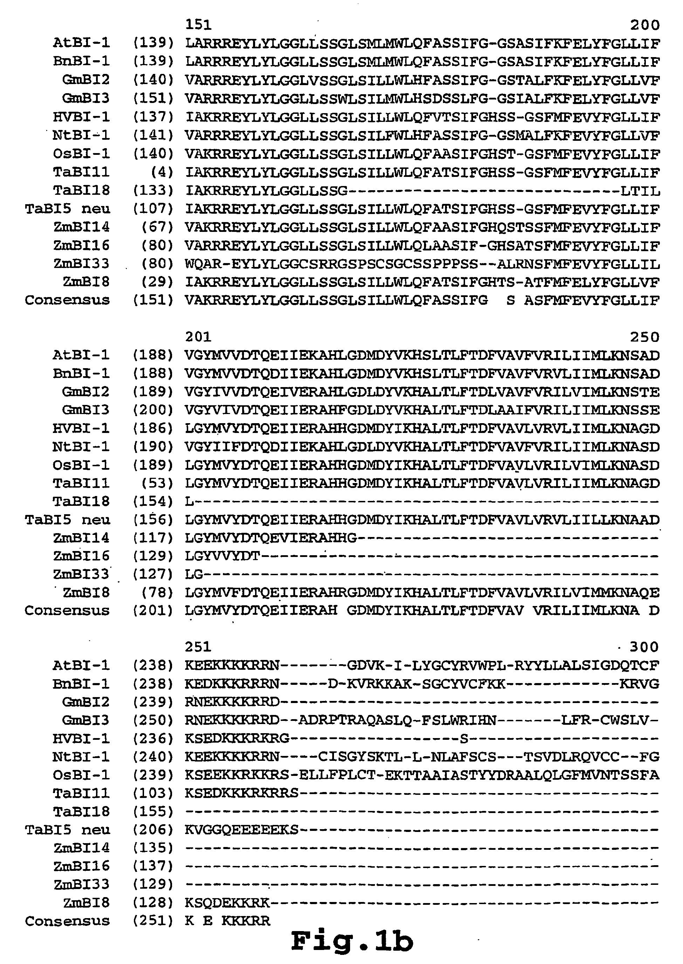

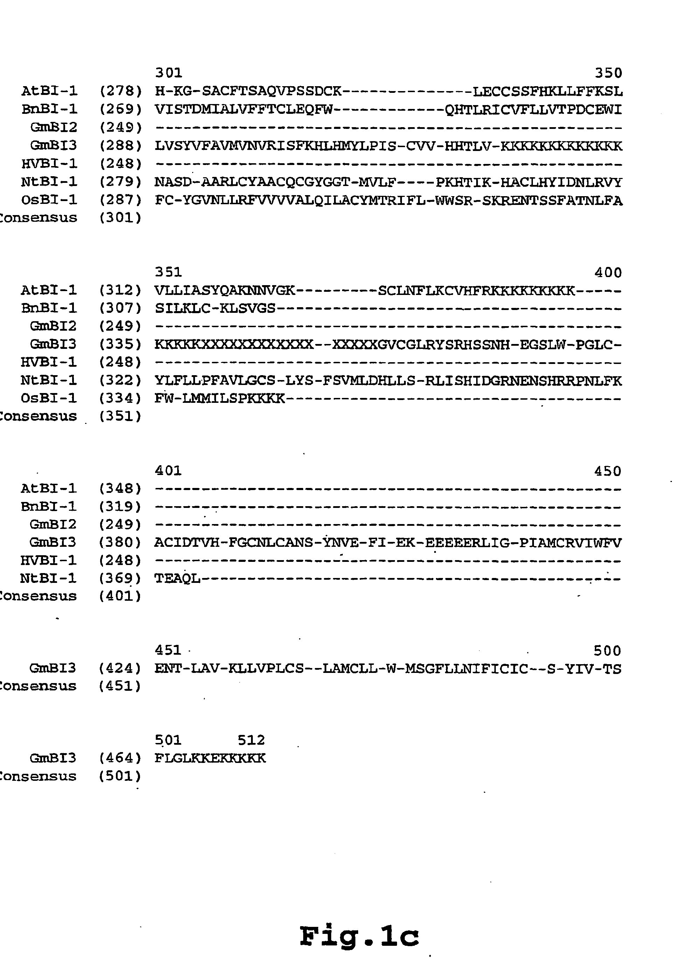

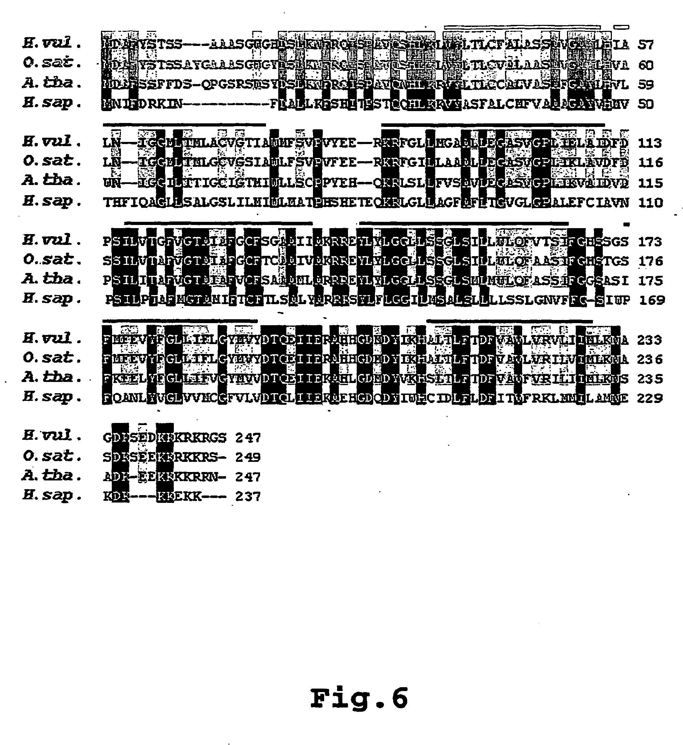

Besonders bevorzugt kommen Sequenzen mit Homologie zu mindestens 2 oder 3 dieser Motive (a bis g) in einem BI1-Protein vor, ganz besonders bevorzugt mindestens 4 oder 5, am meisten bevorzugt alle Motive a bis j. Weitere BI1 typischen Sequenzmotive kann der Fachmann unschwer aus dem Sequenzvergleich von BI1-Proteine - wie in Fig. 1 oder 6 dargestellt - ableiten.

Insbesondere bevorzugt sind BI-Proteine, die kodiert werden durch ein Polypeptid das mindestens eine Sequenz umfaßt ausgewählt aus der Gruppe bestehend aus:
a) den Sequenzen gemäß SEQ ID NO: 2, 4, 6, 8, 10, 12, 14, 16, 18, 20, 22, 24, 26, 28, 30, 32 und 38, und
b) Sequenzen, die eine Identität von mindestens 50%, bevorzugt mindstens 70%, besonders bevorzugt mindstens 90%, ganz besonders bevorzugt mindstens 95% zu einer der Sequenzen gemäß SEQ ID NO: 2, 4, 6, 8, 10, 12, 14, 16, 18, 20, 22, 24, 26, 28, 30, 32 und 38 aufweisen,
c) Sequenzen die mindestens eine Teilsequenz von mindestens 10, bevorzugt 20, besonders bevorzugt 50 zusammenhängenden Aminosäureresten einer der Sequenzen gemäß SEQ ID NO: 2, 4, 6, 8, 10, 12, 14, 16, 18, 20, 22, 24, 26, 28, 30, 32 und 38 umfassen.

Erfindungsgemäß von dem Begriff BI-Protein umfasst sind insbesondere natürliche oder künstliche Mutationen der BI1-Polypeptide gemäß SEQ ID NO: 2, 4, 6, 8, 10 und 38 sowie homologe Polypeptide aus anderen Organismen, bevorzugt Pflanzen, welche weiterhin im wesentlichen gleiche Eigenschaften aufweisen. Mutationen umfassen Substitutionen, Additionen, Deletionen, Inversion oder Insertionen eines oder mehrerer Aminosäurereste.
Umfaßt sind insofern auch Ausführungsformen unter Verwendung von BI1-Proteinen aus nicht-pflanzlichen Organismen wie beispielsweise Mensch (GenBank Acc.-No.: P55061), Ratte (GenBank Acc.-No.: P55062) oder Drosophila (GenBank Acc.-No.: Q9VSH3). Zwischen pflanzlichen und nicht-pflanzlichen BI1-Proteinen konservierte Motive können durch Sequenzvergleiche leicht identifiziert werden (vgl. Alignment in Bolduc et 1. (2003) Planta 216:377-386; Fig. 1 und 6).

Somit werden beispielsweise auch solche Polypeptide durch die vorliegende Erfindung mit umfaßt, welche man durch Modifikation eines Polypeptides gemäß SEQ ID NO: 2, 4, 6, 8, 10 und 38 erhält.

Die zu den im Rahmen dieser Erfindung offenbarten BI1-Sequenzen homologen Sequenzen aus anderen Pflanzen können z.B. durch
a) Datenbanksuche in Banken von Organismen, deren genomische oder cDNA Sequenz ganz oder teilweise bekannt ist, unter Verwendung der bereitgestellten BI1-Sequenzen als Suchsequenz oder
b) Durchmustern von Gen- oder cDNA-Bibliotheken unter Verwendung der bereitgestellten BI1-Sequenzen als Sonde - aufgefunden werden. Die Durchmusterung von cDNA- oder genomischen Bibliotheken (beispielsweise unter Verwendung einer der unter SEQ ID NO: 1, 3, 5, 7, 9, 11, 13 , 15, 17, 19, 21, 23, 25, 27, 29, 31 und 37 beschriebene Nukleinsäuresequenzen oder Teilen derselben als Sonde), ist ein dem Fachmann geläufiges Verfahren, um Homologe in anderen Arte zu identifizieren. Dabei haben die von den Nukleinsäuresequenzen gemäß SEQ ID NO: 1, 3, 5, 7, 9, 11, 13 , 15, 17, 19, 21, 23, 25, 27, 29, 31 und 37 abgeleiteten Sonden eine Länge von mindestens 20 bp, bevorzugt mindestens 50 bp, besonders bevorzugt mindestens 100 bp, ganz besonders bevorzugt mindestens 200 bp, am meisten bevorzugt mindestens 400 bp. Für die Durchmusterung der Bibliotheken kann auch ein zu den unter SEQ ID NO: 1, 3, 5, 7, 9, 11, 13 , 15, 17, 19, 21, 23, 25, 27, 29, 31 und 37 beschriebenen Sequenzen komplementärer DNA-Strang eingesetzt werden.

Unter Homologie zwischen zwei Nukleinsäuresequenzen wird die Identität der Nukleinsäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA; Altschul et al. (1997) Nucleic Acids Res. 25:3389ff) unter Einstellung folgender Parameter berechnet wird:

| | |
|---|---|
| Gap Weight: 50 | Length Weight: 3 |
| Average Match: 10 | Average Mismatch: 0 |

Beispielhaft wird unter einer Sequenz, die eine Homologie von mindestens 80 % auf Nukleinsäurebasis mit der Sequenz SEQ ID NO: 1 aufweist, eine Sequenz verstanden, die bei einem Vergleich mit der Sequenz SEQ ID NO: 1 nach obigem Programmalgorithmus mit obigem Parametersatz eine Homologie von mindestens 80 % aufweist.

Unter Homologie zwischen zwei Polypeptiden wird die Identität der Aminosäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:

| | |
|---|---|
| Gap Weight: 8 | Length Weight: 2 |
| Average Match: 2,912 | Average Mismatch:-2,003 |

Beispielhaft wird unter einer Sequenz, die eine Homologie von mindestens 80 % auf Proteinbasis mit der Sequenz SEQ ID NO: 2 aufweist, eine Sequenz verstanden, die bei einem Vergleich mit der Sequenz SEQ ID NO: 2 nach obigem Programmalgorithmus mit obigem Parametersatz eine Homologie von mindestens 80 % aufweist.

BI1-Proteine umfassen auch solche Polypeptide die durch Nukleinsäuresequenzen kodiert werden, die unter Standardbedingungen mit einer der durch SEQ ID NO: 1, 3, 5, 7, 9, 11, 13 , 15, 17, 19, 21, 23, 25, 27, 29, 31 und 37 beschriebenen BI1 Nukleinsäuresequenz, der zu ihr komplementären Nukleinsäuresequenz oder Teilen der vorgenannten hybridisieren und die gleichen wesentlichen Eigenschaften wie die unter SEQ ID NO: 2, 4, 6, 8, 10 und 38 beschriebenen Proteine haben.

Der Begriff "Standardhybridisierungsbedingungen" ist breit zu verstehen und meint stringente als auch weniger stringente Hybridisierungsbedingungen. Solche Hybridisierungsbedingungen sind unter anderem bei Sambrook J, Fritsch EF, Maniatis T et al., in Molecular Cloning (A Laboratory Manual), 2. Auflage, Cold Spring Harbor Laboratory Press, 1989, Seiten 9.31-9.57) oder in Current Protocols in Molecular Biology, John Wiley &Sons, N.Y. (1989), 6.3.1-6.3.6. beschrieben. Beispielhaft können die Bedingungen während des Waschschrittes ausgewählt sein aus dem Bereich von Bedingungen begrenzt von solchen mit geringer Stringenz (mit ungefähr 2X SSC bei 50°C) und solchen mit hoher Stringenz (mit ungefähr 0.2X SSC bei 50°C bevorzugt bei 65°C) (20X SSC: 0,3M Natriumcitrat, 3M NaCl, pH 7.0). Darüber hinaus kann die Temperatur während des Waschschrittes von niedrig stringenten Bedingungen bei Raumtemperatur, ungefähr 22°C, bis zu stärker stringenten Bedingungen bei ungefähr 65°C angehoben werden. Beide Parameter, Salzkonzentration und Temperatur, können gleichzeitig variiert werden, auch kann einer der beiden Parameter konstant gehalten und nur der andere variiert werden. Während der Hybridisierung können auch denaturierende Agenzien wie zum Beispiel Formamid oder SDS eingesetzt werden. In Gegenwart von 50% Formamid wird die Hybridisierung bevorzugt bei 42°C ausgeführt.

"Wesentliche Eigenschaften" meint in Bezug auf ein BI-Protein beispielsweise eine oder mehr nachfolgender Eigenschaften:
a) Verleihung oder Steigerung der Pathogenresistenz gegen zumindest ein Pathogen bei Erhöhung Proteinmenge oder Funktion des besagten BI-Proteins in mindestens einem Gewebe der Pflanze, wobei besagtes Gewebe nicht die Blattepidermis ist.
b) Ausbleiben eines spontan-induzierten Zelltodes bei Erhöhung der Proteinmenge oder Funktion des besagten BI-Proteins
c) Die Eigenschaft bei transienter co-Transfektion von Bax mit besagtem BI1-Protein beispielsweise in HEK293 Zellen die BAX-induzierte Apoptose signifikant zu inhibieren. Entsprechende Verfahren sind beschrieben (Bolduc N et al. (2003) Planta 216:377-386).
d) Das Vorliegen von fünf bis sieben putativen Transmembrandomänen innerhalb der besagten BI1-Proteins.
e) Eine präferentielle Lokalisation in Zellmembranen, insbesondere der Kernhüll-, ER- und/oder Thylakoidmembran.

Dabei kann die quantitative Ausprägung besagter Eigenschaften eines BI1-Proteins nach unten als auch nach oben im Vergleich zu einem Wert erhalten für das BI1-Protein gemäß SEQ ID NO: 2, 4, 6, 8, 10 oder 38 abweichen.

Der Begriff "Erhöhung der BI1 Proteinmenge" ist im Rahmen dieser Erfindung breit zu verstehen und kann auf unterschiedlichen zellbiologischen Mechanismen beruhen.

"Proteinmenge" meint die Menge eines BI1-Proteins in dem jeweils angegebenen Organismus, Gewebe, Zelle oder Zellkompartiment.

"Erhöhung der Proteinmenge" meint die mengenmäßige Erhöhung der Menge eines BI1-Proteins in dem jeweils angegebenen Organismus, Gewebe, Zelle oder Zellkompartiment. - beispielsweise durch eines der unten beschriebenen Verfahren - im Vergleich zu dem Wildtyp derselben Gattung und Art auf den dieses Verfahren nicht angewendet wurde, aber unter ansonst gleichen Rahmenbedingungen (wie beispielsweise Kulturbedingungen, Alter der Pflanzen etc.). Die Erhöhung beträgt dabei mindestens 10 %, bevorzugt mindestens 30 % oder mindestens 50 %, besonders bevorzugt mindestens 70 % oder 100 %, ganz besonders bevorzugt um mindestens 200 % oder 500 %, am meisten bevorzugt um mindestens 1000 %. Die Bestimmung der Proteinmenge kann durch verschiedene dem Fachmann geläufige Verfahren erfolgen. Beispielhaft jedoch nicht einschränkend seien zu nennen: Das Mikro-Biuret Verfahren (Goa J (1953) Scand J Clin Lab Invest 5:218-222), die Folin-Ciocalteu-Methode (Lowry OH et al. (1951) J Biol Chem 193:265-275) oder die Messung der Adsorption von CBB G-250 (Bradford MM (1976) Analyt Biochem 72:248-254). Ferner kann eine Quantifizierung über immunologische Methoden wie beispielsweise Western-Blot erfolgen. Die Herstellung entsprechender BI1-Antikörper sowie die Durchführung von BI1-Western-Blots ist beschrieben (Bolduc et al. (2002) FEBS Lett 532:111-114). Eine indirekte Quantifizierung kann über Northern-Blots realisiert werden, wobei die mRNA Menge in der Regel gut mit der resultierenden Proteinmenge korreliert. Entsprechende Verfahren sind beschrieben (u.a. Bolduc et al. (2003) Planta 216:377-386; Matsumura H et al. (2003) Plant J 33:425-434).

"Funktion" meint bevorzugt die Eigenschaft eines BI1-Proteins den spontan-induzierten Zelltodes zu vermindern und/oder die Eigenschaft, die apoptose-indizierende Wirkung von Bax zu inhibieren. Entsprechende Funktionen zählen zu den wesentlichen Eigenschaft eines BI1-Proteins.

"Erhöhung" der Funktion meint im Rahmen dieser Erfindung beispielsweise die mengenmäßige Steigerung der inhibitorischen Wirkung auf den Bax-induzierten apoptotischen Zelltod, welche durch dem Fachmann geläufige Verfahren quantifiziert werden kann (s.o.) Der Erhöhung beträgt dabei mindestens 10 %, bevorzugt mindestens 30 % oder mindestens 50 %, besonders bevorzugt mindestens 70 % oder 100 %, ganz besonders bevorzugt um mindestens 200 % oder 500 %, am meisten bevorzugt um mindestens 1000 %. Verfahren zur Erhöhung der Funktion umfassen neben den oben beschriebenen Verfahren zur Erhöhung der Proteinmenge (die auch immer die Funktion erhöht) ferner beispielhaft - jedoch nicht einschränkend - insbesondere das Einführen von Mutationen in ein BI1-protein.

Die BI1-Proteinmenge kann beispielhaft jedoch nicht einschränkend durch eines der nachfolgenden Verfahren erhöht werden:
a) Rekombinante Expression oder Überexpression eines BI1-Proteins durch Einringen einer rekombinanten Expressionskassette umfassend eine Nukleinsäuresequenz kodierend für ein BI1-Protein unter Kontrolle eines gewebespezifischen Promotors, wobei besagter Promotor im wesentlichen keine Aktivität in der Blattepidermis aufweist.
b) Modifikation (z.B. Austausch) der regulatorischen Regionen (z.B. der Promotorregion) eines endogenen BI1-Gens beispielsweise Austausch gegen einen gewebespezifischen Promotor mittels homologer Rekombination, wobei besagter Promotor im wesentlichen keine Aktivität in der Blattepidermis aufweist.
c) Insertion einer Nukleinsäuresequenz kodierend für ein BI1-Protein in das pflanzliche Genom hinter einen gewebespezifischen Promotor mittels homologer Rekombination, wobei besagter Promotor im wesentlichen keine Aktivität in der Blattepidermis aufweist.
d) Erhöhung der Expression eines endogenen BI1-Proteins durch Einbringen eines Transkriptionsfaktors (z.B. eines artifiziellen Transkriptionsfaktors aus der Klasse der Zinkfingerproteine) geeignet zur Induktion der Expression besagten BI1-Proteins. Bevorzugt ist das Einringen einer rekombinanten Expressionskassette umfassend eine Nukleinsäuresequenz kodierend für besagten Transkriptionsfaktor unter Kontrolle eines gewebespezifischen Promotors, wobei besagter Promotor im wesentlichen keine Aktivität in der Blattepidermis aufweist.

Der Begriff "Einbringen" umfaßt im Rahmen der Erfindung allgemein alle Verfahren, die dazu geeignet die einzubringende Verbindung, direkt oder indirekt, in eine Pflanze oder eine Zelle, Kompartiment, Gewebe, Organ oder Samen derselben einzuführen oder dort zu generieren. Direkte und indirekte Verfahren sind umfaßt. Die Einbringen kann zu einer vorübergehenden (transienten) Präsenz besagter Verbindung führen oder aber auch zu einer dauerhaften (stabilen) oder induzierbaren. Einführen umfaßt beispielsweise Verfahren wie Transfektion, Transduktion oder Transformation.

In den im Rahmen der Erfindung zum Einsatz kommenden rekombinanten Expressionskassetten steht ein Nukleinsäuremolekül (beispielsweise kodierend für ein BI1-Protein) in funktioneller Verknüpfung mit mindestens einem gewebespezifischen Promotor, wobei besagter Promotor im wesentlichen keine Aktivität in der Blattepidermis aufweist und wobei der Promotor in Bezug auf die zu exprimierende Nukleinsäuresequenz heterolog ist d.h. natürlicherweise nicht mit derselben kombiniert vorkommt. Die erfindungsgemäßen rekombinanten Expressionskassetten können optional weitere genetische Kontrollelement umfassen.

Unter einer funktionellen Verknüpfung versteht man zum Beispiel die sequentielle Anordnung des besagten Promotors mit der zu exprimierenden Nukleinsäuresequenz und ggf. weiterer regulativer Elemente wie zum Beispiel einem Terminator derart, dass jedes der regulativen Elemente seine Funktion bei der rekombinanten Expression der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die rekombinant zu exprimierende Nukleinsäuresequenz hinter der als Promoter fungierenden Sequenz positioniert wird, so dass beide Sequenzen kovalent miteinander verbunden sind. Bevorzugt ist dabei der Abstand zwischen der Promotorsequenz und der rekombinant zu exprimierende Nukleinsäuresequenz geringer als 200 Basenpaare, besonders bevorzugt kleiner als 100 Basenpaare, ganz besonders bevorzugt kleiner als 50 Basenpaare. Die Herstellung einer funktionellen Verknüpfung als auch die Herstellung einer rekombinanten Expressionskassette kann mittels gängiger Rekombinations- und Klonierungstechniken realisiert werden, wie sie beispielsweise in Maniatis T, Fritsch EF und Sambrook J (1989) Molecular Cloning: A Laboratory Manual, Cold Spring Harbor Laboratory, Cold Spring Harbor (NY), in Silhavy TJ, Berman ML und Enquist LW (1984) Experiments with Gene Fusions, Cold Spring Harbor Laboratory, Cold Spring Harbor (NY), in Ausubel FM et al. (1987) Current Protocols in Molecular Biology, Greene Publishing Assoc. and Wiley Interscience und bei Gelvin et al. (1990) In: Plant Molecular Biology Manual beschrieben sind. Zwischen beide Sequenzen können aber auch weitere Sequenzen positioniert werden, die zum Beispiel die Funktion eines Linkers mit bestimmten Restriktionsenzymschnittstellen oder eines Signalpeptides haben. Auch kann die Insertion von Sequenzen zur Expression von Fusionsproteinen führen.

Bevorzugt kann die rekombinante Expressionskassette, bestehend aus einer Verknüpfung von Promoter und zu exprimierender Nukleinsäuresequenz, integriert in einem Vektor vorliegen und durch zum Beispiel Transformation in ein pflanzliches Genom insertiert werden.

Unter einer rekombinanten Expressionskassette sind aber auch solche Konstruktionen zu verstehen, bei denen der Promoter - zum Beispiel durch eine homologe Rekombination - vor ein endogenes BI1-Gen plaziert wird, und so die Expression des BI1-Proteins steuert. Analog kann auch die zu exprimierende Nukleinsäuresequenz (z.B. kodierend für ein BI1-Protein) derart hinter einen endogenen Promotor plaziert werden, dass der gleiche Effekt auftritt. Beide Ansätze führen zu rekombinanten Expressionskassetten im Sinne der Erfindung.

Unter einem "gewebespezifischen Promotor, der im wesentlichen keine Aktivität in der Blattepidermis aufweist" sind im Rahmen dieser Erfindung allgemein solche Promotoren zu verstehen, die geeignet sind eine rekombinante Expression einer Nukleinsäuresequenz mindestens in einem pflanzlichen Gewebe zu gewährleisten oder zu erhöhen, mit der Maßgabe, dass
a) besagtes pflanzliches Gewebe ausgewählt ist aus allen pflanzlichen Geweben mit Ausnahme der Blattepidermis, und
b) die rekombinante Expression unter Kontrolle des besagten Promotors in besagtem pflanzlichen Gewebe mindestens das fünffache, bevorzugt mindestens das zehnfache, besonders bevorzugt mindestens das einhundertfache der Expression in der pflanzlichen Blattepidermis beträgt.

Dem Fachmann sind zahlreiche Promotoren bekannt, die diesen Anforderungen genügen. Insbesondere geeignet sind gewebespezifische Promotoren, wie beispielsweise, jedoch nicht einschränkend Promotoren mit Spezifitäten für die Antheren, Ovarien, Blüten, Stengel, Wurzeln, Knollen oder Samen.
a) Als Samen spezifische Promotoren bevorzugt sind zum Beispiel die Promotoren des Phaseolins (US 5,504,200; Bustos MM et al. (1989) Plant Cell 1(9):839-53), 2S Albumins (Joseffson LG et al. (1987) J Biol Chem 262:12196-12201), Legumins (Shirsat A et al. (1989) Mol Gen Genet 215(2):326-331) und Legumin B4 (LeB4; Baumlein H et al. (1991) Mol Gen Genet 225:121-128; Baumlein H et al. (1992) Plant J 2(2):233-9; Fiedler U et al. (1995) Biotechnology (NY) 13.(10) :1090f), USP (unknown seed protein; Baumlein H et al. (1991) Mol Gen Genet 225(3):459-67), Napins (US 5,608,152; Stalberg K et al. (1996) L Planta 199:515-519), Saccharosebindeproteins (WO 00/26388), Oleosins (WO 98/45461), oder der Bce4-Promoter aus Brassica (WO 91/13980). Weitere geeignete samenspezifische Promotoren sind die der Gene kodierend für das "High Molecular Weight Glutenin" (HMWG), Gliadin, Verzweigungsenzym, ADP Glucose Pyrophosphatase (AGPase) oder die Stärkesynthase. Bevorzugt sind ferner Promotoren, die eine samenspezifische Expression in Monokotyledonen wie Mais, Gerste, Weizen, Roggen, Reis etc. erlauben. Vorteilhaft eingesetzt werden können der Promoter des lpt2 oder Ipt1-Gen (WO 95/15389, WO 95/23230) oder die Promotoren beschrieben in WO 99/16890 (Promotoren des Hordein-Gens, des Glutelin-Gens, des Oryzin-Gens, des.Prolamin-Gens, des Gliadin-Gens, des Glutelin-Gens, des Zein-Gens, des Kasirin-Gens oder des Secalin-Gens). Weitere samenspezifische Promotoren sind beschrieben in WO 89/03887.
b) Knollen-, Speicherwurzel- oder Wurzel-spezifische Promotoren umfassen beispielsweise den Promotor des Patatin Gens (GenBank Acc.-No.: A08215), den Patatin Promotor Klasse I B33-Promotor (GenBank Acc.-No.: X14483) oder den Promotor des Cathepsin D Inhibitors aus Kartoffel. Insbesondere bevorzugt ist der Promotor beschrieben durch SEQ ID NO: 29. Knollen-spezifische Promotoren sind im Rahmen der Erfindung insbesondere zum Erzielen einer Resistenz gegen *Phytophthora infestans* geeignet. Da obligat-biotrophe Pilze nur Blätter befallen, ist eine Aktivität im epidermalen Knollengewebe unerheblich.
c) Blütenspezifische Promotoren umfassen beispielsweise den Phytoen Synthase Promotor (WO 92/16635) oder den Promotor des P-rr Gens (WO 98/22593).
d) Antheren-spezifische Promotoren umfassen beispielsweise den 5126-Promotor (US 5,689,049, US 5,689,051), den glob-1 Promotor und den γ-Zein Promotor.
e) Ährenspezifische Promotoren, wie beispielsweise der in US 6,291,666. Ähren-spezifische Promotoren sind insbesondere zur Vermittlung einer Resistenz gegen Fusarium vorteilhaft.
f) Mesophyll-spezifische Promotoren wie beispielsweise der Promotor des Weizen Germin 9f-3.8 Gens (GenBank Acc.-No.: M63224) oder der Gerste GerA Promotor (WO 02/057412). Besagte Promotoren sind insbesondere vorteilhaft, da sie sowohl mesophyll-spzifisch und pathogen-induzierbar sind. Ferner geeignet ist der mesophyll-spezifische Arabidopsis CAB-2 Promotor (GenBank Acc.-No.: X15222), sowie der Zea mays PPCZm1 Promotor (GenBank Acc.-No.: X63869). Insbesondere bevorzugt sind die Promotor beschrieben durch SEQ ID NO: 30, 31 oder 32. Die in erfindungsgemäßen Verfahren verwendeten rekombinanten Expressionskassetten oder Vektoren enthaltenen Nukleinsäuresequenzen können mit weiteren genetischen Kontrollsequenzen neben einem Promoter funktionell verknüpft sein. Der Begriff der genetischen Kontrollsequenzen ist breit zu verstehen und meint all solche Sequenzen, die einen Einfluß auf das Zustandekommen oder die Funktion der erfindungsgemäßen rekombinanten Expressionskassette haben. Genetische Kontrollsequenzen umfassen auch weitere Promotoren, Promotorelemente oder Minimalpromotoren, die die expressionssteuernden Eigenschaften modifizieren können. So kann durch genetische Kontrollsequenzen zum Beispiel die gewebespezifische Expression zusätzlich abhängig von bestimmten Streßfaktoren erfolgen. Entsprechende Elemente sind zum Beispiel für Wasserstreß, Abscisinsäure (Lam E & Chua NH (1991) J Biol Chem 266(26):17131-17135) und Hitzestreß (Schoffl F et al. (1989) Mol Gen Genet 217(2-3):246-53) beschrieben.

Genetische Kontrollsequenzen umfassen ferner auch die 5'-untranslatierte Regionen, Introns oder nichtkodierende 3'-Region von Genen wie beipielsweise das Actin-1 Intron, oder die Adh1-S Introns 1, 2 und 6 (allgemein: The Maize Handbook, Chapter 116, Freeling and Walbot, Eds., Springer, New York (1994)). Es ist gezeigt worden, dass diese eine signifikante Funktionen bei der Regulation der Genexpression spielen können. So wurde gezeigt, dass 5'-untranslatierte Sequenzen die transiente Expression heterologer Gene verstärken können. Beispielhaft für Translationsverstärker sei die 5'-Leadersequenz aus dem Tabak-Mosaik-Virus zu nennen (Gallie et al. (1987) Nucl Acids Res 15:8693-8711) und dergleichen. Sie können ferner die Gewebsspezifität fördern (Rouster J et al. (1998) Plant J 15:435-440).

Die rekombinante Expressionskassette kann vorteilhafterweise eine oder mehrere sogenannte "enhancer Sequenzen" funktionell verknüpft mit dem Promoter enthalten, die eine erhöhte rekombinante Expression der Nukleinsäuresequenz ermöglichen. Auch am 3'-Ende der rekombinant zu exprimierenden Nukleinsäuresequenzen können zusätzliche vorteilhafte Sequenzen inseriert werden, wie weitere regulatorische Elemente oder Terminatoren. Die rekombinant zu exprimierenden Nukleinsäuresequenzen können in einer oder mehreren Kopien im Genkonstrukt enthalten sein.

Als Kontrollsequenzen geeignete Polyadenylierungssignale sind pflanzliche Polyadenylierungssignale, vorzugsweise solche, die im wesentlichen T-DNA Polyadenylierungssignale aus Agrobacterium tumefaciens, insbesondere der OCS (Octopin-Synthase)-Terminator und der NOS (Nopalin-Synthase)-Terminator.

Als Kontrollsequenzen sind weiterhin solche zu verstehen, die eine homologe Rekombination bzw. Insertion in das Genom eines Wirtsorganismus ermöglichen oder die Entfernung aus dem Genom erlauben. Bei der homologen Rekombination kann zum Beispiel der natürliche Promoter eines BI1-Gens gegen einen der bevorzugten gewebespezifischen Promoter ausgetauscht werden. Methoden wie die cre/lox-Technologie erlauben eine gewebespezifische, unter Umständen induzierbare Entfernung der rekombinanten Expressionskassette aus dem Genom des Wirtsorganismus (Sauer B (1998) Methods. 14(4) : 381-92). Hier werden bestimmte flankierende Sequenzen dem Zielgen angefügt (lox-Sequenzen), die später eine Entfernung mittels der cre-Rekombinase ermöglichen.

Eine rekombinante Expressionskassetten und die von ihr abgeleiteten Vektoren können weitere Funktionselemente enthalten. Der Begriff Funktionselement ist breit zu verstehen und meint all solche Elemente, die einen Einfluß auf Herstellung, Vermehrung oder Funktion der erfindungsgemäßen rekombinanten Expressionskassetten, Vektoren oder rekombinanten Organismen haben. Beispielhaft aber nicht einschränkend seien zu nennen:
a) Selektionsmarker, die eine Resistenz gegen einen Metabolismusinhibitor wie 2-Desoxyglucose-6-phosphat (WO 98/45456), Antibiotika oder Biozide, bevorzugt Herbizide, wie zum Beispiel Kanamycin, G 418, Bleomycin, Hygromycin, oder Phosphinotricin etc. verleihen. Besonders bevorzugte Selektionsmarker sind solche die eine Resistenz gegen Herbizide verleihen. Beispielhaft seien genannt: DNA Sequenzen, die für Phosphinothricinacetyltransferasen (PAT) kodieren und Glutaminsynthaseinhibitoren inaktivieren (bar und pat Gen), 5-Enolpyruvylshikimat-3-phosphatsynthasegene (EPSP Synthasegene), die eine Resistenz gegen Glyphosat^{®} (N-(phosphonomethyl)glycin) verleihen, das für das Glyphosat^{®} degradierende Enzyme kodierende gox Gen (Glyphosatoxidoreduktase), das deh Gen (kodierend für eine Dehalogenase, die Dalapon^{®} inaktiviert), Sulfonylurea- und Imidazolinon inaktivierende Acetolactatsynthasen sowie bxn Gene, die für Bromoxynil degradierende Nitrilaseenzyme kodieren, das aasa-Gen, das eine Resistenz gegen das Antibiotikum Apectinomycin verleih, das streptomycinphosphotransferase (SPT) Gen, das eine Resistenz gegen Streptomycin gewährt, das Neomycinphosphotransferas (NPTII) Gen, das eine Resistenz gegen Kanamycin oder Geneticidin verleiht, das Hygromycinphosphotransferase (HPT) Gen, das eine Resistenz gegen Hygromycin vermittelt, das Acetolactatsynthas Gen (ALS), das eine Resistenz gegen Sulfonylharnstoff-Herbizide verleiht (z.B. mutierte ALS-Varianten mit z.B. der S4 und/oder Hra Mutation).
b) Reportergene, die für leicht quantifizierbare Proteine kodieren und über Eigenfarbe oder Enzymaktivität eine Bewertung der Transformationseffizienz oder des Expressionsortes oder -zeitpunktes gewährleisten. Ganz besonders bevorzugt sind dabei Reporter-Proteine (schenborn E, Groskreutz D. Mol Biotechnol. 1999; 13(1):29-44) wie das "green fluorescence protein" (GFP) (Sheen et al.(1995) Plant Journal 8(5):777-784; Haseloff et al.(1997) Proc Natl Acad Sci USA 94(6):2122-2127; Reichel et al.(1996) Proc Natl Acad Sci USA 93(12):5888-5893; Tian et al. (1997) Plant Cell Rep 16:267-271; WO 97/41228; Chui WL et al. (1996) Curr Biol 6:325-330; Leffel SM et al. (1997) Biotechniques. 23(5):912-8), die Chloramphenicoltransferase, eine Luziferase (Ow et al. (1986) Science 234:856-859; Millar et al. (1992) Plant Mol Biol Rep 10:324-414), das Aequoringen (Prasher et al. (1985) Biochem Biophys Res Commun 126(3):1259-1268), die β-Galactosidase, R-Locus Gen (kodieren ein Protein, das die Produktion von Anthocyaninpigmenten (rote Färbung) in pflanzlichen Gewebe reguliert und so eine direkte Analyse der Promotoraktivität ohne Zugabe zusätzlicher Hilfstoffe oder chromogener Substrate ermöglicht; Dellaporta et al., In: Chromosome Structure and Function: Impact of New Concepts, 18th Stadler Genetics Symposium, 11:263-282, 1988), ganz besonders bevorzugt ist die β-Glucuronidase (Jefferson et al., EMBO J. 1987, 6, 3901-3907).
c) Replikationsursprünge, die eine Vermehrung der erfindungsgemäßen rekombinanten Expressionskassetten oder Vektoren in zum Beispiel E.coli gewährleisten. Beispielhaft seien genannt ORI (origin of DNA replication), der pBR322 ori oder der P15A ori (Sambrook et al.: Molecular Cloning. A Laboratory Manual, 2nd ed. Cold Spring Harbor Laboratory Press, Cold Spring Harbor, NY, 1989).
d) Elemente, die für eine Agrobakterium vermittelte Pflanzentransformation erforderlich sind, wie zum Beispiel die rechte oder linke Begrenzung der T-DNA oder die vir-Region.

Zur Selektion erfolgreich homolog rekombinierter oder auch transformierter Zellen ist es in der Regel erforderlich, einen selektionierbaren Marker zusätzlich einzuführen, der den erfolgreich rekombinierten Zellen eine Resistenz gegen ein Biozid (zum Beispiel ein Herbizid), einen Metabolismusinhibitor wie 2-Desoxyglucose-6-phosphat (WO 98/45456) oder ein Antibiotikum verleiht. Der Selektionsmarker erlaubt die Selektion der transformierten Zellen von untransformierten (McCormick et al. (1986) Plant Cell Reports 5:81-84).

Die Einführung einer rekombinanten Expressionskassette in einen Organismus oder Zellen, Geweben, Organe, Teile bzw. Samen desselben (bevorzugt in Pflanzen bzw. pflanzliche Zellen, Gewebe, Organe, Teile oder Samen), kann vorteilhaft unter Verwendung von Vektoren realisiert werden, in denen die rekombinanten Expressionskassetten enthalten sind. Die rekombinante Expressionskassette kann in den Vektor (zum Beispiel ein Plasmid) über eine geeignete Restriktionsschnittstelle eingeführt werden. Das entstandene Plasmid wird zunächst in E.coli eingeführt. Korrekt transformierte E.coli werden selektioniert, gezüchtet und das rekombinante Plasmid mit dem Fachmann geläufigen Methoden gewonnen. Restriktionsanalyse und Sequenzierung können dazu dienen, den Klonierungsschritt zu überprüfen.

Vektoren können beispielhaft Plasmide, Cosmide, Phagen, Viren oder auch Agrobacterien sein. In einer vorteilhaften Ausführungsform wird die Einführung der rekombinante Expressionskassette mittels Plasmidvektoren realisiert. Bevorzugt sind solche Vektoren, die eine stabile Integration der rekombinanten Expressionskassette in das Wirtsgenom ermöglichen.

Die Herstellung eines transformierten Organismus (bzw. einer transformierten Zelle oder Gewebes) erfordert, dass die entsprechende DNA, RNA oder Protein in die entsprechende Wirtszelle eingebracht wird.

Für diesen Vorgang, der als Transformation (oder Transduktion bzw. Transfektion) bezeichnet wird, steht eine Vielzahl von Methoden zur Verfügung (Keown et al. (1990) Methods Enzymol 185:527-537; Jenes B et al.(1993) Techniques for Gene Transfer, in: Transgenic Plants, Vol. 1, Engineering and Utilization, herausgegeben von SD Kung und R Wu, Academic Press, S. 128-143 sowie in Potrykus (1991) Annu Rev Plant Physiol Plant Molec Biol 42;205-225).

So kann die DNA oder RNA beispielhaft direkt durch Mikroinjektion oder durch Bombardierung mit DNA-beschichteten Mikropartikeln eingeführt werden. Auch kann die Zelle chemisch, zum Beispiel mit Polyethylenglykol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen erfolgen. Elektroporation ist eine weitere geeignete Methode zur Einführung von DNA, bei der die Zellen reversibel durch einen elektrischen Impuls permeabilisiert werden. Entsprechende Verfahren sind beschrieben (beispielsweise bei Bilang et al. (1991) Gene 100:247-250; Scheid et al. (1991) Mol Gen Genet 228:104-112; Guerche et al. (1987) Plant Science 52:111-116; Neuhause et al. (1987) Theor Appl Genet 75:30-36; Klein et al. (1987) Nature 327:70-73 ; Howell et al. (1980) Science 208:1265; Horsch et al.(1985) Science 227:1229-1231 ; DeBlock et al. (1989) Plant Physiol 91:694-701).

Bei Pflanzen werden dabei die beschriebenen Methoden zur Transformation und Regeneration von Pflanzen aus Pflanzengeweben oder Pflanzenzellen zur transienten oder stabilen Transformation genutzt. Geeignete Methoden sind vor allem die Protoplastentransformation durch Polyethylenglykol-induzierte DNA-Aufnahme, das biolistische Verfahren mit der Genkanone, die sogenannte "particle bombardement" Methode, die Elektroporation, die Inkubation trockener Embryonen in DNA-haltiger Lösung und die Mikroinjektion.

Neben diesen "direkten" Transformationstechniken kann eine Transformation auch durch bakterielle Infektion mittels Agrobacterium tumefaciens oder Agrobacterium rhizogenes durchgeführt werden. Die Agrobacterium-vermittelte Transformation ist am besten für dicotyledone Pflanzenzellen geeignet. Die Verfahren sind beispielsweise beschrieben bei Horsch RB et al. (1985) Science 225: 1229f).

Werden Agrobacterien verwendet, so ist die rekombinante Expressionskassette in spezielle Plasmide zu integrieren, entweder in einen Zwischenvektor (englisch: shuttle or intermediate vector) oder einen binären Vektor. Wird ein Ti oder Ri Plasmid zur Transformation verwendet werden soll, ist zumindest die rechte Begrenzung, meistens jedoch die rechte und die linke Begrenzung der Ti oder Ri Plasmid T-DNA als flankierende Region mit der einzuführenden rekombinanten Expressionskassette verbunden.

Bevorzugt werden binäre Vektoren verwendet. Binäre Vektoren können sowohl in E.coli als auch in Agrobacterium replizieren. Sie enthalten in der Regel ein Selektionsmarkergen und einen Linker oder Polylinker flankiert von der rechten und linken T-DNA Begrenzungssequenz. Sie können direkt in Agrobacterium transformiert werden (Holsters et al. (1978) Mol Gen Genet 163:181-187). Das Selektionsmarkergen erlaubt eine Selektion transformierter Agrobakteria und ist zum Beispiel das nptII Gen, das eine Resistenz gegen Kanamycin verleiht. Das in diesem Fall als Wirtsorganismus fungierende Agrobacterium sollte bereits ein Plasmid mit der vir-Region enthalten. Diese ist für die Übertragung der T-DNA auf die pflanzliche Zelle erforderlich. Ein so transformiertes Agrobacterium kann zur Transformation pflanzlicher Zellen verwendet werden. Die Verwendung von T-DNA zur Transformation pflanzlicher Zellen ist intensiv untersucht und beschrieben (EP 120 516; Hoekema, In: The Binary Plant Vector System, Offsetdrukkerij Kanters B.V., Alblasserdam, Chapter V; An et al. (1985) EMBO J 4:277-287). Verschiedene binäre Vektoren sind bekannt und teilweise kommerziell erhältlich wie zum Beispiel pBI101.2 oder pBIN19 (Bevan et al. (1984) Nucl Acids Res 12:8711f; Clontech Laboratories, Inc. USA). Weitere zur Expression in Pflanzen geeignet Promotoren sind beschrieben (Rogers et al. (1987) Methods Enzymol 153:253-277; Schardl et al. (1987) Gene 61:1-11; Berger et al. (1989) Proc Natl Acad Sci USA 86:8402-8406).

Direkte Transformationstechniken eignen sich im Prinzip für jeden Organismus und Zelltyp. Im Falle von Injektion oder Elektroporation von DNA bzw. RNA in pflanzliche Zellen sind keine besonderen Anforderungen an das verwendete Plasmid gestellt. Einfache Plasmide wie die der pUC-Reihe können verwendet werden. Sollen vollständige Pflanzen aus den transformierten Zellen regeneriert werden, so ist er erforderlich, das sich auf dem Plasmid ein zusätzliches selektionierbares Markergen befindet.

Stabil transformierte Zellen d.h. solche, die die eingeführte DNA integriert in die DNA der Wirtszelle enthalten, können von untransformierten selektioniert werden, wenn ein selektionierbarer Marker Bestandteil der eingeführten DNA ist. Als Marker kann beispielhaft jedes Gen fungieren, dass eine Resistenz gegen Antibiotika oder Herbizide (wie Kanamycin, G 418, Bleomycin, Hygromycin oder Phosphinotricin etc.) zu verleihen vermag (s.o.). Transformierte Zellen, die ein solches Markergen exprimieren, sind in der Lage, in der Gegenwart von Konzentrationen eines entsprechenden Antibiotikums oder Herbizides zu überleben, die einen untransformierten Wildtyp abtöten. Beispiel für geeignete Selektionsmarker sind oben genannt. Sobald eine transformierte Pflanzenzelle hergestellt wurde, kann eine vollständige Pflanze unter Verwendung von dem Fachmann bekannten Verfahren erhalten werden. Hierbei geht man beispielhaft von Kalluskulturen aus. Aus diesen noch undifferenzierten Zellmassen kann die Bildung von Sproß und Wurzel in bekannter Weise induziert werden. Die erhaltenen Sprößlinge können ausgepflanzt und gezüchtet werden. Dem Fachmann sind Verfahren bekannt, um aus Pflanzenzellen, Pflanzenteile und ganze Pflanzen zu regenerieren. Beispielsweise werden hierzu Verfahren beschrieben von Fennell et al. (1992) Plant Cell Rep. 11: 567-570; Stoeger et al (1995) Plant Cell Rep. 14:273-278; Jahne et al. (1994) Theor Appl Genet 89:525-533 verwendet. Die erhaltenen Pflanzen können in der üblichen Weise gezüchtet und/oder gekreuzt werden. Zwei oder mehr Generationen sollten kultiviert werden, um sicherzustellen, dass die genomische Integration stabil und vererblich ist.

Das erfindungsgemäße Verfahren kann vorteilhaft mit weiteren Verfahren die eine Pathogenresistenz (beispielsweise gegen Insekten, Pilze, Bakterien, Nematoden etc.), Streßresistenz oder eine andere Verbesserung der pflanzlichen Eigenschaften bewirken kombiniert werden. Beispiele sind u.a. genannt bei Dunwell JM (2000) J Exp Bot. 51 Spec No:487-96. Offenbart werden Polypeptidsequenzen kodierend für BI1 Protein umfassend mindestens eine Sequenz ausgewählt aus der Gruppe bestehend aus
a) den Sequenzen gemäß SEQ ID NO: 12, 14, 16, 18, 20, 22, 24, 28, 30, 32 oder 38,
b) Sequenzen die eine Homologie von mindestens 90%, bevorzugt mindestens 95%, besonders bevorzugt mindesten 98% zu einer der Sequenzen gemäß SEQ ID NO: 12, 14, 16, 18, 20, 22, 24, 28, 30, 32 oder 38 aufweisen, und
c) Sequenzen die mindestens 10, bevorzugt mindestens 20, besonders bevorzugt mindestens 30 zusammenhängende Aminosäuren einer der Sequenzen gemäß SEQ ID NO: 12, 14, 16, 18, 20, 22, 24, 28, 30, 32 oder 38 umfassen.

Verwendet werden in der Erfindung bevorzugt Nukleinsäuresequenzen kodierend für die erfindungsgemäßen neuen Polypeptidsequenzen kodierend für BIl-Proteine. Bevorzugt sind die Nukleinsäuresequenz gemäß SEQ ID NO: 11, 13, 15, 17, 19, 21, 23, 25, 27, 29, 31 oder 37, die dazu komplementäre Nukleinsäuresequenz und die durch Entartung (Degeneration) des genetischen Codes abgeleitete Sequenzen.

Dazu dienen rekombinante Expressionskassetten, die eine der erfindungsgemäßen Nukleinsäuresequenzen umfassen. In den rekombinanten Expressionskassetten ist die Nukleinsäuresequenz kodierend für das BI1-Protein aus Gerste mit mindestens einem genetischen Kontrollelement nach obiger Definition derart verknüpft, das die Expression (Transkription und ggf. Translation) in einem beliebigen Organismus - bevorzugt in Pflanzen - realisiert werden kann. Dazu geeignete genetische Kontrollelemente sind oben beschrieben. Die rekombinanten Expressionskassetten können auch weitere Funktionselementen gemäß obiger Definition enthalten. Die insertierte Nukleinsäuresequenz kodierend für ein BI1-Protein aus Gerste kann in sense- oder antisense-Orientierung in die Expressionskassette insertiert sein, und damit zu Expression von sense- oder antisense-RNA führen. Erfindungsgemäß verwendet werden auch rekombinante Vektoren, die die rekombinanten Expressionskassetten beinhalten.

"Rekombinant" meint bezüglich zum Beispiel einer Nukleinsäuresequenz, einer Expressionskassette oder einem Vektor enthaltend besagte Nukleinsäuresequenz oder einem Organismus transformiert mit besagter Nukleinsäuresequenz, Expressionskassette oder Vektor alle solche durch gentechnische Methoden zustandegekommene Konstruktionen, in denen sich entweder
a) die BI1 Nukleinsäuresequenz, oder
b) eine mit der BI1 Nukleinsäuresequenz funktionell verknüpfte genetische Kontrollsequenz, zum Beispiel ein Promotor, oder
c) (a) und (b)
sich nicht in ihrer natürlichen, genetischen Umgebung befinden oder durch gentechnische Methoden modifiziert wurden, wobei die Modifikation beispielhaft eine Substitutionen, Additionen, Deletionen, Inversion oder Insertionen eines oder mehrerer Nukleotidreste sein kann. Natürliche genetische Umgebung meint den natürlichen chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des BIl-Promotors mit dem entsprechenden BIl-Gen - wird zu einer rekombinanten Expressionskassette, wenn diese durch nicht-natürliche, synthetische ("künstliche") Verfahren wie beispielsweise einer Mutagenisierung geändert wird. Entsprechende Verfahren sind beschrieben (US 5,565,350; WO 00/15815; siehe auch oben).

In einer Ausführungsform betrifft die Erfindung die Verwendung eines BI-1 codierenden Nukleinsäuremoleküls oder eines BI-1 Proteins zur Herstellung einer Pathogen-resistenten Pflanze, vorzugsweise zur Herstellung einer gegen Pilze resistenten Pflanze oder zur Herstellung eines dies bewirkenden Fungizids oder zur Bekämpfung oder Behandlung von mit Pathogenen befallenen oder durch Pathogene bedrohten Pflanzen.

### Sequenzen

| | | |
|---|---|---|
| 1. | SEQ ID NO: 1 : | Nukleinsäuresequenz kodierend für ein BI1 Protein aus Gerste (Hordeum vulgare). |
| 2. | SEQ ID NO: 2 : | Aminosäuresequenz kodierend für ein BI1 Protein aus Gerste (Hordeum vulgare). |
| 3. | SEQ ID NO: 3 : | Nukleinsäuresequenz kodierend für ein BI1 Protein aus Arabidopsis thaliana. |
| 4. | SEQ ID NO: 4 : | Aminosäuresequenz kodierend für ein BI1 Protein aus Arabidopsis thaliana. |
| 5. | SEQ ID NO: 5 ; | Nukleinsäuresequenz kodierend für ein BI1 Protein aus Tabak. |
| 6. | SEQ ID NO: 6 : | Aminosäuresequenz kodierend für ein BI1 Protein aus Tabak. |
| 7. | SEQ ID NO: 7 : | Nukleinsäuresequenz kodierend für ein BI1 Protein aus Reis. |
| 8. | SEQ ID NO: 8: | Aminosäuresequenz kodierende für ein BI1 Protein aus Reis. |
| 9. | SEQ ID NO: 9 : | Nukleinsäuresequenz kodierend für ein BI1 Protein aus Raps. |
| 10. | SEQ ID NO: 10 : | Aminosäuresequenz kodierend für ein BI1 Protein aus Raps. |
| 11. | SEQ ID NO: 11 : | Nukleinsäuresequenz kodierend für Teil eines BI1 Protein aus Soja. |
| 12. | SEQ ID NO: 12: | Aminosäuresequenz kodierend für Teil eines BI1 Protein aus Soja. |
| 13. | SEQ ID NO: 13 : | Nukleinsäuresequenz kodierend für Teil eines BI1 Protein aus Soja. |
| 14. | SEQ ID NO: 14: | Aminosäuresequenz kodierend für Teil eines BI1 Protein aus Soja. |
| 15. | SEQ ID NO: 15 : | Nukleinsäuresequenz kodierend für Teil eines BI1 Protein aus Weizen. |
| 16. | SEQ ID NO: 16 : | Aminosäuresequenz kodierend für Teil eines BI1 Protein aus Weizen. |
| 17. | SEQ ID NO: 17 : | Nukleinsäuresequenz kodierend für Teil eines BI1 Protein aus Mais. |
| 18. | SEQ ID NO: 18 : | Aminosäuresequenz kodierend für Teil eines BI1 Protein aus Mais. |
| 19. | SEQ ID NO: 19 : | Nukleinsäuresequenz kodierend für Teil eines BI1 Protein aus Weizen. |
| 20. | SEQ ID NO: 20 : | Aminosäuresequenz kodierend für Teil eines BI1 Protein aus Weizen. |
| 21. | SEQ ID NO: 21 : | Nukleinsäuresequenz kodierend für Teil eines BI1 Protein aus Mais. |
| 22. | SEQ ID NO: 22 : | Aminosäuresequenz kodierend für Teil eines BI1 Protein aus Mais. |
| 23. | SEQ ID NO: 23: | Nukleinsäuresequenz kodierend für Teil eines BI1 Protein aus Mais. |
| 24. | SEQ ID NO: 24 : | Aminosäuresequenz kodierend für Teil eines BI1 Protein aus Mais. |
| 25. | SEQ ID No: 25 : | Nukleinsäuresequenz kodierend für Teil eines BI1 Protein aus Weizen. |
| 26. | SEQ ID NO: 26 : | Aminosäuresequenz kodierende für Teil eines BI1 Protein aus Weizen. |
| 27. | SEQ ID NO: 27 : | Nukleinsäuresequenz kodierend für Teil eines BI1 Protein aus Mais. |
| 28. | SEQ ID NO: 28 : | Aminosäuresequenz kodierend für Teil eines BI1 Protein aus Mais. |
| 29. | SEQ ID NO: 29 : | Nukleinsäuresequenz kodierend für den Patatin Promotor aus Kartoffel. |
| 30. | SEQ ID NO: 30 : | Nukleinsäuresequenz kodierend für den Germin 9f-3.8 Promotor aus Weizen. |
| 31. | SEQ ID NO: 31 : | Nukleinsäuresequenz kodierend für den Arabidopsis CAB-2 Promotor |
| 32. | SEQ ID NO: 32 : | Nukleinsäuresequenz kodierend für den PPCZm1 Promotor aus Mais |
| 33. | SEQ ID NO: 33 : | Nukleinsäuresequenz kodierend für rekombinanten Expressionsvektor pUbiBI-1 |
| 34. | SEQ ID NO: 34 : | Nukleinsäuresequenz kodierend für rekombinanten Expressionsvektor pLo114UbiBI-1 |
| 35. | SEQ ID NO: 35 : | Nukleinsäuresequenz kodierend für rekombinanten Expressionsvektor pOXoBI-1 |
| 36. | SEQ ID NO: 36 : | Nukleinsäuresequenz kodierend für rekombinanten Expressionsvektor pLo114OXoBI-1 |
| 37. | SEQ ID NO: 37: | Nukleinsäuresequenz kodierend für BI-1 Protein aus Weizen |
| 38. | SEQ ID NO: 38: | Aminosäuresequenz kodierend für BI-1 Protein aus Weizen |
| 39. | SEQ ID NO: 39: | Nukleinsäuresequnez für PEN1 (= ROR2) aus Gerste |
| 40. | SEQ ID NO: 40: | Aminosäuresequenz kodierend für PEN1 (= ROR2) aus Gerste |
| 41. | SEQ ID NO: 41: | Nukleinsäuresequnez für PEN1 (= ROR2) aus Arabidopsis thaliana |
| 42. | SEQ ID NO: 42: | Aminosäuresequenz kodierend für PEN1 (= ROR2) aus Arabidopsis thaliana |
| 43. | SEQ ID NO: 43: | Nukleinsäuresequenz kodierend für SNAP34 aus Gerste |
| 44. | SEQ ID NO: 44: | Aminosäuresequenz kodierend für SNAP34 aus Gerste |

### Abbildungen

1. Fig. 1a-d: Vergleich von Proteinsequenzen verschiedener BI-1 Proteine aus Pflanzen. AtBI-1: Arabidopsis; BnBI-1: Brassica napus (Raps); GmBI2: Glycine max (Soja; Variante 1); GmBI3: Glycine max (Soja; Variante 2); HVBI-1: Hordeum vulgare (Gerste); NtBI-1: Nicotiana tabacum (Tabak); OsBI-1: Oryza sativa (Reis); TaBI11: Triticum aestivum (Weizen, Variante 1); TaBI18: Triticum aestivum (Weizen, Variante 2); TaBI5 neu: Triticum aestivum (Weizen, Variante 3); ZmBI14: Zea mays (Mais; Variante 1); ZmBI16: Zea mays (Mais; Variante 2); ZmBI33: Zea mays (Mais; Variante 3); ZmBI8: Zea mays (Mais; Variante 4); Consensus: Aus dem Alignment abgeleitete Konsensussequenz.

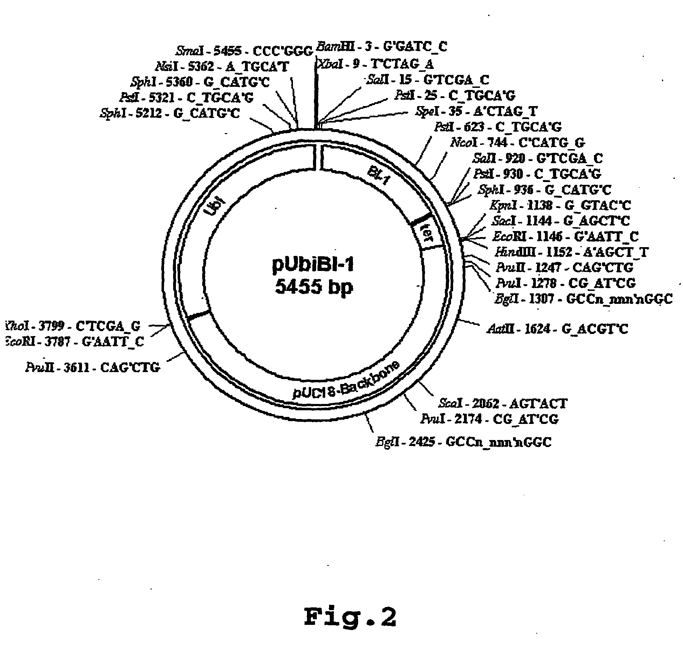

2. Fig. 2: Vektorkarte für Vektor pUbiBI-1 (Ubi: Ubiquitin-Promotor; BI-1 Nukleinsäuresequenz kodierend für Gerste BI1-Protein; ter: Transkriptionsterminator). Angegeben sind ferner die Lokalisation der Schnittstellen verschiedener Restriktionsenzyme.

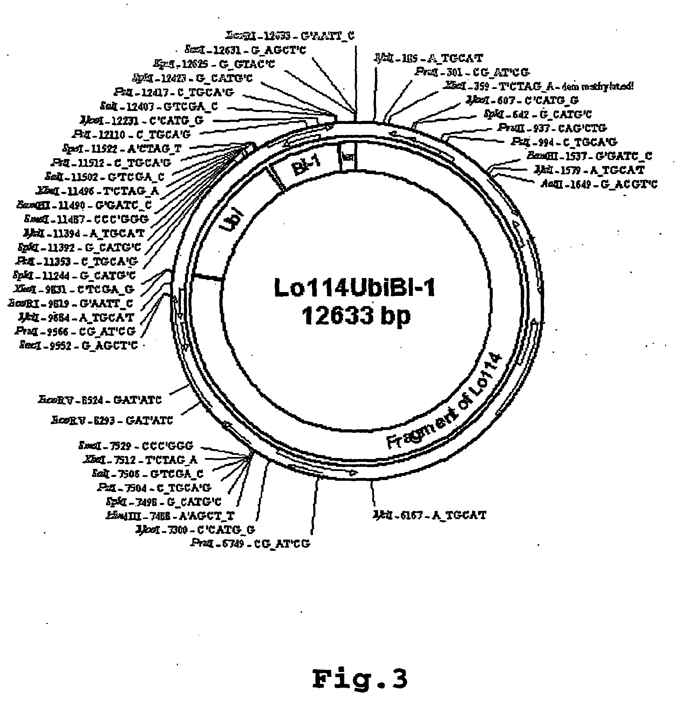

3. Fig. 3: Vektorkarte für Vektor pLO114UbiBI-1 (Ubi: Ubiquitin-Promotor; BI-1 Nukleinsäuresequenz kodierend für Gerste BI1-Protein; ter: Transkriptionsterminator). Angegeben sind ferner die Lokalisation der Schnittstellen verschiedener Restriktionsenzyme.

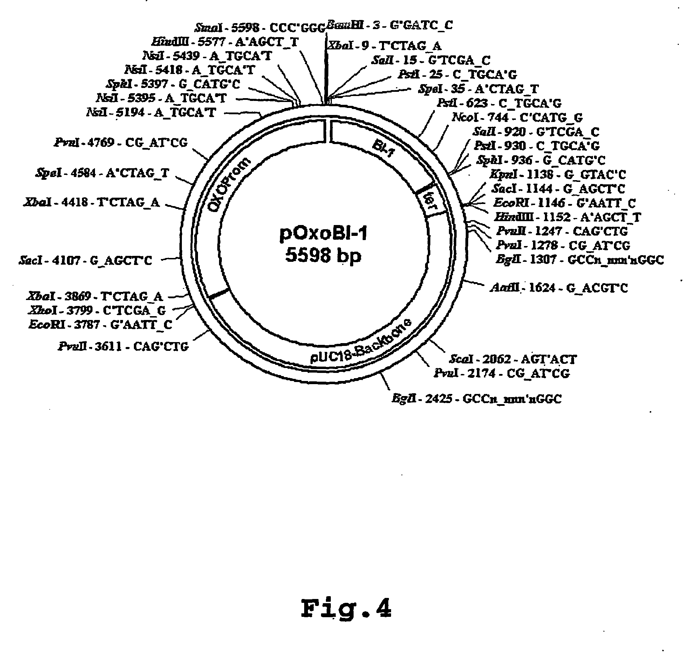

4. Fig. 4: Vektorkarte für Vektor pOxoBI-1 (Oxo: TaGermin 9f-2.8 Promotor; BI-1 Nukleinsäuresequenz kodierend für Gerste BI1-Protein; ter: Transkriptionsterminator). Angegeben sind ferner die Lokalisation der Schnittstellen verschiedener Restriktionsenzyme.

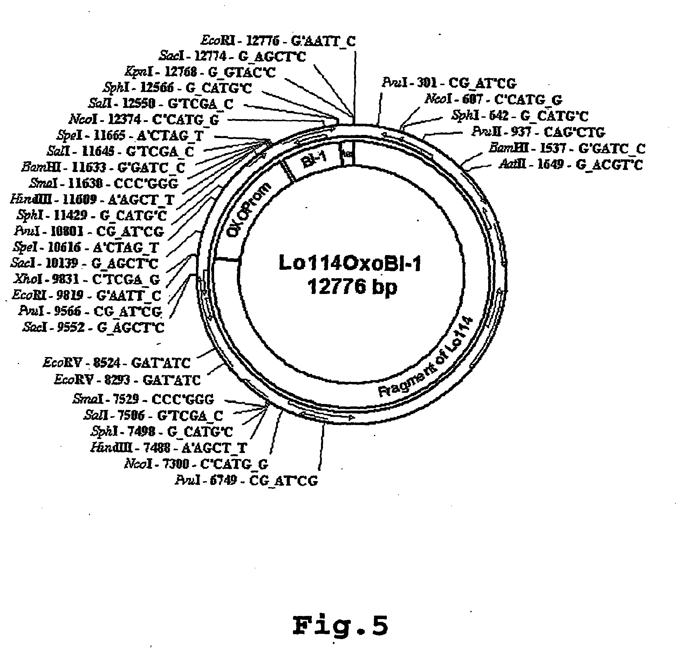

5. Fig. 5: Vektorkarte für Vektor pLO114OxoBI-1 (Oxo: TaGermin 9f-2.8 Promotor; BI-1 Nukleinsäuresequenz kodierend für Gerste BI1-Protein; ter: Transkriptionsterminator). Angegeben sind ferner die Lokalisation der Schnittstellen verschiedener Restriktionsenzyme.
6. Fig. 6: Vergleich der Proteinssequenzen von BI-1 Proteinen aus Gerste (*Hordeum vulgare*, GenBank Acc.-No.: CAC37797), Reis (*Oryza sativa*, GenBank Acc.-No.: Q9MBD8), *Arabidopsis thaliana* (GenBank Acc.-No.: Q9LD45) und Mensch (*Homo sapiens,* GenBank Acc.-No.: AAB87479). Schwarzhinterlegte Aminosäuren sind identisch in allen Arten. Grau hinterlegte Aminosäuren sind nur in Pflanzen identisch. Balken zeigen die vorausgesagten sieben Transmembrandomänen in HvBI-1 an.

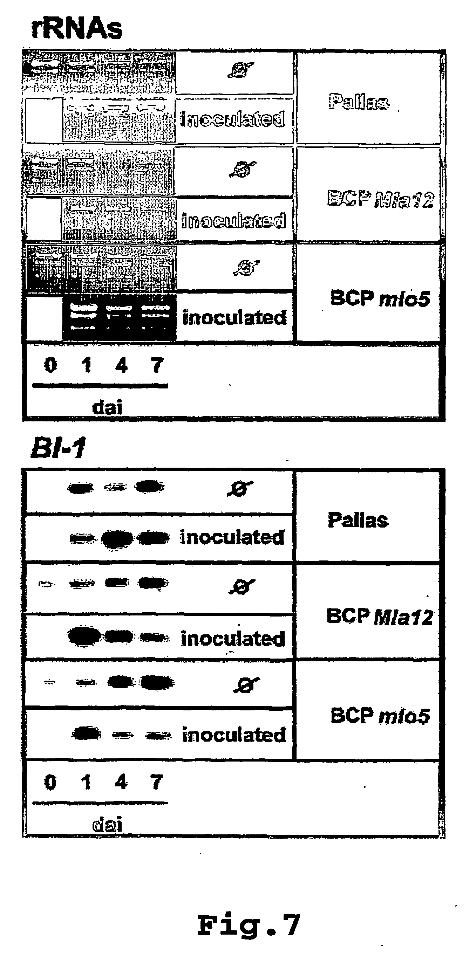

7. Fig. 7: BI-1 Expression in resistenten und suszeptiblen Gersten-Linien (cDNA Gelblot-Analyse): cDNAs wurde mittels RT-PCR ausgehend von Gesamt-RNA synthetisiert. Gesamt-RNA wurde aus suszeptibler Gersten-Linie Pallas, resistenter Gersten-Linie BC*PMla12* und resistenter Gersten-Linie BC*Pmlo*5 zum Zeitpunkt 0 (d.h. unmittlbar vor Inokkulation), sowie jeweils 1, 4 und 7 Tage nach Inokkulation mit *Bgh* und parallel dazu aus nicht-infizierten Kontrollpflanzen (Ø) gewonnen. Die RT-PCR für *BI-1* wurde unter Verwendung von 20 Zyklen ausgeführt (s.u.). Die eingesetzte RNA-Menge (0.5 µg) wurde zusätzlich durch rRNA-Färbung mit Ethidiumbromid in Gelen kontrolliert. Wiederholung der Experimente ergab vergleichbare Resultate.
8. Fig. 8: *BI-1* wird im Mesophyllgewebe exprimiert (cDNA Gelblot-Analyse). RT-PCR wurde ausgehend von RNA isoliert aus Pallas (P) und BC*PM1a12* (P10) (24 h nach Inokulation mit *Bg*hA6) durchgeführt. Für die Extraktion der Gesamt-RNA wurden abaxiale epidermale Streifen (E, inokulierte Positionen der Blätter) vom Mesophyll und der adaxialen Epidermis (M) separiert. *Ubiquitin 1* (*Ubi*) wurde als Marker eine gewebeunspezifischen Genexpression verwendet. RT-PCR wurde unter Verwendung von 30 Zyklen durchgeführt.
9. Fig. 9: *BI-1* Expression wird während chemischer Resistenzinduktion reprimiert.
   (A) chemisch induzierte Resistenz in der Gersten-Linie Pallas gg. Blumeria graminis (DC) Speer f.sp. hordei (Bgh). Gersten Primärblätter wurden mit 2,6-Dichloroisonicotinsäure (DCINA) behandelt und zeigten weniger Mehltau-Pustulen als entsprechende unbehandelte Kontrollpflanzen.
   (B) RNA und cDNA Blots. RNA (10 µg) wurde 0, 1, 2 und 3 Tage nach Bodenbehandlung (soil drench treatment; dpt) mit DCINA bzw. der Kontrolle (Trägersubstanz) und zusätzlich 1 und 4 Tage nach Inokulation (dpi, entspricht 4 bzw. 7 dpt) analysiert. RT-PCR (*Ubi, BI*-1) wurde unter Verwendung von 20 Zyklen realisiert. Wiederholung führte zu vergleichbaren Ergebnissen (siehe Beispiel 2).

   Als Kontrolle wurde BCI-4 eingesetzt. BCI-4 ist ein DCINAinduziertes Gen (Besser et al. (2000) Mol Plant Pahol. 1(5): 277-286) und ein Mitglied der Barley Chemically (=BTH) Induced- Genfamilie.

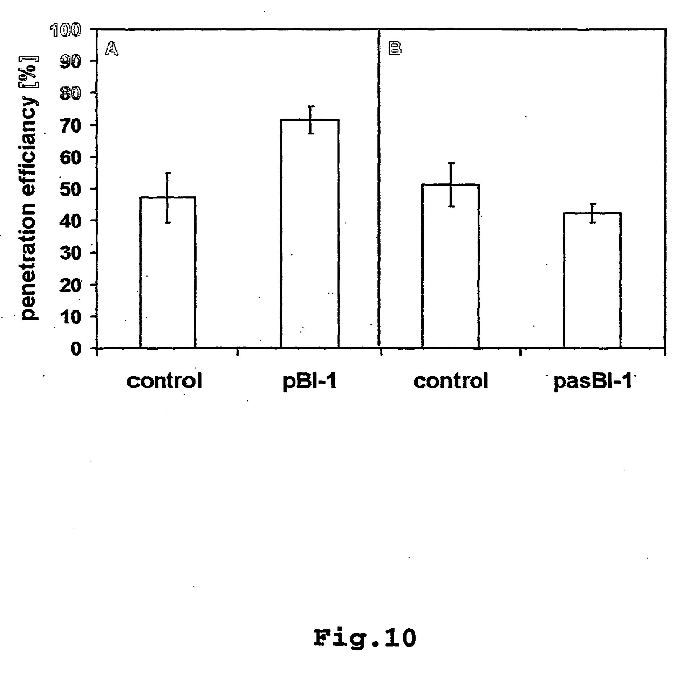

10. Fig. 10: Überexpression von *BI-1* induziert Supersuszeptibilität.
   (A) Durchschnittliche Penetrationseffizienz von *Bgh* in 6 unabhängigen Experimenten mit *Bgh* auf Gersten-Linie Ingrid. PE von *Bgh* war signifikant (p<0.01, Students t-Test) erhöht in Zellen, die mit *pBI-1* transformiert (bombardiert) wurden im Vergleich zu Zellen, die mit der Leervektor-Kontrolle (*pGY1*). bombardiert wurden.
   (B) Die Penetrationseffizienz von *Bgh* auf Zellen die mit einem antisense-*BI-1 Konstrukt* (*pasBI-1*) bombardiert wurden, war nicht-signifikant (p>0.05) vermindert im Vergleich zu Zellen, die mit der Leervektor-Kontrolle (*pGY1*). bombardiert wurden.

   Die Säulen geben jeweils den Mittelwert der einzelnen Experimente wieder. Die Balken stellen den Standardfehler dar.

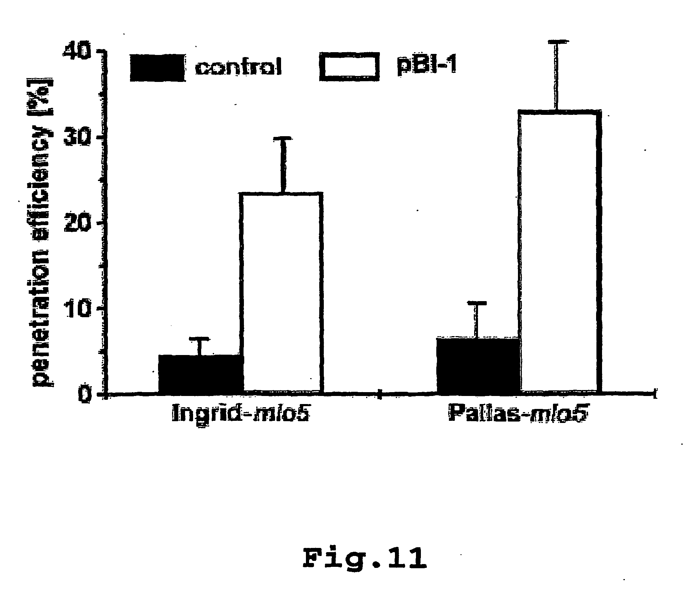

11. Fig. 11: Überexpression von *BI-1* induziert Bruch der *mlo5*-vermittelten Penetrationsresistenz. Penetrationseffizienz von *Bgh* wurde in 3 bis 4 unabhängigen Experimenten mit *Bgh* auf den Gersten Linien Ingrid-*mlo5* bzw. Pallas-*mlo5* bewertet. PE durch *Bgh* war signifikant (p<0.05) erhöht in Zellen, die mit *pBI-1* transformiert (bombardiert) wurden im Vergleich zu Zellen, die mit der Leervektor-Kontrolle (*pG*Y1). bombardiert wurden. Die Säulen geben jeweils den Mittelwert von drei unabhängigen Experimente wieder. Die Balken stellen den Standardfehler dar.

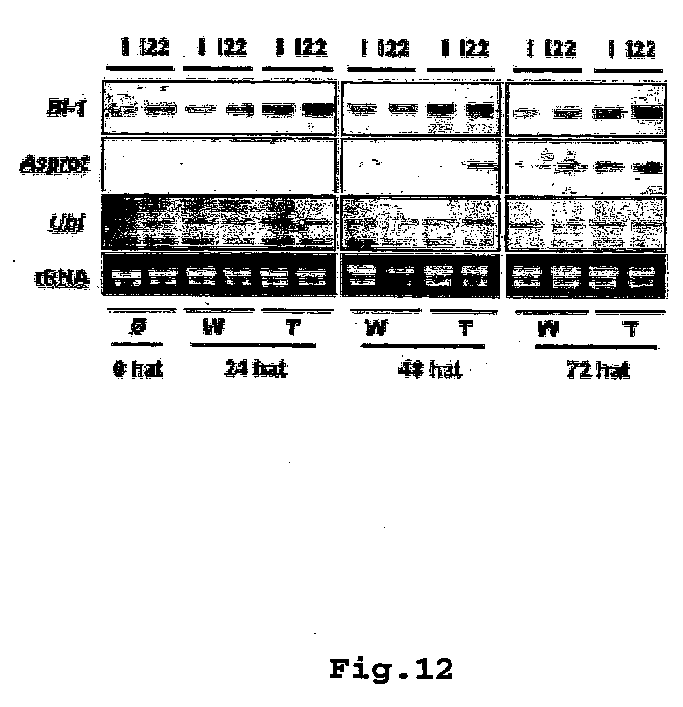

12. Fig. 12: *BI*-*1* Expression wird durch toxische Külturfiltrate aus *Bipolaris sorokiniana* induziert. Northern-Blots (10 µg Gesamt-RNA) mit RNA aus Ingrid (I) und BCIngrid-mlo5 (I22). RNA wurde 0, 24, 48 und 72 h nach Injektion der toxischen Kulturfiltrate von *Bipolaris sorokiniana* (T) bzw. Wasser (W) isoliert. BI-1 mRNAs wurde auf Nylonmembranen nach stringenten Waschen detektiert. BI-1: Detektion von BAX Inhibitor 1 mRNA; *Ubi*: *Detektion von Ubiquitin 1*; *Asprot*: Detection der Aspartatprotease mRNA; hat: Stunden nach Behandlung ("h after treatment")

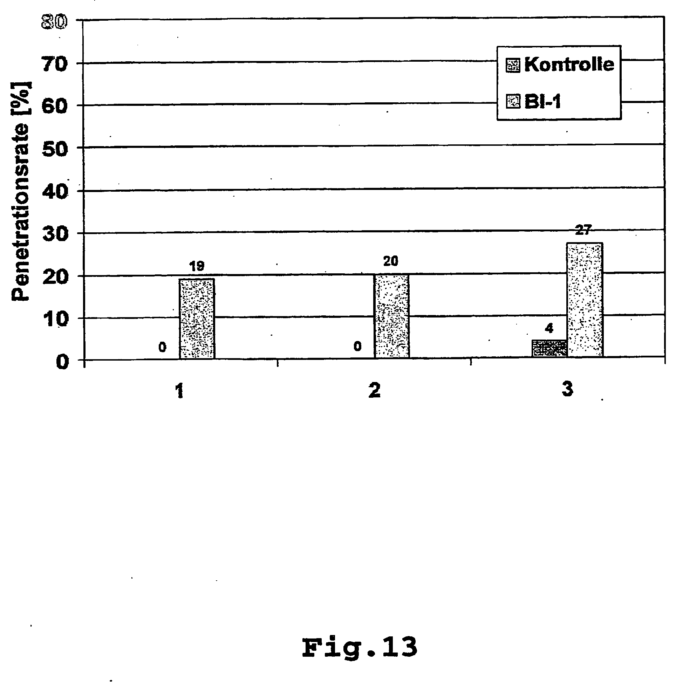

13. Fig. 13: BI-1 Überexpression bricht Nicht-Wirtsresistenz von Gerste (cv. Manchuria) gegen *Blumeria graminis* f.sp. *tritici*. Penetrationsraten wurden in drei unabhängigen Experimenten untersucht.

### Beispiele

### Allgemeine Methoden:

Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die im Rahmen der vorliegenden Erfindung durchgeführten Klonierungsschritte wie z.B. Restriktionsspaltungen, Agarosegelelektrophorese, Reinigung von DNA-Fragmenten, Transfer von Nukleinsäuren auf Nitrozellulose und Nylonmembranen, Verknüpfen von DNA-Fragmenten, Transformation von E. coli Zellen, Anzucht von Bakterien, Vermehrung von Phagen und Sequenzanalyse rekombinanter DNA werden wie bei Sambrook et al. (1989) Cold Spring Harbor Laboratory Press; ISBN 0-87969-309-6 beschrieben durchgeführt. Die Sequenzierung rekombinanter DNA-Moleküle erfolgt mit einem Laserfluoreszenz-DNA-Sequenzierer der Firma MWG-Licor nach der Methode von Sanger (Sanger et al. (1977) Proc Natl Acad Sci USA 74:5463-5467).

### Beispiel 1: Pflanzen, Pathogene und Inokulation

Die Gerstensorten Ingrid, Pallas und die rückgekreuzte Linie BC*PMla12,* BC*Pmlo5* und BCIngrid-*mlo5* (I22) wurde von Lisa Munk, Department of Plant Pathology, Royal Veterinary and Agriculturai University, Kopenhagen, Dänemark zur Verfügung gestellt. Ihre Herstellung ist beschrieben (K⌀lster P et al. (1986) Crop Sci 26: 903-907).

Das 12 bis 36 h im Dunkeln auf feuchtem Filterpapier vorgekeimte Saatgut wurde, wenn nicht anders beschrieben, zu je 5 Körnern an den Rand eines Vierkanttopfes (8x8cm) in Fruhstorfer Erde vom Typ P ausgelegt, mit Erde bedeckt und regelmäßig mit Leitungswasser gegossen. Alle Pflanzen wurden in Klimaschränken oder -klammern bei 18°C und 60 % relativer Luftfeuchte und einem 16-stündigen Licht / 8-stündigen Dunkelheitszyklus mit 3000 bzw. 5000 lux (50 bzw. 60 µmols-¹m-² Photonenflussdichte) 5 bis 8 Tage lang kultiviert und im Keimlingsstadium in den Versuchen verwendet. Bei Experimenten, in denen Applikationen an Primärblättern durchgeführt wurden, waren diese vollständig entwickelt.

Vor Durchführung der transienten Transfektionsexperimente wurden die Pflanzen in Klimaschränken oder -kammern bei tagsüber 24°C, nachts 20°C, 50 bis 60 % relativer Luftfeuchte und einem 16-stündigen Licht / 8-stündigen Dunkelheitszyklus mit 30000 lux kultiviert.

Für die Inokulation von Gerstenpflanzen wurde echter Gerstenmehltau Blumeria graminis (DC) Speer f.sp. hordei Em. Marchal der Rasse A6 (Wiberg A (1974) Hereditas 77: 89-148) (BghA6) verwendet. Dieser wurde vom Institut für Biometrie, JLU Gießen bereitgestellt. Die Nachzucht des Inokulums erfolgte in Klimakammern zu den gleichen Bedingungen, wie sie oben für die Pflanzen beschrieben sind, durch Übertragung der Konidien von befallenem Pflanzenmaterial auf regelmäßig angezogene, 7 Tage alte Gerstenpflanzen cv. Golden Promise bei einer Dichte von 100 Ronidien/mm².

Die Inokulation erfolgte auf primäre Blätter von Gerstenpflanzen mit nachfolgenden Konidien-Dichten: 5 Konidien/ mm² bei chemischer Induktion von Resistenz und makroskopischer Auswertung des Induktionserfolges, 50 Konidien/ mm² bei Genexpressionsstudien und 150 Konidien/ mm² bei Überprüfung der Gentransformation mit transformierten Blattsegmenten. Die Inokulation mit BghA6 erfolgte unter Verwendung von 7 Tagen alten Keimlingen durch Abschütteln der Konidien bereits befalletzer Pflanzen in einem Inokulationsturm(soweit nicht anders angegeben).

### Beispiel 2: Modulation der BI1 Expression mit DCINA

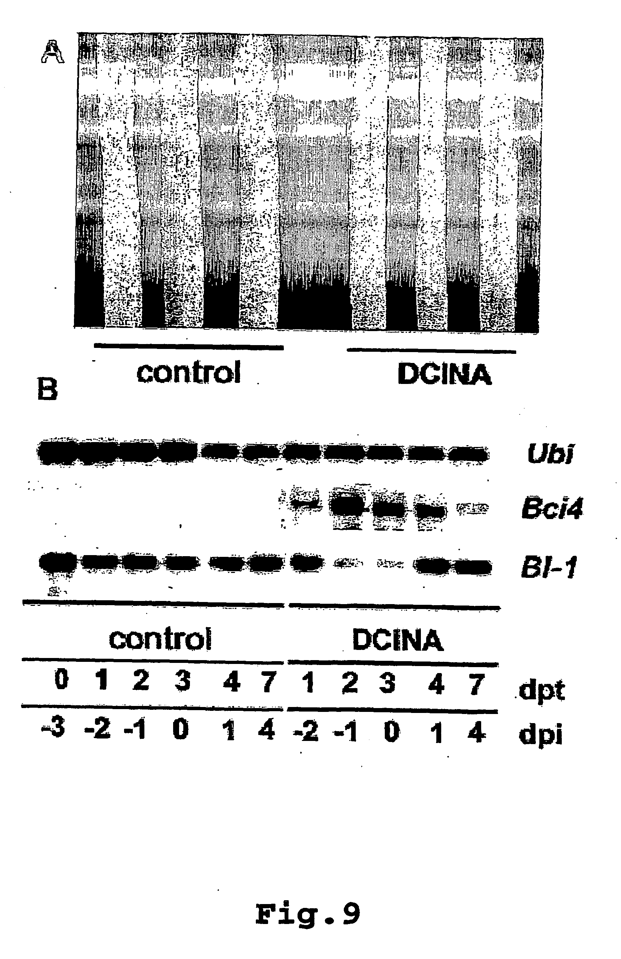

2,6-Dichlorisonikotinsäure (DCINA, Syngenta AG, Basel, Schweiz; als 25% (w/w) Fomulierung) wurde auf 4-Tage alte Gerstenschößlinge der Sorte Pallas mittels Bodenbewässerung ("soil drench") in einer Endkonzentration von 8 mg/l Bodenvolumen appliziert. Die verwendete Suspension wurde mit Leitungswasser angesetzt. Als Kontrolle diente eine Bodenbewässerung mit dem Trägermaterial (benetzbares Puder "wettable powder"). Nach drei Tagen wurden die Pflanzen mit *Blumeria graminis* (DC) Speer f.sp. *hordei* Em. Marchal der Rasse A6 (5 Konidien/ mm²) infiziert. Pflanzen mit chemisch induzierter Resistenz (CIR) wiesen ca. 70% weniger Mehltaukolonien auf als die entsprechenden Kontrollpflanzen, die nur mit der Trägersubstanz behandelt wurden (Fig. 9A).

Northern Blot und RT-PCT Blots wurden zur Bestimmung der *BI1* Transkriptmengen durchgeführt und zeigten eine überraschende Verminderung der *BI1* Expression 1 bis 3 Tage nach chemischer Behandlung (Fig. 9B).

### Beispiel 3: RNA-Extraktion

Gesamt RNA wurde aus 8 bis 10 primären Blattsegmenten (Länge 5 cm) mittels "RNA Extraction Buffer" (AGS, Heidelberg, Germany) extrahiert. Dazu wurden die zentrale Primärblattsegment von 5 cm Länge geerntet und in flüssigem Stickstoff in Mörsern homogenisiert. Das Homogenisat wurde bis zur RNA-Extraktion bei -70°C gelagert. Aus dem tiefgefrorenen Blattmaterial wurde mit Hilfe eines RNA-Extraktions-Kits (AGS, Heidelberg) Gesamt-RNA extrahiert. Dazu wurden 200 mg des tiefgefrorenen Blattmaterials in einem Mikrozentrifugenröhrchen (2 mL) mit 1,7 mL RNA-Extraktionspuffer (AGS) überschichtet und sofort gut durchmischt. Nach Zugabe von 200 µL Chloroform wurde erneut gut gemischt und bei Raumtemperatur 45 min auf einem Horizontalschüttler bei 200 U/min geschüttelt. Anschließend wurde zur Phasentrennung 15 min bei 20000 g und 4°C zentrifugiert, die obere wäßrige Phase in ein neues Mikrozentrifugenröhrchen überführt und die untere verworfen. Die wäßrige Phase wurde erneut mit 900 µL Chloroform gereinigt, indem 3 mal für 10 sec homogenisiert und erneut zentrifugiert (s.o.) und abgehoben wurde. Zur Fällung der DNA wurde dann 850 µL 2-Propanol hinzugegeben, homogenisiert und für 30 bis 60 min auf Eis gestellt. Im Anschluß daran wurde für 20 min zentrifugiert (s.o), vorsichtig der Überstand dekantiert, 2 mL 70 %iges Ethanol (-20°C) hinzu pipettiert, durchmischt und erneut 10 min zentrifugiert. Der Überstand wurde dann wiederum dekantiert und das Pelet vorsichtig mit einer Pipette von Flüssigkeitsresten befreit, bevor es an einem Reinluftarbeitsplatz im Reinluftstrom getrocknet wurde. Danach wurde die RNA in 50 µL DEPC-Wasser auf Eis gelöst, durchmischt und 5 min zentrifugiert (s.o.). 40 µl des Überstandes wurden als RNA-Lösung in ein neues Mikrozentrifugenröhrchen überführt und bei -70°C gelagert.

Die Konzentration der RNA wurde photometrisch bestimmt. Dazu wurde die RNA-Lösung 1:99 (v/v) mit destilliertem Wasser verdünnt und die Extinktion (Photometer DU 7400, Beckman) bei 260 nm gemessen (E_{260 nm} = 1 bei 40 µg RNA/mL). Gemäß der errechneten RNA-Gehalte wurden die Konzentrationen der RNA-Lösungen anschließend mit DEPC-Wasser auf 1 ∝g/∝L angeglichen und im Agarosegel überprüft.

Zur Überprüfung der RNA-Konzentrationen im horizontalen Agarosegel (1% Agarose in 1 x MOPS-Puffer mit 0,2 µg/mL Ethidiumbromid) wurde 1 µL RNA-Lösung mit 1 µL 10 x MOPS, 1 µL Farbmarker und 7 µL DEPC-Wasser versetzt, nach Ihrer Größe bei 120 V Spannung im Gel in 1 x MOPS-Laufpuffer über 1,5 h aufgetrennt und unter UV-Licht fotografiert. Eventuelle Konzentrationsunterschiede der RNA-Extrakte wurden mit DEPC-Wasser ausgeglichen und die Anpassung erneut im Gel überprüft.

### Beipiel 4: Klonierung der BI1 cDNA Sequenz aus Gerste

Der Volllängenklon von hvBI1 (GenBank Acc.-No.: AJ290421) umfaßt am 3'-Ende zwei Stopp-Codons und am 5'-wende ein potentielles Start-Codon. Der ORF überspannt 247 Aminosäuren und zeigt die höchste Sequenzhomologie zu einem BI1-Gen aus Reis, Mais, Brassica napus und Arabidopsis thaliana (jeweils 86% Identität auf Nukleotidebene) sowie einem humanen BI1-Homolog (53% Ähnlichkeit) (Fig. 1 und 6). Die Aminosäuresequenz von hvBI1 umfaßt sieben potentielle Transmembrandomänen mit einer Orientierung des C-Terminus im Cytosol.

Nachfolgende Konstrukte wurden hergestellt:
a) Amplifikation eines 478 bp Fragment der Gerste BI1 cDNA (GenBank Acc.-No.: AJ290421)
   BI1-sense 5'-atggacgccttctactcgacctcg-3'
   BI1-antisense 5'- gccagagcaggatcgacgcc-3'
b) Amplifikation eines 513 bp *Ubi* cDNA Fragment (GenBank Acc.-No.: M60175)
   UBI-sense 5'-ccaagatgcagatcttcgtga-3'
   UBI-antisense 5'-ttcgcgataggtaaaagagca-3'
c) Amplifikation eines 871 bp Volllängen BI1 Leserahmens
   BI1VL sense 5'-ggattcaacgcgagcgcaggacaagc-3'_
   BI1VL antisense 5'-gtcgacgcggtgacggtatctacatg-3'

Die erhaltenen Fragmente wurden in den Vektor pGEM-T mittels T-Überhang-Ligation ligiert und dienten als Ausgangsplasmide für die Herstellung von Sonden (z.B. für Northern-Blot) bzw. dsRNA.

Die einzelnen Konstrukte trugen die Bezeichnung pGEMT-BI1, pGEMT-BI1VL(240) und pGEMT-UBI.

Das BI1-Volllängenprodukt wurde aus pGEMT in die SalI Schnittstelle des pGY-1 vektors (Schweizer, P., Pokorny, J., Abderhalden, O. & Dudler, R. (1999) Mol. Plant-Microbe Interact. 12, 647-654) unter Verwendung der SalI-Schnittstelle in pGEMT und mittels der dem BI1VL antisense Primer angefügten SalI-Schnitstellen umkloniert. Vektoren mit sense (pBI-1) und antisense Orientierung (pasBI-1) wurden isoliert und resequenziert. Die Vektoren enthalten die BI-1 Sequenz unter Kontrolle des CaMV 35S Promotors.

### Beispiel 5: Reverse Transkription - Polymerasekettenreaktion (RT-PCR)

Zum Nachweis von niedrigen Transkriptmengen wurde eine semiquantitative RT-PCR unter Verwendung des "OneStep RT-PCR Kit" (Qiagen, Hilden, Germany) durchgeführt. Dabei wurde RNA (Isolation s.o.) zuerst in cDNA übersetzt (Reverse Transkription) und in einer anschließenden PCR-Reaktion mit spezifischen Primern die gesuchte cDNA amplifiziert. Um die Ausgangsmenge an Matrizen RNA abzuschätzen, wurde die Amplifikation während der exponentiellen Phase (nach 20 Zyklen) unterbrochen um Unterschiede in der Ziel-RNA wiederzuspiegeln. Die PCR Produkte wurden über ein Agarosegel aufgetrennt, denaturiert, auf Nylonmembranen geblottet und mit spezifischen, nicht-radioaktiv-markierten Sonden unter stringenten Standardbedingungen detektiert. Hybridisierung, Waschschritte und Immunodetektion erfolgten wie unter "Northern Blot" beschrieben. Für die einzelnen Reaktionen (25 µL-Ansatz) wurden unter Verwendung des "*One Step RT-PCR Kit*" (Qiagen, Hilden, Deutschland) zusammengegeben:
1000 ng Gesamt-RNA einer bestimmten Probe
0,4 mM dNTPs,
jeweils 0,6 µM sense- und *antisense*-Primer
0,10 µl RNase-Inhibitor
1 µL Enzymmix in 1x RT-Puffer

Die cDNA-Synthese (reverse Transkription) erfolgte für 30 min bei 50°C. Anschließend wurde die Reverse Transkriptase für 15 min bei 95°C inaktiviert, was zugleich Aktivierung der DNA-Polymerase und Denaturierung der cDNA bewirkt. Anschließend folgt eine PCR gemäß nachfolgendem Programm: 1 min mit 94°C; 25 Zyklen mit 1 min mit 94 °C; 1 min mit 54°C und 1 min mit 72°C; abschließend 10 min mit 72°C. Dann Lagerung bei 4°C bis zur Weiterverarbeitung. Die PCR-Produkte wurden im 1xTBE-Agarosegel mit Ethidiumbromid aufgetrennt. Für die einzelnen Ansätze wurden mit den oben angegebenen Primer-Paaren amplifiziert.

### Beispiel 6: Northem-Blot Analyse

Zur Vorbereitung des Northern-Blottings wurde die RNA im Agarosegel unter denaturierenden Bedingungen aufgetrennt. Ein Teil RNA-Lösung (entsprechend 10 µg RNA) wurde dazu mit gleichem Volumen Probenpuffer (mit Ethidiumbromid) gemischt, 5 min bei 94°C denaturiert, 5 min auf Eis gestellt, kurz zentrifugiert und aufs Gel aufgetragen. Das 1 x MOPS-Gel (1,5 % Agarose, ultra pure) enthielt 5 Volumenprozent konzentrierte Formaldehydlösung (36,5 % [v/v]). Die RNA wurde bei 100 V 2 h lang aufgetrennt und anschließend geblottet.

Das Northern-Blotting erfolgte als aufwärtsgerichteter RNA-Transfer im Kapillarstrom. Das Gel wurde dazu zunächst 30 min in 25 mM Natriumhydrogen/dihydrogenphosphat-Puffer (pH 6,5) geschwenkt und zurechtgeschnitten. Ein Whatmanpapier wurde so vorbereitet, dass es auf einer horizontalen Platte auflag und auf 2 Seiten in eine Wanne mit 25 mM Natriumhydrogen/dihydrogenphosphat-Puffer (pH 6,5) ragte. Auf dieses Papier wurde das Gel aufgelegt, wobei nicht bedeckte Teile des Whatmanpapiers mit einer Plastikfolie abgedeckt wurden. Das Gel wurde dann mit einer positiv geladenen Nylonmembran (Boehringer-Mannheim) luftblasenfrei abgedekt, wonach die Membran wiederum mit saugfähigem Papier in mehreren Lagen etwa 5 cm hoch bedeckt wurde. Das saugfähige Papier wurde noch mit einer Glasplatte und einem 100 g Gewicht beschwert. Das Blotting erfolgte über Nacht bei Raumtemperatur. Die Membran wurde kurz in A. bidest. geschwenkt und zur RNA-Fixierung mit einer Lichtenergie von 125 mJ im *Crosslinker* (Biorad) UV-Licht bestrahlt. Die Überprüfung des gleichmäßigen RNA-Transfers auf die Membran erfolgte auf der W-Lichtbank.

Zur Detektion von Gersten mRNA wurden 10 mg Gesamt-RNA aus jeder Probe über ein Agarosegel aufgetrennt und mittels Kapillartransfer auf eine positiv-geladene Nylonmembran geblottet. Die Detektion erfolgte mit dem DIG-Systeme nach Herstellerangaben unter Verwendung von Digoxygenin-markierten antisense-RNA Sonden (wie beschrieben in Hückelhoven R et al. (2001) Plant Mol Biol 47:739-748).

Herstellung der Sonden: Zur Hybridisierung mit den zu detektierenden mRNAs wurden mit Digogygenin oder Fluoreszein markierte RNA Sonden hergestellt. Diese wurden durch *in vitro* Transkription eines PCR-Produktes mittels einer T7 oder SP6 DNA Polymerase mit markierten UTPs erstellt. Als Vorlage für die PCR gestützte Amplifikation dienten die oben beschriebenen Plasmidvektoren pGEMT-BI1, pGEMT-UBI. Je nach Orienttierung des Inserts wurden unterschiedliche RNA-Polymerasen zur Herstellung des antisense-Stranges herangezogen. Die T7-RNA-Polymerase wurde für für pGEMT-BI1 verwendet, die SP6-RNA-Polymerase für pGEMT-UBI. Das Insert der einzelnen Vektor wurde über PCR mit flankierenden Standard-Primern (M13 fwd und rev) amplifiziert. Die Reaktion lief dabei mit folgenden Endkonzentrationen in einem Gesamtvolumen von 50 µL PCR-Puffer (Silverstar) ab:
M13-fwd: 5'-GTAAAACGACGGCCAGTG-3'
M13-Rev: 5'-GGAAACAGCTATGACCATG-3'

10 % Dimethylsulfoxid (v/v)
je 2 ng/µL Primer (M13 *forward* und *reversed*)
1,5 mM MgCl₂,
0,2 mM dNTPs,
4 Units Taq-Polymerase (Silverstar),
2 ng/µL Plasmid-DNA.

Die Amplifikation verlief in einem *Thermocycler* (Perkin-Elmar 2400) temperaturgesteuert mit nachfolgendem Temperaturprogramm: 94°C für 3 min; 30 Zyklen mit 94°C für 30 sek, 58°C für 30 sek und 72°C für 1,2 min; 72°C für 5 min; anschließend Kühlung bei 4°C bis zur Weiterverarbeitung. Der Erfolg der Reaktion wurde im 1 %igen Agarosegel überprüft. Die Produkte wurden anschließend mit einem "*High Pure PCR-Product Purification Kit*" (Boehringer-Mannheim) aufgereinigt. Man erhielt etwa 40 µL Säuleneluat, das erneut im Gel überprüft und bei -20°C gelagert wurde.

Die RNA Polymerisation, die Hybridisierung und die Immunodetektion wurden weitestgehend nach Angaben des Herstellers des Kits zur nicht-radioaktiven RNA-Detektion durchgeführt (*DIG System User's Guide, DIG-Luminescence detection Kit,* Boehringer-Mannheim, Kogel et al. (1994) Plant Physiol 106:1264-1277). 4 µl gereinigtes PCR-Produkt wurden mit 2 µL Transskriptionspuffer, 2 µl NTP-Markierungsrnix, 2 µl-NTP-Mix und 10 µl DEPC-Wasser versetzt. Anschließend wurden 2 µL der T7-RNA-Polymeraselösung zu pipettiert. Die Reaktion wurde dann 2 h bei 37°C durchgeführt und anschließend mit DEPC-Wasser auf 100 µL aufgefüllt. Die RNA-Sonde wurde im Ethidiumbromidgel detektiert und bei -20°C gelagert.

Zur Vorbereitung der Hybridisierung wurden die Membranen zunächst 1 h bei 68°C in 2 x SSC (*Salt, Sodiumcitrate*), 0,1 % SDS-Puffer (Natriumdodecylsulfat) geschwenkt, wobei der Puffer 2 bis 3 mal erneuert wurde. Die Membranen wurden anschließend an die Innenwand auf 68°C vorgeheizter Hybridisierungsröhren angelegt und 30 min mit 10 mL *Dig-Easy-*Hybridisierungspuffer im vorgeheizten Hybridisierungsofen inkubiert. Währenddessen wurden 10 µL Sondenlösung in 80 µL Hybridisierungspuffer bei 94°C für 5 min denaturiert, anschließend auf Eis gestellt und kurz zentrifugiert. Zur Hybridisierung wurde die Sonde dann in 10 mL 68°C warmem Hybridisierungspuffer überführt, und der Puffer in der Hybridisierungsröhre durch diesen Sondenpuffer ersetzt. Die Hybridisierung erfolgte dann ebenfalls bei 68°C über Nacht. Vor Immundetektion von RNA-RNA Hybriden wurden die Blots stringent zweimal für jeweils 20 min in 0.1 % (w/v) SDS, 0.1 x SSC bei 68°C gewaschen. Zur Immunodetektion wurden die Blots zunächst zweimal für 5 min bei RT in 2 x SSC, 0,1 % SDS geschwenkt. Anschließend erfolgten 2 stringente Waschschritte bei 68°C in 0,1 x SSC, 0,1 % SDS für je 15 min. Die Lösung wurde anschließend durch Waschpuffer ohne Tween ersetzt. Es wurde 1 min geschüttelt und die Lösung durch Blocking-Reagenz ausgetauscht. Nach weiteren 30 min Schütteln wurden 10 µL Anti-Fluoreszein-Antikörperlösung hinzugefügt und weitere 60 min geschüttelt. Es folgten zwei 15 minütige Waschschritte in Waschpuffer mit Tween. Die Membran wurde anschließend 2 min in Substratpuffer äquilibriert und nach Abtropfen auf eine Kopierfolie überführt. Auf der "RNA-Seite" der Membran wurde dann ein Gemisch aus 20 µL CDP-Star^{™} und 2 mL Substratpuffer gleichmäßig verteilt. Im Anschluß wurde die Membran mit einer zweiten Kopierfolie abgedeckt und an den Rändern mit Hitze luftblasenfrei und wasserdicht verschweißt. Die Membran wurde dann in einer Dunkelkammer für 10 min mit einem Röntgenfilm bedeckt und dieser anschließend entwickelt. Je nach Stärke der Lumineszenzreaktion wurde die Belichtungszeit variiert.

Wenn nicht extra gekennzeichnet waren die Lösungen im Lieferumfang des *Kits* enthalten (*DIG-Luminescence detection Kit,* Boehringer-Mannheim). Alle anderen wurden aus folgenden Stammlösungen durch Verdünnung mit autoklaviertem, destilliertem Wasser hergestellt. Alle Stammlösungen wurden, wenn nicht anders spezifiziert, mit DEPC (wie DEPC-Wasser) angesetzt und anschließend autoklaviert.
- DEPC-Wasser: Destilliertes Wasser wird über Nacht bei 37°C mit Diethylpyrokarbonat (DEPC, 0,1 %, w/v) behandelt und anschließend autoklaviert
- 10 x MOPS-Puffer: 0,2 M MOPS (Morpholin-3-propansulfonsäure), 0,05 M Natriumacetat, 0,01 M EDTA, pH mit 10 M NaOH auf pH 7,0 eingestellt
- 20 x SSC (Natriumchlorid-Natriumzitrat, *Salt-Sodiumcitrate*): 3 M NaClo, 0,3 M triNatriumcitrat x 2 H₂O, pH mit 4 M HCl auf pH 7,0 eingestellt.
- 1% SDS (Natriumdodecylsufat, *Sodiumdodecylsulfate*)Natriumdodecylsulfat (w/v), ohne DEPC
- RNA-Probenpuffer: 760 µL Formamid, 260 µL Formaldehyd, 100 µL Ethidiumbromid (10 mg/mL), 80 µL Glycerol, 80 µL Bromphenolblau (gesättigt), 160 µL 10 x MOPS, 100 µL Wasser.
- 10 x Waschpuffer ohne Tween: 1,0 M Maleinsäure, 1,5 M NaCl; ohne DEPC, mit NaOH (fest, ca. 77 g) und 10 M NaOH auf pH 7,5 einstellen.
- Waschpuffer mit Tween: aus Waschpuffer ohne Tween mit Tween (0,3 %, v/v)
- 10 x Blockingreagenz: 50 g Blockingpulver (Boehringer-Mannheim) in 500 mL Waschpuffer ohne Tween suspendieren.
- Substratpuffer:100 mM Tris (Trishydroxymethylamino-methan), 150 mM NaCl mit 4 M HCl auf pH 9,5 einstellen.
- 10 x Farbmarker: 50 % Glycerol (v/v), 1,0 mM EDTA pH 8,0, 0,25 % Bromphenolblau (w/v), 0,25 % Xylencyanol (w/v).

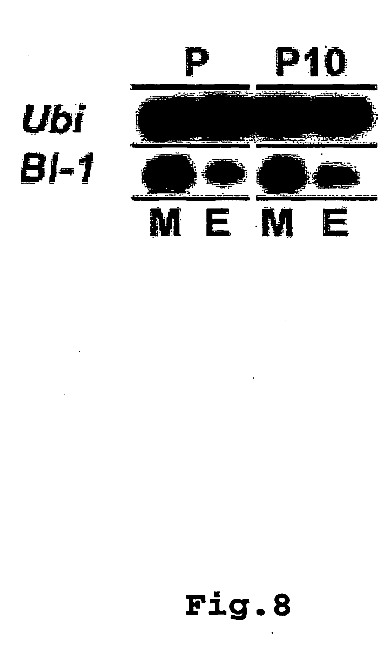

Eine BI1 Expression wurde wie beschrieben mit RT-PCR und cDNA Gelblots untersucht und ergab, dass BI1 überwiegend im Mesophyllgewebe von Blättern exprimiert wird, während Ubiquitin konstitutiv gleichmäßig in Epidermis und Mesophyll exprimiert wird (Fig. 8).

Ferner ist eine Expression von BI1 als Reaktion auf Behandlung der Pflanzen mit toxischen ICulturfiltraten von Bipolaris sorokiniana zu beobachten. Primärblätter der Gerste zeigen typische nekrotische Flecke (leaf spot blotch symptoms) nach Behandlung der Pflanzen mit toxischen Kulturfiltraten von Bipolaris sorokiniana (durchgeführt wie bei Kumar et al. 2001 beschrieben). Die Blattnekrosen waren erkennbar 48 h nach Behandlung. Der beobachtete Gewebeschaden war in der Bgh-resistenten Linie BCIngrid-mlo5 (I22) deutlicher ausgeprägt als in der Elterlinie Ingrid (Mlo-Genotype. Kumar et al. 2001). Die Expression von BI1 korreliert 72 h nach Behandlung (hat) mit der Ausprägung der Blattnekrosen (Fig. 12).

### Beispiel 7:

Die zur stabilen, mesophyll-spezifischen Überexpression wird der Oxalat-Oxidase Promoter (germin 9f-2.8) aus Weizen eingesetzt. In Gerste ist die entsprechende Oxalat-Oxidase Expression Mesophyll-spezifisch, schwach konstitutiv und Pathogen-responsiv (Gregersen PL et al. (1997) Physiol Mol Plant Pathol 51: 85-97). Er kann deshalb zur Mesophyll-spezifischen Expression von BI1-genutzt werden. Zur Kontrolle wird HVBI1 unter Kontrolle des Mais-Ubiquitinpromoters (Christensen AH et al. (1992) Plant Mol Biol 18:675-689) oder des Reis-Aktinpromoters überexprimiert (Zhang W et al. (1991) Plant Cell 3:1155-1165). Eingesetzt werden nachfolgende Konstrukte:
a) pUbiBI-1 (SEQ ID NO: 33;für transiente Gerstentransformation und Weizentransformation mit Partikel Bombardement. Expression von BI-1 unter Kontrolle des Mais Ubiquitin Promotors).
b) pLo114UbiBI-1 (SEQ ID NO: 34; erhalten durch Umklonierung der Ubi/BI-1 Expressionscassette als EcoR1-Fragment aus pUbiBI-1 in pLo114-GUS-Kan; Binärer Vektor für transiente Gerstentransformation mit *A. tumefaciens*)
c) pOXoBI-1 (SEQ ID NO: 35; Mesophyllspezifischer TaGermin 9f-2.8 Promoter vor BI1 zur Weizentrafo über Patikelbombardement.
d) pLo114OXoBI-1 (SEQ ID NO: 36)

Es werden Wildtypgerste und Weizen sowie mlo-Gerste transformiert, vermehrt und geselbstet. Die Transformation von Gerste und Weizen erfolgt wie beschrieben (Repellin A et al. (2001) Plant Cell, Tissue and Organ Culture 64: 159-183): Dazu werden Kalli aus unreifen Weizen- (bzw. Gersten-) embryonen über biolistischen Gentransfer mit Mikroprojektilen transformiert. Dabei werden pUC basierte Vektoren zusammen mit Vektoren die Selektionsmarker tragen kotransformiert. Anschließend werden die Embryonen auf Selektionsmedium kultiviert und regeneriert. Gerste wird mit Hilfe von *Agrobacterium tumefaciens* transformiert. Dabei wird ein binärer Vektor auf Basis von pcambia_1301 eingesetzt. Unreife Embryonen von Gerste werden mit *A. tumefaciens* cokultiviert, selektiert und anschließend regeneriert (Repellin A et al. (2001) Plant Cell, Tissue and Organ Culture 64: 159-183; Horvath H et al. (2003) Proc Natl. Acad Sci USA 100: 365-369; Horvath H et al. (2002) in Barley Science, eds. Slafer, G. A., Molina-Cano, J. L., Savin, R., Araus, J. L. & Romagosa, J. (Harworth, New York), pp. 143-1.76; Tingay S et al. (1997) Plant J. 11: 1369-1376).

Die transgenen (rekombinanten) Gersten- und Weizenpflanzen der T1 oder T2-Generation werden auf Resistenz gegenüber hemibiotrophen und perthotrophen Erregern geprüft. Dazu werden die Blätter mit verschiedenen Pathogenen inokuliert. Als biotrophe Erreger werden Gerstenmehltau (*Blumeria graminis* f.sp. *hordei*) und Braunrost (*Puccinia hordei*) verwendet. Als Maß der Mehltauanfälligkeit wird die Pustelzahl pro Blattfläche 5-7 Tage nach Inokulation mit 2-5 Konidien pro mm² Blattfläche ausgewertet (Beßer K et al. (2000) Mol Plant Pathology 1: 277-286). Als hemibiotrophe Erreger werden *Bipolaris sorokiniana* n und *Magnaporthe grisea* verwendet. Die Inokulation erfolgt wie zuvor beschrieben (Kumar J et al. (2001) Phytopathology 91: 127-133; Jarosch B et al. (1999) Mol Plant Microbe Inter 12: 508-514). Als Maß für die Anfälligkeit dient die Anzahl und Größe der Blattläsionen 2 bis 6 Tage nach Sprühinokulation mit Konidien (Kumar J et al. (2001) Phytopathology 91:127-133; Jarosch B et al. (1999) Mol Plant Microbe Inter 12:508-514; Jarosch B et al. (2003) Mol Plant Microbe Inter 16:107-114.). Als perthotropher Erreger wird *Fusarium graminearum*, verwendet.

Zur Bestimmung der Fusarium Head Blight (FHB) Typ 1-Resistenz werden Weizenähren in einem frühen Blühstadium mit einer Makrokonidien-Suspension (ca. 2 x 10⁵ml⁻¹) von *Fusarium graminearum* bzw. *Fusarium culmorcum* besprüht*.* Die inokulierten Pflanzen werden für 3 Tage in eine Nebenkammer mit 25°C Lufttemperatur und 100% relativer Luftfeuchte transferriert. Danach werden die Pflanzen im Gewächshaus unter Dauerlicht bei einer Temperatur von 20°C inkubiert und die Stärke der FHB-Symptome über die Ähre hinweg nach 5, 7 und 8 Tagen evaluiert.

Zur Quantifizierung der Fusarium Head Blight (FHB) Typ II-Resistenz werden jeweils 10 - 20 µl Aliquots einer Makrokonidien-Suspension (ca. 2 x 10⁵ml⁻¹) von *Fusarium graminearum* bzw. *Fusarium culmorum* in einzelne, relativ zentral gelegene Ährchen von Weizenpflanzen injiziert. Die inokulierten Pflanzen werden für 3 Tage in eine Nebelkaamner mit 25°C Lufttemperatur und 100% relativer Luftfeuchte transferriert. Danach werden die Pflanzen im Gewächshaus unter Dauerlicht bei einer Temperatur von 20°C inkubiert und die Ausbreitung der FHB-Symptome über die Ähre hinweg nach 7, 14 und 21 Tagen evaluiert. Die Ausbreitung der Symptome über die Ähre (das sog. Fusarium-Spreading) wird als Mass für die FHB Typ II-Resistenz genommen.

### Vergleichsbeispiel 1: Transiente BI1 Expression in der Epidermis und Evaluation der Pilzpathogenentwicklung

Gerste cv Ingrid Blattsegmente wurden mit einer pGY-BI1 zusammen mit einem GFP-Expressionsvektor transformiert. Anschließend wurden die Blätter mit Bgh inokuliert und das Ergebnis nach 48 h mittels Licht- und Fluoreszenzmikroskopie analysiert. Die Penetration in GFP-exprimierenden Zellen wurde mittels Detektion von Haustorien in lebenden Zellen und durch Bewertung der Pilzentwicklung aufin eben diesen Zellen beurteilt. Es wurde ein Verfahren zur transienten Transformation eingesetzt, das bereits für die biolistische Einführung von DNA und RNA in epidermale Zellen von Gerstenblättern beschrieben wurde (Schweizer P et al. (1999) Mol Plant Microbe Interact 12:647-54; Schweizer P et al. (2000) Plant J 2000 24:895-903).

Für Microcarrier-Präparation wurden 55 mg Wolframpartikel (M 17, Durchmesser 1,1 µm; Bio-Rad, München) zweimal mit 1 ml autoklaviertem Destilliertem Wasser und einmal mit 1 mL absolutem Ethanol gewaschen, getrocknet und in 1 ml 50 %igem Glycerin aufgenommen (ca. 50 mg/ml Stammlösung). Die Lösung wurde mit 50%igem Glycerin auf 25 mg/ml verdünnt, vor Gebrauch gut gemischt und im Ultraschallbad suspendiert.

Zur Microcarrier-Beschichtung wurden pro Schuß 0,3 µg Plasmid *pGFP* (GFP unter Kontrolle des CaMV 35S Promotors; Schweizer P et al. (1999) Mol Plant-Microbe Interact 12:647-654.), 0,7 µg Leervektor pGY bzw. pGY-BI1 (1 µL), 12,5 µl Wolframpartikel-Suspension (25 mg/ml; entsprechend 312 µg Wolframpartikel), 12,5 µl 1 M Ca(NO3)2-Lösung (pH 10) tropfenweise unter ständigem Mischen zusammengegeben, 10 min bei RT stehengelassen, kurz zentrifugiert und 20 µl vom Überstand abgenommen. Der Rest mit den Wolframpartikel wird resuspendiert (Ultraschallbad) und ins Experiment eingesetzt.

Es wurden ca. 4 cm lange Segmente von Gerstenprimärblättern verwendet. Die Gewebe wurden auf 0,5 % Phytagar (GibcoBRL™ Life Technologies™, Karlsruhe) mit 20 µg/ml Benzimidazol in Petrischalen (6,5 cm Durchmesser) gelegt und direkt vor dem Partikelbeschuss an den Rändern mit einer Schablone mit einer rechteckigen Aussparung von 2,2 cm x 2,3 cm abgedeckt. Die Schalen wurden nacheinander auf den Boden der Vakuumkammer (Schweizer P et al. (1999) Mol Plant Microbe Interact 12:647-54) gestellt, über dem ein Nylonnetz (Maschenweite 0,2 mm, Millipore, Eschborn) als Diffusor auf einer Lochplatte eingeschoben war (5 cm über dem Boden, 11 cm unterhalb des Macrocarriers, s.u.), um Partikelklumpen zu zerstreuen und den Partikelstrom abzubremsen. Der oben an der Kammer angebrachte Macrocarrier (Plastik-Sterilfilterhalter, 13 mm, Gelman Sciences, Swinney, UK) wurde je Schuss mit 5,8 µL DNA-beschichteten Wolframpartikeln (Microcarrier, s.u.) beladen. Mit einer Membranvakuumpumpe (Vacuubrand, Wertheim) wurde der Druck um 0,9 bar in der Kammer reduziert und die Wolframpartikel mit 9 bar Heliumgasdruck auf die Oberfläche des Pflanzengewebes geschossen. Sofort danach wurde die Kammer belüftet. Zur Markierung transformierter Zellen wurden die Blätter mit dem Plasmid (pGFP; Vektor auf pUC18-Basis, CaMV 35S-Promoter/Terminator-Kassette mit insertiertem GFP-Gen; Schweizer P et al. (1999) Mol Plant Microbe Interact 12:647-54; zur Verfügung gestellt von Dr. P. Schweizer, Institut für Pflanzengenetik IPK, Gatersleben, Deutschland) beschossen. Vor dem Schießen eines anderen Plasmids wurde der Macrocarrier jeweils gründlich mit Wasser gereinigt. Nach vierstündiger Inkubation nach dem Beschuß bei leicht geöffneten Petrischalen, RT und Tageslicht wurden die Blätter mit 100 Konidien/mm² des Echten Gerstenmehltaupilzes (Rasse A6; *Blumeria graminis f.sp. hordei* Mehltau A6) inokuliert und für weitere 40 h unter gleichen Bedingungen inkubiert. Anschließend wurde die Penetration ausgewertet. Das Ergebnis (z.B. die Penetrationseffizienz, definiert als prozentualer Anteil angegriffener Zellen, die ein mit reifems Haustorium und einer Sekundärhyphae ("secondary elongating hyphae"), wurde mittels Fluoreszens- und Lichtmikroskopie analysiert. Eine Inokulation mit 150 Conidia/mm² ergibt eine Angriffsfrequenz von ca. 50 % der transformierten Zellen. Für jedes einzelne Experiment wurde eine minimale Anzahl von 100 Interaktionsstellen ausgewertet. Transformierte (GFP exprimierenden) Zellen wurden unter Anregung mit blauem Licht identifiziert. Drei verschiedene Kategorien von transformierten Zellen konnten unterschieden werden:
1 Penetrierte Zellen, die ein leicht erkennbares Haustorium beinhalten. Eine Zelle mit mehr als einem Haustorium wurde als eine Zelle gewertet.
2. Zellen, die durch ein Pilz-Appressorium zwar angegriffen wurden, aber kein Haustorium beinhalten. Eine Zelle die mehrfach von Bgh angegriffen wurden, aber kein Haustorium enthält, wurde als eine Zelle gewertet.
3. Zellen die nicht durch Bgh angegriffen sind.

Stomatazellen und Stomatanebenzellen wurden von der Bewertung ausgeschlossen. Oberflächenstrukturen von Bgh wurden mittels Lichtmikroskopie oder Fluoreszenzfärbung des Pilzes mit 0,3 % Calcofluor (w/v in Wasser) für 30 sec analysiert. Die Entwicklung des Pilzes kann leicht durch Fluoreszenzmikroskopie nach Anfärbung mit Calcofluor evaluiert werden. In BI1-dsRNA transformierten Zellen entwickelt der Pilz zwar ein primäres und ein appressoriales Keimschlauch ("Germ-Tube") aber kein Haustorium. Haustoriumausbildung ist eine Vorbedingung für die Bildung einer Sekundärhyphae.
Die Penetrationseffizien (Penetrationsraten) errechnet sich als Anzahl der penetrierten Zellen durch Anzahl der attackierten Zellen multipliziert mit 100.

Die Penetrationseffizienz dient der Bestimmung des Suszeptibilität von Zellen, die mit pGY-BI1 transfiziert sind im Vergleich zu Zellen die mit einer Leervektorkontrolle transformiert sind (Fig. 10). Es wurde beobachtet, dass die BI1 Überexpression die Penetrationshäufigkeit von Bgh signifikant erhöht (Fig. 10). In sechs unabhängigen Experimenten bewirkte die Überexpression in der suszeptiblen Gerstensorte Ingrid eine signifikante Erhöhung der durchschnittlichen Penetrationseffizienz (PE) von 47 % auf 72 % (165 % der Kontrollen) bei Zellen die BI1 überexprimieren im Vergleich zu Zellen, die mit Leervektor transformiert wurden (Kontrolle) (Fig. 10).

Ferner wurden epidermale Zellen der *Bg*h-resistenten mlo5-Gerste mit dem BI1 Überexpressionskonstrukt pGY-1 wie oben beschrieben transient transformiert. Der mlo5-Genotyp in einem Pallas bzw. Ingrid Hintergrund zeigt eine geringfügige Anfälligkeit gegen *Bg*h. In 7 unabhängigen Experimenten wurde in Kontrollpflanzen (Transformation mit Leervektor und GFP-Vektor) eine Penetrationseffizienz von minimal 0 bis maximal 11 % gefunden. Überraschenderweise ergab eine *BI1* Überexpression (pGY-BI1) eine nahezu vollständige Rekonstitution der suszeptiblen Phänotyps, d.h. es erfolgte ein nahezu kompletter Bruch der mlo-Resistenz. Die durchschnittliche Penetrationseffizienz von Bgh auf Ingrid-*mlo5* und Pallas-*mlo5* Blattsegmenten steigt von 4 % auf 23 %, bzw. von 6 % auf 33 % (Fig. 11). Dies bedeutet einen relativen Anstieg der Penetration auf 520 % bzw. 510 % der Kontrollen. Desweiteren erhöhte die Überexpression von BI1 im Gerste cv Manchuria die Anfälligkeit gegen das Weizenpathogen *Blumeria graminis* f.sp. *tritici* in drei unabhängigen Experimenten von 0 bis 4 % auf 19 bis 27 % (Fig. 13).

## Patentansprüche

1. Verfahren zur Erzeugung oder Erhöhung der Resistenz gegen mindestens einen biotischen Streßfaktor in Pflanzen, wobei nachfolgende Arbeitsschritte umfasst sind
a) Erhöhung der Proteinmenge mindestens eines Bax Inhibitor-1 (BI1) Proteins in mindestens einem pflanzlichen Gewebe mit der Maßgabe, dass die Expression in der Blattepidermis im wesentlichen unverändert bleibt oder reduziert wird, und
b) Auswahl der Pflanzen, bei denen im Vergleich zur Ausgangspflanze eine Resistenz gegen mindestens einen biotischen Stressfaktor besteht oder erhöht ist;
und
wobei der Streßfaktor ein pflanzliches Pathogen ist.

2. Verfahren nach Anspruch 1, wobei der Streßfaktor ein nekrotrophes oder hemibiotrophes Pathogen ist.

3. Verfahren nach einem der Ansprüche 1 bis 2, wobei das BI-1 Protein mindestens eine Sequenz umfaßt, die eine Homologie von mindestens 50% aufweist zu mindestens einem BI1-Konsensusmotiv ausgewählt aus der Gruppe bestehend aus
a) H (L/I) KXVY,
b) AXGA (Y/F) XH,
c) NIGG,
d) P (V/P) (Y/F) E (E/Q) (R/Q) KR,
e) (E/Q) G (A/S) S (V/I) GPL,
f) DP(S/G)(L/I)(I/L),
g) V (G/A) T (A/S) (L/I) AF (A/G) CF (S/T),
h) YL (Y/F) LGG,
i) L (L/V) SS (G/W) L (S/T) (I/M) L (L/M) W, und
j) DTGX(I/V) (I/V)E.

4. Verfahren nach einem der Ansprüche 1 bis 3, wobei das BI1-Protein kodiert wird durch ein Polypeptid, das mindestens eine Sequenz umfaßt ausgewählt aus der Gruppe bestehend aus:
a) den Sequenzen gemäß SEQ ID NO: 2, 4, 6, 8, 10, 12, 14, 16, 18, 20, 22, 24, 26, 28, 30, 32 oder 38, und
b) Sequenzen, die eine Identität von mindestens 50% zu einer der Sequenzen gemäß SEQ ID NO: 2, 4, 6, 8, 10, 12, 14, 16, 18, 20, 22, 24, 26, 28, 30, 32 oder 38 aufweisen,
c) Sequenzen die mindestens eine Teilsequenz von mindestens 10 zusammenhängenden Aminosäureresten einer der Sequenzen gemäß SEQ ID NO: 2, 4, 6, 8, 10, 12, 14, 16, 18, 20, 22, 24, 26, 28, 30, 32 oder 38 umfassen.

5. Verfahren nach einem der Ansprüche 1 bis 4, wobei die Erhöhung der Proteinmenge mindestens eines BI1-Proteins durch rekombinante Expression des besagten BI1-Proteins unter Kontrolle eines wurzel-, knollen- oder mesophyll-spezifischen Promotors realisiert wird.

6. Verfahren nach einem der Ansprüche 1 bis 5, umfassend
(a) stabile Transformation einer pflanzlichen Zelle mit einer rekombinanten Expressionskassette enthaltend eine für ein BI-Protein kodierende Nukleinsäuresequenz in funktioneller Verknüpfung mit einem gewebespezifischen Promotor, wobei der Promotor im wesentlichen keine Aktivität in der Blattepidermis aufweist und wobei der Promotor in Bezug auf die besagte das BI-Protein kodierende Nukleinsäuresequenz heterolog ist,
(b) Regeneration der Pflanze aus der pflanzlichen Zelle, und
(c) Expression besagter für ein BI-Protein kodierende Nukleinsäuresequenz in einer Menge und für eine Zeit hinreichend um eine Pathogenresistenz in besagter Pflanze zu erzeugen oder zu erhöhen.

7. Verfahren nach einem der Ansprüche 1 bis 6, wobei die Pflanze aus den monokotyledonen und dikotyledonen Pflanzen ausgewählt ist.

8. Verfahren nach einem der Ansprüche 1 bis 7, wobei die Pflanze ausgewählt ist aus der Gruppe der monokotyledonen Pflanzen bestehend aus Weizen, Hafer, Hirse, Gerste, Roggen, Mais, Reis, Buchweizen, Sorghum, Triticale, Dinkel, Leinsamen oder Zuckerrohr.

9. Verfahren nach einem der Ansprüche 1 bis 8, wobei die Expression des Bax Inhibitor-1 (BI-1) im Mesophyll erhöht wird.

10. Verfahren nach einem der Ansprüche 1 bis 9, wobei die Pflanze einen mlo-resistenten Phänotyp aufweist, oder die Expression oder Funktion von MLO, RacB und/oder NaOx mindestens in der Epidermis inhibiert oder im Vergleich zu einer Kontrollpflanze reduziert ist und/oder die Expression oder Funktion von PEN2, SNAP34 und/oder PEN1 mindestens in der Epidermis erhöht wird im Vergleich zu einer Kontrollpflanze.

## Claims

1. A method for generating or increasing the resistance, in plants, to at least one biotic stress factor, comprising the following steps:
a) increasing the amount of protein of at least one Bax inhibitor-1 (BI1) protein in at least one plant tissue with the proviso that the expression in the leaf epidermis remains essentially unchanged or is reduced, and
b) selection of the plants in which, in comparison with the starting plant, a resistance to at least one biotic stress factor exists or is increased;
and
the stress factor being a plant pathogen.

2. The method according to claim 1, wherein the stress factor is a necrotrophic or hemibiotrophic pathogen.

3. The method according to claim 1 or 2, wherein the BI-1 protein comprises at least one sequence which has at least 50% homology with at least one BI1 consensus motif selected from the group consisting of
a) H(L/I)KXVY,
b) AXGA(Y/F)XH,
c) NIGG,
d) P(V/P) (Y/F) E (E/Q) (R/Q) KR,
e) (E/Q)G(A/S)S(V/I)GPL,
f) DP(S/G)(L/I)(I/L),
g) V(G/A)T(A/S)(L/I)AF(A/G)CF(S/T),
h) YL(Y/F)LGG,
i) L(L/V)SS(G/W)L(S/T)(I/M)L(L/M)W, and
j) DTGX(I/V)(I/V)E.

4. The method according to any of claims 1 to 3, wherein the BI-1 protein is encoded by a polypeptide comprising at least one sequence selected from the group consisting of:
a) the sequences as shown in SEQ ID NO: 2, 4, 6, 8, 10, 12, 14, 16, 18, 20, 22, 24, 26, 28, 30, 32 and 38, and
b) sequences which have at least 50% identity with one of the sequences as shown in SEQ ID NO: 2, 4, 6, 8, 10, 12, 14, 16, 18, 20, 22, 24, 26, 28, 30, 32 and 38,
c) sequences which comprise at least one part-sequence of at least 10 contiguous amino acid residues of one of the sequences as shown in SEQ ID NO: 2, 4, 6, 8, 10, 12, 14, 16, 18, 20, 22, 24, 26, 28, 30, 32 and 38.

5. The method according to any of claims 1 to 4, wherein the increase in the protein quantity of at least one BI1 protein is effected by recombinant expression of said BI1 protein under the control of a root-, tuber- or mesophyll-specific promoter.

6. The method according to any of claims 1 to 5, comprising the
(a) stable transformation of a plant cell with a recombinant expression cassette comprising a nucleic acid sequence coding for a BI protein in functional linkage with a tissue-specific promoter, the promoter having essentially no activity in the leaf epidermis and the promoter being heterologous with regard to said nucleic acid sequence which codes for the BI protein;
(b) regeneration of the plant from the plant cell; and
(c) expression of said nucleic acid sequence which codes for a BI protein in an amount and for a period sufficient to generate or to increase a pathogen resistance in said plant.

7. The method according to any of claims 1 to 6, wherein the plant is selected from among the monocotyledonous and dicotyledonous plants.

8. The method according to any of claims 1 to 7, wherein the plant is selected from the group of the monocotyledonous plants consisting of wheat, oats, millet, barley, rye, maize, rice, buckwheat, sorghum, triticale, spelt, linseed and sugar cane.

9. The method according to any of claims 1 to 8, wherein the expression of the Bax inhibitor-1 (BI-1) in the mesophyll is increased.

10. The method according to any of claims 1 to 9, wherein the plant has an mlo-resistant phenotype, or the expression or function of MLO, RacB and/or NaOx is inhibited or, in comparison with a control plant, is reduced at least in the epidermis and/or the expression or function of PEN2, SNAP34 and/or PEN1 is increased at least in the epidermis in comparison with a control plant.

## Revendications

1. Procédé pour la production ou l'augmentation de la résistance à au moins un facteur de stress biotique chez des plantes, dans lequel sont comprises les étapes opératoires suivantes
a) augmentation de la quantité de protéine d'au moins une protéine inhibitrice Bax 1 (BI1) dans au moins un tissu végétal, étant entendu que l'expression dans l'épiderme foliaire reste pratiquement inchangée ou est réduite, et
b) sélection des plantes dans lesquelles une résistance à au moins un facteur de stress biotique existe ou est augmentée par comparaison à la plante de départ ;
et
le facteur de stress est un agent phytopathogène.

2. Procédé selon la revendication 1, dans lequel le facteur de stress est un agent pathogène nécrotrophe ou hémibiotrophe.

3. Procédé selon la revendication 1 ou 2, dans lequel la protéine BI-1 comprend au moins une séquence qui présente une homologie d'au moins 50 % avec au moins un motif consensus de BI1, choisi dans le groupe constitué par
a) H (L/I) KXVY,
b) AXGA (Y/F) XH,
c) NIGG,
d) P(V/P) (Y/F) E (E/Q) (R/Q) KR,
e) (E/Q) G (A/S) S (V/I) GPL,
f) DP(S/G)(L/I)(I/L),
g) V(G/A)T(A/S) (L/I)AF(A/G)CF(S/T),
h) YL (Y/F) LGG,
i) L (L/V) SS (G/W) L (S/T) (I/M) L (L/M) W, et
j) DTGX (I/V) (I/V) E.

4. Procédé selon l'une quelconque des revendications 1 à 3, dans lequel la protéine BI-1 est codée par un polypeptide qui comprend au moins une séquence choisie dans le groupe constitué par :
a) les séquences selon SEQ ID n° 2, 4, 6, 8, 10, 12, 14, 16, 18, 20, 22, 24, 26, 28, 30, 32 ou 38, et
b) les séquences qui présentent une identité d'au moins 50 % avec l'une des séquences selon SEQ ID n° 2, 4, 6, 8, 10, 12, 14, 16, 18, 20, 22, 24, 26, 28, 30, 32 ou 38,
c) les séquences qui comportent au moins une séquence partielle d'au moins 10 résidus aminoacide successifs de l'une des séquences selon SEQ ID n° 2, 4, 6, 8, 10, 12, 14, 16, 18, 20, 22, 24, 26, 28, 30, 32 ou 38.

5. Procédé selon l'une quelconque des revendications 1 à 4, dans lequel l'augmentation de la quantité de protéine d'au moins une protéine BI1 est réalisée par expression recombinante de ladite protéine BI1 sous contrôle d'un promoteur spécifique de la racine, du tubercule ou du mésophylle.

6. Procédé selon l'une quelconque des revendications 1 à 5, comprenant
(a) la transformation stable d'une cellule végétale par une cassette d'expression recombinante contenant une séquence d'acide nucléique codant pour une protéine BI, en liaison fonctionnelle avec un promoteur spécifique de tissu, le promoteur ne présentant pratiquement pas d'activité dans l'épiderme foliaire et le promoteur étant hétérologue eu égard à ladite séquence d'acide nucléique codant la protéine BI,
(b) régénération de la plante à partir de la cellule végétale, et
(c) expression de ladite séquence d'acide nucléique codant une protéine BI, en une quantité suffisante et pendant un temps suffisant pour produire ou augmenter une résistance à un agent pathogène chez ladite plante.

7. Procédé selon l'une quelconque des revendications 1 à 6, dans lequel la plante est choisie parmi les plantes monocotylédones et les plantes dicotylédones.

8. Procédé selon l'une quelconque des revendications 1 à 7, dans lequel la plante est choisie dans le groupe des plantes monocotylédones constitué par le blé, l'avoine, le millet, l'orge, le seigle, le maïs, le riz, le sarrasin, le sorgho, le triticale, l'épeautre, le lin et la canne à sucre.

9. Procédé selon l'une quelconque des revendications 1 à 8, dans lequel l'expression de l'inhibiteur Bax 1(BI-1) est augmentée dans le mésophylle.

10. Procédé selon l'une quelconque des revendications 1 à 9, dans lequel la plante présente un phénotype mlo-résistant, ou l'expression ou la fonction de MLO, RacB et/ou NaOx est inhibée au moins dans l'épiderme ou est réduite par comparaison avec une plante témoin et/ou l'expression ou la fonction de PEN2, SNAP34 et/ou PEN1 est augmentée au moins dans l'épiderme, par comparaison avec une plante témoin.


### SEQUENZPROTOKOLL

<110> BASF Plant Science GmbH
<120> Verfahren zur Erhöhung der Resistenz gegen Streßfaktoren in Pflanzen
<130> PF54350-AT
<140>
   <141>
<160> 44
<170> PatentIn Ver. 2.1
<210> 1
   <211> 744
   <212> DNA
   <213> Hordeum vulgare
<220>
   <221> CDS
   <222> (1)..(741)
   <223> coding for BI1-protein
<400> 1
   atg gac gcc ttc tac tcg acc tcg tcg gcg gcg gcg agc ggc tgg ggc    48
   Met Asp Ala Phe Tyr Ser Thr Ser Ser Ala Ala Ala Ser Gly Trp Gly
   1               5                   10                  15
   cac gac tcc ctc aag aac ttc cgc cag atc tcc ccc gcc gtg cag tcc    96
   His Asp Ser Leu Lys Asn Phe Arg Gln Ile Ser Pro Ala Val Gln Ser
               20                  25                  30
   cac ctc aag ctc gtt tac ctg act cta tgc ttt gca ctg gcc tca tct    144
   His Leu Lys Leu Val Tyr Leu Thr Leu Cys Phe Ala Leu Ala Ser Ser
           35                  40                  45
   gcc gtg ggt gct tac cta cac att gcc ctg aac atc ggc ggg atg ctg    192
   Ala Val Gly Ala Tyr Leu His Ile Ala Leu Asn Ile Gly Gly Met Leu
       50                  55                  60
   aca atg ctc gct tgt gtc gga act atc gcc tgg atg ttc tcg gtg cca    240
   Thr Met Leu Ala Cys Val Gly Thr Ile Ala Trp Met Phe Ser Val Pro
   65                  70                  75                  80
   gtc tat gag gag agg aag agg ttt ggg ctg ctg atg ggt gca gcc ctc    288
   Val Tyr Glu Glu Arg Lys Arg Phe Gly Leu Leu Met Gly Ala Ala Leu
                   85                  90                  95
   ctg gaa ggg gct tcg gtt gga cct ctg att gag ctt gcc ata gac ttt    336
   Leu Glu Gly Ala Ser Val Gly Pro Leu Ile Glu Leu Ala Ile Asp Phe
               100                 105                 110
   gac cca agc atc ctc gtg aca ggg ttt gtc gga acc gcc atc gcc ttt    384
   Asp Pro Ser Ile Leu Val Thr Gly Phe Val Gly Thr Ala Ile Ala Phe
           115                 120                 125
   ggg tgc ttc tct ggc gcc gcc atc atc gcc aag cgc agg gag tac ctg    432
   Gly Cys Phe Ser Gly Ala Ala Ile Ile Ala Lys Arg Arg Glu Tyr Leu
       130                 135                 140
   tac ctc ggt ggc ctg ctc tcg tct ggc ctg tcg atc ctg ctc tgg ctg    480
   Tyr Leu Gly Gly Leu Leu Ser Ser Gly Leu Ser Ile Leu Leu Trp Leu
   145                 150                 155                 160
   cag ttt gtc acg tcc atc ttt ggc cac tcc tct ggc agc ttc atg ttt    528
   Gln Phe Val Thr Ser Ile Phe Gly His Ser Ser Gly Ser Phe Met Phe
                   165                 170                 175
   gag gtt tac ttt ggc ctg ttg atc ttc ctg ggg tac atg gtg tac gac    576
   Glu Val Tyr Phe Gly Leu Leu Ile Phe Leu Gly Tyr Met Val Tyr Asp
               180                 185                 190
   acg cag gag atc atc gag agg gcg cac cat ggc gac atg gac tac atc    624
   Thr Gln Glu Ile Ile Glu Arg Ala His His Gly Asp Met Asp Tyr Ile
           195                 200                 205
   aag cac gcc ctc acc ctc ttc acc gac ttt gtt gcc gtc ctc gtc cga    672
   Lys His Ala Leu Thr Leu Phe Thr Asp Phe Val Ala Val Leu Val Arg
       210                 215                 220
   gtc ctc atc atc atg ctc aag aac gca ggc gac aag tcg gag gac aag    720
   Val Leu Ile Ile Met Leu Lys Asn Ala Gly Asp Lys Ser Glu Asp Lys
   225                 230                 235                 240
   aag aag agg aag agg ggg tcc tga                                    744
   Lys Lys Arg Lys Arg Gly Ser
                   245
<210> 2
   <211> 247
   <212> PRT
   <213> Hordeum vulgare
<400> 2
   Met Asp Ala Phe Tyr Ser Thr Ser Ser Ala Ala Ala Ser Gly Trp Gly
   1               5                   10                  15
   His Asp Ser Leu Lys Asn Phe Arg Gln Ile Ser Pro Ala Val Gln Ser
               20                  25                  30
   His Leu Lys Leu Val Tyr Leu Thr Leu Cys Phe Ala Leu Ala Ser Ser
           35                  40                  45
   Ala Val Gly Ala Tyr Leu His Ile Ala Leu Asn Ile Gly Gly Met Leu
       50                  55                  60
   Thr Met Leu Ala Cys Val Gly Thr Ile Ala Trp Met Phe Ser Val Pro
   65                  70                  75                  80
   Val Tyr Glu Glu Arg Lys Arg Phe Gly Leu Leu Met Gly Ala Ala Leu
                   85                  90                  95
   Leu Glu Gly Ala Ser Val Gly Pro Leu Ile Glu Leu Ala Ile Asp Phe
               100                 105                 110
   Asp Pro Ser Ile Leu Val Thr Gly Phe Val Gly Thr Ala Ile Ala Phe
           115                 120                 125
   Gly Cys Phe Ser Gly Ala Ala Ile Ile Ala Lys Arg Arg Glu Tyr Leu
       130                 135                 140
   Tyr Leu Gly Gly Leu Leu Ser Ser Gly Leu Ser Ile Leu Leu Trp Leu
   145                 150                 155                 160
   Gln Phe Val Thr Ser Ile Phe Gly His Ser Ser Gly Ser Phe Met Phe
                   165                 170                 175
   Glu Val Tyr Phe Gly Leu Leu Ile Phe Leu Gly Tyr Met Val Tyr Asp
               180                 185                 190
   Thr Gln Glu Ile Ile Glu Arg Ala His His Gly Asp Met Asp Tyr Ile
           195                 200                 205
   Lys His Ala Leu Thr Leu Phe Thr Asp Phe Val Ala Val Leu Val Arg
       210                 215                 220
   Val Leu Ile Ile Met Leu Lys Asn Ala Gly Asp Lys Ser Glu Asp Lys
   225                 230                 235                 240
   Lys Lys Arg Lys Arg Gly Ser
                   245
<210> 3
   <211> 1067
   <212> DNA
   <213> Arabidopsis thaliana
<220>
   <221> CDS
   <222> (1)..(741)
   <223> coding for BI1-protein
<400> 3
   atg gat gcg ttc tct tcc ttc ttc gat tct caa cct ggt agc aga agc    48
   Met Asp Ala Phe Ser Ser Phe Phe Asp Ser Gln Pro Gly Ser Arg Ser
    1               5                  10                  15
   tgg agc tat gat tct ctt aaa aac ttc cgt cag att tct cca gcc gtt    96
   Trp Ser Tyr Asp Ser Leu Lys Asn Phe Arg Gln Ile Ser Pro Ala Val
               20                  25                  30
   cag aat cat ctt aaa cgg gtt tat ttg acc tta tgt tgt gct ctt gtg   144
   Gln Asn His Leu Lys Arg Val Tyr Leu Thr Leu Cys Cys Ala Leu Val
           35                  40                  45
   gcg tct gcc ttt gga gct tac ctc cat gtg ctc tgg aat atc ggc ggt   192
   Ala Ser Ala Phe Gly Ala Tyr Leu His Val Leu Trp Asn Ile Gly Gly
       50                  55                  60
   att ctt aca acg att gga tgt att gga act atg att tgg ctc ctt tca   240
   Ile Leu Thr Thr Ile Gly Cys Ile Gly Thr Met Ile Trp Leu Leu Ser
   65                  70                  75                  80
   tgt cct cct tat gaa cac caa aaa agg ctt tct ctt ctg ttt gtg tct   288
   Cys Pro Pro Tyr Glu His Gln Lys Arg Leu Ser Leu Leu Phe Val Ser
                   85                  90                  95
   gct gtt ctt gaa ggt gct tct gtt ggc ccc ttg atc aaa gtg gca att   336
   Ala Val Leu Glu Gly Ala Ser Val Gly Pro Leu Ile Lys Val Ala Ile
               100                 105                 110
   gat gtt gac cca agc atc ctt atc act gca ttt gtt gga act gcg ata   384
   Asp Val Asp Pro Ser Ile Leu Ile Thr Ala Phe Val Gly Thr Ala Ile
           115                 120                 125
   gcg ttt gtc tgt ttc tca gca gca gca atg tta gca aga cgc agg gag   432
   Ala Phe Val Cys Phe Ser Ala Ala Ala Met Leu Ala Arg Arg Arg Glu
       130                 135                 140
   tat ctc tac ctt gga gga ctg ctt tca tct ggc ttg tct atg cta atg   480
   Tyr Leu Tyr Leu Gly Gly Leu Leu Ser Ser Gly Leu Ser Met Leu Met
   145                 150                 155                 160
   tgg ctc cag ttt gcc tct tca atc ttt ggt ggc tct gca tct atc ttt   528
   Trp Leu Gln Phe Ala Ser Ser Ile Phe Gly Gly Ser Ala Ser Ile Phe
                   165                 170                 175
   aag ttt gag ttg tac ttt gga ctt ttg atc ttt gtg gga tac atg gtg   576
   Lys Phe Glu Leu Tyr Phe Gly Leu Leu Ile Phe Val Gly Tyr Met Val
               180                 185                 190
   gtg gac aca caa gag att ata gaa aag gca cac ctc ggt gac atg gac   624
   Val Asp Thr Gln Glu Ile Ile Glu Lys Ala His Leu Gly Asp Met Asp
           195                 200                 205
   tat gta aaa cat tcg ttg acc ctt ttc act gac ttt gta gct gtg ttt   672
   Tyr Val Lys His Ser Leu Thr Leu Phe Thr Asp Phe Val Ala Val Phe
       210                 215                 220
   gtt cgg att ctc atc ata atg ttg aag aac tca gca gat aaa gaa gag   720
   Val Arg Ile Leu Ile Ile Met Leu Lys Asn Ser Ala Asp Lys Glu Glu
   225                 230                 235                 240
   aag aag aag aaa agg aga aac tgaggggatg taaagtaaat ttaactttat      771
   Lys Lys Lys Lys Arg Arg Asn
                       245
   ggttgttatc gtgtgtggcc actttgaaga tattacttgt tagcactctc tattggtgac 831
   cagacatgtt tccactaaaa aggatctgct tgtttcactt ctgcacaagt accatcttca 891
   gattgtaaat gactcgagtg ttgttcttct tttcataaac ttttgttctt taagagtttg 951
   gttctactga ttgcatctta ccaagctaag aataatgtag gaaaatgata atcctgttta 1011
   aattttctaa aatgtgtgca tttcagaaaa aaaaaaaaaa aaaaaaaaaa aaaaaa     1067
<210> 4
   <211> 247
   <212> PRT
   <213> Arabidopsis thaliana
<400> 4
   Met Asp Ala Phe Ser Ser Phe Phe Asp Ser Gln Pro Gly Ser Arg Ser
   1               5                   10                  15
   Trp Ser Tyr Asp Ser Leu Lys Asn Phe Arg Gln Ile Ser Pro Ala Val
               20                  25                  30
   Gln Asn His Leu Lys Arg Val Tyr Leu Thr Leu Cys Cys Ala Leu Val
           35                  40                  45
   Ala Ser Ala Phe Gly Ala Tyr Leu His Val Leu Trp Asn Ile Gly Gly
       50                  55                  60
   Ile Leu Thr Thr Ile Gly Cys Ile Gly Thr Met Ile Trp Leu Leu Ser
   65                  70                  75                  80
   Cys Pro Pro Tyr Glu His Gln Lys Arg Leu Ser Leu Leu Phe Val Ser
                   85                  90                  95
   Ala Val Leu Glu Gly Ala Ser Val Gly Pro Leu Ile Lys Val Ala Ile
               100                 105                 110
   Asp Val Asp Pro Ser Ile Leu Ile Thr Ala Phe Val Gly Thr Ala Ile
           115                 120                 125
   Ala Phe Val Cys Phe Ser Ala Ala Ala Met Leu Ala Arg Arg Arg Glu
       130                 135                 140
   Tyr Leu Tyr Leu Gly Gly Leu Leu Ser Ser Gly Leu Ser Met Leu Met
   145                 150                 155                 160
   Trp Leu Gln Phe Ala Ser Ser Ile Phe Gly Gly Ser Ala Ser Ile Phe
                   165                 170                 175
   Lys Phe Glu Leu Tyr Phe Gly Leu Leu Ile Phe Val Gly Tyr Met Val
               180                 185                 190
   Val Asp Thr Gln Glu Ile Ile Glu Lys Ala His Leu Gly Asp Met Asp
           195                 200                 205
   Tyr Val Lys His Ser Leu Thr Leu Phe Thr Asp Phe Val Ala Val Phe
       210                 215                 220
   Val Arg Ile Leu Ile Ile Met Leu Lys Asn Ser Ala Asp Lys Glu Glu
   225                 230                 235                 240
   Lys Lys Lys Lys Arg Arg Asn
                   245
<210> 5
   <211> 1160
   <212> DNA
   <213> Nicotiana tabacum
<220>
   <221> CDS
   <222> (1)..(747)
   <223> coding for BI1-protein
<400> 5
   atg gag tct tgc aca tcg ttc ttc aat tca cag tcg gcg tcg tct cgc 48
   Met Glu Ser Cys Thr Ser Phe Phe Asn Ser Gln Ser Ala Ser Ser Arg
   1 5 10 15
   aat cgc tgg agt tac gat tct ctt aag aac ttc cgc cag atc tct ccc 96
   Asn Arg Trp Ser Tyr Asp Ser Leu Lys Asn Phe Arg Gln Ile Ser Pro
   20 25 30
   ttt gtt caa act cat ctc aaa aag gtc tac ctt tca tta tgt tgt gct 144
   Phe Val Gln Thr His Leu Lys Lys Val Tyr Leu Ser Leu Cys Cys Ala
   35 40 45
   tta gtt gct tcg gct gct gga gct tac ctt cac att ctt tgg aac att 192
   Leu Val Ala Ser Ala Ala Gly Ala Tyr Leu His Ile Leu Trp Asn Ile
   50 55 60
   ggt ggc tta ctt acg aca ttg gga tgt gtg gga agc ata gtg tgg ctg 240
   Gly Gly Leu Leu Thr Thr Leu Gly Cys Val Gly Ser Ile Val Trp Leu
   65 70 75 80
   atg gcg aca cct ctg tat gaa gag caa aag agg ata gca ctt ctg atg 288
   Met Ala Thr Pro Leu Tyr Glu Glu Gln Lys Arg Ile Ala Leu Leu Met
   85 90 95
   gca gct gca ctg ttt aaa gga gca tct att ggt cca ctg att gaa ttg 336
   Ala Ala Ala Leu Phe Lys Gly Ala Ser Ile Gly Pro Leu Ile Glu Leu
   100 105 110
   gct att gac ttt gac cca agc att gtg atc ggt gct ttt gtt ggt tgt 384
   Ala Ile Asp Phe Asp Pro Ser Ile Val Ile Gly Ala Phe Val Gly Cys
   115 120 125
   gct gtg gct ttt ggt tgc ttc tca gct gct gcc atg gtg gca agg cgc 432
   Ala Val Ala Phe Gly Cys Phe Ser Ala Ala Ala Met Val Ala Arg Arg
   130 135 140
   aga gag tac ttg tat ctt gga ggt ctt ctt tca tct ggt ctc tct atc 480
   Arg Glu Tyr Leu Tyr Leu Gly Gly Leu Leu Ser Ser Gly Leu Ser Ile
   145 150 155 160
   ctt ttc tgg ttg cac ttc gcg tcc tcc att ttt ggt ggt tct atg gcc 528
   Leu Phe Trp Leu His Phe Ala Ser Ser Ile Phe Gly Gly Ser Met Ala
   165 170 175
   ttg ttc aag ttc gag gtt tat ttt ggg ctc ttg gtg ttt gtg ggc tat 576
   Leu Phe Lys Phe Glu Val Tyr Phe Gly Leu Leu Val Phe Val Gly Tyr
   180 185 190
   atc att ttt gac acc caa gat ata att gag aag gca cac ctt ggg gat 624
   Ile Ile Phe Asp Thr Gln Asp Ile Ile Glu Lys Ala His Leu Gly Asp
   195 200 205
   ttg gac tac gtg aag cat gct ctg acc ctc ttt aca gat ttt gtt gct 672
   Leu Asp Tyr Val Lys His Ala Leu Thr Leu Phe Thr Asp Phe Val Ala
   210 215 220
   gtt ttt gtg cga ata tta atc ata atg ctg aag aat gca tcc gac aag 720
   Val Phe Val Arg Ile Leu Ile Ile Met Leu Lys Asn Ala Ser Asp Lys
   225 230 235 240
   gaa gag aag aag aag aag agg aga aac taatgcataa gcggttattc 767
   Glu Glu Lys Lys Lys Lys Arg Arg Asn
   245
   aaagactctg taactctaga atctggcatt ttcttgttca taaacttctg tagaccttcg 827
   acaagtatgt tgttaatagt ttggtaacgc ctcagattaa gctgcgaggc tctgttatgc 887
   cgcatgccaa tgtggttatg gtggtacata gatggttttg tttccgaagc ataccatcaa 947
   ataacatgca tgtttacact atatcgataa cctacgagtg tactacttat ttctgctccc 1007
   ttttgctgtg ttaggttgtt catgattgta tagttgattt tccgttatgt tagaccatct 1067
   tctttcttga cgtttaattt ctcatattga tgggagaaat gaaaattcac accgtcgccc 1127
   caacttgttt aagactgagg cgcaattgta gtt                              1160
<210> 6
   <211> 249
   <212> PRT
   <213> Nicotiana tabacum
<400> 6
   Met Glu Ser Cys Thr Ser Phe Phe Asn Ser Gln Ser Ala Ser Ser Arg
   1               5                   10                  15
   Asn Arg Trp Ser Tyr Asp Ser Leu Lys Asn Phe Arg Gln Ile Ser Pro
               20                  25                  30
   Phe Val Gln Thr His Leu Lys Lys Val Tyr Leu Ser Leu Cys Cys Ala
           35                  40                  45
   Leu Val Ala Ser Ala Ala Gly Ala Tyr Leu His Ile Leu Trp Asn Ile
       50                  55                  60
   Gly Gly Leu Leu Thr Thr Leu Gly Cys Val Gly Ser Ile Val Trp Leu
   65                  70                  75                  80
   Met Ala Thr Pro Leu Tyr Glu Glu Gln Lys Arg Ile Ala Leu Leu Met
                   85                  90                  95
   Ala Ala Ala Leu Phe Lys Gly Ala Ser Ile Gly Pro Leu Ile Glu Leu
               100                 105                 110
   Ala Ile Asp Phe Asp Pro Ser Ile Val Ile Gly Ala Phe Val Gly Cys
           115                 120                 125
   Ala Val Ala Phe Gly Cys Phe Ser Ala Ala Ala Met Val Ala Arg Arg
       130                 135                 140
   Arg Glu Tyr Leu Tyr Leu Gly Gly Leu Leu Ser Ser Gly Leu Ser Ile
   145                 150                 155                 160
   Leu Phe Trp Leu His Phe Ala Ser Ser Ile Phe Gly Gly Ser Met Ala
                   165                 170                 175
   Leu Phe Lys Phe Glu Val Tyr Phe Gly Leu Leu Val Phe Val Gly Tyr
               180                 185                 190
   Ile Ile Phe Asp Thr Gln Asp Ile Ile Glu Lys Ala His Leu Gly Asp
           195                 200                 205
   Leu Asp Tyr Val Lys His Ala Leu Thr Leu Phe Thr Asp Phe Val Ala
       210                 215                 220
   Val Phe Val Arg Ile Leu Ile Ile Met Leu Lys Asn Ala Ser Asp Lys
   225                 230                 235                 240
   Glu Glu Lys Lys Lys Lys Arg Arg Asn
                   245
<210> 7
   <211> 1056
<212> DNA
   <213> Oryza sativa
<220>
   <221> CDS
   <222> (1)..(747)
   <223> coding for BI1-protein
<400> 7
   atg gac gcc ttc tac tcg acc tcg tcg gcg tac gga gcg gcg gcg agc      48
   Met Asp Ala Phe Tyr Ser Thr Ser Ser Ala Tyr Gly Ala Ala Ala Ser
    1               5                  10                  15
   ggc tgg ggc tac gac tcg ctg aag aac ttc cgc cag atc tcc ccc gcc      96
   Gly Trp Gly Tyr Asp Ser Leu Lys Asn Phe Arg Gln Ile Ser Pro Ala
               20                  25                  30
   gtc cag tcc cac ctc aag ctc gtt tac ctg aca cta tgc gtc gcc ctg     144
   Val Gln Ser His Leu Lys Leu Val Tyr Leu Thr Leu Cys Val Ala Leu
           35                  40                  45
   gct gcg tcg gcg gtg ggc gca tac ctg cac gtc gcc ttg aac atc ggc     192
   Ala Ala Ser Ala Val Gly Ala Tyr Leu His Val Ala Leu Asn Ile Gly
       50                  55                  60
   ggg atg ttg act atg ctc ggg tgc gtg ggg agc atc gcc tgg ttg ttc     240
   Gly Met Leu Thr Met Leu Gly Cys Val Gly Ser Ile Ala Trp Leu Phe
   65                  70                  75                  80
   tcg gtg cct gtc ttt gag gag agg aag agg ttt ggg att ctc ttg gcc     288
   Ser Val Pro Val Phe Glu Glu Arg Lys Arg Phe Gly Ile Leu Leu Ala
                   85                  90                  95
   gct gcc ctg ctg gaa ggg gct tca gtt ggg cct ctg atc aag ctt gct     336
   Ala Ala Leu Leu Glu Gly Ala Ser Val Gly Pro Leu Ile Lys Leu Ala
               100                 105                 110
   gta gac ttt gac tca agc att ctc gta aca gca ttt gtt gga act gcc     384
   Val Asp Phe Asp Ser Ser Ile Leu Val Thr Ala Phe Val Gly Thr Ala
           115                 120                 125
   att gca ttt ggg tgc ttc act tgc gct gcc atc gtt gcc aag cgt agg     432
   Ile Ala Phe Gly Cys Phe Thr Cys Ala Ala Ile Val Ala Lys Arg Arg
       130                 135                 140
   gag tac ctc tac ctt ggt ggt ttg ctc tct tct ggc ctc tcc atc ctg     480
   Glu Tyr Leu Tyr Leu Gly Gly Leu Leu Ser Ser Gly Leu Ser Ile Leu
   145                 150                 155                 160
   ctc tgg ctg cag ttt gcc gca tcc atc ttt ggc cac tcc acc ggc agc     528
   Leu Trp Leu Gln Phe Ala Ala Ser Ile Phe Gly His Ser Thr Gly Ser
                   165                 170                 175
   ttc atg ttt gag gtt tac ttt ggc ctg ttg atc ttc ctg ggg tac atg     576
   Phe Met Phe Glu Val Tyr Phe Gly Leu Leu Ile Phe Leu Gly Tyr Met
               180                 185                 190
   gtg tat gac acg cag gag atc atc gag agg gct cac cac ggt gac atg     624
   Val Tyr Asp Thr Gln Glu Ile Ile Glu Arg Ala His His Gly Asp Met
           195                 200                 205
   gac tac atc aag cac gca ctc acc ctc ttc act gac ttc gtg gcc gtc     672
   Asp Tyr Ile Lys His Ala Leu Thr Leu Phe Thr Asp Phe Val Ala Val
       210                 215                 220
   ctt gtc cgg atc ctc gtc atc atg ctc aag aac gcg tct gac aag tcg     720
   Leu Val Arg Ile Leu Val Ile Met Leu Lys Asn Ala Ser Asp Lys Ser
   225                 230                 235                 240
   gag gag aag aag agg aag aag agg tct tgagagcttc tcttcccgct           767
   Glu Glu Lys Lys Arg Lys Lys Arg Ser
                   245
   ttgcacataa gaaaaaacca ccgcggctat tgcctctacg tattatgaca gagccgcact 827
   tcaactgggt tttatggtga atacaagttc ttttgcattt tgttgatacg gtgtgaatct 887
   tctcaggttt gtcgtcgtag tagctttgca aatactagca tgctacatga cacggatctt 947
   tctgtaatgg tggtcgcgtt gatcgaaacg tgaaaacaca tcttcatttg cgactaattt 1007
   gtttgccttt tggtgattga tgatgatcct ttccccaaaa aaaaaaaaa 1056
<210> 8
   <211> 249
   <212> PRT
   <213> Oryza sativa
<400> 8
   Met Asp Ala Phe Tyr Ser Thr Ser Ser Ala Tyr Gly Ala Ala Ala Ser
   1 5 10 15
   Gly Trp Gly Tyr Asp Ser Leu Lys Asn Phe Arg Gln Ile Ser Pro Ala
   20 25 30
   Val Gln Ser His Leu Lys Leu Val Tyr Leu Thr Leu Cys Val Ala Leu
   35 40 45
   Ala Ala Ser Ala Val Gly Ala Tyr Leu His Val Ala Leu Asn Ile Gly
   50 55 60
   Gly Met Leu Thr Met Leu Gly Cys Val Gly Ser Ile Ala Trp Leu Phe
   65 70 75 80
   Ser Val Pro Val Phe Glu Glu Arg Lys Arg Phe Gly Ile Leu Leu Ala
   85 90 95
   Ala Ala Leu Leu Glu Gly Ala Ser Val Gly Pro Leu Ile Lys Leu Ala
   100 105 110
   Val Asp Phe Asp Ser Ser Ile Leu Val Thr Ala Phe Val Gly Thr Ala
   115 120 125
   Ile Ala Phe Gly Cys Phe Thr Cys Ala Ala Ile Val Ala Lys Arg Arg
   130 135 140
   Glu Tyr Leu Tyr Leu Gly Gly Leu Leu Ser Ser Gly Leu Ser Ile Leu
   145 150 155 160
   Leu Trp Leu Gln Phe Ala Ala Ser Ile Phe Gly His Ser Thr Gly Ser
   165 170 175
   Phe Met Phe Glu Val Tyr Phe Gly Leu Leu Ile Phe Leu Gly Tyr Met
   180 185 190
   Val Tyr Asp Thr Gln Glu Ile Ile Glu Arg Ala His His Gly Asp Met
   195 200 205
   Asp Tyr Ile Lys His Ala Leu Thr Leu Phe Thr Asp Phe Val Ala Val
   210 215 220
   Leu Val Arg Ile Leu Val Ile Met Leu Lys Asn Ala Ser Asp Lys Ser
   225 230 235 240
   Glu Glu Lys Lys Arg Lys Lys Arg Ser
   245
<210> 9
   <211> 973
   <212> DNA
   <213> Brassica napus
<220>
   <221> CDS
   <222> (1)..(741)
   <223> coding for BI1-protein
<400> 9
   atg gat tca ttc tcg tcc ttc ttc gat tct caa cct ggt agc aga agc    48
   Met Asp Ser Phe Ser Ser Phe Phe Asp Ser Gln Pro Gly Ser Arg Ser
    1               5                  10                  15
   tgg agc tat gat tct ctc aaa aac ctc cgt cag att tct ccc tcc gtc    96
   Trp Ser Tyr Asp Ser Leu Lys Asn Leu Arg Gln Ile Ser Pro Ser Val
               20                  25                  30
   cag aat cat ctc aag agg gtt tat ctc act ctg tgt tgt gct ctc gtt   144
   Gln Asn His Leu Lys Arg Val Tyr Leu Thr Leu Cys Cys Ala Leu Val
           35                  40                  45
   gcg tct gcg ttt gga gct tac ctc cac gtg ctc tgg aac ata ggt ggt   192
   Ala Ser Ala Phe Gly Ala Tyr Leu His Val Leu Trp Asn Ile Gly Gly
       50                  55                  60
   att ctc act acc att gga tgc ttt gga agc atg att tgg ctg ctc tcc   240
   Ile Leu Thr Thr Ile Gly Cys Phe Gly Ser Met Ile Trp Leu Leu Ser
   65                  70                  75                  80
   tgt cct cct tat gaa caa caa aag agg ctt tcc ctt ctg ttt ctg tct   288
   Cys Pro Pro Tyr Glu Gln Gln Lys Arg Leu Ser Leu Leu Phe Leu Ser
                   85                  90                  95
   gct gtt ctc gaa ggt gct tca gtt ggt ccc ttg atc aaa gtg gca gtt   336
   Ala Val Leu Glu Gly Ala Ser Val Gly Pro Leu Ile Lys Val Ala Val
               100                 105                 110
   gat ttt gac cca agc atc ctc atc act gcg ttt gtc gga act gcg ata   384
   Asp Phe Asp Pro Ser Ile Leu Ile Thr Ala Phe Val Gly Thr Ala Ile
           115                 120                 125
   gcc ttt atc tgt ttc tca ggg gca gcg atg ttg gca aga cgc aga gag   432
   Ala Phe Ile Cys Phe Ser Gly Ala Ala Met Leu Ala Arg Arg Arg Glu
       130                 135                 140
   tac ctc tac ctc gga gga ctg ctt tca tct ggc ttg tcc atg ctt atg   480
   Tyr Leu Tyr Leu Gly Gly Leu Leu Ser Ser Gly Leu Ser Met Leu Met
   145                 150                 155                 160
   tgg ctt cag ttt gcc tct tcc atc ttt ggt ggc tct gca tcc atc ttt   528
   Trp Leu Gln Phe Ala Ser Ser Ile Phe Gly Gly Ser Ala Ser Ile Phe
                   165                 170                 175
   aag ttt gag ctc tac ttt gga ctc ttg atc ttt gtg gga tac atg gtg   576
   Lys Phe Glu Leu Tyr Phe Gly Leu Leu Ile Phe Val Gly Tyr Met Val
               180                 185                 190
   gtg gac act caa gat att ata gag aag gcc cac ctc ggt gac atg gat   624
   Val Asp Thr Gln Asp Ile Ile Glu Lys Ala His Leu Gly Asp Met Asp
           195                 200                 205
   tac gtg aaa cat tcg ttg acc ctt ttc acc gat ttt gta gct gtg ttt   672
   Tyr Val Lys His Ser Leu Thr Leu Phe Thr Asp Phe Val Ala Val Phe
       210                 215                 220
   gtt cgt gtt ctc atc att atg ctg aag aac tcg gca gat aaa gaa gat   720
   Val Arg Val Leu Ile Ile Met Leu Lys Asn Ser Ala Asp Lys Glu Asp
   225                 230                 235                 240
   aaa aag aag agg agg agg aac tgagactaaa aagtgagaaa gaaagctaaa      771
   Lys Lys Lys Arg Arg Arg Asn
                   245
   tagagtgggt gttatgtgtg tttcaaaaaa taaaaaagag tgggtgttat aagtacagac 831
   atgatagcgt tggtgttttt tacttgtttg gaacagtttt ggtaacaaca cacgttacgt 891
   atttgtgtat tcctcttagt gactccagat tgtgaatgga tcagtatctt gaaactgtgt 951
   tgaaaattat cagttgggag ct 973
<210> 10
   <211> 247
   <212> PRT
   <213> Brassica napus
<400> 10
   Met Asp Ser Phe Ser Ser Phe Phe Asp Ser Gln Pro Gly Ser Arg Ser
   1               5                   10                  15
   Trp Ser Tyr Asp Ser Leu Lys Asn Leu Arg Gln Ile Ser Pro Ser Val
               20                  25                  30
   Gln Asn His Leu Lys Arg Val Tyr Leu Thr Leu Cys Cys Ala Leu Val
           35                  40                  45
   Ala Ser Ala Phe Gly Ala Tyr Leu His Val Leu Trp Asn Ile Gly Gly
       50                  55                  60
   Ile Leu Thr Thr Ile Gly Cys Phe Gly Ser Met Ile Trp Leu Leu Ser
   65                  70                  75                  80
   Cys Pro Pro Tyr Glu Gln Gln Lys Arg Leu Ser Leu Leu Phe Leu Ser
                   85                  90                  95
   Ala Val Leu Glu Gly Ala Ser Val Gly Pro Leu Ile Lys Val Ala Val
               100                 105                 110
   Asp Phe Asp Pro Ser Ile Leu Ile Thr Ala Phe Val Gly Thr Ala Ile
           115                 120                 125
   Ala Phe Ile Cys Phe Ser Gly Ala Ala Met Leu Ala Arg Arg Arg Glu
       130                 135                 140
   Tyr Leu Tyr Leu Gly Gly Leu Leu Ser Ser Gly Leu Ser Met Leu Met
   145                 150                 155                 160
   Trp Leu Gln Phe Ala Ser Ser Ile Phe Gly Gly Ser Ala Ser Ile Phe
                   165                 170                 175
   Lys Phe Glu Leu Tyr Phe Gly Leu Leu Ile Phe Val Gly Tyr Met Val
               180                 185                 190
   Val Asp Thr Gln Asp Ile Ile Glu Lys Ala His Leu Gly Asp Met Asp
           195                 200                 205
   Tyr Val Lys His Ser Leu Thr Leu Phe Thr Asp Phe Val Ala Val Phe
       210                 215                 220
   Val Arg Val Leu Ile Ile Met Leu Lys Asn Ser Ala Asp Lys Glu Asp
   225                 230                 235                 240
   Lys Lys Lys Arg Arg Arg Asn
                   245
<210> 11
   <211> 747
   <212> DNA
   <213> Glycine max
<220>
   <221> CDS
   <222> (1)..(744)
   <223> coding for BI1-protein
<400> 11
   cga ttg caa gca atg gac gcc ttc aat tcc ttc ttc gat tca aga aac 48
   Arg Leu Gln Ala Met Asp Ala Phe Asn Ser Phe Phe Asp Ser Arg Asn
    1               5                  10                  15
   cga tgg aat tac gat act ctc aaa aac ttc cgt cag att tct ccg gtc     96
   Arg Trp Asn Tyr Asp Thr Leu Lys Asn Phe Arg Gln Ile Ser Pro Val
               20                  25                  30
   gtg cag aat cac ctg aag cag gtt tat ttt act ctg tgt ttt gcc gtg    144
   Val Gln Asn His Leu Lys Gln Val Tyr Phe Thr Leu Cys Phe Ala Val
           35                  40                  45
   gtt gct gcg gct gtc ggg gct tac ctt cat gtc ctc ttg aac att ggg    192
   Val Ala Ala Ala Val Gly Ala Tyr Leu His Val Leu Leu Asn Ile Gly
       50                  55                  60
   ggt ttt ctt act aca gtg gca tgc atg gga agc agc ttt tgg tta ctc    240
   Gly Phe Leu Thr Thr Val Ala Cys Met Gly Ser Ser Phe Trp Leu Leu
   65                  70                  75                  80
   tcc aca cct cct ttt gaa gag agg aag agg gtg act ttg ttg atg gcc    288
   Ser Thr Pro Pro Phe Glu Glu Arg Lys Arg Val Thr Leu Leu Met Ala
                   85                  90                  95
   gca tca ctg ttt cag ggt tcc tct att gga ccc ttg att gat ttg gct    336
   Ala Ser Leu Phe Gln Gly Ser Ser Ile Gly Pro Leu Ile Asp Leu Ala
               100                 105                 110
   att cat atc gat cca agc ctt atc ttt agt gca ttt gtg gga aca gcc    384
   Ile His Ile Asp Pro Ser Leu Ile Phe Ser Ala Phe Val Gly Thr Ala
           115                 120                 125
   ttg gcc ttt gca tgc ttc tca gga gca gct ttg gtt gct agg cgt agg    432
   Leu Ala Phe Ala Cys Phe Ser Gly Ala Ala Leu Val Ala Arg Arg Arg
       130                 135                 140
   gag tac ctg tac ctt ggt ggc ttg gtt tct tct gga ttg tcc atc ctt    480
   Glu Tyr Leu Tyr Leu Gly Gly Leu Val Ser Ser Gly Leu Ser Ile Leu
   145                 150                 155                 160
   ctc tgg ttg cac ttt gct tct tcc atc ttt gga ggc tca aca gct ctc    528
   Leu Trp Leu His Phe Ala Ser Ser Ile Phe Gly Gly Ser Thr Ala Leu
                   165                 170                 175
   ttt aag ttt gag ttg tac ttt ggg ctt ttg gtg ttt gta ggt tac att    576
   Phe Lys Phe Glu Leu Tyr Phe Gly Leu Leu Val Phe Val Gly Tyr Ile
               180                 185                 190
   gta gta gac acc caa gaa ata gtt gag agg gca cac ttg ggc gat ctg    624
   Val Val Asp Thr Gln Glu Ile Val Glu Arg Ala His Leu Gly Asp Leu
           195                 200                 205
   gac tat gta aag cat gcc ttg acc ttg ttt acc gat ttg gtc gca gtt    672
   Asp Tyr Val Lys His Ala Leu Thr Leu Phe Thr Asp Leu Val Ala Val
       210                 215                 220
   ttt gtc cgg att ctt gtt att atg ttg aag aat tcg act gag agg aat    720
   Phe Val Arg Ile Leu Val Ile Met Leu Lys Asn Ser Thr Glu Arg Asn
   225                 230                 235                 240
   gag aag aaa aag aag aga aga gat tga                                747
   Glu Lys Lys Lys Lys Arg Arg Asp
                   245
<210> 12
   <211> 248
   <212> PRT
   <213> Glycine max
<400> 12
   Arg Leu Gln Ala Met Asp Ala Phe Asn Ser Phe Phe Asp Ser Arg Asn
   1               5                   10                  15
   Arg Trp Asn Tyr Asp Thr Leu Lys Asn Phe Arg Gln Ile Ser Pro Val
               20                  25                  30
   Val Gln Asn His Leu Lys Gln Val Tyr Phe Thr Leu Cys Phe Ala Val
           35                  40                  45
   Val Ala Ala Ala Val Gly Ala Tyr Leu His Val Leu Leu Asn Ile Gly
       50                  55                  60
   Gly Phe Leu Thr Thr Val Ala Cys Met Gly Ser Ser Phe Trp Leu Leu
   65                  70                  75                  80
   Ser Thr Pro Pro Phe Glu Glu Arg Lys Arg Val Thr Leu Leu Met Ala
                   85                  90                  95
   Ala Ser Leu Phe Gln Gly Ser Ser Ile Gly Pro Leu Ile Asp Leu Ala
               100                 105                 110
   Ile His Ile Asp Pro Ser Leu Ile Phe Ser Ala Phe Val Gly Thr Ala
           115                 120                 125
   Leu Ala Phe Ala Cys Phe Ser Gly Ala Ala Leu Val Ala Arg Arg Arg
       130                 135                 140
   Glu Tyr Leu Tyr Leu Gly Gly Leu Val Ser Ser Gly Leu Ser Ile Leu
   145                 150                 155                 160
   Leu Trp Leu His Phe Ala Ser Ser Ile Phe Gly Gly Ser Thr Ala Leu
                   165                 170                 175
   Phe Lys Phe Glu Leu Tyr Phe Gly Leu Leu Val Phe Val Gly Tyr Ile
               180                 185                 190
   Val Val Asp Thr Gln Glu Ile Val Glu Arg Ala His Leu Gly Asp Leu
           195                 200                 205
   Asp Tyr Val Lys His Ala Leu Thr Leu Phe Thr Asp Leu Val Ala Val
       210                 215                 220
   Phe Val Arg Ile Leu Val Ile Met Leu Lys Asn Ser Thr Glu Arg Asn
   225                 230                 235                 240
   Glu Lys Lys Lys Lys Arg Arg Asp
                   245
<210> 13
   <211> 1510
   <212> DNA
   <213> Glycine max
<220>
   <221> CDS
   <222> (1)..(777)
   <223> coding for BI-1 protein
<400> 13
   atc acg aaa act ata cga ttc gat tcc ttg ttt tcg atg gac act ttc   48
   Ile Thr Lys Thr Ile Arg Phe Asp Ser Leu Phe Ser Met Asp Thr Phe
    1               5                  10                  15
   ttc aag tcc cca tct tct tct tct tcg aga agc cgc tgg agt tac gat   96
   Phe Lys Ser Pro Ser Ser Ser Ser Ser Arg Ser Arg Trp Ser Tyr Asp
               20                  25                  30
   act ctc aag aat ttc cgc gag atc tct ccg ctc gtt cag aat cac atc   144
   Thr Leu Lys Asn Phe Arg Glu Ile Ser Pro Leu Val Gln Asn His Ile
           35                  40                  45
   aaa ctg gtt tat ttt acg tta tgt tgc gct gtg gtg gct gct gct gtt   192
   Lys Leu Val Tyr Phe Thr Leu Cys Cys Ala Val Val Ala Ala Ala Val
       50                  55                  60
   gga gct ttc ctt cat gtt ctg tgg aac att ggc ggt ttt ctc acc acg    240
   Gly Ala Phe Leu His Val Leu Trp Asn Ile Gly Gly Phe Leu Thr Thr
    65                  70                  75                  80
   ttg gct tcc att ggg agc atg ttt tgg ttg cta tct aca ccc cct ttt    288
   Leu Ala Ser Ile Gly Ser Met Phe Trp Leu Leu Ser Thr Pro Pro Phe
                    85                  90                  95
   gaa gag caa aag agg ttg tct ctg ttg atg gct tcg gcc ctg ttt cag    336
   Glu Glu Gln Lys Arg Leu Ser Leu Leu Met Ala Ser Ala Leu Phe Gln
               100                 105                 110
   ggt gct tcc att gga cct ctg att gat ttg gct ttt gcc att gat cct    384
   Gly Ala Ser Ile Gly Pro Leu Ile Asp Leu Ala Phe Ala Ile Asp Pro
           115                 120                 125
   ggc ctt atc att ggc gca ttt gtg gca act tct ttg gct ttt gct tgc    432
   Gly Leu Ile Ile Gly Ala Phe Val Ala Thr Ser Leu Ala Phe Ala Cys
       130                 135                 140
   ttt tct gca gta gcc tta gtt gca agg cga agg gag tac ctc tac ctt    480
   Phe Ser Ala Val Ala Leu Val Ala Arg Arg Arg Glu Tyr Leu Tyr Leu
   145                 150                 155                 160
   ggt ggt ttg ctt tct tct tgg ctt tcc att ctt atg tgg ttg cac tct    528
   Gly Gly Leu Leu Ser Ser Trp Leu Ser Ile Leu Met Trp Leu His Ser
                   165                 170                 175
   gat tcc tct ctc ttt ggg ggc tca att gca ctc ttc aag ttt gag ctg    576
   Asp Ser Ser Leu Phe Gly Gly Ser Ile Ala Leu Phe Lys Phe Glu Leu
               180                 185                 190
   tac ttt ggg ctt ttg gtg ttt gtg ggc tac gtt ata gta gac act caa    624
   Tyr Phe Gly Leu Leu Val Phe Val Gly Tyr Val Ile Val Asp Thr Gln
           195                 200                 205
   gaa att att gaa agg gct cac ttt ggt gac ctg gat tat gtg aag cat    672
   Glu Ile Ile Glu Arg Ala His Phe Gly Asp Leu Asp Tyr Val Lys His
       210                 215                 220
   gca ttg aca ttg ttc act gat ttg gct gca atc ttt gtg cga att ctt    720
   Ala Leu Thr Leu Phe Thr Asp Leu Ala Ala Ile Phe Val Arg Ile Leu
   225                 230                 235                 240
   att ata atg ttg aag aat tca tct gag aga aat gag aag aag aag aaa    768
   Ile Ile Met Leu Lys Asn Ser Ser Glu Arg Asn Glu Lys Lys Lys Lys
                   245                 250                 255
   agg aga gat tagtaggctg accgaccgac tcgagctcag gcttctctac           817
   Arg Arg Asp
   agtaatttag tttgtggaga atacataatt agctgtttag atgatgttgg tcccttgtgt 877
   agttagttag ctatgtgttt gctgtaatgg taaatgtcag gatttctttt aaacatcttc 937
   atatgtattt gccaatatca taatgtgtcg tataacatca taccttggtt taaaaaaaaa 997
   aaaaaaaaaa aaaaaaaaaa aaaaaaaaaa aaaaaaaaaa aaaaaaaann nnnnnnnnnn 1057
   nnnnnnnnnn nnnnnnnnnn nnnnnnnnnn nnnnnnnngg tgtttgtggg ctacgttata 1117
   gtagacactc aagtaatcat tgagagggct cactttggtg acctggatta tgttaagcat 1177
   gcattgacac tgttcactga tttggctgca atctttgtgc gaattcttaa tataatgttg 1237
   aataattcat ctaagagaaa tgagaagaag aggaggagag attaataggt tgaccgattg 1297
   ctatgtgtag agtaatttgg tttgtagaga atacataatt agctgtttag aagttgttgg 1357
   tccccttgtg tagttagtag ttagctatgt gtttgctgta atggtaaatg tcaggatttc 1417
   ttttaaacat tttcatatgt atttgctaat aatcataata tatagtataa acatcattcc 1477
   ttggtttaaa aaaagaaaaa aaaaaaaaaa aaa 1510
<210> 14
   <211> 259
   <212> PRT
   <213> Glycine max
<400> 14
   Ile Thr Lys Thr Ile Arg Phe Asp Ser Leu Phe Ser Met Asp Thr Phe
   1 5 10 15
   Phe Lys Ser Pro Ser Ser Ser Ser Ser Arg Ser Arg Trp Ser Tyr Asp
   20 25 30
   Thr Leu Lys Asn Phe Arg Glu Ile Ser Pro Leu Val Gln Asn His Ile
   35 40 45
   Lys Leu Val Tyr Phe Thr Leu Cys Cys Ala Val Val Ala Ala Ala Val
   50 55 60
   Gly Ala Phe Leu His Val Leu Trp Asn Ile Gly Gly Phe Leu Thr Thr
   65 70 75 80
   Leu Ala Ser Ile Gly Ser Met Phe Trp Leu Leu Ser Thr Pro Pro Phe
   85 90 95
   Glu Glu Gln Lys Arg Leu Ser Leu Leu Met Ala Ser Ala Leu Phe Gln
   100 105 110
   Gly Ala Ser Ile Gly Pro Leu Ile Asp Leu Ala Phe Ala Ile Asp Pro
   115 120 125
   Gly Leu Ile Ile Gly Ala Phe Val Ala Thr Ser Leu Ala Phe Ala Cys
   130 135 140
   Phe Ser Ala Val Ala Leu Val Ala Arg Arg Arg Glu Tyr Leu Tyr Leu
   145 150 155 160
   Gly Gly Leu Leu Ser Ser Trp Leu Ser Ile Leu Met Trp Leu His Ser
   165 170 175
   Asp Ser Ser Leu Phe Gly Gly Ser Ile Ala Leu Phe Lys Phe Glu Leu
   180 185 190
   Tyr Phe Gly Leu Leu Val Phe Val Gly Tyr Val Ile Val Asp Thr Gln
   195 200 205
   Glu Ile Ile Glu Arg Ala His Phe Gly Asp Leu Asp Tyr Val Lys His
   210 215 220
   Ala Leu Thr Leu Phe Thr Asp Leu Ala Ala Ile Phe Val Arg Ile Leu
   225 230 235 240
   Ile Ile Met Leu Lys Asn Ser Ser Glu Arg Asn Glu Lys Lys Lys Lys
   245 250 255
   Arg Arg Asp
<210> 15
   <211> 651
   <212> DNA
   <213> Triticum aestivum
<220>
   <221> CDS
   <222> (1)..(651)
   <223> coding for BI-1 protein
<400> 15
   gtc gca atg ccg ggt cga cga ttt cgt ctg acc tat gct ttg cct ggc      48
   Val Ala Met Pro Gly Arg Arg Phe Arg Leu Thr Tyr Ala Leu Pro Gly
   1               5                   10                  15
   ctc atc tgc cgt ggg tgc tta cct gca cat tgc cct gaa cat tgg cgg      96
   Leu Ile Cys Arg Gly Cys Leu Pro Ala His Cys Pro Glu His Trp Arg
               20                  25                  30
   gat gct gac aat gct cgc gtg tat cgg aac cat cgc ctg gat gtt ctc      144
   Asp Ala Asp Asn Ala Arg Val Tyr Arg Asn His Arg Leu Asp Val Leu
           35                  40                  45
   ggt gcc agt cta cga gga gag gaa gag gtt tgg gct gct gat ggg tgc      192
   Gly Ala Ser Leu Arg Gly Glu Glu Glu Val Trp Ala Ala Asp Gly Cys
       50                  55                  60
   agc ctc ctg gaa ggg gct tca gtt gga cct ctg att gag ctt gcc ata      240
   Ser Leu Leu Glu Gly Ala Ser Val Gly Pro Leu Ile Glu Leu Ala Ile
   65                  70                  75                  80
   gac ttt gac cca agt atc ctc gtg aca ggg ttt gtc gga acc gcc atc      288
   Asp Phe Asp Pro Ser Ile Leu Val Thr Gly Phe Val Gly Thr Ala Ile
                   85                  90                  95
   gcc ttc ggg tgc ttc tct ggc gcc gcc atc atc gcc aag cgc agg gag      336
   Ala Phe Gly Cys Phe Ser Gly Ala Ala Ile Ile Ala Lys Arg Arg Glu
               100                 105                 110
   tac ctg tac ctc ggt ggt ctg ctc tcc tcc ggc ctg tcg atc ctg ctc      384
   Tyr Leu Tyr Leu Gly Gly Leu Leu Ser Ser Gly Leu Ser Ile Leu Leu
           115                 120                 125
   tgg ctg cag ttt gcc acg tcc atc ttt ggc cac tcc tct ggc agc ttc      432
   Trp Leu Gln Phe Ala Thr Ser Ile Phe Gly His Ser Ser Gly Ser Phe
       130                 135                 140
   atg ttt gag gtt tac ttt ggc ctg ttg atc ttc ctg gga tac atg gtg      480
   Met Phe Glu Val Tyr Phe Gly Leu Leu Ile Phe Leu Gly Tyr Met Val
   145                 150                 155                 160
   tac gac acg cag gag atc atc gag agg gcg cac cac ggc gac atg gat      528
   Tyr Asp Thr Gln Glu Ile Ile Glu Arg Ala His His Gly Asp Met Asp
                   165                 170                 175
   tac atc aag cac gcg ctc acc ctc ttc acc gac ttc gtc gcc gtt ctc      576
   Tyr Ile Lys His Ala Leu Thr Leu Phe Thr Asp Phe Val Ala Val Leu
               180                 185                 190
   gtc cgc gtc ctc atc atc ttg ctc aag aac gca gcg gac aag gtc gga      624
   Val Arg Val Leu Ile Ile Leu Leu Lys Asn Ala Ala Asp Lys Val Gly
           195                 200                 205
   ggc caa gaa gag gag gaa gag aag tcc                                  651
   Gly Gln Glu Glu Glu Glu Glu Lys Ser
       210                 215
<210> 16
   <211> 217
   <212> PRT
   <213> Triticum aestivum
<400> 16
   Val Ala Met Pro Gly Arg Arg Phe Arg Leu Thr Tyr Ala Leu Pro Gly
   1 5 10 15
   Leu Ile Cys Arg Gly Cys Leu Pro Ala His Cys Pro Glu His Trp Arg
   20 25 30
   Asp Ala Asp Asn Ala Arg Val Tyr Arg Asn His Arg Leu Asp Val Leu
   35 40 45
   Gly Ala Ser Leu Arg Gly Glu Glu Glu Val Trp Ala Ala Asp Gly Cys
   50 55 60
   Ser Leu Leu Glu Gly Ala Ser Val Gly Pro Leu Ile Glu Leu Ala Ile
   65 70 75 80
   Asp Phe Asp Pro Ser Ile Leu Val Thr Gly Phe Val Gly Thr Ala Ile
   85 90 95
   Ala Phe Gly Cys Phe Ser Gly Ala Ala Ile Ile Ala Lys Arg Arg Glu
   100 105 110
   Tyr Leu Tyr Leu Gly Gly Leu Leu Ser Ser Gly Leu Ser Ile Leu Leu
   115 120 125
   Trp Leu Gln Phe Ala Thr Ser Ile Phe Gly His Ser Ser Gly Ser Phe
   130 135 140
   Met Phe Glu Val Tyr Phe Gly Leu Leu Ile Phe Leu Gly Tyr Met Val
   145 150 155 160
   Tyr Asp Thr Gln Glu Ile Ile Glu Arg Ala His His Gly Asp Met Asp
   165 170 175
   Tyr Ile Lys His Ala Leu Thr Leu Phe Thr Asp Phe Val Ala Val Leu
   180 185 190
   Val Arg Val Leu Ile Ile Leu Leu Lys Asn Ala Ala Asp Lys Val Gly
   195 200 205
   Gly Gln Glu Glu Glu Glu Glu Lys Ser
   210 215
<210> 17
   <211> 412
   <212> DNA
   <213> Zea mays
<220>
   <221> CDS
   <222> (3)..(410)
   <223> coding for BI1-protein
<400> 17
   tt gtt att gac ttg gat tcg agg att ctc gtc act gcg ttc gtc ggg      47
      Val Ile Asp Leu Asp Ser Arg Ile Leu Val Thr Ala Phe Val Gly
      1               5                   10                  15
   acc gca gtt gct ttt gca tgc ttc tct ggc gct gcc atc atc gcc aag     95
   Thr Ala Val Ala Phe Ala Cys Phe Ser Gly Ala Ala Ile Ile Ala Lys
                   20                  25                  30
   cgc agg gaa tac ctg tac ctc ggc ggt ctg ctt tca tct ggc ctc tcc    143
   Arg Arg Glu Tyr Leu Tyr Leu Gly Gly Leu Leu Ser Ser Gly Leu Ser
               35                  40                  45
   att ctt ctc tgg ctg cag ttt gct act tca atc ttt ggc cac acc agc    191
   Ile Leu Leu Trp Leu Gln Phe Ala Thr Ser Ile Phe Gly His Thr Ser
           50                  55                  60
   gcg acc ttc atg ttt gag ctc tac ttt ggc ctc ctg gtt ttc ctg gga    239
   Ala Thr Phe Met Phe Glu Leu Tyr Phe Gly Leu Leu Val Phe Leu Gly
       65                  70                  75
   tat atg gtg ttt gac acc cag gag atc atc gag agg gcg cac cgt ggg    287
   Tyr Met Val Phe Asp Thr Gln Glu Ile Ile Glu Arg Ala His Arg Gly
   80                  85                  90                  95
   gac atg gac tac atc aag cac gcg ctg act ctc ttc acc gac ttt gtt    335
   Asp Met Asp Tyr Ile Lys His Ala Leu Thr Leu Phe Thr Asp Phe Val
                   100                 105                 110
   gcg gtt ctt gtt cga atc ctt gtc atc atg atg aag aat gca cag gag    383
   Ala Val Leu Val Arg Ile Leu Val Ile Met Met Lys Asn Ala Gln Glu
               115                 120                 125
   aaa tcc caa gac gag aag aag agg aag aa                             412
   Lys Ser Gln Asp Glu Lys Lys Arg Lys
           130                 135
<210> 18
   <211> 136
   <212> PRT
   <213> Zea mays
<400> 18
   Val Ile Asp Leu Asp Ser Arg Ile Leu Val Thr Ala Phe Val Gly Thr
   1               5                   10                  15
   Ala Val Ala Phe Ala Cys Phe Ser Gly Ala Ala Ile Ile Ala Lys Arg
               20                  25                  30
   Arg Glu Tyr Leu Tyr Leu Gly Gly Leu Leu Ser Ser Gly Leu Ser Ile
           35                  40                  45
   Leu Leu Trp Leu Gln Phe Ala Thr Ser Ile Phe Gly His Thr Ser Ala
       50                  55                  60
   Thr Phe Met Phe Glu Leu Tyr Phe Gly Leu Leu Val Phe Leu Gly Tyr
   65                  70                  75                  80
   Met Val Phe Asp Thr Gln Glu Ile Ile Glu Arg Ala His Arg Gly Asp
                   85                  90                  95
   Met Asp Tyr Ile Lys His Ala Leu Thr Leu Phe Thr Asp Phe Val Ala
               100                 105                 110
   Val Leu Val Arg Ile Leu Val Ile Met Met Lys Asn Ala Gln Glu Lys
           115                 120                 125
   Ser Gln Asp Glu Lys Lys Arg Lys
       130                 135
<210> 19
   <211> 345
   <212> DNA
   <213> Triticum aestivum
<220>
   <221> CDS
   <222> (1)..(342)
<400> 19
   gcc gcc atc atc gcc aag cgc agg gag tac ctg tac ctc ggt ggc ctg     48
   Ala Ala Ile Ile Ala Lys Arg Arg Glu Tyr Leu Tyr Leu Gly Gly Leu
   1               5                   10                  15
   ctc tcc tcc ggc ctg tcg atc ctg ctc tgg ctg cag ttt gcc acg tcc     96
   Leu Ser Ser Gly Leu Ser Ile Leu Leu Trp Leu Gln Phe Ala Thr Ser
               20                  25                  30
   atc ttt ggc cac tcc tct ggc agc ttc atg ttt gag gtt tac ttt ggc     144
   Ile Phe Gly His Ser Ser Gly Ser Phe Met Phe Glu Val Tyr Phe Gly
           35                  40                  45
   ctg ttg atc ttt ctg gga tac atg gtg tac gac acg cag gag atc atc     192
   Leu Leu Ile Phe Leu Gly Tyr Met Val Tyr Asp Thr Gln Glu Ile Ile
       50                  55                  60
   gag agg gcg cac cac ggc gac atg gac tac atc aag cac gcg ctc acc     240
   Glu Arg Ala His His Gly Asp Met Asp Tyr Ile Lys His Ala Leu Thr
   65                  70                  75                  80
   ctc ttc acc gac ttt gtc gcc gtc ctc gtc cgg atc ctc atc atc atg    288
   Leu Phe Thr Asp Phe Val Ala Val Leu Val Arg Ile Leu Ile Ile Met
                   85                  90                  95
   ctc aag aac gca ggc gac aag tcg gag gac aag aag aag agg aag agg    336
   Leu Lys Asn Ala Gly Asp Lys Ser Glu Asp Lys Lys Lys Arg Lys Arg
               100                 105                 110
   agg tcc tga                                                        345
   Arg Ser
<210> 20
   <211> 114
   <212> PRT
   <213> Triticum aestivum
<400> 20
   Ala Ala Ile Ile Ala Lys Arg Arg Glu Tyr Leu Tyr Leu Gly Gly Leu
   1               5                   10                  15
   Leu Ser Ser Gly Leu Ser Ile Leu Leu Trp Leu Gln Phe Ala Thr Ser
               20                  25                  30
   Ile Phe Gly His Ser Ser Gly Ser Phe Met Phe Glu Val Tyr Phe Gly
           35                  40                  45
   Leu Leu Ile Phe Leu Gly Tyr Met Val Tyr Asp Thr Gln Glu Ile Ile
       50                  55                  60
   Glu Arg Ala His His Gly Asp Met Asp Tyr Ile Lys His Ala Leu Thr
   65                  70                  75                  80
   Leu Phe Thr Asp Phe Val Ala Val Leu Val Arg Ile Leu Ile Ile Met
                   85                  90                  95
   Leu Lys Asn Ala Gly Asp Lys Ser Glu Asp Lys Lys Lys Arg Lys Arg
               100                 105                 110
   Arg Ser
<210> 21
   <211> 403
   <212> DNA
   <213> Zea mays
<220>
   <221> CDS
   <222> (1)..(402)
   <223> coding for BI1-protein
<400> 21
   ggc agc atc gcc tgg ctc ttc tcg gtg ccc gtc tac gag gag agg aag      48
   Gly Ser Ile Ala Trp Leu Phe Ser Val Pro Val Tyr Glu Glu Arg Lys
    1               5                  10                  15
   agg tac tgg ctg ctg atg gcg gct gcc ctc ctg gaa ggg gcg tcg gtt      96
   Arg Tyr Trp Leu Leu Met Ala Ala Ala Leu Leu Glu Gly Ala Ser Val
               20                  25                  30
   gga ccc ctc atc aag ctc gcc gtg gaa ttt gac cca agc atc ctg gtg     144
   Gly Pro Leu Ile Lys Leu Ala Val Glu Phe Asp Pro Ser Ile Leu Val
           35                  40                  45
   aca gcg ttc gtg ggg act gcc att gcg ttc gcg tgc ttc tct tgc gcg     192
   Thr Ala Phe Val Gly Thr Ala Ile Ala Phe Ala Cys Phe Ser Cys Ala
       50                  55                  60
   gcc atg gtg gcc aag cgc agg gag tac ctc tac ctg ggc ggg ctg ctc     240
   Ala Met Val Ala Lys Arg Arg Glu Tyr Leu Tyr Leu Gly Gly Leu Leu
   65                  70                  75                  80
   tct tct ggc ctc tcc atc ctg ctc tgg ctg cag ttc gcc gcc tcc atc     288
   Ser Ser Gly Leu Ser Ile Leu Leu Trp Leu Gln Phe Ala Ala Ser Ile
                   85                  90                  95
   ttc ggc cac caa tcc act agc agc ttc atg ttt gag gtc tac ttt ggg     336
   Phe Gly His Gln Ser Thr Ser Ser Phe Met Phe Glu Val Tyr Phe Gly
               100                 105                 110
   ctg ctc atc ttc ctg ggc tac atg gtg tac gac acg cag gag gtc atc     384
   Leu Leu Ile Phe Leu Gly Tyr Met Val Tyr Asp Thr Gln Glu Val Ile
           115                 120                 125
   gag agg gcg cac cac ggc g                                           403
   Glu Arg Ala His His Gly
       130
<210> 22
   <211> 134
   <212> PRT
   <213> Zea mays
<400> 22
   Gly Ser Ile Ala Trp Leu Phe Ser Val Pro Val Tyr Glu Glu Arg Lys
   1               5                   10                  15
   Arg Tyr Trp Leu Leu Met Ala Ala Ala Leu Leu Glu Gly Ala Ser Val
               20                  25                  30
   Gly Pro Leu Ile Lys Leu Ala Val Glu Phe Asp Pro Ser Ile Leu Val
           35                  40                  45
   Thr Ala Phe Val Gly Thr Ala Ile Ala Phe Ala Cys Phe Ser Cys Ala
       50                  55                  60
   Ala Met Val Ala Lys Arg Arg Glu Tyr Leu Tyr Leu Gly Gly Leu Leu
   65                  70                  75                  80
   Ser Ser Gly Leu Ser Ile Leu Leu Trp Leu Gln Phe Ala Ala Ser Ile
                   85                  90                  95
   Phe Gly His Gln Ser Thr Ser Ser Phe Met Phe Glu Val Tyr Phe Gly
               100                 105                 110
   Leu Leu Ile Phe Leu Gly Tyr Met Val Tyr Asp Thr Gln Glu Val Ile
           115                 120                 125
   Glu Arg Ala His His Gly
       130
<210> 23
   <211> 410
   <212> DNA
   <213> Zea mays
<220>
   <221> CDS
   <222> (3)..(410)
   <223> coding for BI1-protein
<400> 23
   gc tgg aac atc ggc gtg agg ctg aca atg ctc ggt tgc atc ggc agc     47
      Trp Asn Ile Gly Val Arg Leu Thr Met Leu Gly Cys Ile Gly Ser
      1               5                   10                  15
   atc gac tgg ctc ttc tcg gtg ccc gtc tac gag gag agg aag agg tat    95
   Ile Asp Trp Leu Phe Ser Val Pro Val Tyr Glu Glu Arg Lys Arg Tyr
                           20                  25                  30
   ggg ctg ctg atg gcg gct gcc ctc ctg gaa ggc gct tcg gtc gga ccc      143
   Gly Leu Leu Met Ala Ala Ala Leu Leu Glu Gly Ala Ser Val Gly Pro
                   35                  40                  45
   ctc gtc aag ctc gcc gtg gaa ttt gac cca agc atc ctg gtg acg gcg      191
   Leu Val Lys Leu Ala Val Glu Phe Asp Pro Ser Ile Leu Val Thr Ala
               50                  55                  60
   ttc gtg ggg act gcc atc gcg ttc gcg tgc ttc tcc ggc gcg gcc atg      239
   Phe Val Gly Thr Ala Ile Ala Phe Ala Cys Phe Ser Gly Ala Ala Met
           65                  70                  75
   gtg gcc agg cgc agg gag tac ctc tac ctg ggc ggg ctg ctc tcg tcg      287
   Val Ala Arg Arg Arg Glu Tyr Leu Tyr Leu Gly Gly Leu Leu Ser Ser
    80                     85                  90                  95
   ggg ctc tcc atc ctg ctc tgg ctg cag ctc gcc gcc tcc atc ttc ggc      335
   Gly Leu Ser Ile Leu Leu Trp Leu Gln Leu Ala Ala Ser Ile Phe Gly
                   100                 105                 110
   cac tcc gca acc agc ttc atg ttc gag gtc tac ttc ggg ctg ctc atc      383
   His Ser Ala Thr Ser Phe Met Phe Glu Val Tyr Phe Gly Leu Leu Ile
               115                 120                 125
   ttc ctg ggc tac gtg gtg tac gac acg                                  410
   Phe Leu Gly Tyr Val Val Tyr Asp Thr
           130                 135
<210> 24
   <211> 136
   <212> PRT
   <213> Zea mays
<400> 24
   Trp Asn Ile Gly Val Arg Leu Thr Met Leu Gly Cys Ile Gly Ser Ile
    1               5                  10                  15
   Asp Trp Leu Phe Ser Val Pro Val Tyr Glu Glu Arg Lys Arg Tyr Gly
               20                  25                  30
   Leu Leu Met Ala Ala Ala Leu Leu Glu Gly Ala Ser Val Gly Pro Leu
           35                  40                  45
   Val Lys Leu Ala Val Glu Phe Asp Pro Ser Ile Leu Val Thr Ala Phe
       50                  55                  60
   Val Gly Thr Ala Ile Ala Phe Ala Cys Phe Ser Gly Ala Ala Met Val
   65                  70                  75                  80
   Ala Arg Arg Arg Glu Tyr Leu Tyr Leu Gly Gly Leu Leu Ser Ser Gly
                   85                  90                  95
   Leu Ser Ile Leu Leu Trp Leu Gln Leu Ala Ala Ser Ile Phe Gly His
               100                 105                 110
   Ser Ala Thr Ser Phe Met Phe Glu Val Tyr Phe Gly Leu Leu Ile Phe
           115                 120                 125
   Leu Gly Tyr Val Val Tyr Asp Thr
       130                 135
<210> 25
   <211> 463
   <212> DNA
   <213> Triticum aestivum
<220>
   <221> CDS
   <222> (1)..(462)
   <223> coding for BI1-protein
<400> 25
   ttc tca ggt acg ttc cgc aat tcc cgg agc gac gat ttc gtg ctc tgc      48
   Phe Ser Gly Thr Phe Arg Asn Ser Arg Ser Asp Asp Phe Val Leu Cys
   1               5                   10                  15
   gaa ctt cag cga gag ctc ccc cga tgc cgg gac gca acc ttg acg gtc      96
   Glu Leu Gln Arg Glu Leu Pro Arg Cys Arg Asp Ala Thr Leu Thr Val
               20                  25                  30
   gta tac gtg atc cca ata gtg ggc cga ata aaa tct gcc gcg ggt gct      144
   Val Tyr Val Ile Pro Ile Val Gly Arg Ile Lys Ser Ala Ala Gly Ala
           35                  40                  45
   tac ctg cac att gcc ctg aac atc ggt ggg atg ctg aca atg ctt gcg      192
   Tyr Leu His Ile Ala Leu Asn Ile Gly Gly Met Leu Thr Met Leu Ala
       50                  55                  60
   tgt atc gga acc att gcc tgg atg ttc tct gtg cca gtc tat gag gag      240
   Cys Ile Gly Thr Ile Ala Trp Met Phe Ser Val Pro Val Tyr Glu Glu
   65                  70                  75                  80
   agg aag agg ttt ggg ctg ctg atg ggt gca gcc ctc ctg gaa ggg gct      288
   Arg Lys Arg Phe Gly Leu Leu Met Gly Ala Ala Leu Leu Glu Gly Ala
                   85                  90                  95
   tcg gtt gga cct ctg att gag ctt gcc ata gac ttt gac cca agc atc      336
   Ser Val Gly Pro Leu Ile Glu Leu Ala Ile Asp Phe Asp Pro Ser Ile
               100                 105                 110
   ctc gtg aca ggg ttt gtt gga acc gcc atc gcc ttt ggg tgc ttc tct      384
   Leu Val Thr Gly Phe Val Gly Thr Ala Ile Ala Phe Gly Cys Phe Ser
           115                 120                 125
   ggc gcc gcc atc atc gcc aag cgc agg gag tac ctg tac ctc gga ggc      432
   Gly Ala Ala Ile Ile Ala Lys Arg Arg Glu Tyr Leu Tyr Leu Gly Gly
       130                 135                 140
   ctg ctc tcc tcc ggc ctg acg atc ctg ctc t                            463
   Leu Leu Ser Ser Gly Leu Thr Ile Leu Leu
   145                 150
<210> 26
   <211> 154
   <212> PRT
   <213> Triticum aestivum
<400> 26
   Phe Ser Gly Thr Phe Arg Asn Ser Arg Ser Asp Asp Phe Val Leu Cys
   1 5 10 15
   Glu Leu Gln Arg Glu Leu Pro Arg Cys Arg Asp Ala Thr Leu Thr Val
   20 25 30
   Val Tyr Val Ile Pro Ile Val Gly Arg Ile Lys Ser Ala Ala Gly Ala
   35 40 45
   Tyr Leu His Ile Ala Leu Asn Ile Gly Gly Met Leu Thr Met Leu Ala
   50 55 60
   Cys Ile Gly Thr Ile Ala Trp Met Phe Ser Val Pro Val Tyr Glu Glu
   65 70 75 80
   Arg Lys Arg Phe Gly Leu Leu Met Gly Ala Ala Leu Leu Glu Gly Ala
   85 90 95
   Ser Val Gly Pro Leu Ile Glu Leu Ala Ile Asp Phe Asp Pro Ser Ile
   100 105 110
   Leu Val Thr Gly Phe Val Gly Thr Ala Ile Ala Phe Gly Cys Phe Ser
   115 120 125
   Gly Ala Ala Ile Ile Ala Lys Arg Arg Glu Tyr Leu Tyr Leu Gly Gly
   130 135 140
   Leu Leu Ser Ser Gly Leu Thr Ile Leu Leu
   145 150
<210> 27
   <211> 388
   <212> DNA
   <213> Zea mays
<220>
   <221> CDS
   <222> (3)..(386)
   <223> coding for BI1-protein
<400> 27
   tc tgg aac atc ggc ggg acg ctg aca atg ctc ggt tgc gtc ggc agc      47
      Trp Asn Ile Gly Gly Thr Leu Thr Met Leu Gly Cys Val Gly Ser
       1               5                  10                  15
   atc gcc tgg ctc ttc tcg gtg ccc gtc tac gag gag agg aag agg tat     95
   Ile Ala Trp Leu Phe Ser Val Pro Val Tyr Glu Glu Arg Lys Arg Tyr
                   20                  25                  30
   ggg ctg ctg atg gcg gct gcc ctc ctg gaa ggc gct tcg gtc gga ccc    143
   Gly Leu Leu Met Ala Ala Ala Leu Leu Glu Gly Ala Ser Val Gly Pro
               35                  40                  45
   ctc gtc aag ctc gcc gtg gaa ttt gac cca agc atc ctg gtg acg gcg    191
   Leu Val Lys Leu Ala Val Glu Phe Asp Pro Ser Ile Leu Val Thr Ala
           50                  55                  60
   ttc gtg ggg act gcc atc gcg ttc gcg tgc ttc tcc ggc gcg cca tgg    239
   Phe Val Gly Thr Ala Ile Ala Phe Ala Cys Phe Ser Gly Ala Pro Trp
       65                  70                  75
   tgg cag gcc agg gag tac ctc tac ctg ggc ggc tgc tct cgt cga ggc    287
   Trp Gln Ala Arg Glu Tyr Leu Tyr Leu Gly Gly Cys Ser Arg Arg Gly
   80                  85                  90                  95
   tct cca tcc tgc tct ggc tgc agc tcg ccg cct cca tct tcg gca ctc    335
   Ser Pro Ser Cys Ser Gly Cys Ser Ser Pro Pro Pro Ser Ser Ala Leu
                   100                 105                 110
   cgc aac agc ttc atg ttc gag gtc tac ttc ggg ctg ctc att ctt ctg    383
   Arg Asn Ser Phe Met Phe Glu Val Tyr Phe Gly Leu Leu Ile Leu Leu
               115                 120                 125
   ggc ta                                                             388
   Gly
<210> 28
   <211> 128
   <212> PRT
   <213> Zea mays
<400> 28
   Trp Asn Ile Gly Gly Thr Leu Thr Met Leu Gly Cys Val Gly Ser Ile
   1               5                   10                  15
   Ala Trp Leu Phe Ser Val Pro Val Tyr Glu Glu Arg Lys Arg Tyr Gly
               20                  25                  30
   Leu Leu Met Ala Ala Ala Leu Leu Glu Gly Ala Ser Val Gly Pro Leu
           35                  40                  45
   Val Lys Leu Ala Val Glu Phe Asp Pro Ser Ile Leu Val Thr Ala Phe
   50 55 60
   Val Gly Thr Ala Ile Ala Phe Ala Cys Phe Ser Gly Ala Pro Trp Trp
   65 70 75 80
   Gln Ala Arg Glu Tyr Leu Tyr Leu Gly Gly Cys Ser Arg Arg Gly Ser
   85 90 95
   Pro Ser Cys Ser Gly Cys Ser Ser Pro Pro Pro Ser Ser Ala Leu Arg
   100 105 110
   Asn Ser Phe Met Phe Glu Val Tyr Phe Gly Leu Leu Ile Leu Leu Gly
   115 120 125
<210> 29
   <211> 1737
   <212> DNA
   <213> Solanum tuberosum
<220>
   <221> promoter
   <222> (1)..(1737)
   <223> patatin promotor
<400> 29
   aagcttatgt tgccatatag agtagtttgt gatggtatac ttcataaact ttaacttatg 60
   ttaaatttgt aatgataaaa tttttattgt aaattaaaaa ttacttataa aattgggcat 120
   tataacatat gaaagacaaa ttgtgttaca tattttactt ttgactttaa tatgaatatt 180
   tcaatttaaa tcattgtttt attttctctt tctttttaca ggtataaaag gtgaaaattg 240
   aagcaagatt gattgcaagc tatgtgtcac cacgttattg atactttgga agaaattttt 300
   acttatatgt ctttgtttag gagtaatatt tgatatgttt tagttagatt ttcttgtcat 360
   ttatgcttta gtataatttt agttattttt attatatgat catgggtgaa ttttgataca 420
   aatatttttg tcattaaata aattaattta tcacaacttg attactttca gtgacaaaaa 480
   atgtattgtc gtagtaccct tttttgttga atatgaataa ttttttttat tttgtgacaa 540
   ttgtaattgt cactacttat gataatattt agtgacatat atgtcgtcgg taaaagcaaa 600
   cactttcagt gacaaaataa tagatttaat cacaaaatta ttaacctttt ttataataat 660
   aaatttatcc ctaatttata catttaagga caaagtattt tttttatata taaaaaatag 720
   tctttagtga cgatcgtagt gttgagtcta gaaatcataa tgttgaatct agaaaaatct 780
   catgcagtgt aaaataaacc tcaaaaagga cgttcagtcc atagaggggg tgtatgtgac 840
   accccaacct cagcaaaaga aaacctccct tcaacaagga catttgcggt gctaaacaat 900
   ttcaagtctc atcacacata tatttattat ataatactaa taaagaatag aaaaggaaag 960
   gtaaacatca ttaaatcgtc tttgtatatt tttagtgaca actgattgac gaaatctttt 1020
   tcgtcacaca aaatttttag tgacgaaaca tgatttatag atgatgaaat tatttgtccc 1080
   tcataatcta atttgttgta gtgatcatta ctcctttgtt tgttttattt gtcatgttag 1140
   tccattaaaa aaaaatatct ctcttcttat gtacgtgaat ggttggaacg gatctattat 1200
   ataatactaa taaagaatag aaaaaggaaa gtgagtgagg ttcgagggag agaatctgtt 1260
   taatatcaga gtcgatcatg tgtcaatttt atcgatatga ccctaacttc aactgagttt 1320
   aaccaattcc gataaggcga gaaatatcat agtattgagt ctagaaaaat ctcatgtagt 1380
   gtggggtaaa cctcagcaag gacgttgagt ccatagaggg gggtgtatgt gacaccccaa 1440
   cctcagcaaa agaaaacctc ccctcaagaa ggacatttgc ggtgctaaac aatttcaagt 1500
   ctcatcacac atatatatat attatataat actaataaat aatagaaaaa ggaaaggtaa 1560
   acatcactaa cgacagttgc ggtgcaaact gagtgaggta ataaacatca ctaactttta 1620
   ttggttatgt caaactcaaa gtaaaatttc tcaacttgtt tacgtgccta tatataccat 1680
   gcttgttata tgctcaaagc accaacaaaa tttaaaaaca ctttgaacat ttgcaaa    1737
<210> 30
   <211> 1317
   <212> DNA
   <213> Triticum aestivum
<220>
   <221> promoter
   <222> (1)..(1317)
   <223> germin 9f-3.8 gene promotor
<400> 30
   gaattcaagc tatcactctc gaaccaagca cattgatgta aggtatcatt ggattccaga 60
   tgtcgtgagt tccaagttgc tgaaacttga gaagatccat accgacgaca atggttcaga 120
   tatgatgacc aagatattgc gaaataagaa gctacaagca tgttgcaagg tagcgggcat 180
   ggcggtgccc ccatcatgag tcggaggggg agatttgttg ggatatcctc ctcatgtggg 240
   ttctgaggag atgaccattt gaggcctttt agccagccca aagaggtgca gaagcccact 300
   acccattagg gttatgacct agggtcattt tggactttgc acatgagtgg atggggatgc 360
   tttaccctcc atccagcagc caccaccaag ggtgacgaaa atcagttcat cctccaagag 420
   agaagaagag agaaaaccaa gagagcaagg gaagaagagg aagattgaag gaagaagaaa 480
   agggagctcc tccccaaggt tgtgatggtc catatccact atcttgtctc cttcaaactt 540
   cggttccacc atctttggta agattgttct aatccctagt tcttgagccc caaatcttgt 600
   tgtgttcatc caagattcag aaatcttgat gtatgagatc ctctagtgct gtctagagaa 660
   gaatttgttg tatcccacat ttgataatag tggaagagga tttgggtggc ttcggcccat 720
   ggtttttccc ctcaagttga ggggttttcc acgtaaaatc tggtgtctct ttgttgatgc 780
   ttggtgttgt ccagaaactt actcctacca caagacacta ggggccagtt cttttgggaa 840
   attctcccag aattgaccct ctccccagct tctcccagaa ttgtcactcc atttttcttt 900
   acaattccta gctagttaag gtctaattag ttaggaattg taaaaaaata tcaagtggca 960
   attctgggag aagctgggga gggggtcaat tctggaagaa ttgcccaaaa gaactggccc 1020
   taggctgagg agtgtcttgc ctgctgctta acattttctg cctccatata tgttgttgca 1080
   tatgtttcct tccgtgctaa gcaacgatcc ttgagttagt acatgatgtg gtgctgagat 1140
   tactttgttt tcgctgcagt tatcagttaa ccacaagtgc atttgcgtgc taattcccaa 1200
   caatatgcca cccgcaactc atccaccata gctcagcagc aaccaccaat gccatagaca 1260
   ctctcggtaa acaacctgta gcttatcagt ctagctaagc gtgctgcata gcaagca 1317
<210> 31
   <211> 959
   <212> DNA
   <213> Arabidopsis thaliana
<220>
   <221> promoter
   <222> (1)..(959)
   <223> CAB-2 promotor
<400> 31
   gaattcatgt gtgagggcaa ttagtgattg taaaaataaa attgtgtttt gtaaaaaact 60
   tttactgtcg aaattattta gggtgatgaa aaaatcagta aactacgaat gatagcttaa 120
   agagtttcta tcaaagtgat tgaggaatag tttgttgcaa attaaacctc taacaaaatg 180
   ttttctgttg tggtttttca tctctacaaa ttttgaattt tatgatgaat tagaaagata 240
   gaatgagtta ctttagattt taaaaggttg ttcaagttta caaaacagat tactagaatc 300
   atgattaaaa atttacaagc tacatattgt ctaaaccaat gatgttgaac ataccagatg 360
   atagtttttc agtgtttgaa caatcaattg gatagttttt atgtttctgc aaaatatgca 420
   aataatcagt gtttttgagt ctttgcattt tgatttaaaa gcaaaaacaa ctgagtttca 480
   aggttaaatt aattacatta ttcatgagat ttatcaggtt agtggataaa ctgacaatgg 540
   aatcaatgtt attgtaaatt ggtagtgatg ttggacttct aatgttactc tctatgatgt 600
   ttcggtcatc ggtatcacac tatctttact tttatttaaa ggaaagatca cacaaataag 660
   ttatctctat tcagaactat taagctgctt ccaaaagact tgcaacatgt ggtctcgaaa 720
   tgctttggct gcaatgaaaa aatcatagca aaagctagtg gactagagac tgccacataa 780
   gaatagtaaa cgttaaaacc aaaatctcaa aaatccaatg agtaaagaga tatagattac 840
   ttcatagata acaaacgtta ctcgcaattt tcctatataa tccaacccta cctaaccatt 900
   ttcaatcact ctcactcaca agttagtcac caaaaaaaaa aaaaacacaa aaagtttca 959
<210> 32
   <211> 445
   <212> DNA
   <213> Zea mays
<220>
   <221> promoter
   <222> (1)..(445)
   <223> PPCZm1 promoter
<400> 32
   gaattccaaa aatagacacg gcaattttct tattcacaga aaaaatataa ctacaactaa 60
   tccccaagtc cacagggatt agggatcaat ctgcaaaact aaaagtactt ttacagttgt 120
   acttggcatg agtcatgtga ccatgagaga ggcgcacggt tcagcaaagc aacataaaat 180
   tctccaaacg ggccccgcca cacacgatca ccatcacccc cgggctcccg acccagtaca 240
   aatagacacg cacactccca actccccacc catctccgcc gcgcacaccg cccaatcagc 300
   caatctcctc ctcctcctcc gctctcagac gagcagcggt tgccatcact ctccacttcc 360
   cacgcccgct gcgggctcgc aggcggcaga gaattgtctg tgccgccggg tgggaatttg 420
   attcggtcgg attccgtgcg ccgcg 445
<210> 33
   <211> 5455
   <212> DNA
   <213> Künstliche Sequenz
<220>
   <223> Beschreibung der künstlichen Sequenz: recombinant expression vector pUbiB1-1
<400> 33
   ggggatcctc tagagtcgac ctgcaggcgg ccgcactagt gattaggatt ccaacgcgag 60
   ccaggacaag cgaggaacct tgcgtgcgag gcgaggccgc cccgctccga ttcgattcga 120
   cgcgcaggcg caggcgcagg gatggacgcc ttctactcga cctcgtcggc ggcggcgagc 180
   ggctggggcc acgactccct caagaacttc cgccagatct cccccgccgt gcagtcccac 240
   ctcaagctcg tttacctgac tctatgcttt gcactggcct catctgccgt gggtgcttac 300
   ctacacattg ccctgaacat cggcgggatg ctgacaatgc tcgcttgtgt cggaactatc 360
   gcctggatgt tctcggtgcc agtctatgag gagaggaaga ggtttgggct gctgatgggt 420
   gcagccctcc tggaaggggc ttcggttgga cctctgattg agcttgccat agactttgac 480
   ccaagcatcc tcgtgacagg gtttgtcgga accgccatcg cctttgggtg cttctctggc 540
   gccgccatca tcgccaagcg cagggagtac ctgtacctcg gtggcctgct ctcgtctggc 600
   ctgtcgatcc tgctctggct gcagtttgtc acgtccatct ttggccactc ctctggcagc 660
   ttcatgtttg aggtttactt tggcctgttg atcttcctgg ggtacatggt gtacgacacg 720
   caggagatca tcgagagggc gcaccatggc gacatggact acatcaagca cgccctcacc 780
   ctcttcaccg actttgttgc cgtcctcgtc cgagtcctca tcatcatgct caagaacgca 840
   ggcgacaagt cggaggacaa gaagaagagg aagagggggt cctgaacgtw tctcccgcac 900
   atgtagatac cgtcaccgcg tcgacctgca ggcatgcccg ctgaaatcac cagtctctct 960
   ctacaaatct atctctctca taataatgtg tgagtagttc ccagataagg gaattagggt 1020
   tcttataggg tttcgctcat gtgttgagca tataagaaac ccttagtatg tatttgtatt 1080
   tgtaaaatac ttctatcaat aaaatttcta attcctaaaa ccaaaatcca gtgggtaccg 1140
   agctcgaatt caagcttggc actggccgtc gttttacaac gtcgtgactg ggaaaaccct 1200
   ggcgttaccc aacttaatcg ccttgcagca catccccctt tcgccagctg gcgtaatagc 1260
   gaagaggccc gcaccgatcg cccttcccaa cagttgcgca gcctgaatgg cgaatggcgc 1320
   ctgatgcggt attttctcct tacgcatctg tgcggtattt cacaccgcat atggtgcact 1380
   ctcagtacaa tctgctctga tgccgcatag ttaagccagc cccgacaccc gccaacaccc 1440
   gctgacgcgc cctgacgggc ttgtctgctc ccggcatccg cttacagaca agctgtgacc 1500
   gtctccggga gctgcatgtg tcagaggttt tcaccgtcat caccgaaacg cgcgagacga 1560
   aagggcctcg tgatacgcct atttttatag gttaatgtca tgataataat ggtttcttag 1620
   acgtcaggtg gcacttttcg gggaaatgtg cgcggaaccc ctatttgttt atttttctaa 1680
   atacattcaa atatgtatcc gctcatgaga caataaccct gataaatgct tcaataatat 1740
   tgaaaaagga agagtatgag tattcaacat ttccgtgtcg cccttattcc cttttttgcg 1800
   gcattttgcc ttcctgtttt tgctcaccca gaaacgctgg tgaaagtaaa agatgctgaa 1860
   gatcagttgg gtgcacgagt gggttacatc gaactggatc tcaacagcgg taagatcctt 1920
   gagagttttc gccccgaaga acgttttcca atgatgagca cttttaaagt tctgctatgt 1980
   ggcgcggtat tatcccgtat tgacgccggg caagagcaac tcggtcgccg catacactat 2040
   tctcagaatg acttggttga gtactcacca gtcacagaaa agcatcttac ggatggcatg 2100
   acagtaagag aattatgcag tgctgccata accatgagtg ataacactgc ggccaactta 2160
   cttctgacaa cgatcggagg accgaaggag ctaaccgctt ttttgcacaa catgggggat 2220
   catgtaactc gccttgatcg ttgggaaccg gagctgaatg aagccatacc aaacgacgag 2280
   cgtgacacca cgatgcctgt agcaatggca acaacgttgc gcaaactatt aactggcgaa 2340
   ctacttactc tagcttcccg gcaacaatta atagactgga tggaggcgga taaagttgca 2400
   ggaccacttc tgcgctcggc ccttccggct ggctggttta ttgctgataa atctggagcc 2460
   ggtgagcgtg ggtctcgcgg tatcattgca gcactggggc cagatggtaa gccctcccgt 2520
   atcgtagtta tctacacgac ggggagtcag gcaactatgg atgaacgaaa tagacagatc 2580
   gctgagatag gtgcctcact gattaagcat tggtaactgt cagaccaagt ttactcatat 2640
   atactttaga ttgatttaaa acttcatttt taatttaaaa ggatctaggt gaagatcctt 2700
   tttgataatc tcatgaccaa aatcccttaa cgtgagtttt cgttccactg agcgtcagac 2760
   cccgtagaaa agatcaaagg atcttcttga gatccttttt ttctgcgcgt aatctgctgc 2820
   ttgcaaacaa aaaaaccacc gctaccagcg gtggtttgtt tgccggatca agagctacca 2880
   actctttttc cgaaggtaac tggcttcagc agagcgcaga taccaaatac tgttcttcta 2940
   gtgtagccgt agttaggcca ccacttcaag aactctgtag caccgcctac atacctcgct 3000
   ctgctaatcc tgttaccagt ggctgctgcc agtggcgata agtcgtgtct taccgggttg 3060
   gactcaagac gatagttacc ggataaggcg cagcggtcgg gctgaacggg gggttcgtgc 3120
   acacagccca gcttggagcg aacgacctac accgaactga gatacctaca gcgtgagctt 3180
   tgagaaagcg ccacgcttcc cgaagggaga aaggcggaca ggtatccggt aagcggcagg 3240
   gtcggaacag gagagcgcac gagggagctt ccagggggaa acgcctggta tctttatagt 3300
   cctgtcgggt ttcgccacct ctgacttgag cgtcgatttt tgtgatgctc gtcagggggg 3360
   cggagcctat ggaaaaacgc cagcaacgcg gcctttttac ggttcctggc cttttgctgg 3420
   ccttttgctc acatgttctt tcctgcgtta tcccctgatt ctgtggataa ccgtattacc 3480
   gcctttgagt gagctgatac cgctcgccgc agccgaacga ccgagcgcag cgagtcagtg 3540
   agcgaggaag cggaagagcg cccaatacgc aaaccgcctc tccccgcgcg ttggccgatt 3600
   cattaatgca gctggcacga caggtttccc gactggaaag cgggcagtga gcgcaacgca 3660
   attaatgtga gttagctcac tcattaggca ccccaggctt tacactttat gcttccggct 3720
   cgtatgttgt gtggaattgt gagcggataa caatttcaca caggaaacag ctatgaccat 3780
   gattacgaat tcccatgcct cgaggatcta acatgcttag atacatgaag taacatgctg 3840
   ctacggttta ataattcttg agttgatttt tactggtact tagatagatg tatatacatg 3900
   cttagataca tgaagtaaca tgctcctaca gttcctttaa tcattattga gtacctatat 3960
   attctaataa atcagtatgt tttaaattat tttgatttta ctggtactta gatagatgta 4020
   tatatacatg ctcaaacatg cttagataca tgaagtaaca tgctgctacg gtttagtcat 4080
   tattgagtgc ctataatttc taataaatca gtatgtttta aattattttg attttactgg 4140
   tacttagata gatgtatata tacatgctca aacatgctta gatacatgaa gtaatatgct 4200
   actacggttt aattgttctt gagtacctat atattctaat aaatcagtat gttttaaatt 4260
   atttcgattt tactggtact tagatagatg tatatataca tgcttagata catgaagtaa 4320
   catgctacta cggtttaatt gttcttgaat acctatatat tctaataaat cagtatgttt 4380
   taaattattt cgattttact ggtacttaga tagatgtata tatacatgct cgaacatgct 4440
   tagatacatg aagtaacatg ctacatatat attataataa atcagtatgt cttaaattat 4500
   tttgatttta ctggtactta gatagatgta tatacatgct caaacatgct tagatacatg 4560
   aagtaacatg ctactacggt ttaatcatta ttgagtacct atatattcta ataaatcagt 4620
   atgttttcaa ttgttttgat tttactggta cttagatata tgtatatata catgctcgaa 4680
   catgcttaga tacgtgaagt aacatgctac tatggttaat tgttcttgag tacctatata 4740
   ttctaataaa tcagtatgtt ttaaattatt tcgattttac tggtacttag atagatgtat 4800
   atatacatgc tcgaacatgc ttagatacat gaagtaacat gctactacgg tttaatcgtt 4860
   cttgagtacc tatatattct aataaatcag tatgtcttaa attatcttga ttttactggt 4920
   acttagatag atgtatatac atgcttagat acatgaagta acatgctact atgatttaat 4980
   cgttcttgag tacctatata ttctaataaa tcagtatgtt tttaattatt ttgattttac 5040
   tggtacttag atagatgtat atatacatgc tcgaacatgc ttagatacat gaagtaacat 5100
   gctactacgg tttaatcatt cttgagtacc tatatattct aataaatcag tatgttttta 5160
   attattttga tattactggt acttaacatg tttagataca tcatatagca tgcacatgct 5220
   gctactgttt aatcattcgt gaatacctat atattctaat atatcagtat gtcttctaat 5280
   tattatgatt ttgatgtact tgtatggtgg catatgctgc agctatgtgt agattttgaa 5340
   tacccagtgt gatgagcatg catggcgcct tcatagttca tatgctgttt atttcctttg 5400
   agactgttct tttttgttga tagtcaccct gttgtttggt gattcttatg caccc      5455
<210> 34
   <211> 12633
   <212> DNA
   <213> Künstliche Sequenz
<220>
   <223> Beschreibung der künstlichen Sequenz: recombinant expression vector pLol14UbiBI-1
<400> 34
   aattcactgg ccgtcgtttt acaacgactc agagcttgac aggaggcccg atctagtaac 60
   atagatgaca ccgcgcgcga taatttatcc tagtttgcgc gctatatttt gttttctatc 120
   gcgtattaaa tgtataattg cgggactcta atcataaaaa cccatctcat aaataacgtc 180
   atgcattaca tgttaattat tacatgctta acgtaattca acagaaatta tatgataatc 240
   atcgcaagac cggcaacagg attcaatctt aagaaacttt attgccaaat gtttgaacga 300
   tcggggatca tccgggtctg tggcgggaac tccacgaaaa tatccgaacg cagcaagatc 360
   tagagcttgg gtcccgctca gaagaactcg tcaagaaggc gatagaaggc gatgcgctgc 420
   gaatcgggag cggcgatacc gtaaagcacg aggaagcggt cagcccattc gccgccaagc 480
   tcttcagcaa tatcacgggt agccaacgct atgtcctgat agcggtccgc cacacccagc 540
   cggccacagt cgatgaatcc agaaaagcgg ccattttcca ccatgatatt cggcaagcag 600
   gcatcgccat gggtcacgac gagatcctcg ccgtcgggca tgcgcgcctt gagcctggcg 660
   aacagttcgg ctggcgcgag cccctgatgc tcttcgtcca gatcatcctg atcgacaaga 720
   ccggcttcca tccgagtacg tgctcgctcg atgcgatgtt tcgcttggtg gtcgaatggg 780
   caggtagccg gatcaagcgt atgcagccgc cgcattgcat cagccatgat ggatactttc 840
   tcggcaggag caaggtgaga tgacaggaga tcctgccccg gcacttcgcc caatagcagc 900
   cagtcccttc ccgcttcagt gacaacgtcg agcacagctg cgcaaggaac gcccgtcgtg 960
   gccagccacg atagccgcgc tgcctcgtcc tgcagttcat tcagggcacc ggacaggtcg 1020
   gtcttgacaa aaagaaccgg gcgcccctgc gctgacagcc ggaacacggc ggcatcagag 1080
   cagccgattg tctgttgtgc ccagtcatag ccgaatagcc tctccaccca agcggccgga 1140
   gaacctgcgt gcaatccatc ttgttcaatc atgcgaaacg atccagatcc ggtgcagatt 1200
   atttggattg agagtgaata tgagactcta attggatacc gaggggaatt tatggaacgt 1260
   cagtggagca tttttgacaa gaaatatttg ctagctgata gtgaccttag gcgacttttg 1320
   aacgcgcaat aatggtttct gacgtatgtg cttagctcat taaactccag aaacccgcgg 1380
   ctgagtggct ccttcaacgt tgcggttctg tcagttccaa acgtaaaacg gcttgtcccg 1440
   cgtcatcggc gggggtcata acgtgactcc cttaattctc cgctcatgat cagattgtcg 1500
   tttcccgcct tcagtttaaa ctatcagtgt ttgacaggat cctgcttggt aataattgtc 1560
   attagattgt ttttatgcat agatgcactc gaaatcagcc aattttagac aagtatcaaa 1620
   cggatgttaa ttcagtacat taaagacgtc cgcaatgtgt tattaagttg tctaagcgtc 1680
   aatttgttta caccacaata tatcctgcca ccagccagcc aacagctccc cgaccggcag 1740
   ctcggcacaa aatcaccacg cgttaccacc acgccggccg gccgcatggt gttgaccgtg 1800
   ttcgccggca ttgccgagtt cgagcgttcc ctaatcatcg accgcacccg gagcgggcgc 1860
   gaggccgcca aggcccgagg cgtgaagttt ggcccccgcc ctaccctcac cccggcacag 1920
   atcgcgcacg cccgcgagct gatcgaccag gaaggccgca ccgtgaaaga ggcggctgca 1980
   ctgcttggcg tgcatcgctc gaccctgtac cgcgcacttg agcgcagcga ggaagtgacg 2040
   cccaccgagg ccaggcggcg cggtgccttc cgtgaggacg cattgaccga ggccgacgcc 2100
   ctggcggccg ccgagaatga acgccaagag gaacaagcat gaaaccgcac caggacggcc 2160
   aggacgaacc gtttttcatt accgaagaga tcgaggcgga gatgatcgcg gccgggtacg 2220
   tgttcgagcc gcccgcgcac gtctcaaccg tgcggctgca tgaaatcctg gccggtttgt 2280
   ctgatgccaa gctggcggcc tggccggcca gcttggccgc tgaagaaacc gagcgccgcc 2340
   gtctaaaaag gtgatgtgta tttgagtaaa acagcttgcg tcatgcggtc gctgcgtata 2400
   tgatgcgatg agtaaataaa caaatacgca aggggaacgc atgaaggtta tcgctgtact 2460
   taaccagaaa ggcgggtcag gcaagacgac catcgcaacc catctagccc gcgccctgca 2520
   actcgccggg gccgatgttc tgttagtcga ttccgatccc cagggcagtg cccgcgattg 2580
   ggcggccgtg cgggaagatc aaccgctaac cgttgtcggc atcgaccgcc cgacgattga 2640
   ccgcgacgtg aaggccatcg gccggcgcga cttcgtagtg atcgacggag cgccccaggc 2700
   ggcggacttg gctgtgtccg cgatcaaggc agccgacttc gtgctgattc cggtgcagcc 2760
   aagcccttac gacatatggg ccaccgccga cctggtggag ctggttaagc agcgcattga 2820
   ggtcacggat ggaaggctac aagcggcctt tgtcgtgtcg cgggcgatca aaggcacgcg 2880
   catcggcggt gaggttgccg aggcgctggc cgggtacgag ctgcccattc ttgagtcccg 2940
   tatcacgcag cgcgtgagct acccaggcac tgccgccgcc ggcacaaccg ttcttgaatc 3000
   agaacccgag ggcgacgctg cccgcgaggt ccaggcgctg gccgctgaaa ttaaatcaaa 3060
   actcatttga gttaatgagg taaagagaaa atgagcaaaa gcacaaacac gctaagtgcc 3120
   ggccgtccga gcgcacgcag cagcaaggct gcaacgttgg ccagcctggc agacacgcca 3180
   gccatgaagc gggtcaactt tcagttgccg gcggaggatc acaccaagct gaagatgtac 3240
   gcggtacgcc aaggcaagac cattaccgag ctgctatctg aatacatcgc gcagctacca 3300
   gagtaaatga gcaaatgaat aaatgagtag atgaatttta gcggctaaag gaggcggcat 3360
   ggaaaatcaa gaacaaccag gcaccgacgc cgtggaatgc cccatgtgtg gaggaacggg 3420
   cggttggcca ggcgtaagcg gctgggttgt ctgccggccc tgcaatggca ctggaacccc 3480
   caagcccgag gaatcggcgt gagcggtcgc aaaccatccg gcccggtaca aatcggcgcg 3540
   gcgctgggtg atgacctggt ggagaagttg aaggccgcgc aggccgccca gcggcaacgc 3600
   atcgaggcag aagcacgccc cggtgaatcg tggcaagcgg ccgctgatcg aatccgcaaa 3660
   gaatcccggc aaccgccggc agccggtgcg ccgtcgatta ggaagccgcc caagggcgac 3720
   gagcaaccag attttttcgt tccgatgctc tatgacgtgg gcacccgcga tagtcgcagc 3780
   atcatggacg tggccgtttt ccgtctgtcg aagcgtgacc gacgagctgg cgaggtgatc 3840
   cgctacgagc ttccagacgg gcacgtagag gtttccgcag ggccggccgg catggccagt 3900
   gtgtgggatt acgacctggt actgatggcg gtttcccatc taaccgaatc catgaaccga 3960
   taccgggaag ggaagggaga caagcccggc cgcgtgttcc gtccacacgt tgcggacgta 4020
   ctcaagttct gccggcgagc cgatggcgga aagcagaaag acgacctggt agaaacctgc 4080
   attcggttaa acaccacgca cgttgccatg cagcgtacga agaaggccaa gaacggccgc 4140
   ctggtgacgg tatccgaggg tgaagccttg attagccgct acaagatcgt aaagagcgaa 4200
   accgggcggc cggagtacat cgagatcgag ctagctgatt ggatgtaccg cgagatcaca 4260
   gaaggcaaga acccggacgt gctgacggtt caccccgatt actttttgat cgatcccggc 4320
   atcggccgtt ttctctaccg cctggcacgc cgcgccgcag gcaaggcaga agccagatgg 4380
   ttgttcaaga cgatctacga acgcagtggc agcgccggag agttcaagaa gttctgtttc 4440
   accgtgcgca agctgatcgg gtcaaatgac ctgccggagt acgatttgaa ggaggaggcg 4500
   gggcaggctg gcccgatcct agtcatgcgc taccgcaacc tgatcgaggg cgaagcatcc 4560
   gccggttcct aatgtacgga gcagatgcta gggcaaattg ccctagcagg ggaaaaaggt 4620
   cgaaaaggtc tctttcctgt ggatagcacg tacattggga acccaaagcc gtacattggg 4680
   aaccggaacc cgtacattgg gaacccaaag ccgtacattg ggaaccggtc acacatgtaa 4740
   gtgactgata taaaagagaa aaaaggcgat ttttccgcct aaaactcttt aaaacttatt 4800
   aaaactctta aaacccgcct ggcctgtgca taactgtctg gccagcgcac agccgaagag 4860
   ctgcaaaaag cgcctaccct tcggtcgctg cgctccctac gccccgccgc ttcgcgtcgg 4920
   cctatcgcgg ccgctggccg ctcaaaaatg gctggcctac ggccaggcaa tctaccaggg 4980
   cgcggacaag ccgcgccgtc gccactcgac cgccggcgcc cacatcaagg caccctgcct 5040
   cgcgcgtttc ggtgatgacg gtgaaaacct ctgacacatg cagctcccgg agacggtcac 5100
   agcttgtctg taagcggatg ccgggagcag acaagcccgt cagggcgcgt cagcgggtgt 5160
   tggcgggtgt cggggcgcag ccatgaccca gtcacgtagc gatagcggag tgtatactgg 5220
   cttaactatg cggcatcaga gcagattgta ctgagagtgc accatatgcg gtgtgaaata 5280
   ccgcacagat gcgtaaggag aaaataccgc atcaggcgct cttccgcttc ctcgctcact 5340
   gactcgctgc gctcggtcgt tcggctgcgg cgagcggtat cagctcactc aaaggcggta 5400
   atacggttat ccacagaatc aggggataac gcaggaaaga acatgtgagc aaaaggccag 5460
   caaaaggcca ggaaccgtaa aaaggccgcg ttgctggcgt ttttccatag gctccgcccc 5520
   cctgacgagc atcacaaaaa tcgacgctca agtcagaggt ggcgaaaccc gacaggacta 5580
   taaagatacc aggcgtttcc ccctggaagc tccctcgtgc gctctcctgt tccgaccctg 5640
   ccgcttaccg gatacctgtc cgcctttctc ccttcgggaa gcgtggcgct ttctcatagc 5700
   tcacgctgta ggtatctcag ttcggtgtag gtcgttcgct ccaagctggg ctgtgtgcac 5760
   gaaccccccg ttcagcccga ccgctgcgcc ttatccggta actatcgtct tgagtccaac 5820
   ccggtaagac acgacttatc gccactggca gcagccactg gtaacaggat tagcagagcg 5880
   aggtatgtag gcggtgctac agagttcttg aagtggtggc ctaactacgg ctacactaga 5940
   aggacagtat ttggtatctg cgctctgctg aagccagtta ccttcggaaa aagagttggt 6000
   agctcttgat ccggcaaaca aaccaccgct ggtagcggtg gtttttttgt ttgcaagcag 6060
   cagattacgc gcagaaaaaa aggatctcaa gaagatcctt tgatcttttc tacggggtct 6120
   gacgctcagt ggaacgaaaa ctcacgttaa gggattttgg tcatgcatga tatatctccc 6180
   aatttgtgta gggcttatta tgcacgctta aaaataataa aagcagactt gacctgatag 6240
   tttggctgtg agcaattatg tgcttagtgc atctaacgct tgagttaagc cgcgccgcga 6300
   agcggcgtcg gcttgaacga atttctagct agacattatt tgccgactac cttggtgatc 6360
   tcgcctttca cgtagtggac aaattcttcc aactgatctg cgcgcgaggc caagcgatct 6420
   tcttcttgtc caagataagc ctgtctagct tcaagtatga cgggctgata ctgggccggc 6480
   aggcgctcca ttgcccagtc ggcagcgaca tccttcggcg cgattttgcc ggttactgcg 6540
   ctgtaccaaa tgcgggacaa cgtaagcact acatttcgct catcgccagc ccagtcgggc 6600
   ggcgagttcc atagcgttaa ggtttcattt agcgcctcaa atagatcctg ttcaggaacc 6660
   ggatcaaaga gttcctccgc cgctggacct accaaggcaa cgctatgttc tcttgctttt 6720
   gtcagcaaga tagccagatc aatgtcgatc gtggctggct cgaagatacc tgcaagaatg 6780
   tcattgcgct gccattctcc aaattgcagt tcgcgcttag ctggataacg ccacggaatg 6840
   atgtcgtcgt gcacaacaat ggtgacttct acagcgcgga gaatctcgct ctctccaggg 6900
   gaagccgaag tttccaaaag gtcgttgatc aaagctcgcc gcgttgtttc atcaagcctt 6960
   acggtcaccg taaccagcaa atcaatatca ctgtgtggct tcaggccgcc atccactgcg 7020
   gagccgtaca aatgtacggc cagcaacgtc ggttcgagat ggcgctcgat gacgccaact 7080
   acctctgata gttgagtcga tacttcggcg atcaccgctt cccccatgat gtttaacttt 7140
   gttttagggc gactgccctg ctgcgtaaca tcgttgctgc tccataacat caaacatcga 7200
   cccacggcgt aacgcgcttg ctgcttggat gcccgaggca tagactgtac cccaaaaaaa 7260
   cagtcataac aagccatgaa aaccgccact gcgggggttc catggacata caaatggacg 7320
   aacggataaa ccttttcacg cccttttaaa tatccgatta ttctaataaa cgctcttttc 7380
   tcttaggttt acccgccaat atatcctgtc aaacactgat agtttaaact gaaggcggga 7440
   aacgacaatc agatctagta ggaaacagct atgaccatga ttacgccaag cttgcatgcc 7500
   tgcaggtcga ctctagagga tcgatccccg ggtaggtcag tcccttatgt tacgtcctgt 7560
   agaaacccca acccgtgaaa tcaaaaaact cgacggcctg tgggcattca gtctggatcg 7620
   cgaaaactgt ggaattggtc agcgttggtg ggaaagcgcg ttacaagaaa gccgggcaat 7680
   tgctgtgcca ggcagtttta acgatcagtt cgccgatgca gatattcgta attatgcggg 7740
   caacgtctgg tatcagcgcg aagtctttat accgaaaggt tgggcaggcc agcgtatcgt 7800
   gctgcgtttc gatgcggtca ctcattacgg caaagtgtgg gtcaataatc aggaagtgat 7860
   ggagcatcag ggcggctata cgccatttga agccgatgtc acgccgtatg ttattgccgg 7920
   gaaaagtgta cgtaagtttc tgcttctacc tttgatatat atataataat tatcattaat 7980
   tagtagtaat ataatatttc aaatattttt ttcaaaataa aagaatgtag tatatagcaa 8040
   ttgcttttct gtagtttata agtgtgtata ttttaattta taacttttct aatatatgac 8100
   caaaatttgt tgatgtgcag gtatcaccgt ttgtgtgaac aacgaactga actggcagac 8160
   tatcccgccg ggaatggtga ttaccgacga aaacggcaag aaaaagcagt cttacttcca 8220
   tgatttcttt aactatgccg gaatccatcg cagcgtaatg ctctacacca cgccgaacac 8280
   ctgggtggac gatatcaccg tggtgacgca tgtcgcgcaa gactgtaacc acgcgtctgt 8340
   tgactggcag gtggtggcca atggtgatgt cagcgttgaa ctgcgtgatg cggatcaaca 8400
   ggtggttgca actggacaag gcactagcgg gactttgcaa gtggtgaatc cgcacctctg 8460
   gcaaccgggt gaaggttatc tctatgaact gtgcgtcaca gccaaaagcc agacagagtg 8520
   tgatatctac ccgcttcgcg tcggcatccg gtcagtggca gtgaagggcg aacagttcct 8580
   gattaaccac aaaccgttct actttactgg ctttggtcgt catgaagatg cggacttgcg 8640
   tggcaaagga ttcgataacg tgctgatggt gcacgaccac gcattaatgg actggattgg 8700
   ggccaactcc taccgtacct cgcattaccc ttacgctgaa gagatgctcg actgggcaga 8760
   tgaacatggc atcgtggtga ttgatgaaac tgctgctgtc ggctttaacc tctctttagg 8820
   cattggtttc gaagcgggca acaagccgaa agaactgtac agcgaagagg cagtcaacgg 8880
   ggaaactcag caagcgcact tacaggcgat taaagagctg atagcgcgtg acaaaaacca 8940
   cccaagcgtg gtgatgtgga gtattgccaa cgaaccggat acccgtccgc aaggtgcacg 9000
   ggaatatttc gcgccactgg cggaagcaac gcgtaaactc gacccgacgc gtccgatcac 9060
   ctgcgtcaat gtaatgttct gcgacgctca caccgatacc atcagcgatc tctttgatgt 9120
   gctgtgcctg aaccgttatt acggatggta tgtccaaagc ggcgatttgg aaacggcaga 9180
   gaaggtactg gaaaaagaac ttctggcctg gcaggagaaa ctgcatcagc cgattatcat 9240
   caccgaatac ggcgtggata cgttagccgg gctgcactca atgtacaccg acatgtggag 9300
   tgaagagtat cagtgtgcat ggctggatat gtatcaccgc gtctttgatc gcgtcagcgc 9360
   cgtcgtcggt gaacaggtat ggaatttcgc cgattttgcg acctcgcaag gcatattgcg 9420
   cgttggcggt aacaagaaag ggatcttcac tcgcgaccgc aaaccgaagt cggcggcttt 9480
   tctgctgcaa aaacgctgga ctggcatgaa cttcggtgaa aaaccgcagc agggaggcaa 9540
   acaatgagag ctcgaatttc cccgatcggt caaacatttg gcaataaagn ttcttaagat 9600
   tgaatcctgt tgccggtctt gcgatgatta tcatataatt tctgttgaat tacgttaagc 9660
   atgtaataat taacatgtaa tgcatgacgt tatttatgag atgggttttt atgattagag 9720
   tcccgcaatt atacatttaa tacgcgatag aaaacaaaat atancgcgca aactaggata 9780
   aattatcgcg cgcggtgtca tctatgttac tagatcggga attcccatgc ctcgaggatc 9840
   taacatgctt agatacatga agtaacatgc tgctacggtt taataattct tgagttgatt 9900
   tttactggta cttagataga tgtatataca tgcttagata catgaagtaa catgctccta 9960
   cagttccttt aatcattatt gagtacctat atattctaat aaatcagtat gttttaaatt 10020
   attttgattt tactggtact tagatagatg tatatataca tgctcaaaca tgcttagata 10080
   catgaagtaa catgctgcta cggtttagtc attattgagt gcctataatt tctaataaat 10140
   cagtatgttt taaattattt tgattttact ggtacttaga tagatgtata tatacatgct 10200
   caaacatgct tagatacatg aagtaatatg ctactacggt ttaattgttc ttgagtacct 10260
   atatattcta ataaatcagt atgttttaaa ttatttcgat tttactggta cttagataga 10320
   tgtatatata catgcttaga tacatgaagt aacatgctac tacggtttaa ttgttcttga 10380
   atacctatat attctaataa atcagtatgt tttaaattat ttcgatttta ctggtactta 10440
   gatagatgta tatatacatg ctcgaacatg cttagataca tgaagtaaca tgctacatat 10500
   atattataat aaatcagtat gtcttaaatt attttgattt tactggtact tagatagatg 10560
   tatatacatg ctcaaacatg cttagataca tgaagtaaca tgctactacg gtttaatcat 10620
   tattgagtac ctatatattc taataaatca gtatgttttc aattgttttg attttactgg 10680
   tacttagata tatgtatata tacatgctcg aacatgctta gatacgtgaa gtaacatgct 10740
   actatggtta attgttcttg agtacctata tattctaata aatcagtatg ttttaaatta 10800
   tttcgatttt actggtactt agatagatgt atatatacat gctcgaacat gcttagatac 10860
   atgaagtaac atgctactac ggtttaatcg ttcttgagta cctatatatt ctaataaatc 10920
   agtatgtctt aaattatctt gattttactg gtacttagat agatgtatat acatgcttag 10980
   atacatgaag taacatgcta ctatgattta atcgttcttg agtacctata tattctaata 11040
   aatcagtatg tttttaatta ttttgatttt actggtactt agatagatgt atatatacat 11100
   gctcgaacat gcttagatac atgaagtaac atgctactac ggtttaatca ttcttgagta 11160
   cctatatatt ctaataaatc agtatgtttt taattatttt gatattactg gtacttaaca 11220
   tgtttagata catcatatag catgcacatg ctgctactgt ttaatcattc gtgaatacct 11280
   atatattcta atatatcagt atgtcttcta attattatga ttttgatgta cttgtatggt 11340
   ggcatatgct gcagctatgt gtagattttg aatacccagt gtgatgagca tgcatggcgc 11400
   cttcatagtt catatgctgt ttatttcctt tgagactgtt cttttttgtt gatagtcacc 11460
   ctgttgtttg gtgattctta tgcacccggg gatcctctag agtcgacctg caggcggccg 11520
   cactagtgat taggattcca acgcgagcca ggacaagcga ggaaccttgc gtgcgaggcg 11580
   aggccgcccc gctccgattc gattcgacgc gcaggcgcag gcgcagggat ggacgccttc 11640
   tactcgacct cgtcggcggc ggcgagcggc tggggccacg actccctcaa gaacttccgc 11700
   cagatctccc ccgccgtgca gtcccacctc aagctcgttt acctgactct atgctttgca 11760
   ctggcctcat ctgccgtggg tgcttaccta cacattgccc tgaacatcgg cgggatgctg 11820
   acaatgctcg cttgtgtcgg aactatcgcc tggatgttct cggtgccagt ctatgaggag 11880
   aggaagaggt ttgggctgct gatgggtgca gccctcctgg aaggggcttc ggttggacct 11940
   ctgattgagc ttgccataga ctttgaccca agcatcctcg tgacagggtt tgtcggaacc 12000
   gccatcgcct ttgggtgctt ctctggcgcc gccatcatcg ccaagcgcag ggagtacctg 12060
   tacctcggtg gcctgctctc gtctggcctg tcgatcctgc tctggctgca gtttgtcacg 12120
   tccatctttg gccactcctc tggcagcttc atgtttgagg tttactttgg cctgttgatc 12180
   ttcctggggt acatggtgta cgacacgcag gagatcatcg agagggcgca ccatggcgac 12240
   atggactaca tcaagcacgc cctcaccctc ttcaccgact ttgttgccgt cctcgtccga 12300
   gtcctcatca tcatgctcaa gaacgcaggc gacaagtcgg aggacaagaa gaagaggaag 12360
   agggggtcct gaacgtwtct cccgcacatg tagataccgt caccgcgtcg acctgcaggc 12420
   atgcccgctg aaatcaccag tctctctcta caaatctatc tctctcataa taatgtgtga 12480
   gtagttccca gataagggaa ttagggttct tatagggttt cgctcatgtg ttgagcatat 12540
   aagaaaccct tagtatgtat ttgtatttgt aaaatacttc tatcaataaa atttctaatt 12600
   cctaaaacca aaatccagtg ggtaccgagc tcg                               12633
<210> 35
   <211> 5598
   <212> DNA
   <213> Künstliche Sequenz
<220>
   <223> Beschreibung der künstlichen Sequenz: recombinant expression vector pOXoBI-1
<400> 35
   ggggatcctc tagagtcgac ctgcaggcgg ccgcactagt gattaggatt ccaacgcgag 60
   ccaggacaag cgaggaacct tgcgtgcgag gcgaggccgc cccgctccga ttcgattcga 120
   cgcgcaggcg caggcgcagg gatggacgcc ttctactcga cctcgtcggc ggcggcgagc 180
   ggctggggcc acgactccct caagaacttc cgccagatct cccccgccgt gcagtcccac 240
   ctcaagctcg tttacctgac tctatgcttt gcactggcct catctgccgt gggtgcttac 300
   ctacacattg ccctgaacat cggcgggatg ctgacaatgc tcgcttgtgt cggaactatc 360
   gcctggatgt tctcggtgcc agtctatgag gagaggaaga ggtttgggct gctgatgggt 420
   gcagccctcc tggaaggggc ttcggttgga cctctgattg agcttgccat agactttgac 480
   ccaagcatcc tcgtgacagg gtttgtcgga accgccatcg cctttgggtg cttctctggc 540
   gccgccatca tcgccaagcg cagggagtac ctgtacctcg gtggcctgct ctcgtctggc 600
   ctgtcgatcc tgctctggct gcagtttgtc acgtccatct ttggccactc ctctggcagc 660
   ttcatgtttg aggtttactt tggcctgttg atcttcctgg ggtacatggt gtacgacacg 720
   caggagatca tcgagagggc gcaccatggc gacatggact acatcaagca cgccctcacc 780
   ctcttcaccg actttgttgc cgtcctcgtc cgagtcctca tcatcatgct caagaacgca 840
   ggcgacaagt cggaggacaa gaagaagagg aagagggggt cctgaacgtw tctcccgcac 900
   atgtagatac cgtcaccgcg tcgacctgca ggcatgcccg ctgaaatcac cagtctctct 960
   ctacaaatct atctctctca taataatgtg tgagtagttc ccagataagg gaattagggt 1020
   tcttataggg tttcgctcat gtgttgagca tataagaaac ccttagtatg tatttgtatt 1080
   tgtaaaatac ttctatcaat aaaatttcta attcctaaaa ccaaaatcca gtgggtaccg 1140
   agctcgaatt caagcttggc actggccgtc gttttacaac gtcgtgactg ggaaaaccct 1200
   ggcgttaccc aacttaatcg ccttgcagca catccccctt tcgccagctg gcgtaatagc 1260
   gaagaggccc gcaccgatcg cccttcccaa cagttgcgca gcctgaatgg cgaatggcgc 1320
   ctgatgcggt attttctcct tacgcatctg tgcggtattt cacaccgcat atggtgcact 1380
   ctcagtacaa tctgctctga tgccgcatag ttaagccagc cccgacaccc gccaacaccc 1440
   gctgacgcgc cctgacgggc ttgtctgctc ccggcatccg cttacagaca agctgtgacc 1500
   gtctccggga gctgcatgtg tcagaggttt tcaccgtcat caccgaaacg cgcgagacga 1560
   aagggcctcg tgatacgcct atttttatag gttaatgtca tgataataat ggtttcttag 1620
   acgtcaggtg gcacttttcg gggaaatgtg cgcggaaccc ctatttgttt atttttctaa 1680
   atacattcaa atatgtatcc gctcatgaga caataaccct gataaatgct tcaataatat 1740
   tgaaaaagga agagtatgag tattcaacat ttccgtgtcg cccttattcc cttttttgcg 1800
   gcattttgcc ttcctgtttt tgctcaccca gaaacgctgg tgaaagtaaa agatgctgaa 1860
   gatcagttgg gtgcacgagt gggttacatc gaactggatc tcaacagcgg taagatcctt 1920
   gagagttttc gccccgaaga acgttttcca atgatgagca cttttaaagt tctgctatgt 1980
   ggcgcggtat tatcccgtat tgacgccggg caagagcaac tcggtcgccg catacactat 2040
   tctcagaatg acttggttga gtactcacca gtcacagaaa agcatcttac ggatggcatg 2100
   acagtaagag aattatgcag tgctgccata accatgagtg ataacactgc ggccaactta 2160
   cttctgacaa cgatcggagg accgaaggag ctaaccgctt ttttgcacaa catgggggat 2220
   catgtaactc gccttgatcg ttgggaaccg gagctgaatg aagccatacc aaacgacgag 2280
   cgtgacacca cgatgcctgt agcaatggca acaacgttgc gcaaactatt aactggcgaa 2340
   ctacttactc tagcttcccg gcaacaatta atagactgga tggaggcgga taaagttgca 2400
   ggaccacttc tgcgctcggc ccttccggct ggctggttta ttgctgataa atctggagcc 2460
   ggtgagcgtg ggtctcgcgg tatcattgca gcactggggc cagatggtaa gccctcccgt 2520
   atcgtagtta tctacacgac ggggagtcag gcaactatgg atgaacgaaa tagacagatc 2580
   gctgagatag gtgcctcact gattaagcat tggtaactgt cagaccaagt ttactcatat 2640
   atactttaga ttgatttaaa acttcatttt taatttaaaa ggatctaggt gaagatcctt 2700
   tttgataatc tcatgaccaa aatcccttaa cgtgagtttt cgttccactg agcgtcagac 2760
   cccgtagaaa agatcaaagg atcttcttga gatccttttt ttctgcgcgt aatctgctgc 2820
   ttgcaaacaa aaaaaccacc gctaccagcg gtggtttgtt tgccggatca agagctacca 2880
   actctttttc cgaaggtaac tggcttcagc agagcgcaga taccaaatac tgttcttcta 2940
   gtgtagccgt agttaggcca ccacttcaag aactctgtag caccgcctac atacctcgct 3000
   ctgctaatcc tgttaccagt ggctgctgcc agtggcgata agtcgtgtct taccgggttg 3060
   gactcaagac gatagttacc ggataaggcg cagcggtcgg gctgaacggg gggttcgtgc 3120
   acacagccca gcttggagcg aacgacctac accgaactga gatacctaca gcgtgagctt 3180
   tgagaaagcg ccacgcttcc cgaagggaga aaggcggaca ggtatccggt aagcggcagg 3240
   gtcggaacag gagagcgcac gagggagctt ccagggggaa acgcctggta tctttatagt 3300
   cctgtcgggt ttcgccacct ctgacttgag cgtcgatttt tgtgatgctc gtcagggggg 3360
   cggagcctat ggaaaaacgc cagcaacgcg gcctttttac ggttcctggc cttttgctgg 3420
   ccttttgctc acatgttctt tcctgcgtta tcccctgatt ctgtggataa ccgtattacc 3480
   gcctttgagt gagctgatac cgctcgccgc agccgaacga ccgagcgcag cgagtcagtg 3540
   agcgaggaag cggaagagcg cccaatacgc aaaccgcctc tccccgcgcg ttggccgatt 3600
   cattaatgca gctggcacga caggtttccc gactggaaag cgggcagtga gcgcaacgca 3660
   attaatgtga gttagctcac tcattaggca ccccaggctt tacactttat gcttccggct 3720
   cgtatgttgt gtggaattgt gagcggataa caatttcaca caggaaacag ctatgaccat 3780
   gattacgaat tcccatgcct cgagcagaaa gatataatat gtaaaaaaat gggtctatat 3840
   atatggaagg tttcaggaag acaaaggttc tagaaacttc caaaaaaaat ccagaatata 3900
   ttttggaaga aataccctct tgggttggcc ccggcgcagc ccctagtggg ccaaaaagcc 3960
   acgatctaat cccggtctaa ttggtctaat agtttagact tctaattaga cgggctctta 4020
   tgccggtcta attggtctaa ttagattaaa atcctaatta aatatgaacg caactaggct 4080
   tcccctctct ctagtttcct cggagctctt tttcatggac cttgaagtat tgccggatca 4140
   ctacttcgga actcgtggat acttcagagt gcacatctac tttgaatctt gattggtaga 4200
   tcatctcgga gaaattctca cagttgggag gtataaccag ttgccgaaat tgccatgctt 4260
   cactcacagc caggatcagc ccatgtccca aggcaaccct tgtagctaca tgccgaggcc 4320
   tgactacttg gggcctcgcg ccctgcattt ttgcatgttc atgtgacacg ttaaatgttg 4380
   agagaaatag attactaaat atcacccatt tcgttattct agatgagtat cctacaatat 4440
   gtataccgaa aaatgtattt taaactgtgg taggtgagaa agatctatta aaaagaactc 4500
   tacgtatact cccccctccc aatccccatc caggtttgta agacactttc gtcttttttt 4560
   gccgaatttt aaccgtaaat ttgactagta aaaataagtt atactgaatg taataaatat 4620
   cgtacattcg gatgttggag acagggagag gctggctggt gcgctggatg gatcacggtc 4680
   agaaagtctg acttgcaacg ccacaggccc gttgattgcc actgacaacc aagttttcgt 4740
   tgtttcgctg gtgccatatt ttccgcgatc gaatatttaa actgcgagga gaaaggcaag 4800
   cagggcgcca tatcagcact tgatcactca ctgatcgatc agtagtagcc accttctctg 4860
   cgccgacgtg ttatatatta ttggcaacaa gtcatcgatt gagaacagaa acaaaacaag 4920
   aagagaacta tttgagagag agtagttacg ccgcagcgag tagcctccca tttctgacga 4980
   tcatgccata cgataaaccg gccggcggcg agaccagtta gcaaggttga aatgccaaca 5040
   catgtcgcgc tcatttctcg gctttttcat tttgcatgtc gtcatgcagg ccctggacac 5100
   tgacatttct ctcttttgct gttgaatgaa gaccctaacc tttcaccatc agcacgcccc 5160
   tcaacttgat aagcctagac gaaacccata tgcatgattg atgagtaatg gtgtgcacga 5220
   atattatgaa cccgtttcca agagcaatac tccattgaga tacacctcct ccttgtatct 5280
   gttcgttggt cccatttcca tagcagccgg cagtggcctt gactctgact gccacgcaag 5340
   taatatatct ttaataaact cgctgccttg cttcgtgtgt ccatttgcaa atgcatgcag 5400
   tgacgacatg cacatgcata gcttaattag ctccatgcat ccactgcttc cattaatccc 5460
   ctatataaag gactccatat gcctcaccat tcactcatcc accacagctt agcagcagca 5520
   acaaccagtg ccatagacac tctccatcaa caaactctag ctgatcaatc ctagctaagc 5580
   ttattacata gcaagccc                                               5598
<210> 36
   <211> 12776
   <212> DNA
   <213> Künstliche Sequenz
<220>
   <223> Beschreibung der künstlichen Sequenz: recombinant expression vector pLo114oXoB1-1
<400> 36
   aattcactgg ccgtcgtttt acaacgactc agagcttgac aggaggcccg atctagtaac 60
   atagatgaca ccgcgcgcga taatttatcc tagtttgcgc gctatatttt gttttctatc 120
   gcgtattaaa tgtataattg cgggactcta atcataaaaa cccatctcat aaataacgtc 180
   atgcattaca tgttaattat tacatgctta acgtaattca acagaaatta tatgataatc 240
   atcgcaagac cggcaacagg attcaatctt aagaaacttt attgccaaat gtttgaacga 300
   tcggggatca tccgggtctg tggcgggaac tccacgaaaa tatccgaacg cagcaagatc 360
   tagagcttgg gtcccgctca gaagaactcg tcaagaaggc gatagaaggc gatgcgctgc 420
   gaatcgggag cggcgatacc gtaaagcacg aggaagcggt cagcccattc gccgccaagc 480
   tcttcagcaa tatcacgggt agccaacgct atgtcctgat agcggtccgc cacacccagc 540
   cggccacagt cgatgaatcc agaaaagcgg ccattttcca ccatgatatt cggcaagcag 600
   gcatcgccat gggtcacgac gagatcctcg ccgtcgggca tgcgcgcctt gagcctggcg 660
   aacagttcgg ctggcgcgag cccctgatgc tcttcgtcca gatcatcctg atcgacaaga 720
   ccggcttcca tccgagtacg tgctcgctcg atgcgatgtt tcgcttggtg gtcgaatggg 780
   caggtagccg gatcaagcgt atgcagccgc cgcattgcat cagccatgat ggatactttc 840
   tcggcaggag caaggtgaga tgacaggaga tcctgccccg gcacttcgcc caatagcagc 900
   cagtcccttc ccgcttcagt gacaacgtcg agcacagctg cgcaaggaac gcccgtcgtg 960
   gccagccacg atagccgcgc tgcctcgtcc tgcagttcat tcagggcacc ggacaggtcg 1020
   gtcttgacaa aaagaaccgg gcgcccctgc gctgacagcc ggaacacggc ggcatcagag 1080
   cagccgattg tctgttgtgc ccagtcatag ccgaatagcc tctccaccca agcggccgga 1140
   gaacctgcgt gcaatccatc ttgttcaatc atgcgaaacg atccagatcc ggtgcagatt 1200
   atttggattg agagtgaata tgagactcta attggatacc gaggggaatt tatggaacgt 1260
   cagtggagca tttttgacaa gaaatatttg ctagctgata gtgaccttag gcgacttttg 1320
   aacgcgcaat aatggtttct gacgtatgtg cttagctcat taaactccag aaacccgcgg 1380
   ctgagtggct ccttcaacgt tgcggttctg tcagttccaa acgtaaaacg gcttgtcccg 1440
   cgtcatcggc gggggtcata acgtgactcc cttaattctc cgctcatgat cagattgtcg 1500
   tttcccgcct tcagtttaaa ctatcagtgt ttgacaggat cctgcttggt aataattgtc 1560
   attagattgt ttttatgcat agatgcactc gaaatcagcc aattttagac aagtatcaaa 1620
   cggatgttaa ttcagtacat taaagacgtc cgcaatgtgt tattaagttg tctaagcgtc 1680
   aatttgttta caccacaata tatcctgcca ccagccagcc aacagctccc cgaccggcag 1740
   ctcggcacaa aatcaccacg cgttaccacc acgccggccg gccgcatggt gttgaccgtg 1800
   ttcgccggca ttgccgagtt cgagcgttcc ctaatcatcg accgcacccg gagcgggcgc 1860
   gaggccgcca aggcccgagg cgtgaagttt ggcccccgcc ctaccctcac cccggcacag 1920
   atcgcgcacg cccgcgagct gatcgaccag gaaggccgca ccgtgaaaga ggcggctgca 1980
   ctgcttggcg tgcatcgctc gaccctgtac cgcgcacttg agcgcagcga ggaagtgacg 2040
   cccaccgagg ccaggcggcg cggtgccttc cgtgaggacg cattgaccga ggccgacgcc 2100
   ctggcggccg ccgagaatga acgccaagag gaacaagcat gaaaccgcac caggacggcc 2160
   aggacgaacc gtttttcatt accgaagaga tcgaggcgga gatgatcgcg gccgggtacg 2220
   tgttcgagcc gcccgcgcac gtctcaaccg tgcggctgca tgaaatcctg gccggtttgt 2280
   ctgatgccaa gctggcggcc tggccggcca gcttggccgc tgaagaaacc gagcgccgcc 2340
   gtctaaaaag gtgatgtgta tttgagtaaa acagcttgcg tcatgcggtc gctgcgtata 2400
   tgatgcgatg agtaaataaa caaatacgca aggggaacgc atgaaggtta tcgctgtact 2460
   taaccagaaa ggcgggtcag gcaagacgac catcgcaacc catctagccc gcgccctgca 2520
   actcgccggg gccgatgttc tgttagtcga ttccgatccc cagggcagtg cccgcgattg 2580
   ggcggccgtg cgggaagatc aaccgctaac cgttgtcggc atcgaccgcc cgacgattga 2640
   ccgcgacgtg aaggccatcg gccggcgcga cttcgtagtg atcgacggag cgccccaggc 2700
   ggcggacttg gctgtgtccg cgatcaaggc agccgacttc gtgctgattc cggtgcagcc 2760
   aagcccttac gacatatggg ccaccgccga cctggtggag ctggttaagc agcgcattga 2820
   ggtcacggat ggaaggctac aagcggcctt tgtcgtgtcg cgggcgatca aaggcacgcg 2880
   catcggcggt gaggttgccg aggcgctggc cgggtacgag ctgcccattc ttgagtcccg 2940
   tatcacgcag cgcgtgagct acccaggcac tgccgccgcc ggcacaaccg ttcttgaatc 3000
   agaacccgag ggcgacgctg cccgcgaggt ccaggcgctg gccgctgaaa ttaaatcaaa 3060
   actcatttga gttaatgagg taaagagaaa atgagcaaaa gcacaaacac gctaagtgcc 3120
   ggccgtccga gcgcacgcag cagcaaggct gcaacgttgg ccagcctggc agacacgcca 3180
   gccatgaagc gggtcaactt tcagttgccg gcggaggatc acaccaagct gaagatgtac 3240
   gcggtacgcc aaggcaagac cattaccgag ctgctatctg aatacatcgc gcagctacca 3300
   gagtaaatga gcaaatgaat aaatgagtag atgaatttta gcggctaaag gaggcggcat 3360
   ggaaaatcaa gaacaaccag gcaccgacgc cgtggaatgc cccatgtgtg gaggaacggg 3420
   cggttggcca ggcgtaagcg gctgggttgt ctgccggccc tgcaatggca ctggaacccc 3480
   caagcccgag gaatcggcgt gagcggtcgc aaaccatccg gcccggtaca aatcggcgcg 3540
   gcgctgggtg atgacctggt ggagaagttg aaggccgcgc aggccgccca gcggcaacgc 3600
   atcgaggcag aagcacgccc cggtgaatcg tggcaagcgg ccgctgatcg aatccgcaaa 3660
   gaatcccggc aaccgccggc agccggtgcg ccgtcgatta ggaagccgcc caagggcgac 3720
   gagcaaccag attttttcgt tccgatgctc tatgacgtgg gcacccgcga tagtcgcagc 3780
   atcatggacg tggccgtttt ccgtctgtcg aagcgtgacc gacgagctgg cgaggtgatc 3840
   cgctacgagc ttccagacgg gcacgtagag gtttccgcag ggccggccgg catggccagt 3900
   gtgtgggatt acgacctggt actgatggcg gtttcccatc taaccgaatc catgaaccga 3960
   taccgggaag ggaagggaga caagcccggc cgcgtgttcc gtccacacgt tgcggacgta 4020
   ctcaagttct gccggcgagc cgatggcgga aagcagaaag acgacctggt agaaacctgc 4080
   attcggttaa acaccacgca cgttgccatg cagcgtacga agaaggccaa gaacggccgc 4140
   ctggtgacgg tatccgaggg tgaagccttg attagccgct acaagatcgt aaagagcgaa 4200
   accgggcggc cggagtacat cgagatcgag ctagctgatt ggatgtaccg cgagatcaca 4260
   gaaggcaaga acccggacgt gctgacggtt caccccgatt actttttgat cgatcccggc 4320
   atcggccgtt ttctctaccg cctggcacgc cgcgccgcag gcaaggcaga agccagatgg 4380
   ttgttcaaga cgatctacga acgcagtggc agcgccggag agttcaagaa gttctgtttc 4440
   accgtgcgca agctgatcgg gtcaaatgac ctgccggagt acgatttgaa ggaggaggcg 4500
   gggcaggctg gcccgatcct agtcatgcgc taccgcaacc tgatcgaggg cgaagcatcc 4560
   gccggttcct aatgtacgga gcagatgcta gggcaaattg ccctagcagg ggaaaaaggt 4620
   cgaaaaggtc tctttcctgt ggatagcacg tacattggga acccaaagcc gtacattggg 4680
   aaccggaacc cgtacattgg gaacccaaag ccgtacattg ggaaccggtc acacatgtaa 4740
   gtgactgata taaaagagaa aaaaggcgat ttttccgcct aaaactcttt aaaacttatt 4800
   aaaactctta aaacccgcct ggcctgtgca taactgtctg gccagcgcac agccgaagag 4860
   ctgcaaaaag cgcctaccct tcggtcgctg cgctccctac gccccgccgc ttcgcgtcgg 4920
   cctatcgcgg ccgctggccg ctcaaaaatg gctggcctac ggccaggcaa tctaccaggg 4980
   cgcggacaag ccgcgccgtc gccactcgac cgccggcgcc cacatcaagg caccctgcct 5040
   cgcgcgtttc ggtgatgacg gtgaaaacct ctgacacatg cagctcccgg agacggtcac 5100
   agcttgtctg taagcggatg ccgggagcag acaagcccgt cagggcgcgt cagcgggtgt 5160
   tggcgggtgt cggggcgcag ccatgaccca gtcacgtagc gatagcggag tgtatactgg 5220
   cttaactatg cggcatcaga gcagattgta ctgagagtgc accatatgcg gtgtgaaata 5280
   ccgcacagat gcgtaaggag aaaataccgc atcaggcgct cttccgcttc ctcgctcact 5340
   gactcgctgc gctcggtcgt tcggctgcgg cgagcggtat cagctcactc aaaggcggta 5400
   atacggttat ccacagaatc aggggataac gcaggaaaga acatgtgagc aaaaggccag 5460
   caaaaggcca ggaaccgtaa aaaggccgcg ttgctggcgt ttttccatag gctccgcccc 5520
   cctgacgagc atcacaaaaa tcgacgctca agtcagaggt ggcgaaaccc gacaggacta 5580
   taaagatacc aggcgtttcc ccctggaagc tccctcgtgc gctctcctgt tccgaccctg 5640
   ccgcttaccg gatacctgtc cgcctttctc ccttcgggaa gcgtggcgct ttctcatagc 5700
   tcacgctgta ggtatctcag ttcggtgtag gtcgttcgct ccaagctggg ctgtgtgcac 5760
   gaaccccccg ttcagcccga ccgctgcgcc ttatccggta actatcgtct tgagtccaac 5820
   ccggtaagac acgacttatc gccactggca gcagccactg gtaacaggat tagcagagcg 5880
   aggtatgtag gcggtgctac agagttcttg aagtggtggc ctaactacgg ctacactaga 5940
   aggacagtat ttggtatctg cgctctgctg aagccagtta ccttcggaaa aagagttggt 6000
   agctcttgat ccggcaaaca aaccaccgct ggtagcggtg gtttttttgt ttgcaagcag 6060
   cagattacgc gcagaaaaaa aggatctcaa gaagatcctt tgatcttttc tacggggtct 6120
   gacgctcagt ggaacgaaaa ctcacgttaa gggattttgg tcatgcatga tatatctccc 6180
   aatttgtgta gggcttatta tgcacgctta aaaataataa aagcagactt gacctgatag 6240
   tttggctgtg agcaattatg tgcttagtgc atctaacgct tgagttaagc cgcgccgcga 6300
   agcggcgtcg gcttgaacga atttctagct agacattatt tgccgactac cttggtgatc 6360
   tcgcctttca cgtagtggac aaattcttcc aactgatctg cgcgcgaggc caagcgatct 6420
   tcttcttgtc caagataagc ctgtctagct tcaagtatga cgggctgata ctgggccggc 6480
   aggcgctcca ttgcccagtc ggcagcgaca tccttcggcg cgattttgcc ggttactgcg 6540
   ctgtaccaaa tgcgggacaa cgtaagcact acatttcgct catcgccagc ccagtcgggc 6600
   ggcgagttcc atagcgttaa ggtttcattt agcgcctcaa atagatcctg ttcaggaacc 6660
   ggatcaaaga gttcctccgc cgctggacct accaaggcaa cgctatgttc tcttgctttt 6720
   gtcagcaaga tagccagatc aatgtcgatc gtggctggct cgaagatacc tgcaagaatg 6780
   tcattgcgct gccattctcc aaattgcagt tcgcgcttag ctggataacg ccacggaatg 6840
   atgtcgtcgt gcacaacaat ggtgacttct acagcgcgga gaatctcgct ctctccaggg 6900
   gaagccgaag tttccaaaag gtcgttgatc aaagctcgcc gcgttgtttc atcaagcctt 6960
   acggtcaccg taaccagcaa atcaatatca ctgtgtggct tcaggccgcc atccactgcg 7020
   gagccgtaca aatgtacggc cagcaacgtc ggttcgagat ggcgctcgat gacgccaact 7080
   acctctgata gttgagtcga tacttcggcg atcaccgctt cccccatgat gtttaacttt 7140
   gttttagggc gactgccctg ctgcgtaaca tcgttgctgc tccataacat caaacatcga 7200
   cccacggcgt aacgcgcttg ctgcttggat gcccgaggca tagactgtac cccaaaaaaa 7260
   cagtcataac aagccatgaa aaccgccact gcgggggttc catggacata caaatggacg 7320
   aacggataaa ccttttcacg cccttttaaa tatccgatta ttctaataaa cgctcttttc 7380
   tcttaggttt acccgccaat atatcctgtc aaacactgat agtttaaact gaaggcggga 7440
   aacgacaatc agatctagta ggaaacagct atgaccatga ttacgccaag cttgcatgcc 7500
   tgcaggtcga ctctagagga tcgatccccg ggtaggtcag tcccttatgt tacgtcctgt 7560
   agaaacccca acccgtgaaa tcaaaaaact cgacggcctg tgggcattca gtctggatcg 7620
   cgaaaactgt ggaattggtc agcgttggtg ggaaagcgcg ttacaagaaa gccgggcaat 7680
   tgctgtgcca ggcagtttta acgatcagtt cgccgatgca gatattcgta attatgcggg 7740
   caacgtctgg tatcagcgcg aagtctttat accgaaaggt tgggcaggcc agcgtatcgt 7800
   gctgcgtttc gatgcggtca ctcattacgg caaagtgtgg gtcaataatc aggaagtgat 7860
   ggagcatcag ggcggctata cgccatttga agccgatgtc acgccgtatg ttattgccgg 7920
   gaaaagtgta cgtaagtttc tgcttctacc tttgatatat atataataat tatcattaat 7980
   tagtagtaat ataatatttc aaatattttt ttcaaaataa aagaatgtag tatatagcaa 8040
   ttgcttttct gtagtttata agtgtgtata ttttaattta taacttttct aatatatgac 8100
   caaaatttgt tgatgtgcag gtatcaccgt ttgtgtgaac aacgaactga actggcagac 8160
   tatcccgccg ggaatggtga ttaccgacga aaacggcaag aaaaagcagt cttacttcca 8220
   tgatttcttt aactatgccg gaatccatcg cagcgtaatg ctctacacca cgccgaacac 8280
   ctgggtggac gatatcaccg tggtgacgca tgtcgcgcaa gactgtaacc acgcgtctgt 8340
   tgactggcag gtggtggcca atggtgatgt cagcgttgaa ctgcgtgatg cggatcaaca 8400
   ggtggttgca actggacaag gcactagcgg gactttgcaa gtggtgaatc cgcacctctg 8460
   gcaaccgggt gaaggttatc tctatgaact gtgcgtcaca gccaaaagcc agacagagtg 8520
   tgatatctac ccgcttcgcg tcggcatccg gtcagtggca gtgaagggcg aacagttcct 8580
   gattaaccac aaaccgttct actttactgg ctttggtcgt catgaagatg cggacttgcg 8640
   tggcaaagga ttcgataacg tgctgatggt gcacgaccac gcattaatgg actggattgg 8700
   ggccaactcc taccgtacct cgcattaccc ttacgctgaa gagatgctcg actgggcaga 8760
   tgaacatggc atcgtggtga ttgatgaaac tgctgctgtc ggctttaacc tctctttagg 8820
   cattggtttc gaagcgggca acaagccgaa agaactgtac agcgaagagg cagtcaacgg 8880
   ggaaactcag caagcgcact tacaggcgat taaagagctg atagcgcgtg acaaaaacca 8940
   cccaagcgtg gtgatgtgga gtattgccaa cgaaccggat acccgtccgc aaggtgcacg 9000
   ggaatatttc gcgccactgg cggaagcaac gcgtaaactc gacccgacgc gtccgatcac 9060
   ctgcgtcaat gtaatgttct gcgacgctca caccgatacc atcagcgatc tctttgatgt 9120
   gctgtgcctg aaccgttatt acggatggta tgtccaaagc ggcgatttgg aaacggcaga 9180
   gaaggtactg gaaaaagaac ttctggcctg gcaggagaaa ctgcatcagc cgattatcat 9240
   caccgaatac ggcgtggata cgttagccgg gctgcactca atgtacaccg acatgtggag 9300
   tgaagagtat cagtgtgcat ggctggatat gtatcaccgc gtctttgatc gcgtcagcgc 9360
   cgtcgtcggt gaacaggtat ggaatttcgc cgattttgcg acctcgcaag gcatattgcg 9420
   cgttggcggt aacaagaaag ggatcttcac tcgcgaccgc aaaccgaagt cggcggcttt 9480
   tctgctgcaa aaacgctgga ctggcatgaa cttcggtgaa aaaccgcagc agggaggcaa 9540
   acaatgagag ctcgaatttc cccgatcggt caaacatttg gcaataaagn ttcttaagat 9600
   tgaatcctgt tgccggtctt gcgatgatta tcatataatt tctgttgaat tacgttaagc 9660
   atgtaataat taacatgtaa tgcatgacgt tatttatgag atgggttttt atgattagag 9720
   tcccgcaatt atacatttaa tacgcgatag aaaacaaaat atancgcgca aactaggata 9780
   aattatcgcg cgcggtgtca tctatgttac tagatcggga attcccatgc ctcgagcaga 9840
   aagatataat atgtaaaaaa atgggtctat atatatggaa ggtttcagga agacaaaggt 9900
   tctagaaact tccaaaaaaa atccagaata tattttggaa gaaataccct cttgggttgg 9960
   ccccggcgca gcccctagtg ggccaaaaag ccacgatcta atcccggtct aattggtcta 10020
   atagtttaga cttctaatta gacgggctct tatgccggtc taattggtct aattagatta 10080
   aaatcctaat taaatatgaa cgcaactagg cttcccctct ctctagtttt ctcggagctc 10140
   tttttcatgg accttgaagt attgccggat cactacttcg gaactcgtgg atacttcaga 10200
   gtgcacatct actttgaatc ttgattggta gatcatctcg gagaaattct cacagttggg 10260
   aggtataacc agttgccgaa attgccatgc ttcactcaca gccaggatca gcccatgtcc 10320
   caaggcaacc cttgtagcta catgccgagg cctgactact tggggcctcg cgccctgcat 10380
   ttttgcatgt tcatgtgaca cgttaaatgt tgagagaaat agattactaa atatcaccca 10440
   tttcgttatt ctagatgagt atcctacaat atgtataccg aaaaatgtat tttaaactgt 10500
   ggtaggtgag aaagatctat taaaaagaac tctacgtata ctcccccctc ccaatcccca 10560
   tccaggtttg taagacactt tcgtcttttt ttgccgaatt ttaaccgtaa atttgactag 10620
   taaaaataag ttatactgaa tgtaataaat atcgtacatt cggatgttgg agacagggag 10680
   aggctggctg gtgcgctgga tggatcacgg tcagaaagtc tgacttgcaa cgccacaggc 10740
   ccgttgattg ccactgacaa ccaagttttc gttgtttcgc tggtgccata ttttccgcga 10800
   tcgaatattt aaactgcgag gagaaaggca agcagggcgc catatcagca cttgatcact 10860
   cactgatcga tcagtagtag ccaccttctc tgcgccgacg tgttatatat tattggcaac 10920
   aagtcatcga ttgagaacag aaacaaaaca agaagagaac tatttgagag agagtagtta 10980
   cgccgcagcg agtagcctcc catttctgac gatcatgcca tacgataaac cggccggcgg 11040
   cgagaccagt tagcaaggtt gaaatgccaa cacatgtcgc gctcatttct cggctttttc 11100
   attttgcatg tcgtcatgca ggccctggac actgacattt ctctcttttg ctgttgaatg 11160
   aagaccctaa cctttcacca tcagcacgcc cctcaacttg ataagcctag acgaaaccca 11220
   tatgcatgat tgatgagtaa tggtgtgcac gaatattatg aacccgtttc caagagcaat 11280
   actccattga gatacacctc ctccttgtat ctgttcgttg gtcccatttc catagcagcc 11340
   ggcagtggcc ttgactctga ctgccacgca agtaatatat ctttaataaa ctcgctgcct 11400
   tgcttcgtgt gtccatttgc aaatgcatgc agtgacgaca tgcacatgca tagcttaatt 11460
   agctccatgc atccactgct tccattaatc ccctatataa aggactccat atgcctcacc 11520
   attcactcat ccaccacagc ttagcagcag caacaaccag tgccatagac actctccatc 11580
   aacaaactct agctgatcaa tcctagctaa gcttattaca tagcaagccc ggggatcctc 11640
   tagagtcgac ctgcaggcgg ccgcactagt gattaggatt ccaacgcgag ccaggacaag 11700
   cgaggaacct tgcgtgcgag gcgaggccgc cccgctccga ttcgattcga cgcgcaggcg 11760
   caggcgcagg gatggacgcc ttctactcga cctcgtcggc ggcggcgagc ggctggggcc 11820
   acgactccct caagaacttc cgccagatct cccccgccgt gcagtcccac ctcaagctcg 11880
   tttacctgac tctatgcttt gcactggcct catctgccgt gggtgcttac ctacacattg 11940
   ccctgaacat cggcgggatg ctgacaatgc tcgcttgtgt cggaactatc gcctggatgt 12000
   tctcggtgcc agtctatgag gagaggaaga ggtttgggct gctgatgggt gcagccctcc 12060
   tggaaggggc ttcggttgga cctctgattg agcttgccat agactttgac ccaagcatcc 12120
   tcgtgacagg gtttgtcgga accgccatcg cctttgggtg cttctctggc gccgccatca 12180
   tcgccaagcg cagggagtac ctgtacctcg gtggcctgct ctcgtctggc ctgtcgatcc 12240
   tgctctggct gcagtttgtc acgtccatct ttggccactc ctctggcagc ttcatgtttg 12300
   aggtttactt tggcctgttg atcttcctgg ggtacatggt gtacgacacg caggagatca 12360
   tcgagagggc gcaccatggc gacatggact acatcaagca cgccctcacc ctcttcaccg 12420
   actttgttgc cgtcctcgtc cgagtcctca tcatcatgct caagaacgca ggcgacaagt 12480
   cggaggacaa gaagaagagg aagagggggt cctgaacgtw tctcccgcac atgtagatac 12540
   cgtcaccgcg tcgacctgca ggcatgcccg ctgaaatcac cagtctctct ctacaaatct 12600
   atctctctca taataatgtg tgagtagttc ccagataagg gaattagggt tcttataggg 12660
   tttcgctcat gtgttgagca tataagaaac ccttagtatg tatttgtatt tgtaaaatac 12720
   ttctatcaat aaaatttcta attcctaaaa ccaaaatcca gtgggtaccg agctcg     12776
<210> 37
   <211> 744
   <212> DNA
   <213> Triticum aestivum
<220>
   <221> CDS
   <222> (1)..(741)
   <223> coding for TaBI-1
<400> 37
   atg gac gcc ttc tac tcg acc tcg tcg gcg gcg gcg agc ggc tgg ggc    48
   Met Asp Ala Phe Tyr Ser Thr Ser Ser Ala Ala Ala Ser Gly Trp Gly
    1               5                  10                  15
   tac gac tcc ctc aag aac ttc cgc gag atc tcc ccc gcc gtg cag tcc    96
   Tyr Asp Ser Leu Lys Asn Phe Arg Glu Ile Ser Pro Ala Val Gln Ser
               20                  25                  30
   cac ctc aag ctc gtt tac ctg acc cta tgc ttt gcc ctg gcc tca tct   144
   His Leu Lys Leu Val Tyr Leu Thr Leu Cys Phe Ala Leu Ala Ser Ser
           35                  40                  45
   gcc gtg ggt gct tac ctg cac att gcc ctg aac atc ggt ggg atg ctg   192
   Ala Val Gly Ala Tyr Leu His Ile Ala Leu Asn Ile Gly Gly Met Leu
       50                  55                  60
   aca atg ctc gcg tgt gtt gga acc atc gcc tgg atg ttc tct gtg cca   240
   Thr Met Leu Ala Cys Val Gly Thr Ile Ala Trp Met Phe Ser Val Pro
   65                  70                  75                  80
   gtc tat gag gag agg aag agg ttt ggg ctg ctg atg ggt gca gcc ctc   288
   Val Tyr Glu Glu Arg Lys Arg Phe Gly Leu Leu Met Gly Ala Ala Leu
                   85                  90                  95
   ctg gaa ggg gct tcg gtt gga cct ctg att gag ctt gcc ata gac ttt   336
   Leu Glu Gly Ala Ser Val Gly Pro Leu Ile Glu Leu Ala Ile Asp Phe
               100                 105                 110
   gac cca agt atc ctc gtg aca ggg ttt gtc gga acc gcc atc gcc ttc   384
   Asp Pro Ser Ile Leu Val Thr Gly Phe Val Gly Thr Ala Ile Ala Phe
           115                 120                 125
   ggg tgc ttc tct ggc gcc gcc atc atc gcc aag cgc agg gag tac ctg   432
   Gly Cys Phe Ser Gly Ala Ala Ile Ile Ala Lys Arg Arg Glu Tyr Leu
       130                 135                 140
   tac ctc ggt ggt ctg ctc tcc tcc ggc ctg tcg atc ctg ctc tgg ctg   480
   Tyr Leu Gly Gly Leu Leu Ser Ser Gly Leu Ser Ile Leu Leu Trp Leu
   145                 150                 155                 160
   cag ttt gcc acg tcc atc ttt ggc cac tcc tct ggc agc ttc atg ttt   528
   Gln Phe Ala Thr Ser Ile Phe Gly His Ser Ser Gly Ser Phe Met Phe
                   165                 170                 175
   gag gtt tac ttt ggc ctg ttg atc ttc ctg gga tac atg gtg tac gac   576
   Glu Val Tyr Phe Gly Leu Leu Ile Phe Leu Gly Tyr Met Val Tyr Asp
               180                 185                 190
   acg cag gag atc atc gag agg gcg cac cac ggc gac atg gat tac atc   624
   Thr Gln Glu Ile Ile Glu Arg Ala His His Gly Asp Met Asp Tyr Ile
           195                 200                 205
   aag cac gcg ctc acc ctc ttc acc gac ttc gtc gcc gtt ctc gtc cgc   672
   Lys His Ala Leu Thr Leu Phe Thr Asp Phe Val Ala Val Leu Val Arg
       210                 215                 220
   gtc ctc atc atc atg ctc aag aac gca ggc gac aag tcg gag gac aag   720
   Val Leu Ile Ile Met Leu Lys Asn Ala Gly Asp Lys Ser Glu Asp Lys
   225                 230                 235                 240
   aag aag agg aag agg ggg tcc tga                                   744
   Lys Lys Arg Lys Arg Gly Ser
                   245
<210> 38
   <211> 247
   <212> PRT
   <213> Triticum aestivum
<400> 38
   Met Asp Ala Phe Tyr Ser Thr Ser Ser Ala Ala Ala Ser Gly Trp Gly
   1               5                   10                  15
   Tyr Asp Ser Leu Lys Asn Phe Arg Glu Ile Ser Pro Ala Val Gln Ser
               20                  25                  30
   His Leu Lys Leu Val Tyr Leu Thr Leu Cys Phe Ala Leu Ala Ser Ser
           35                  40                  45
   Ala Val Gly Ala Tyr Leu His Ile Ala Leu Asn Ile Gly Gly Met Leu
       50                  55                  60
   Thr Met Leu Ala Cys Val Gly Thr Ile Ala Trp Met Phe Ser Val Pro
   65                  70                  75                  80
   Val Tyr Glu Glu Arg Lys Arg Phe Gly Leu Leu Met Gly Ala Ala Leu
                   85                  90                  95
   Leu Glu Gly Ala Ser Val Gly Pro Leu Ile Glu Leu Ala Ile Asp Phe
               100                 105                 110
   Asp Pro Ser Ile Leu Val Thr Gly Phe Val Gly Thr Ala Ile Ala Phe
           115                 120                 125
   Gly Cys Phe Ser Gly Ala Ala Ile Ile Ala Lys Arg Arg Glu Tyr Leu
       130                 135                 140
   Tyr Leu Gly Gly Leu Leu Ser Ser Gly Leu Ser Ile Leu Leu Trp Leu
   145                 150                 155                 160
   Gln Phe Ala Thr Ser Ile Phe Gly His Ser Ser Gly Ser Phe Met Phe
                   165                 170                 175
   Glu Val Tyr Phe Gly Leu Leu Ile Phe Leu Gly Tyr Met Val Tyr Asp
               180                 185                 190
   Thr Gln Glu Ile Ile Glu Arg Ala His His Gly Asp Met Asp Tyr Ile
           195                 200                 205
   Lys His Ala Leu Thr Leu Phe Thr Asp Phe Val Ala Val Leu Val Arg
       210                 215                 220
   Val Leu Ile Ile Met Leu Lys Asn Ala Gly Asp Lys Ser Glu Asp Lys
   225                 230                 235                 240
   Lys Lys Arg Lys Arg Gly Ser
                   245
<210> 39
   <211>
   <212> DNA
   <213> Hordeum vulgare
<220>
   <221> CDS
   <222> (173)..(1126)
   <223> coding for Hordeum vulgare subsp. vulgare syntaxin (Ror2)
<400> 39
<210> 40
   <211> 318
   <212> PRT
   <213> Hordeum vulgare
<400> 40
   Met Asn Asn Leu Phe Ser Ser Ser Trp Lys Arg Ala Gly Ala Gly Gly
   1               5                   10                  15
   Asp Gly Asp Leu Glu Ser Gly Gly Gly Gly Val Glu Met Thr Ala Pro
               20                  25                  30
   Pro Gly Ala Ala Ala Gly Ala Ser Leu Asp Arg Phe Phe Glu Asp Val
           35                  40                  45
   Glu Ser Ile Lys Asp Asp Leu Arg Glu Leu Glu Arg Ile Gln Arg Ser
       50                  55                  60
   Leu His Asp Gly Asn Glu Ser Gly Lys Ser Leu His Asp Ala Ser Ala
   65                  70                  75                  80
   Val Arg Ala Leu Arg Ser Arg Met Asp Ala Asp Val Ala Ala Ala Ile
                   85                  90                  95
   Lys Lys Ala Lys Val Val Lys Leu Arg Leu Glu Ser Leu Asp Arg Ala
               100                 105                 110
   Asn Ala Ala Asn Arg Ser Val Ala Gly Cys Gly Pro Gly Ser Ser Thr
           115                 120                 125
   Asp Arg Thr Arg Thr Ser Val Val Ala Gly Leu Arg Lys Lys Leu Arg
       130                 135                 140
   Asp Ala Met Glu Ser Phe Ser Ser Leu Arg Ser Arg Ile Thr Ser Glu
   145                 150                 155                 160
   Tyr Arg Glu Thr Val Ala Arg Arg Tyr Phe Thr Val Thr Gly Ser Gln
                   165                 170                 175
   Pro Asp Glu Ala Thr Leu Asp Thr Leu Ala Glu Thr Gly Glu Gly Glu
               180                 185                 190
   Arg Leu Leu Gln Arg Ala Ile Ala Glu Gln Gln Gly Arg Gly Glu Val
           195                 200                 205
   Leu Gly Val Val Ala Glu Ile Gln Glu Arg His Gly Ala Val Ala Asp
       210                 215                 220
   Leu Glu Arg Ser Leu Leu Glu Leu Gln Gln Val Phe Asn Asp Met Ala
   225                 230                 235                 240
   Val Leu Val Ala Ala Gln Gly Glu Gln Leu Asp Asp Ile Glu Gly His
                   245                 250                 255
   Val Gly Arg Ala Arg Ser Phe Val Asp Arg Gly Arg Glu Gln Leu Gln
               260                 265                 270
   Val Ala Arg Lys His Gln Lys Ser Ser Arg Lys Trp Thr Phe Ile Gly
           275                 280                 285
   Ile Gly Ile Leu Leu Val Val Ile Leu Ile Ile Val Ile Pro Ile Val
       290                 295                 300
   Leu Lys Asn Thr Asn Lys Ser Asn Asn Asn Asn Ser Gln Gln
   305                 310                 315
<210> 41
   <211> 948
   <212> DNA
   <213> Arabidopsis thaliana
<220>
   <221> CDS
   <222> (1)..(945)
   <223> coding for Arabidopsis thaliana syntaxin 121 (SYP121) / syntaxin-related protein (SYR1) (At3g11820)
<400> 41
   atg gcg aat ccc gcg gga tca acc ggt ggt gtg aac ctc gac aag ttc    48
   Met Ala Asn Pro Ala Gly Ser Thr Gly Gly Val Asn Leu Asp Lys Phe
   1               5                   10                  15
   ttc gaa gat gtt gaa tct gtg aaa gaa gag cta aag gag cta gat cgg    96
   Phe Glu Asp Val Glu Ser Val Lys Glu Glu Leu Lys Glu Leu Asp Arg
               20                  25                  30
   ctc aac gaa aca ctc tct tca tgt cac gag cag agc aag acg ctt cac    144
   Leu Asn Glu Thr Leu Ser Ser Cys His Glu Gln Ser Lys Thr Leu His
           35                  40                  45
   aat gct aaa gcc gtt aaa gat ctc cgg tct aaa atg gac ggt gac gtt    192
   Asn Ala Lys Ala Val Lys Asp Leu Arg Ser Lys Met Asp Gly Asp Val
       50                  55                  60
   gga gtc gcg ttg aag aag gcg aag atg att aaa gtt aaa ctc gag gcg    240
   Gly Val Ala Leu Lys Lys Ala Lys Met Ile Lys Val Lys Leu Glu Ala
   65                  70                  75                  80
   cta gat cgt gcc aat gct gct aat cgg agt ctc cct ggc tgt gga cct    288
   Leu Asp Arg Ala Asn Ala Ala Asn Arg Ser Leu Pro Gly Cys Gly Pro
                   85                  90                  95
   ggt tct tcc tcc gat cga acc agg acc tct gtc ctc aat ggt ctc agg    336
   Gly Ser Ser Ser Asp Arg Thr Arg Thr Ser Val Leu Asn Gly Leu Arg
               100                 105                 110
   aag aaa ttg atg gac tct atg gat agt ttc aac cga ttg agg gag ctt    384
   Lys Lys Leu Met Asp Ser Met Asp Ser Phe Asn Arg Leu Arg Glu Leu
           115                 120                 125
   atc tcg tcc gag tat aga gaa act gta cag agg agg tac ttc acc gtc    432
   Ile Ser Ser Glu Tyr Arg Glu Thr Val Gln Arg Arg Tyr Phe Thr Val
       130                 135                 140
   acc ggc gag aat ccg gat gaa cga acc cta gat cga ctg att tcc act    480
   Thr Gly Glu Asn Pro Asp Glu Arg Thr Leu Asp Arg Leu Ile Ser Thr
   145                 150                 155                 160
   gga gag agt gag aga ttc ttg cag aaa gca ata caa gaa caa gga aga    528
   Gly Glu Ser Glu Arg Phe Leu Gln Lys Ala Ile Gln Glu Gln Gly Arg
                   165                 170                 175
   gga agg gtg tta gac acc att aac gag att caa gaa agg cat gat gcg    576
   Gly Arg Val Leu Asp Thr Ile Asn Glu Ile Gln Glu Arg His Asp Ala
               180                 185                 190
   gtt aaa gac att gag aag aat ctc agg gag ctt cac cag gtg ttt cta     624
   Val Lys Asp Ile Glu Lys Asn Leu Arg Glu Leu His Gln Val Phe Leu
           195                 200                 205
   gac atg gcc gtg ctg gta gag cac cag gga gct cag ctt gat gac atc     672
   Asp Met Ala Val Leu Val Glu His Gln Gly Ala Gln Leu Asp Asp Ile
       210                 215                 220
   gag agt cat gtg ggt cga gct agc tcc ttt atc aga ggc gga act gac     720
   Glu Ser His Val Gly Arg Ala Ser Ser Phe Ile Arg Gly Gly Thr Asp
   225                 230                 235                 240
   cag cta caa acc gct cgg gtt tac cag aag aac acg cga aaa tgg aca     768
   Gln Leu Gln Thr Ala Arg Val Tyr Gln Lys Asn Thr Arg Lys Trp Thr
                   245                 250                 255
   tgt att gcc att att att ctc atc atc atc ata act gtt gtg gtt ctt     816
   Cys Ile Ala Ile Ile Ile Leu Ile Ile Ile Ile Thr Val Val Val Leu
               260                 265                 270
   gct gtt tta aaa ccg tgg aac aac agc agt ggc ggc ggc ggc ggt ggt     864
   Ala Val Leu Lys Pro Trp Asn Asn Ser Ser Gly Gly Gly Gly Gly Gly
           275                 280                 285
   ggt ggt ggg ggt acc act gga gga agt caa cca aat tca ggg aca cca     912
   Gly Gly Gly Gly Thr Thr Gly Gly Ser Gln Pro Asn Ser Gly Thr Pro
       290                 295                 300
   cca aat cct cct cag gca agg cgt cta ttg cgt tga                     948
   Pro Asn Pro Pro Gln Ala Arg Arg Leu Leu Arg
   305                 310                 315
<210> 42
   <211> 315
   <212> PRT
   <213> Arabidopsis thaliana
<400> 42
   Met Ala Asn Pro Ala Gly Ser Thr Gly Gly Val Asn Leu Asp Lys Phe
   1 5 10 15
   Phe Glu Asp Val Glu Ser Val Lys Glu Glu Leu Lys Glu Leu Asp Arg
   20 25 30
   Leu Asn Glu Thr Leu Ser Ser Cys His Glu Gln Ser Lys Thr Leu His
   35 40 45
   Asn Ala Lys Ala Val Lys Asp Leu Arg Ser Lys Met Asp Gly Asp Val
   50 55 60
   Gly Val Ala Leu Lys Lys Ala Lys Met Ile Lys Val Lys Leu Glu Ala
   65 70 75 80
   Leu Asp Arg Ala Asn Ala Ala Asn Arg Ser Leu Pro Gly Cys Gly Pro
   85 90 95
   Gly Ser Ser Ser Asp Arg Thr Arg Thr Ser Val Leu Asn Gly Leu Arg
   100 105 110
   Lys Lys Leu Met Asp Ser Met Asp Ser Phe Asn Arg Leu Arg Glu Leu
   115 120 125
   Ile Ser Ser Glu Tyr Arg Glu Thr Val Gln Arg Arg Tyr Phe Thr Val
   130 135 140
   Thr Gly Glu Asn Pro Asp Glu Arg Thr Leu Asp Arg Leu Ile Ser Thr
   145 150 155 160
   Gly Glu Ser Glu Arg Phe Leu Gln Lys Ala Ile Gln Glu Gln Gly Arg
   165 170 175
   Gly Arg Val Leu Asp Thr Ile Asn Glu Ile Gln Glu Arg His Asp Ala
   180 185 190
   Val Lys Asp Ile Glu Lys Asn Leu Arg Glu Leu His Gln Val Phe Leu
   195 200 205
   Asp Met Ala Val Leu Val Glu His Gln Gly Ala Gln Leu Asp Asp Ile
   210 215 220
   Glu Ser His Val Gly Arg Ala Ser Ser Phe Ile Arg Gly Gly Thr Asp
   225 230 235 240
   Gln Leu Gln Thr Ala Arg Val Tyr Gln Lys Asn Thr Arg Lys Trp Thr
   245 250 255
   Cys Ile Ala Ile Ile Ile Leu Ile Ile Ile Ile Thr Val Val Val Leu
   260 265 270
   Ala Val Leu Lys Pro Trp Asn Asn Ser Ser Gly Gly Gly Gly Gly Gly
   275 280 285
   Gly Gly Gly Gly Thr Thr Gly Gly Ser Gln Pro Asn Ser Gly Thr Pro
   290 295 300
   Pro Asn Pro Pro Gln Ala Arg Arg Leu Leu Arg
   305 310 315
<210> 43
   <211> 1275
   <212> DNA
   <213> Hordeum vulgare
<220> .
   <221> CDS
   <222> (80)..(1006)
   <223> coding for Hordeum vulgare subsp. vulgare SNAP-34
<400> 43
   ggcccctcca ccccacccca cccagtcgct gcggatactt gattctgcta ctcggccagc 60
   gatcgatctc gcctccgcc atg agc gcc acc agg ccc tcc ttc ttc ccc tcc  112
                        Met Ser Ala Thr Arg Pro Ser Phe Phe Pro Ser
                        1               5                   10
   aac aac aac agg aac aag ccc gcc acc cgg aac ccc ttc gac tcc gac   160
   Asn Asn Asn Arg Asn Lys Pro Ala Thr Arg Asn Pro Phe Asp Ser Asp
               15                  20                  25
   tcg gac gac gac ggc ggc atg gcc cgg cgc ggc ccg gcg cgg gcc tcg   208
   Ser Asp Asp Asp Gly Gly Met Ala Arg Arg Gly Pro Ala Arg Ala Ser
           30                  35                  40
   tcc gtc ccg acc ccc gcc gcg ggg ccg gcc agg gcc tcc tcg gcc ccg   256
   Ser Val Pro Thr Pro Ala Ala Gly Pro Ala Arg Ala Ser Ser Ala Pro
       45                  50                  55
   atc ccc gcc gac gag gcg gac cag cgg ggc gcc ctg ttc ggc gcg ggc   304
   Ile Pro Ala Asp Glu Ala Asp Gln Arg Gly Ala Leu Phe Gly Ala Gly
   60                  65                  70                  75
   ccc gcg ccg tcc ggc ttc gcg tcc tcc tcc tcc gcg gcc gcc agg ggc   352
   Pro Ala Pro Ser Gly Phe Ala Ser Ser Ser Ser Ala Ala Ala Arg Gly
                   80                  85                  90
   cgg tac agg aac gac ttc cgc gac tcg ggc ggc gtg gag gcg cag tcc   400
   Arg Tyr Arg Asn Asp Phe Arg Asp Ser Gly Gly Val Glu Ala Gln Ser
               95                  100                 105
   gtg cag gag ctc gag ggc tac gcg gcc tac aag gcc gag gag acc acg   448
   Val Gln Glu Leu Glu Gly Tyr Ala Ala Tyr Lys Ala Glu Glu Thr Thr
           110                 115                 120
   cgc cgg gtc gac ggc tgc ctc cgg gtc gcc gag gag atg cgg gac acc      496
   Arg Arg Val Asp Gly Cys Leu Arg Val Ala Glu Glu Met Arg Asp Thr
       125                 130                 135
   gcg tca aag acc ctg ctc cag gtg cac cag cag ggc cag cag atc agg      544
   Ala Ser Lys Thr Leu Leu Gln Val His Gln Gln Gly Gln Gln Ile Arg
   140                 145                 150                 155
   cgc acc cac gcc atg gcc gtc gac atc gac cag gat ctc tcc agg ggg      592
   Arg Thr His Ala Met Ala Val Asp Ile Asp Gln Asp Leu Ser Arg Gly
                   160                 165                 170
   gaa aag cta cta ggt gat ctt ggt ggt ttg ttt tcc aag aag tgg aag      640
   Glu Lys Leu Leu Gly Asp Leu Gly Gly Leu Phe Ser Lys Lys Trp Lys
               175                 180                 185
   cca aag aag aac ggc gca atc agg ggc cct atg ctg acc aga gac gat      688
   Pro Lys Lys Asn Gly Ala Ile Arg Gly Pro Met Leu Thr Arg Asp Asp
           190                 195                 200
   tcc ttc ata cgc aag ggc agc cat atg gag cag agg cat aaa ctg ggg      736
   Ser Phe Ile Arg Lys Gly Ser His Met Glu Gln Arg His Lys Leu Gly
       205                 210                 215
   ctg tca gat cgt ccg cat cga tcc aat gca cgc cag ttc cta tct gaa      784
   Leu Ser Asp Arg Pro His Arg Ser Asn Ala Arg Gln Phe Leu Ser Glu
   220                 225                 230                 235
   ccc aca tca ggc ctt gag aaa gtc gag gtg gag aag gca aag cag gat      832
   Pro Thr Ser Gly Leu Glu Lys Val Glu Val Glu Lys Ala Lys Gln Asp
                   240                 245                 250
   gat ggc ctg tct gac ctt agc gac ata ctg aca gag ttg aaa gga atg      880
   Asp Gly Leu Ser Asp Leu Ser Asp Ile Leu Thr Glu Leu Lys Gly Met
               255                 260                 265
   gcc att gac atg gga act gag att gag ggg caa aca aag gat ctt ggt      928
   Ala Ile Asp Met Gly Thr Glu Ile Glu Gly Gln Thr Lys Asp Leu Gly
           270                 275                 280
   cat gcg gag aag gac ttt gac gaa ctt aac tac agg gtc aag ggg gca      976
   His Ala Glu Lys Asp Phe Asp Glu Leu Asn Tyr Arg Val Lys Gly Ala
       285                 290                 295
   aac gct cga aca cgt cgc ctg ctt ggc aga taggcaagaa gcatatgttg       1026
   Asn Ala Arg Thr Arg Arg Leu Leu Gly Arg
   300                 305
   ttcaccagag gattctgtga cactccttat cttctgcatt tgctttcgtg ggctgttaat 1086
   tcagatcatt ttgtgcataa aactctggtt aggaaggtct gttggggagt tgtatcaggg 1146
   tttattgtgt atatacgcta gacgggcggt tcgttttcta tgttgcagtt gtactacatt 1206
   tgctatggac agtagatacg tttgtattcg gttttcttgt tttgcaatcg ctatgctgca 1266
   ggaaagcac                                                         1275
<210> 44
   <211> 309
   <212> PRT
   <213> Hordeum vulgare
<400> 44
   Met Ser Ala Thr Arg Pro Ser Phe Phe Pro Ser Asn Asn Asn Arg Asn
   1               5                   10                  15
   Lys Pro Ala Thr Arg Asn Pro Phe Asp Ser Asp Ser Asp Asp Asp Gly
               20                  25                  30
   Gly Met Ala Arg Arg Gly Pro Ala Arg Ala Ser Ser Val Pro Thr Pro
           35                  40                  45
   Ala Ala Gly Pro Ala Arg Ala Ser Ser Ala Pro Ile Pro Ala Asp Glu
       50                  55                  60
   Ala Asp Gln Arg Gly Ala Leu Phe Gly Ala Gly Pro Ala Pro Ser Gly
   65                  70                  75                      80
   Phe Ala Ser Ser Ser Ser Ala Ala Ala Arg Gly Arg Tyr Arg Asn Asp
                   85                  90                  95
   Phe Arg Asp Ser Gly Gly Val Glu Ala Gln Ser Val Gln Glu Leu Glu
               100                 105                 110
   Gly Tyr Ala Ala Tyr Lys Ala Glu Glu Thr Thr Arg Arg Val Asp Gly
           115                 120                 125
   Cys Leu Arg Val Ala Glu Glu Met Arg Asp Thr Ala Ser Lys Thr Leu
       130                 135                 140
   Leu Gln Val His Gln Gln Gly Gln Gln Ile Arg Arg Thr His Ala Met
   145                 150                 155                     160
   Ala Val Asp Ile Asp Gln Asp Leu Ser Arg Gly Glu Lys Leu Leu Gly
                   165                 170                 175
   Asp Leu Gly Gly Leu Phe Ser Lys Lys Trp Lys Pro Lys Lys Asn Gly
               180                 185                 190
   Ala Ile Arg Gly Pro Met Leu Thr Arg Asp Asp Ser Phe Ile Arg Lys
           195                 200                 205
   Gly Ser His Met Glu Gln Arg His Lys Leu Gly Leu Ser Asp Arg Pro
       210                 215                 220
   His Arg Ser Asn Ala Arg Gln Phe Leu Ser Glu Pro Thr Ser Gly Leu
   225                 230                 235                     240
   Glu Lys Val Glu Val Glu Lys Ala Lys Gln Asp Asp Gly Leu Ser Asp
                   245                 250                 255
   Leu Ser Asp Ile Leu Thr Glu Leu Lys Gly Met Ala Ile Asp Met Gly
               260                 265                 270
   Thr Glu Ile Glu Gly Gln Thr Lys Asp Leu Gly His Ala Glu Lys Asp
           275                 280                 285
   Phe Asp Glu Leu Asn Tyr Arg Val Lys Gly Ala Asn Ala Arg Thr Arg
       290                 295                 300
   Arg Leu Leu Gly Arg
   305